(19) Europäisches Patentamt
European Patent Office
Office européen des brevets

(11) **EP 4 129 965 B1**

(12) **EUROPEAN PATENT SPECIFICATION**

(45) Date of publication and mention of the grant of the patent:
**17.12.2025 Bulletin 2025/51**

(21) Application number: **21780957.3**

(22) Date of filing: **30.03.2021**

(51) International Patent Classification (IPC):
***C08G 64/02*** *(2006.01)* ***C07C 31/27*** *(2006.01)*

(52) Cooperative Patent Classification (CPC):
**C07C 31/278; C07C 43/196; C08G 63/64; C08G 64/0208; C08G 64/302; C08G 64/305;**
C07C 2603/90

(86) International application number:
**PCT/JP2021/013550**

(87) International publication number:
**WO 2021/200952 (07.10.2021 Gazette 2021/40)**

(54) **THERMOPLASTIC RESIN HAVING CARBONATE BOND**

THERMOPLASTISCHES HARZ MIT CARBONATBINDUNG

RÉSINE THERMOPLASTIQUE PRÉSENTANT UNE LIAISON CARBONATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2020 JP 2020065276**
**31.03.2020 JP 2020065356**

(43) Date of publication of application:
**08.02.2023 Bulletin 2023/06**

(60) Divisional application:
**24172949.0 / 4 400 490**

(73) Proprietor: **Mitsubishi Chemical Corporation**
**Chiyoda-ku**
**Tokyo 100-8251 (JP)**

(72) Inventors:
- **SUGIHARA, Yusuke**
  **Tokyo 100-8251 (JP)**
- **HAYASHI, Hiroyuki**
  **Tokyo 100-8251 (JP)**
- **TAKAHASHI, Takayoshi**
  **Tokyo 100-8251 (JP)**
- **FUJI, Michiaki**
  **Tokyo 100-8251 (JP)**
- **NAKAGAWA, Takafumi**
  **Tokyo 100-8251 (JP)**

(74) Representative: **Vossius & Partner**
**Patentanwälte Rechtsanwälte mbB**
**Siebertstraße 3**
**81675 München (DE)**

(56) References cited:
**JP-A- 2001 011 166** **JP-A- 2002 265 584**
**JP-A- 2002 265 584** **JP-A- H05 105 746**
**JP-A- H05 148 351** **JP-A- S6 377 933**

- **SIEMS K ET AL: "Rigidol, an unusual diterpene from Sapium rigidifolium", PHYTOCHEMISTRY, ELSEVIER, AMSTERDAM , NL, vol. 33, no. 6, 12 August 1993 (1993-08-12), pages 1465 - 1468, XP026647252, ISSN: 0031-9422, [retrieved on 19930812], DOI: 10.1016/0031-9422(93)85112-5**
- **SIEMS, K. ET AL.: "An unusual diterpene from sapium rigidiforium", PHYTOCHEMISTRY, vol. 33, no. 6, 1993, pages 1465 - 1468, XP026647252, DOI: 10.1016/0031-9422(93)85112-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 129 965 B1

## Description

TECHNICAL FIELD

The present invention relates to a thermoplastic resin having a carbonate bond.

BACKGROUND ART

As thermoplastic resins, resins having a carbonate bond, such as polycarbonate resins and polyester carbonate resins, are widely used. For example, polycarbonate resins generally use bisphenols as monomer components and are widely used as so-called engineering plastics in the fields of electrical and electronic parts, automobile parts, medical parts, building materials, films, sheets, bottles, optical recording media, lenses, etc. by making use of their superiority in transparency, heat resistance, mechanical strength, etc. Further, polycarbonate diols are used as, for example, raw materials of polyurethanes by reacting with isocyanate compounds.
In recent years, polycarbonate resins using, as monomer components, compounds other than bisphenols have been developed. For example, Patent Literatures 1 and 2 each disclose a polycarbonate resin using, as a monomer component,
a compound containing a fluorene ring. Patent Literatures 6 and 7 each propose a polycarbonate resin using, as a monomer component, a fused-ring
compound containing a norbornane skeleton. Patent Literature 8 discloses a process for producing a polycarbonate resin by subjecting a pentacyclopentadecanedimethanol with a diol to melt polycondensation with a carbonic diester. Non-patent Literature 1 discloses the compound rigidol.

PRIOR ART LITERATURE

Patent Documents

Patent Literature 1: WO2004/111106
Patent Literature 2: WO2007/063823
Patent Literature 3: Japanese Patent No. 5119250
Patent Literature 4: Japanese Patent No. 5204200
Patent Literature 5: JP 2015-25111 A
Patent Literature 6: JP 6-43302 A
Patent Literature 7: JP 2002-322267 A
Patent Literature 8: JP 2002-265584 A

Non-patent Documents

Non-patent Literature 1: Siems K. et al.: "Rigidol, an unusual diterpene from Sapium rigidifolium", PHYTOCHEMISTRY, vol. 33, no. 6, pp. 1465-1468, 1993.

SUMMARY OF THE INVENTION

In recent years, thermoplastic resins having a polycarbonate bond, such as polycarbonate resins, have more widely been used, and have been required to satisfy various performance requirements such as mechanical properties and heat resistance. Therefore, there have been demands for development of a thermoplastic resin having a novel structure. Further, there have been demands for development of a polymerizable compound used as a raw material monomer thereof.
In light of such circumstances, it is an object of the present invention to provide a novel thermoplastic resin having a carbonate bond and a fused ring.

MEANS FOR SOLVING THE PROBLEMS

The present invention is as defined in the appended claims.
The present invention is directed to a thermoplastic resin containing a structure represented by the following formula (A1).

[Formula 1]

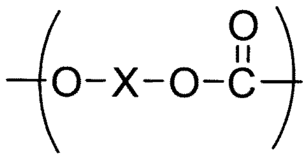

(A1)

[Formula 2]

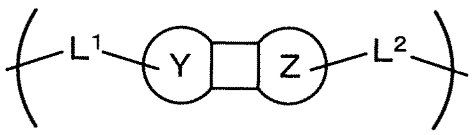

(B1)

X in the formula (A1) represents the above formula (B1). In the formula (B1), a ring Y and a ring Z forming a fused ring with cyclobutane are each independently an optionally-substituted alicyclic carbon ring having 15 or less carbon atoms. The alicyclic carbon ring optionally contains a hetero atom. In the formula (B1), $L^1$ and $L^2$ are each independently a direct bond or a divalent hydrocarbon group having 1 to 5 carbon atoms.

Disclosed herein is a polymerizable alicyclic compound represented by the following formula (P1) that can be used as a monomer for producing the thermoplastic resin of the present invention.

[Formula 3]

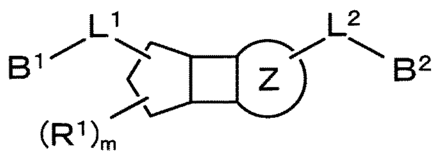

(P1)

In the formula (P1), a ring Z is an optionally-substituted alicyclic carbon ring having 6 to 15 carbon atoms. The alicyclic carbon ring optionally contains a hetero atom. $L^1$ and $L^2$ are each independently a direct bond or a divalent hydrocarbon group having 1 to 5 carbon atoms. $B^1$ and $B^2$ are hydroxy groups. $R^1$ is a substituent. m represents the number of the substituents $R^1$ and is an integer of 0 to 4.

ADVANTAGEOUS EFFECTS OF INVENTION

According to the present invention, it is possible to provide a novel thermoplastic resin having a carbonate bond and a fused ring.

Further, the polymerizable alicyclic compound is a compound having a fused-ring structure and has hydroxy groups. Therefore, the polymerizable alicyclic compound can be used as, for example, a monomer for producing a thermoplastic resin having a carbonate bond.

BRIEF DESCRIPTION OF DRAWINGS

Fig. 1 is a diagram showing a reaction for synthesizing a dihydroxy compound P1 by a production method A.
Fig. 2 is a diagram showing a reaction for synthesizing a dihydroxy compound P1 by a production method B.
Fig. 3 is a diagram showing a reaction for synthesizing a dihydroxy compound P1 by a production method C.
Fig. 4 is a diagram showing a reaction for synthesizing a dihydroxy compound P1 by a production method D.
Fig. 5 is a diagram showing a reaction for synthesizing a dihydroxy compound P1 by a production method E.
Fig. 6 is a diagram showing a reaction for synthesizing a compound (P1) from a compound (N1) and a compound (N2).
Fig. 7 is a diagram showing a first group of examples of a combination of starting materials and a dihydroxy compound obtained from the starting materials.

Fig. 8 is a diagram showing a second group of examples of a combination of starting materials and a dihydroxy compound obtained from the starting materials.
Fig. 9 is a diagram showing a reaction for synthesizing a dihydroxy compound P1-1 by a production method A.
Fig. 10 is a diagram showing a reaction for synthesizing a dihydroxy compound P1-1 by a production method B.
Fig. 11 is a diagram showing a reaction for synthesizing a dihydroxy compound P1-1 by a production method C.
Fig. 12 is a diagram showing a reaction for synthesizing a compound (P1) from a compound (N1) and a compound (N2).
Fig. 13 is a diagram showing polymerizable alicyclic compounds obtained from corresponding starting materials.
Fig. 14 is an NMR chart of a polycarbonate copolymer obtained in Example 1-1.
Fig. 15 is an NMR chart of a polycarbonate copolymer obtained in Example 1-2.
Fig. 16 is an NMR chart of a polycarbonate copolymer obtained in Example 1-3.
Fig. 17 is an NMR chart of a polycarbonate copolymer obtained in Example 1-4.
Fig. 18 is an NMR chart of a polycarbonate copolymer obtained in Example 1-5.

DESCRIPTION OF EMBODIMENTS

Embodiments of the present invention will be described in detail below, however, the description of the configurations etc. described later is an example of the embodiments of the present invention, and the present invention is not limited to the following contents as long as it does not go beyond the scope of the invention as defined by the appended claims. Further, when the word "to" is used to represent a range in this specification, numerical values or physical values before and after "to" are inclusive. Further, a numerical value or a physical value defined as an upper or lower limit is inclusive. Further, "ppm" and "%" represent "ppm by weight" and "% by weight", respectively, unless otherwise specified. Further, "part(s) by weight" and "part(s) by mass" are practically used synonymously with each other, and "weight %" and "mass%" are practically used synonymously with each other.

[I] First, a thermoplastic resin according to the present invention will be described.

The thermoplastic resin having a carbonate bond has a structure represented by the following formula (A1). In this specification, the thermoplastic resin having a carbonate bond is referred to as a "carbonate-based resin" as appropriate. Examples of the carbonate-based resin include a polycarbonate resin having a carbonate bond and a polyester carbonate resin having a carbonate bond and an ester bond.
The carbonate-based resin is a concept that includes a copolymer repeatedly having the structure represented by the formula (A1) and a structure other than the formula (A1) (specifically, a copolymeric polycarbonate, a copolymeric polyester carbonate) even when it is a homopolymer repeatedly having the structure represented by the formula (A1).

[Formula 4]

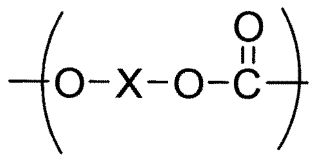

(A1)

The carbonate-based resin has a structure in which a plurality of divalent structural units (hereinafter also simply referred to as "structural units") are linked through divalent linking groups (hereinafter also simply referred to as "linking groups"). The linking group or a polymerization reaction group may be bonded to one of the structural units located at the end.
When at least part of the linking groups of the carbonate-based resin are carbonate bonds (-O-C(=O)-O-) and a plurality of linking groups are present in the carbonate-based resin, these linking groups may be a carbonate bond and other bond except for the carbonate bond. Examples of the other bond include an ester bond, an amide bond, a phosphonate bond, and a sulfone bond.
From the viewpoint that the carbonate-based resin can inexpensively and easily be synthesized, the linking groups in the carbonate-based resin are preferably all carbonate bonds or carbonate bonds and ester bonds. Further, from the viewpoint that the mole ratio of a monomer used for polymerization can easily be adjusted and polymerization can easily be performed, the linking groups in the thermoplastic resin are more preferably all carbonate bonds. That is, the carbonate-based resin is preferably a polycarbonate resin or a polyester carbonate resin, more preferably a polycarbonate

resin. When the linking group is asymmetric like an ester bond, the linking group may link together two adjacent structural units in any direction.

A structure represented by a formula (B1) is referred to as a "structural unit B1" as appropriate. The formula (B1) represents X in the formula (A1), and is a partial structural formula of the formula (A1).

[Formula 5]

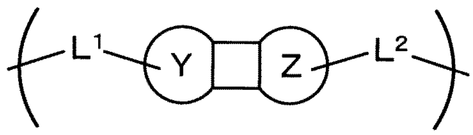

(B1)

The carbonate-based resin has the structural unit B1. As shown in the formula (B1), a ring Y and a ring Z sandwich cyclobutane, and each of the ring Y and the ring Z forms a fused ring with cyclobutane. In other words, the ring Y and the ring Z sandwich cyclobutane to be fused to the cyclobutane. In the formula (B1), the ring Y and the ring Z are each independently an alicyclic carbon ring having 15 or less carbon atoms. The alicyclic carbon ring means a cyclic skeleton of an alicyclic compound and is a concept that includes not only a ring constituted from only carbon but also a heterocycle. If the number of carbon atoms of the alicyclic carbon ring exceeds 15, there is no room for bond rotation due to many rigid bonds, and therefore there is a possibility that the carbonate-based resin becomes brittle. Further, if the number of carbon atoms exceeds 15, inexpensive synthesis is difficult, and therefore production costs increase. From the viewpoint of further preventing the resin from becoming brittle and production costs from increasing, the number of carbon atoms of the alicyclic carbon ring is preferably 12 or less, more preferably 10 or less. The lower limit of the number of carbon atoms of the alicyclic carbon ring is 3. It is noted that the number of carbon atoms of the alicyclic carbon ring specifically represents the number of atoms circularly linked in the alicyclic carbon ring, and in the case of a heterocycle, the number of carbon atoms includes not only the number of carbon atoms but also the number of atoms other than carbon (specifically, hetero atoms).

At least one of the alicyclic carbon rings of the ring Y and the ring Z may have one or two or more substituents. Examples of the substituent that at least one of the alicyclic carbon rings of the ring Y and the ring Z may have include a hydrocarbon group having 1 to 14 carbon atoms, an acyl group having 1 to 10 carbon atoms, an alkoxy group having 1 to 12 carbon atoms, an aryloxy group having 3 to 14 carbon atoms, an acyloxy group having 1 to 10 carbon atoms, a silyl group, a sulfinyl group, a sulfo group, an alkylthio group, an arylthio group, an amino group, a halogen atom, a nitro group, and a cyano group.

Specific examples of the hydrocarbon group having 1 to 14 carbon atoms include an alkyl group having 1 to 12 carbon groups, an alkenyl group having 2 to 10 carbon atoms, an alkynyl group having 2 to 10 carbon atoms, and an aryl group having 3 to 14 carbon atoms. Examples of the alkyl group having 1 to 12 carbon atoms include a methyl group, an ethyl group, a propyl group, an isopropyl group, and a butyl group. Examples of the alkenyl group having 2 to 10 carbon atoms include a vinyl group, an allyl group, and a butenyl group. Examples of the alkynyl group having 2 to 10 carbon atoms include an acetylene group and a propynyl group. Examples of the aryl group having 3 to 14 carbon atoms include a phenyl group, a tolyl group, and a naphthyl group.

Examples of the acyl group having 1 to 10 carbon atoms include an acetyl group and the like. Examples of the alkoxy group having 1 to 12 carbon atoms include a methoxy group, an ethoxy group, and a propoxy group. Examples of the aryloxy group having 3 to 14 carbon atoms include a phenoxy group and the like. Examples of the acyloxy group having 1 to 10 carbon atoms include a methoxyacetyl group and a phenoxyacetyl group. Examples of the silyl group include a trimethylsilyl group and the like. Examples of the sulfo group include a sulfo group, a methylsulfonyl group, and an ethylsulfonyl group. Examples of the sulfinyl group include a methyl sulfinyl group and an ethyl sulfinyl group. Examples of the alkylthio group include a methylthio group and an ethylthio group. Examples of the arylthio group include a phenylthio group and the like. Examples of the amino group include an amino group and a dimethylamino group.

The substituent that the ring Y and the ring Z may have, such as the hydrocarbon group having 1 to 14 carbon atoms, may further have one or more substituents, and examples of such a substituent include an alkoxy group, a halogen atom, a nitro group, and a cyano group.

From the viewpoint of increasing the ratio of a rigid skeleton to obtain a resin having excellent heat resistance, it is preferred that the alicyclic carbon ring in the structural unit of the carbonate-based resin has no substituent and is constituted from carbon atoms bonded to hydrogen atoms or has, as a substituent, a hydrocarbon group having 1 to 14 carbon atoms. Further, from the viewpoint of improving toughness and the viewpoint of reducing melt viscosity to improve flowability of the resin during, for example, molding, the substituent is preferably an alkyl group having 5 to 12 carbon atoms. Further, from the viewpoint of improving heat resistance of the resin, the substituent is preferably an alkyl group having 1 to 4 carbon atoms. From the viewpoint of further improving heat resistance of the resin and ease of synthesis of a monomer, the substituent is preferably an alkyl group having 1 to 2 carbon atoms, more preferably an alkyl group having 1

carbon atom (specifically, a methyl group). Further, from the viewpoint that heat resistance of the resin is further improved and raw materials are inexpensively available, it is preferred that each of the alicyclic carbon rings in the ring Y and the ring Z has one methyl group as a substituent, and other carbon atoms constituting the alicyclic carbon ring (i.e., carbon atoms other than a carbon atom bonded to the methyl group) are bonded to hydrogen atoms without being bonded to substituents. Further, from the viewpoint that the resin can achieve both excellent heat resistance and toughness and raw materials are inexpensively available, the alicyclic carbon rings of the ring Y and the ring Z preferably have no substituent. It is noted that $L^1$ and $L^2$ in the formula (B1) represent linking groups and are not regarded as substituents.

From the viewpoint of toughness and heat resistance of the carbonate-based resin, the substituent that the alicyclic carbon ring of the ring Y or the ring Z has is preferably an alkoxy group having 1 to 10 carbon atoms, and from the same viewpoint, the substituent is more preferably an alkoxy group having 1 to 3 carbon atoms. From the viewpoint of being more excellent in toughness and heat resistance, the substituent is even more preferably a methoxy group.

When a hydrogen atom on the β carbon atom relative to a hydroxy group is substituted by an alkyl group, elimination of the hydroxy group from a monomer can be prevented in production of the resin. This makes it possible to further increase a polymerization temperature to obtain a resin having a larger molecular weight. Therefore, when $L^1$ and $L^2$ are, for example, methylene groups, carbon atoms of the alicyclic carbon rings in the ring Y and the ring Z bonded to $L^1$ and $L^2$ are preferably further bonded to substituents.

In this specification, the alicyclic carbon ring is a concept that includes one having a fused ring. At least one of the alicyclic carbon rings of the ring Y and the ring Z may have a fused ring. In this case, as described above, the number of carbon atoms of the alicyclic carbon ring containing a fused ring (specifically, the ring Y or the ring Z) is 15 or less. When the alicyclic carbon ring contains a fused ring, it is possible to increase the bond distance between monomer units constituting the resin (specifically, the bond distance between the structural units B1). This makes it possible to improve mechanical properties, such as toughness, of the resin.

At least one of the alicyclic carbon rings of the ring Y and the ring Z may contain a hetero atom. Examples of the hetero atom include an oxygen atom, a sulfur atom, a nitrogen atom, and a halogen atom. From the viewpoint of imparting polarity to the structural unit B1 to improve affinity between the thermoplastic resin having the structural unit B1 and a dissimilar polymer or compatibility with a dissimilar polymer in blending, the hetero atom is preferably an oxygen atom and/or a sulfur atom. The alicyclic carbon ring may contain a hetero atom as, for example, the functional group described above. Further, the alicyclic carbon ring may contain a hetero atom as a cross-linked structure that will be described later. From the viewpoint that a monomer can easily be synthesized, the viewpoint that a condensation polymerization reaction is less likely to be inhibited in production of the carbonate-based resin, and the viewpoint that the polarity of the carbonate-based resin can be reduced to reduce a water absorption rate, the ring Y and the ring Z preferably contain no hetero atoms.

At least one of the alicyclic carbon rings of the ring Y and the ring Z preferably has at least one carbon ring constituted from 4 or more carbon atoms. In this case, the resin has a rigid structural skeleton and therefore has improved heat resistance.

At least one of the alicyclic carbon rings of the ring Y and the ring Z preferably has, like a norbornane skeleton or the like, a structure having a ring formed by bonding a hydrocarbon group having 5 or less carbon atoms and bonded to a carbon atom constituting the alicyclic carbon ring to another carbon atom constituting the alicyclic carbon ring. That is, the alicyclic carbon ring preferably has a structure formed by cross-linking a hydrocarbon group having 5 or less carbon atoms thereto. In this case, the resin has a rigid structural skeleton and therefore has improved heat resistance and pencil hardness. From the viewpoint of improving such an effect, the number of carbon atoms the hydrocarbon group of the cross-linked structure is more preferably 3 or less, even more preferably 2 or less.

Further, at least one of the alicyclic carbon rings of the ring Y and the ring Z preferably has, like oxabicyclo[2.2.1]heptane or the like, a structure having a ring formed by bonding an oxygen atom bonded to a carbon atom constituting the alicyclic carbon ring to another carbon atom constituting the alicyclic carbon ring. That is, the alicyclic carbon ring preferably has a structure formed by cross-linking an oxygen atom thereto (i.e., a cyclic ether bond). In this case, compatibility between the carbonate resin and a dissimilar polymer improves due to high polarity of the hetero atom. Further, at least one of the alicyclic carbon rings of the ring Y and the ring Z preferably has, like thiabicyclo[2.2.1]heptane or the like, a structure having a ring formed by bonding a sulfur atom bonded to a carbon atom constituting the alicyclic carbon ring to another carbon atom constituting the alicyclic carbon ring. That is, the alicyclic carbon ring preferably has a structure formed by cross-linking a sulfur atom thereto (i.e., a cyclic thioether bond). Also in this case, the effect of improving compatibility between the carbonate-based resin and a dissimilar polymer can be obtained due to high polarity of the hetero atom.

$L^1$ and $L^2$ are each independently a direct bond or a divalent hydrocarbon group having 1 to 5 carbon atoms. The hydrocarbon group is, for example, an alkylene group, and may have a straight chain or a branched chain. From the viewpoint of further improving toughness and molding processability (specifically, flowability of a melted resin during molding) of the resin, the number of carbon atoms of each of the hydrocarbon groups of $L^1$ and $L^2$ is preferably 3 to 5. On the other hand, from the viewpoint of achieving toughness of the resin and further improving heat resistance due to the formation of a rigid skeleton, the number of carbon atoms of each of the hydrocarbon groups of $L^1$ and $L^2$ is preferably 1 to 2.

Further, from the viewpoint that raw materials for synthesizing a monomer in production of the carbonate-based resin are inexpensive, $L^1$ and $L^2$ are preferably methylene groups. A methylene group is also referred to as a methanediyl group.

The alicyclic skeletons of the ring Y and the ring Z in the formula (B1) are preferably different from each other. That is, in the formula (B1), the ring Y and the ring Z are preferably asymmetric with respect to the cyclobutane ring. In this case, it is possible to obtain the effect of improving solubility, which is advantageous for synthesis of a monomer or formation of a polymer. Further, it is possible to obtain the effect of reducing the photoelastic coefficient of the carbonate-based resin having the structure represented by the formula (B1).

The formula (B1) is preferably represented by the following formula (B1') or (B1"). The formula (B1') corresponds to the formula (B1) in which one substituent is bonded to each of the ring Y and the ring Z, and $L^1$ and $L^2$ are methylene groups. The formula (B1") corresponds to the formula (B1) in which one substituent is bonded to each of the ring Y and the ring Z, and $L^1$ and $L^2$ are direct bonds. In this case, both the toughness and heat resistance of the resin can be achieved at high levels. In the formula (B1') and the formula (B1"), the ring Y and the ring Z forming a fused ring with cyclobutane are each independently an alicyclic carbon ring constituted from 4 or more and 15 or less carbon atoms. Similarly to the formula (B1), this alicyclic carbon ring may contain a hetero atom and may have a fused ring. $R^1$ and $R^2$ are each independently a hydrogen atom, a hydrocarbon group having 1 to 14 carbon atoms, or an alkoxy group having 1 to 12 carbon atoms. The hydrocarbon group and the alkoxy group are the same as those described above.

[Formula 6]

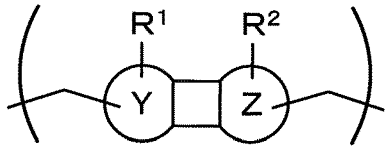

(B1')

[Formula 7]

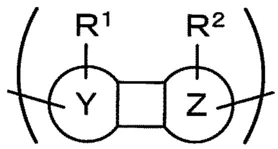

(B1'')

In the formula (B1'), $R^1$ and $R^2$ are preferably each independently a hydrogen atom or a methyl group. Further, $R^1$ and $R^2$ in the formula (B1') are preferably methyl groups. That is, carbon atoms other than carbon atoms bonded to methyl groups are bonded to hydrogen atoms without being bonded to substituents. In this case, raw materials are inexpensively available, and the carbonate-based resin has improved heat resistance due to its rigid structural skeleton. From the same viewpoint, $R^1$ and $R^2$ in the formula (B1") are preferably each independently a methoxy group or an ethoxy group. Further, $R^1$ and $R^2$ in the formula (B1') are more preferably hydrogen atoms. That is, the alicyclic carbon rings in the ring Y and the ring Z have no substituents, and carbon atoms constituting the alicyclic carbon rings are bonded to hydrogen atoms. Also in this case, raw materials are inexpensively available, and the carbonate-based resin has improved heat resistance due to its rigid structural skeleton.

The ring Y and the ring Z in the formula (B1), the formula (B1'), and the formula (B1") are preferably each independently any one of rings selected from among the following formulas (C1) to (C11), and are more preferably each independently any one of alicyclic carbon rings represented by the formulas (C1) to (C7). In this case, both heat resistance and toughness can be achieved. It is noted that * in the formulas (C1) to (C11) represents a site where the fused ring is formed. Each of the rings represented by the formulas (C1) to (C11) forms a fused ring with cyclobutane at a position between * and * in the formula (B1), the formula (B1'), and the formula (B1"). The formulas (C1) to (C11) are partial structural formulas representing the ring Y and the ring Z in the formula (B1), the formula (B1'), and the formula (B1").

[Formula 8]

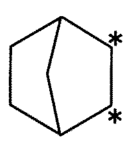

(C1)

[Formula 9]

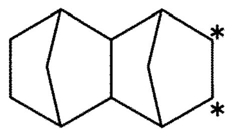

(C2)

[Formula 10]

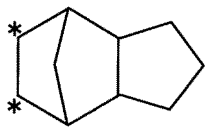

(C3)

[Formula 11]

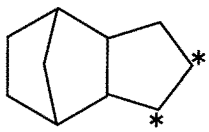

(C4)

[Formula 12]

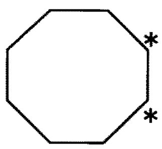

(C5)

[Formula 13]

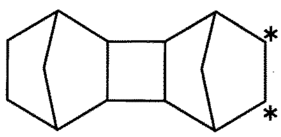

(C6)

[Formula 14]

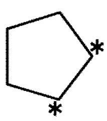

(C7)

[Formula 15]

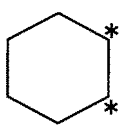

(C8)

[Formula 16]

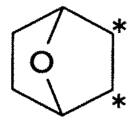

(C9)

[Formula 17]

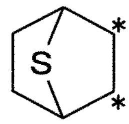

(C10)

[Formula 18]

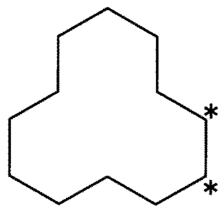

(C11)

From the viewpoint of achieving both toughness and heat resistance of the carbonate-based resin, the ring Y and the ring Z in the formula (B1), the formula (B1'), and the formula (B1") are preferably each independently any one selected from the group consisting of the formula (C5), the formula (C7), the formula (C8), and the formula (C11). Further, from the viewpoint that heat resistance of the carbonate-based resin is improved and raw materials are inexpensively available, the ring Y and the ring Z in the formula (B1), the formula (B1'), and the formula (B1") are preferably each independently the formula (C2) or the formula (C6). Further, from the viewpoint that polarity is imparted to the structural unit B1 to improve affinity between the carbonate-based resin and a dissimilar polymer (specifically, a polar resin) or adhesiveness at the interface with a dissimilar polymer, heat resistance is improved, and raw materials are inexpensively available, the ring Y and the ring Z in the formula (B1), the formula (B1'), and the formula (B1") are preferably each independently the formula (C9) or the formula (C10). Further, from the viewpoint that toughness and heat resistance of the carbonate-based resin are improved and raw materials are inexpensively available, the ring Y and the ring Z in the formula (B1), the formula (B1'), and the formula (B1") are preferably each independently any one selected from the group consisting of the formula (C1), the formula (C3), the formula (C4), and the formula (C7). Further, from the viewpoint that toughness of the carbonate-based resin is further improved and raw materials are inexpensively available, the ring Y and the ring Z in the formula (B1), the formula (B1'), and the formula (B1") are preferably each independently any one selected from the group consisting of the formula (C3), the formula (C4), and the formula (C7).

Specific examples of the formula (B1') and the formula(B1") include the following formulas (B2) to (B23).

[Formula 19]

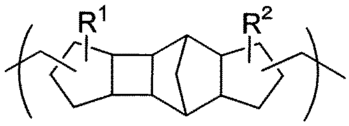

(B2)

[Formula 20]

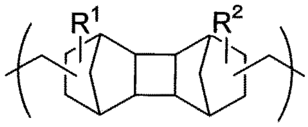

(B3)

[Formula 21]

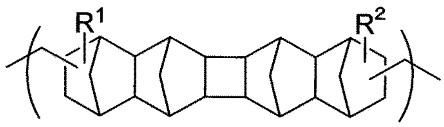

(B4)

[Formula 22]

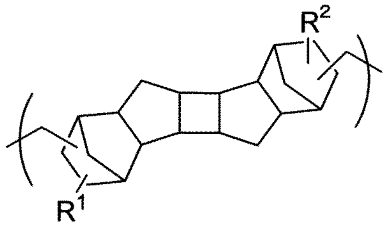

(B5)

[Formula 23]

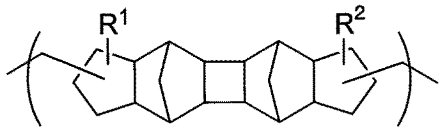

(B6)

[Formula 24]

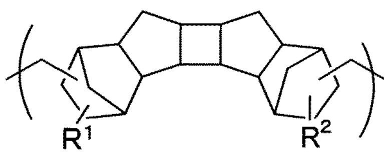

(B7)

[Formula 25]

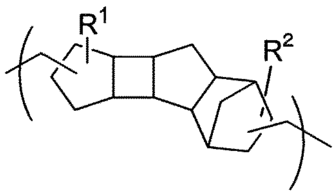

(B8)

[Formula 26]

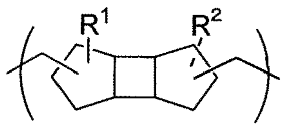

(B9)

[Formula 27]

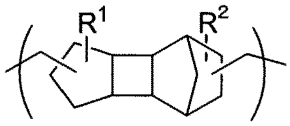

(B10)

[Formula 28]

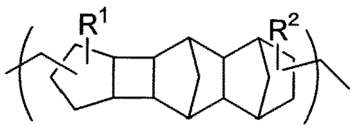

(B11)

[Formula 29]

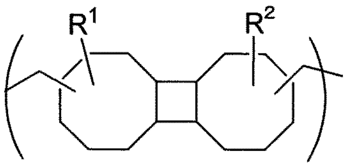

(B12)

[Formula 30]

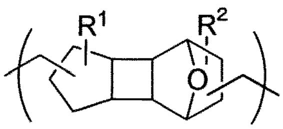

(B13)

[Formula 31]

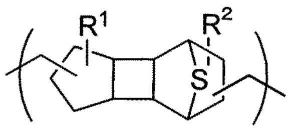

(B14)

[Formula 32]

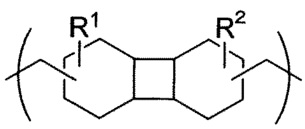

(B15)

[Formula 33]

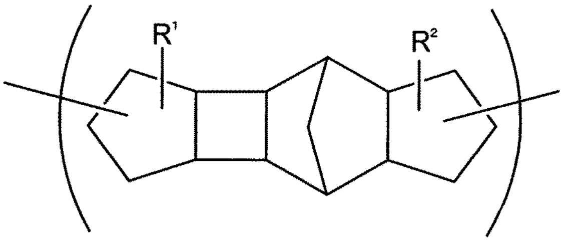

(B16)

[Formula 34]

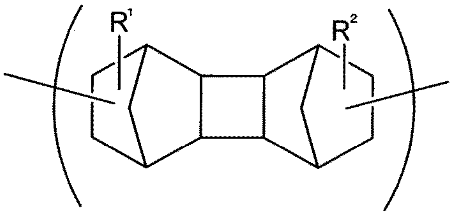

(B17)

[Formula 35]

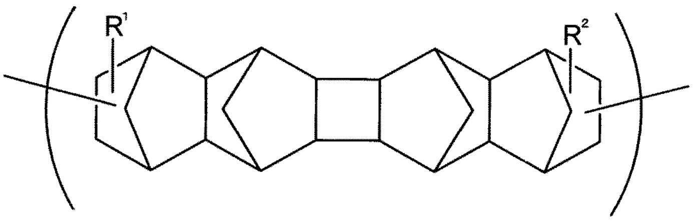

(B18)

[Formula 36]

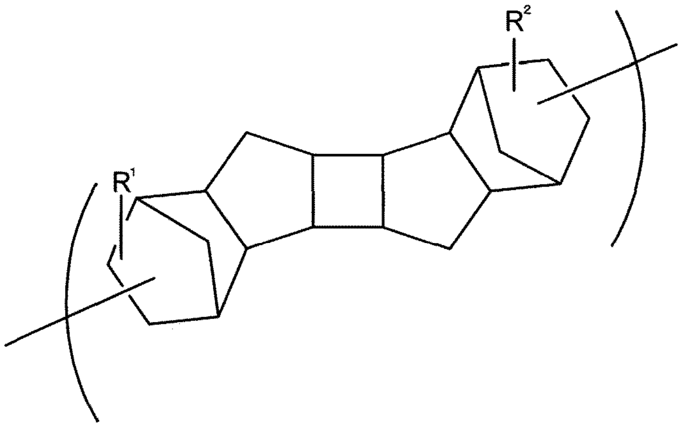

(B19)

[Formula 37]

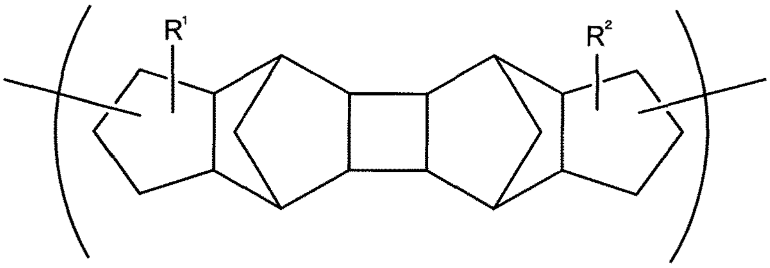

(B20)

[Formula 38]

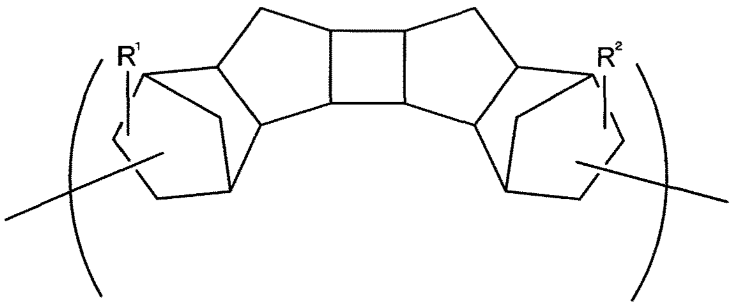

(B21)

[Formula 39]

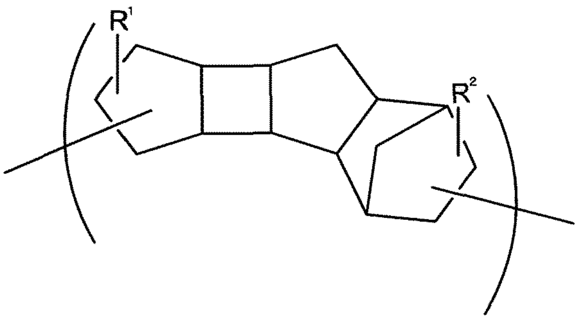

(B22)

[Formula 40]

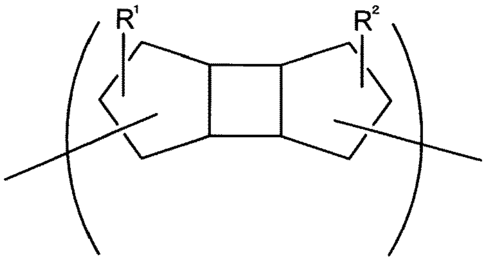

(B23)

[Formula 41]

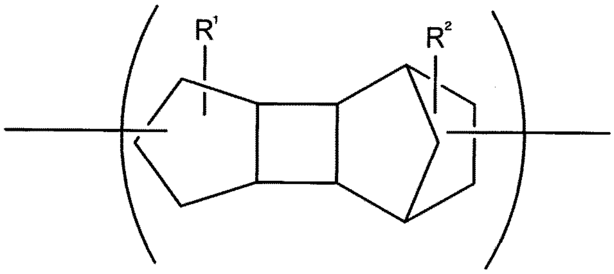

(B24)

[Formula 42]

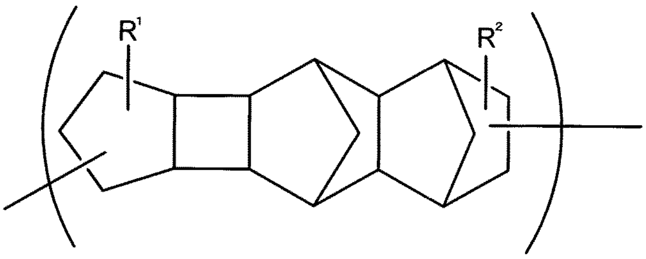

(B25)

[Formula 43]

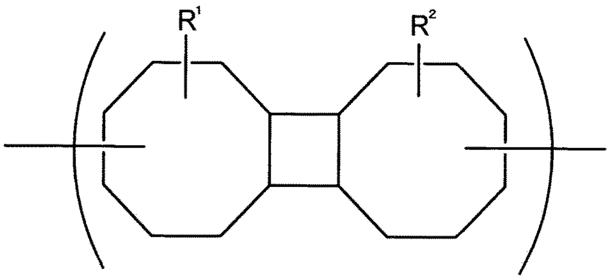

(B26)

$R^1$ and $R^2$ in the formulas (B2) to (B26) are each independently a hydrogen atom, a hydrocarbon group having 1 to 14 carbon atoms, or an alkoxy group having 1 to 12 carbon atoms. The hydrocarbon group having 1 to 14 carbon atoms and the alkoxy group having 1 to 12 carbon atoms are the same as those described above with reference to the formula (B1).

From the viewpoint that raw materials are inexpensively available, $R^1$ and $R^2$ in the formulas (B2) to (B15) are preferably each independently a hydrogen atom or an alkyl group having 1 to 12 carbon atoms and are more preferably each independently a hydrogen atom or a methyl group. The alkyl group having 1 to 12 carbon atoms is the same as that described above with reference to the formula (B1).

Further, from the viewpoint that raw materials are inexpensively available, $R^1$ and $R^2$ in the formulas (B16) to (B26) are preferably each independently an alkoxy group having 1 to 4 carbon atoms and are more preferably each independently a methoxy group or an ethoxy group.

From the viewpoint of further improving heat resistance of the carbonate-based resin, the formula (B1') is

preferably the formula (B4), the formula (B5), the formula (B7), or the formula (B11), and $R^1$ and $R^2$ are preferably each independently a hydrogen atom or a methyl group. From the viewpoint of imparting polarity to the structural unit B1 to improve affinity between the carbonate-based resin and a dissimilar polymer or adhesiveness at the interface with a dissimilar polymer, the formula (B1') is preferably the formula (B13) or the formula (B14), and $R^1$ and $R^2$ are preferably each independently a hydrogen atom or a methyl group. From the viewpoint that both heat resistance and toughness of the carbonate-based resin can be achieved at high levels, the formula (B1') is preferably the formula (B2), the formula (B3), or the formula (B11), and $R^1$ and $R^2$ are preferably each independently a hydrogen atom or a methyl group. From the viewpoint that both heat resistance and toughness of the carbonate-based resin can be achieved at high levels and raw material are inexpensively available, the formula (B1') is preferably the formula (B2) or the formula (B3), and $R^1$ and $R^2$ are preferably each independently a hydrogen atom or a methyl group. From the viewpoint that both heat resistance and toughness of the carbonate-based resin can be achieved at high levels, raw materials are inexpensively available, and a monomer can easily be synthesized and is inexpensive, the formula (B1') is preferably the formula (B2), and $R^1$ and $R^2$ are preferably each independently a hydrogen atom or a methyl group.

From the viewpoint of further improving heat resistance of the carbonate-based resin, the formula (B1") is preferably the formula (B18), the formula (B19), the formula (B21), or the formula (B25), and $R^1$ and $R^2$ are preferably each independently a methoxy group or an ethoxy group. From the viewpoint that both heat resistance and toughness of the carbonate-based resin can be achieved at high levels, the formula (B1") is preferably the formula (B16), the formula (B17), or the formula (B25), and $R^1$ and $R^2$ are preferably each independently a methoxy group or an ethoxy group. From the viewpoint that both heat resistance and toughness of the carbonate-based resin can be achieved at high levels and raw material are inexpensively available, the formula (B1") is preferably the formula (B2) or the formula (B17), and $R^1$ and $R^2$ are preferably each independently a methoxy group or an ethoxy group. From the viewpoint that both heat resistance and toughness of the carbonate-based resin can be achieved at high levels, raw materials are inexpensively available, and a monomer can easily be synthesized and is inexpensive, the formula (B1") is preferably the formula (B16), and $R^1$ and $R^2$ are preferably each independently a methoxy group or an ethoxy group.

Hereinbelow, a monomer for forming the structural unit B1 will be described. The carbonate-based resin has a repeating structural unit represented by the formula (B1). The repeating structural unit represented by the formula (B1) is referred to as a "first structural unit B1" as appropriate. The carbonate-based resin having such a repeating structural unit is produced using, as a monomer material, a dihydroxy compound represented by the following formula (P1). That is, a dihydroxy compound is used in which a hydroxy group is bonded to the end of each of $L^1$ and $L^2$ that function as linking groups in the formula (B1). As described above, each of $L^1$ and $L^2$ may be a direct bond. It is noted that the dihydroxy compound forms a polymer chain of the carbonate-based resin with the oxygen atom of the hydroxy group. Therefore, in the carbonate-based resin, the structure represented by the formula (B1) containing no oxygen atom derived from the hydroxy group of the dihydroxy compound can be regarded as a structural unit, but the structure represented by the formula (B1) in which -O- is bonded to the end of each of $L^1$ and $L^2$ can also be regarded as a structural unit. The structural unit represented by the formula (B1) in which -O- is bonded to the end of each of $L^1$ and $L^2$ is referred to as a "second structural unit B1" as appropriate.

[Formula 44]

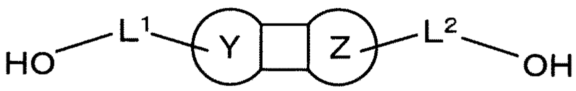

(P1)

A ring Y, a ring Z, a substituent, a hetero atom, a fused ring, $L^1$, and $L^2$ in the formula (P1) are the same as those in the formula (B1) except that the formula (P1) represents a dihydroxy compound, and preferred modes of the formula (P1) are also the same as those of the formula (B1) described above with reference to the formula (B1') and the formula (B1"). Preferred modes of the formula (P1) are shown by formulas (P2) to (P15). $R^1$ and $R^2$ in the formulas (P2) to (P15) are the same as those in the formulas (B2) to (B15). It is noted that specifically, the formulas (P2) to (P15) are preferred modes of a monomer for forming the formula (B1'). Examples of a preferred mode of a monomer for forming the formula(B1") include monomers represented by the formulas (P2) to (P15) in which two hydroxymethyl groups are replaced with hydroxy groups, and their chemical formulas are not shown except for a formula (P16).

[Formula 45]

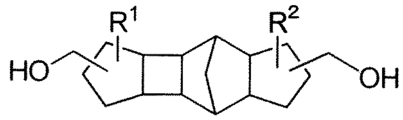

(P2)

[Formula 46]

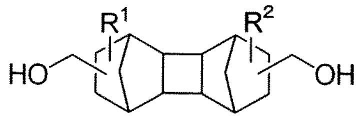

(P3)

[Formula 47]

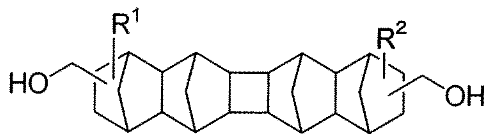

(P4)

[Formula 48]

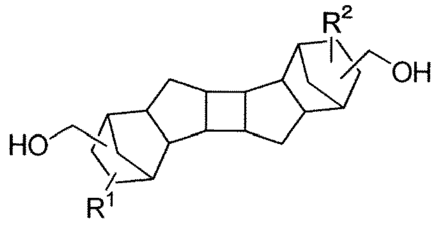

(P5)

[Formula 49]

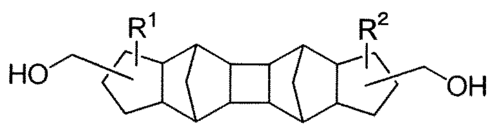

(P6)

[Formula 50]

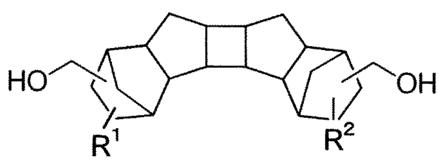

(P7)

[Formula 51]

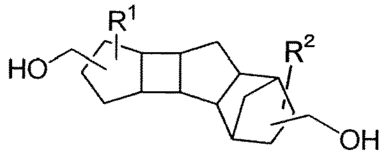

(P8)

[Formula 52]

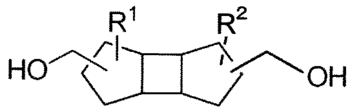

(P9)

[Formula 53]

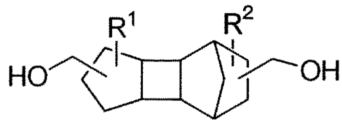

(P10)

[Formula 54]

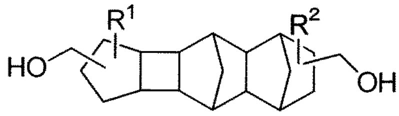

(P11)

[Formula 55]

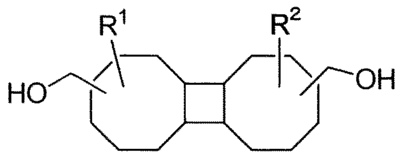

(P12)

[Formula 56]

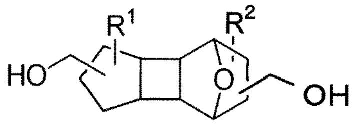

(P13)

[Formula 57]

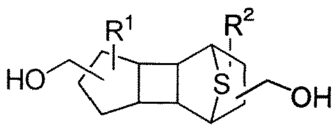

(P14)

[Formula 58]

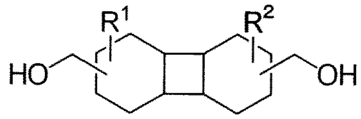

(P15)

[Formula 59]

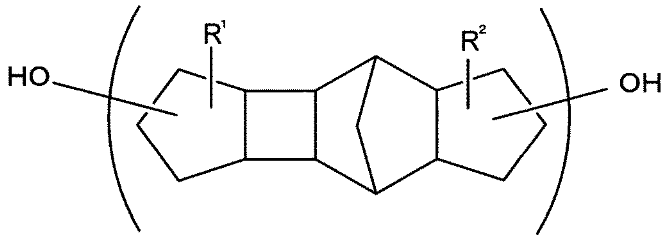

(P16)

When a polycarbonate resin is produced using, as a monomer, a compound represented by the following formula (P24), a polycarbonate resin having a structural unit represented by the following formula (B24) is obtained. The brittle fracture stress of a homopolycarbonate of the compound represented by the formula (P24) was predicted by software for predicting physical properties of polymers, Polymer-Design Tools version 1.1 produced by DTW Associates, Inc. (USA), and as a result, a value predicted by the Bicerano method was 153 MPa. This exceeds the brittle fracture stress of a homopolycarbonate of 2,3-di(hydroxymethyl)-perhydro-1,4:5,8-dimethanonaphthalene

(DMNDM) represented by the following formula (Z1) predicted by the same method. Brittle fracture means that when a force is exerted on a resin, the resin fractures with little deformation, and stress at which brittle fracture occurs is referred to as brittle fracture stress. In general, it is considered that when the brittle fracture stress of a resin is larger, the resin can withstand larger stress and therefore has excellent toughness. That is, it is supposed that the polycarbonate resin having the structural unit represented by the formula (B24) has excellent toughness. A polycarbonate resin having the structural unit represented by the formula (B1) having a structure similar to that of the formula (B24) is also considered to have excellent toughness.

Further, the glass transition temperature Tg of a polycarbonate resin produced using, as a monomer, the compound represented by the formula (P24) estimated by the Bicerano method of the software described above was 120°C (extrapolation value). This exceeds the glass transition temperature of a homopolycarbonate of tricyclodecanedimethanol that will be described later. That is, it is supposed that the polycarbonate resin having the structural unit represented by the formula (B24) has excellent heat resistance. A polycarbonate resin having the structural unit represented by the formula (B1) having a structure similar to the that of the formula (B24) is also considered to have excellent heat resistance.

[Formula 60]

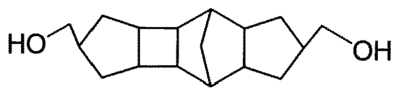

(P24)

[Formula 61]

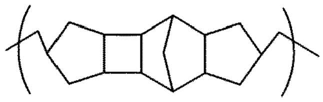

(B24)

[Formula 62]

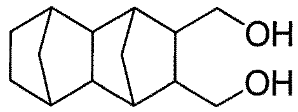

(Z1)

A method for producing the dihydroxy compound represented by the formula (P1) is not particularly limited, but for example, any of the production methods A to C shown in Fig. 1 to Fig. 3 is available. Alternatively, a production method D shown in Fig. 4 or a production method E shown in Fig. 5 is also available.

In Figs. 1 to 3, a ring Y1 and a ring Z1 are each independently an alicyclic carbon ring having two double bonds and 15 or less carbon atoms. This alicyclic carbon ring may have a substituent, may contain a hetero atom, and may have a fused ring. The substituent, the hetero atom, and the fused ring are the same as those described above with reference to the formula (B1).

In Figs. 1 to 3, a ring Y2 and a ring Z2 are each independently an alicyclic carbon ring having one double bond and 15 or less carbon atoms. This alicyclic carbon ring may have a substituent, may contain a hetero atom, and may have a fused ring. The substituent, the hetero atom, and the fused ring are the same as those described above with reference to the formula (B1).

In Figs. 1 to 3, $M^1$ and $M^2$ are each independently a direct bond or a divalent hydrocarbon group having 1 to 4 carbon atoms. The divalent hydrocarbon group may have a substituent having 1 to 5 carbon atoms. Specifically, the divalent hydrocarbon group is an alkylene group. $L^1$ and $L^2$ are the same as those described above with reference to the formula (B1). P is a hydrogen atom, an alkyl group having 1 to 5 carbon atoms, or an aryl group having 6 to 12 carbon atoms.

As shown in Fig. 1, a compound (M3) is produced from a compound (M1) and a compound (M2). Specifically, two unsaturated molecules, one of which has 2m $\pi$ electrons and the other of which has 2n $\pi$ electrons, such as the compound (M1) and the compound (M2) form a metallacycle (not shown) in the presence of a transition metal active species (specifically, a metallic catalyst) due to formation of two metal-carbon bonds and one carbon-carbon bond and cyclization. At this time, the valence of a metallic center formally increases by 2 due to its oxidation state. Then, reductive elimination occurs so that a carbon-carbon bond is formed, and the oxidation state of the transition metal center is reduced and an active species is regenerated. This allows a plurality of reactions, such as multimerization of unsaturated molecules, to proceed. At this time, when Pd or Ni is used, a four-membered ring is formed (see "Organotransition Metal Catalyzed Cycloaddition" written by Itoh Kenji, 2002, vol. 60. No. 1, p.28 - p.41). Further, when an Fe catalyst is used, a four-membered ring is formed from two unsaturated molecules (see Jordan M. Hoyt et al., "Iron-catalyzed intermolecular [2+2] cycloadditions of unactivated alkenes", "Science" Vol. 349, Issue 6251, Aug. 28, 2015, p. 960 - p. 963). Further, a four-membered ring is formed by using Ni in an unsaturated structure having a hetero atom at a bridgehead position or another position, such as 1,4-dihydro-epoxynaphthalene (see Daw-Jen Huang et al., "[2+2]Dimerization of norbornadiene and its derivatives in the presence of nickel complexes and zinc metal", "Journal of Organometallic Chemistry", 490, 1995, C1 - C7).

In the production methods shown in Fig. 1 to Fig. 3, a cyclic diene compound such as a cyclopentadiene skeleton and a compound having a distorted unsaturated bond such as a norbornene skeleton can be used as the compound (M1), the compound (M2), the compound (M5), and the compound (M8). When a compound having a cyclopentadiene skeleton and a compound having a norbornene skeleton are used as raw materials, reactivity of the raw materials increases so that a four-membered ring structure (specifically, cyclobutane) is easily formed.

Further, a compound having a distorted unsaturated bond such as a norbornene skeleton forms a four-membered ring by using a photocyclization reaction. In fact, it is known that dicyclopentadiene is dimerized by light irradiation in the presence of a metallic catalyst (see Robert G. Salomon et al., "Copper(I) catalysis in photocycloadditions. I. Norbornene", "Journal of the American Chemical Society", 1974, 96, 4, p. 1137). Therefore, a compound having a four-membered ring can be produced using a photocyclization reaction. However, the yield of photoreaction is generally low, and photoreaction is not suitable for synthesis of an alicyclic compound having a structure asymmetric with respect to a four-membered ring, such as alicyclic compounds shown in Figs. 1 to 3 and Fig. 6.

As shown in Fig. 1, in the production method A, a cyclization reaction between the compound (M1) and the compound (M2) occurs so that a compound (M3) is obtained. Then, an aldehyde is added to the compound (M3) by an oxo reaction to obtain a compound (M4). Then, the compound (M4) is subjected to a hydrogenation reaction (i.e., a reduction

reaction) to obtain a compound P1.

In the production method A, the cyclization reaction between the compound (M1) and the compound (M2) uses $Pd(dba)_2$, $Pd(acac)_2$, $Pd(PPh_3)_4$, $PdCl_2(PPh_3)_2$, $PdCl_2(dppp)$, $PdCl_2(H_2NCH_2CH_2NH_2)$, $NiCl_2$, $NiBr_2$, $NiCl_2(PPh_3)_2$, or $Ni(Cod)_2$ as a transition metal catalyst, $FeCl_3$ as an iron catalyst, or $TiCl_4$ as a titanium catalyst. As a ligand, $P(p\text{-tolyl})_3$, $PPh_3$, $P[(p\text{-MeO})Ph]_3$, or $P(Cy)_3$ is used. However, a ligand may not be used. As a reducing agent, for example, zinc can be used. A reaction temperature is, for example, 60 to 200°C. As a solvent, acetonitrile, tetrahydrofuran (THF), toluene, xylene, trimethylbenzene, tetralin, decalin, or the like is used. When a low-boiling solvent is used, pressure may be applied. For example, the reaction is performed under a pressure condition of 0.5 MPa to 10 MPa. During the reaction, for the purpose of adjusting pH, acetic acid, propionic acid, 4-methoxybenzoic acid, benzoic acid, formic acid, chloroacetic acid, trifluoroacetic acid, pyridinium p-toluenesulfonate (PPTS), tosic acid, methanesulfonic acid, sulfuric acid, or the like is used.

The compound (M4) shown in Fig. 1, in which $M^1$ and $M^2$ are direct bonds, is obtained from the compound (M3) by, for example, an oxo reaction. A reaction temperature is, for example, 20 to 200°C. A rhodium compound used in this step may be in any form as long as it forms a complex with an organophosphorus compound and exhibits hydroformylation activity in the presence of hydrogen and carbon monoxide, and may be in the form of a precursor. That is, a catalyst precursor material such as $Rh(acac)(CO)_2$, $Rh_2O_3$, $Rh_4(CO)_{12}$, $Rh_6(CO)_{16}$, $Rh(NO_3)_3$, or the like may be introduced into a reaction mixture together with an organophosphorus compound to form a rhodium hydride carbonyl phosphorus complex having catalytic activity in a reaction container, or a rhodium hydride carbonyl phosphorus complex catalyst previously prepared may be introduced into a reaction container. A suitable amount of the rhodium catalyst is as follows. A suitable amount of the rhodium catalyst per 100 parts by weight of the compound (M3) as a raw material is preferably 50 to 50000 ppm, more preferably 50 to 2000 ppm in terms of the amount of rhodium metal. The mole ratio between the ligand and the rhodium metal (ligand/rhodium) is in the range of 1 to 50. Examples of the organophosphorus compound include a phosphine represented by $P(-R^1)(-R^2)(-R^3)$ and a phosphite represented by $P(-OR^1)(-OR^2)(-OR^3)$. Specific examples of $R^1$, $R^2$, and $R^3$ in the phosphine and the phosphite include an optionally-substituted alkyl group having 1 to 12 carbon atoms, an optionally-substituted alicyclic alkyl group having 1 to 12 carbon atoms, and an optionally-substituted aryl group having 1 to 12 carbon atoms. Preferred specific examples of the phosphite used in this step include tris(2-t-butylphenyl) phosphite, tris(3-methyl-6-t-butylphenyl) phosphite, tris(3-methoxy-6-t-butylphenyl) phosphite, tris(2,4-di-t-butylphenyl) phosphite, and di(2-t-butylphenyl)t-butyl phosphite. The organophosphorus compound is not limited to these phosphine and phosphite. Further, the phosphines and the phosphites may be used singly or in combination of two or more of them. As a solvent, methylcyclohexane, cyclohexane, toluene, xylene, trimethylbenzene, an aliphatic hydrocarbon having 5 or more carbon atoms, ethylbenzene, propanol, butanol, or the like is used. A pressure condition during the reaction is, for example, 1 MPa to 15 MPa. The reaction uses, for example, a mixed gas of hydrogen and carbon monoxide, and the mole ratio between hydrogen and carbon monoxide in this mixed gas is preferably in the range of 0.2 to 5.0. The mole ratio in this mixed gas is a compositional ratio in an introduced gas. A method for synthesizing the compound (M4) containing aldehyde groups is not limited to the oxo reaction, and may be other known reaction.

The compound (M4) can be converted to a compound (P1) by a reduction reaction. The reduction reaction can use a hydrogenation catalyst (i.e., a reducing catalyst). As the hydrogenation catalyst, a metallic catalyst such as a transition metal catalyst can be used. Examples of such a metallic catalyst to be used include: the rhodium catalysts mentioned above with reference to the oxo reaction; metals belonging to Group VIII of the Periodic Table and known to have hydrogen reduction ability, such as nickel, cobalt, ruthenium, palladium, and platinum; copper chromite; and copper-zinc. These metallic catalysts may be used as an elemental metal or a metal oxide, or may be used in the form of an elemental metal or a metal oxide supported by an inorganic carrier such as silica, alumina, diatomite, or carbon, or may be used in the form of a metallic complex. From the viewpoint of a hydrogen reduction reaction speed and catalyst separation after reaction, among these hydrogenation catalysts, a Rh catalyst, raney nickel, nickel/diatomite, copper chromite, ruthenium/carbon, or a ruthenium/alumina catalyst is particularly suitably used. As a solvent in the reduction reaction, methylcyclohexane, cyclohexane, toluene, xylene, trimethylbenzene, an aliphatic hydrocarbon having 5 or more carbon atoms, ethylbenzene, propanol, butanol, or the like is used. A pressure condition during the reaction is, for example, 1 MPa to 15 MPa. As a ligand, a ligand preferably used in the oxo reaction may be used, or a ligand may not be used. The mole ratio between hydrogen and carbon monoxide in a carbon monoxide mixed gas is preferably in the range of 0.2 to 5.0 in terms of the composition of an introduced gas (hydrogen/carbon monoxide), or only hydrogen gas is preferably used. It is noted that after the oxo reaction, hydrogen reduction may be performed without performing quenching. In this case, the same catalyst as that used in the oxo reaction step is added, or a preferred catalyst is separately added. The synthesis of (P1) from (M4) containing aldehyde groups is not limited to the hydrogen reduction reaction, and may be performed by another known reaction. An example of another reducing agent includes sodium boron hydride. The reducing agent such as sodium boron hydride is used together with a solvent used in the above-described reduction step or an alcohol solvent such as methanol or ethanol.

Abbreviations for ligands are as follows.

dba: dibenzylideneacetone
acac: acetylacetonate
PPh: phenylphosphine
dppp: diphenylphosphinopropane
tolyl: methylphenyl group
Cy: cyclohexyl group
Me: methyl group
Ph: phenyl group

As shown in Fig. 2, the production method B is a method in which a compound (M1) and a compound (M5) are used as starting materials of a cyclization reaction. Unlike the compound (M2) in the production method A, the compound (M5) in the production method B has only one unsaturated bond, and therefore excessive cyclization is prevented in the production method B. In this point, the production method B is preferably performed. A compound (M6) is obtained by a cyclization reaction between the compound (M1) and the compound (M5). Then, an aldehyde is added to the compound (M6) by, for example, an oxo reaction to obtain a compound (M7). Then, the compound (M7) is subjected to a hydrogenation reaction to obtain a compound P1. The reaction conditions of the cyclization reaction, the oxo reaction, and the hydrogenation reaction are the same as those in the production method A.

As shown in Fig. 3, the production method C is a method in which a compound (M1) and a compound (M8) are used as starting materials of a cyclization reaction. Unlike the compound (M2) in the production method A, the compound (M8) in the production method C has only one unsaturated bond, and therefore excessive cyclization is prevented in the production method C. In this point, the production method C is preferably performed. A compound (M9) is obtained by a cyclization reaction between the compound (M1) and the compound (M8). Then, an aldehyde is added to the compound (M9) by, for example, an oxo reaction to obtain a compound (M10). Then, the compound (M10) is subjected to a hydrogenation reaction to obtain a compound P1. The reaction conditions of the cyclization reaction, the oxo reaction, and the hydrogenation reaction are the same as those in the production method A.

As shown in Fig. 4, the production method D is a method in which a compound (M5) and a compound (M5') synthesized by the same method as the compound (M5) are used as starting materials of a cyclization reaction. Unlike the compound (M1) and the compound (M2) in the production method A, the compound (M5) and the compound (M5') in the production method D each have only one unsaturated bond. Therefore, excessive cyclization is prevented in the production method D. In this point, the production method D is preferably performed. A compound (P1) is obtained by a cyclization reaction between the compound (M5) and the compound (M5'). The compound (M5) and the compound (M5') may be the same. The reaction conditions of the cyclization reaction are the same as those in the production method A.

As shown in Fig. 5, the production method E is a method in which a compound (M8) and a compound (M8') synthesized by the same method as the compound (M8) are used as starting materials of a cyclization reaction. Unlike the compound (M1) and the compound (M2) in the production method A, the compound (M8) and the compound (M8') in the production method E each have only one unsaturated bond. Therefore, excessive cyclization is prevented in the production method E. In this point, the production method E is preferably performed. A compound (M11) is obtained by a cyclization reaction between the compound (M8) and the compound (M8'). Then, the compound (M11) is subjected to a hydrogenation reaction to obtain a compound P1. The compound (M8) and the compound (M8') may be the same. The reaction conditions of the cyclization reaction and the hydrogenation reaction are the same as those in the production method A.

It is noted that in Fig. 1 to Fig. 5, substituents are not shown, but a compound P1 having substituents bonded to the alicyclic carbon rings can be produced by, for example, using compounds having a substituent as the compound (M1), the compound (M2), the compound (M5), the compound (M5'), the compound (M8), and the compound (M8').

As shown in Fig. 6, a compound (P2) is obtained by, for example, the production method A. A compound (N3) and a compound (N4) are obtained by a cyclization reaction between a compound (N1) and a compound (N2). It is noted that also when only (N1) is used as a starting material without using (N2) in this step, (N2) is generated due to the occurrence of cracking in the reaction, and as a result, (N3) and (N4) can be obtained by a cyclization reaction between the compound (N1) and the compound (N2). Further, also when only (N2) is used without using (N1) in this step, (N1) is generated due to the occurrence of dimerization of (N2) in the reaction, and as a result, (N3) and (N4) can be obtained by a cyclization reaction between the compound (N1) and the compound (N2) (see Kyung-sun Son, "Selective synthesis of tricyclopentadiene from dicyclopentadiene with homogeneous Pd catalysts", "Applied Organometallic Chemistry", 2014, Vol. 28, p. 151-155). Then, a compound (N5) is obtained from the compound (N3) and the compound (N4) by an oxo reaction, and a compound (P2) is obtained by subjecting the compound (N5) to a reduction reaction. It is noted that in Fig. 6, substituents are not shown, but as described above, a compound P2 having substituents can be obtained by, for example, using compounds having a substituent as the compound (N1) and the compound (N2).

Fig. 7 shows raw materials used for synthesis of the compound (P2), a compound (P8), a compound (P9), a compound (P11), a compound (P13), and a compound (P14) as examples of the compound (P1). In Fig. 7, $B^1$ and $B^2$ are

hydroxymethyl groups.

The compound (P2) shown in Fig. 7 is produced by the production method A in which only a compound (Q1) or a compound (Q2) is used as a starting material or a compound (Q1) and a compound (Q2) are used as starting materials. The compound (P8) is produced by the production method B or C in which a compound (Q1) and a compound (Q3) are used as raw materials. The compound (P9) is produced by the production method B or C in which a compound (Q1) and a compound (Q4) are used as raw materials. The compound (P11) is produced by the production method B or C in which a compound (Q1) and a compound (Q5) are used as raw materials. The compound (P13) is produced by the production method B or C in which a compound (Q1) and a compound (Q6) are used as raw materials. The compound (P14) is produced by the production method B or C in which a compound (Q1) and a compound (Q7) are used as raw materials. Other compounds represented by the formula (P1) can also be produced by, for example, the production methods A to C by appropriately selecting starting materials.

Fig. 8 shows raw materials used for synthesis of the compound (P4), a compound (P5), a compound (P6), a compound (P12), and a compound (P15) as examples of the compound (P1). In Fig. 8, $B^1$ and $B^2$ are hydroxymethyl groups. Further, $D^1$ and $D^2$ are hydroxymethyl groups, carboxy groups, methoxycarbonyl groups, ethoxycarbonyl groups, or phenoxycarbonyl groups. The compound (P4), the compound (P5), the compound (P6), the compound (P12), and the compound (P15) can be produced by the production method D or E.

Further, when the compound (M3) is epoxidized and subjected to ring opening using a solvent such as an alcohol, a compound (W2) and a compound (W3) are obtained in which $L^1$ and $L^2$ are direct bonds and hydroxy groups are contained. Preferred examples of an oxidizing agent used for epoxidation include m-chlorobenzoic acid (mCPBA), hydrogen peroxide, and peracetic acid, and examples of the alcohol used for ring opening include methanol, ethanol, and propanol.

[Formula 63]

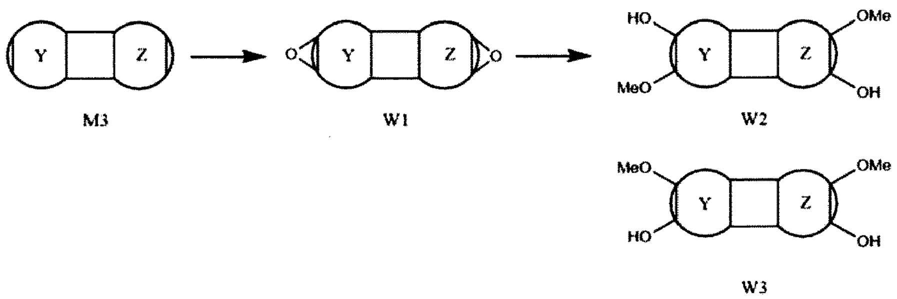

M3 W1 W2 W3

(Z-3)

In the formula (Z-3), one of two epoxy groups of a compound (W1) is on the ring Y, and the other epoxy group is on the ring Z. Further, in the compound (W2), a carbon atom bonded to the hydroxy group and a carbon atom bonded to the methoxy group are carbon atoms adjacent to each other in the same alicyclic carbocyclic skeleton. That is, in the formula (Z-3), the hydroxy group and the methoxy group of the compound (W2) are bonded to any two respective carbon atoms adjacent to each other in the alicyclic carbocyclic skeleton.

The carbonate-based resin is preferably constituted from a copolymeric polycarbonate further containing a structural unit derived from another dihydroxy compound other than the structural unit B1. Specifically, the carbonate-based resin preferably contains, in addition to the structural unit B1, one or two or more structural units derived from another (other) dihydroxy compound(s) other than the compound represented by the formula (P1). In this case, desired characteristics obtained by introduction of the structural unit(s) derived from another (other) dihydroxy compound(s) can be imparted to the carbonate-based resin.

From the viewpoint of improving surface hardness of the carbonate-based resin or reducing photoelastic coefficient of the carbonate-based resin, the content of the structural unit B1 in the carbonate-based resin based on the amount of substance is preferably 1 mol% or more, more preferably 5 mol% or more, even more preferably 15 mol% or more, even more preferably 25 mol% or more, particularly preferably 37.5 mol% or more. The content of the structural unit B1 is preferably 50 mol% or less. From the same viewpoint, the content of the structural unit B1 in the carbonate-based resin based on mass is preferably 1 mass% or more, more preferably 10 mass% or more, even more preferably 40 mass% or more, even more preferably 55 mass% or more, particularly preferably 75 mass% or more. It is noted that the content of the structural unit B1 is the content of the above-described second structural unit. That is, in this specification, the content of a structural unit derived from a dihydroxy compound (e.g., the structural unit B1, a structural unit D4 described later) in the carbonate-based resin is the content of the structural unit containing oxygen atoms derived from hydroxy groups at both ends. Similarly to the oxygen atom of a dihydroxy compound, the content of a structural unit derived from a

dicarboxylic acid compound in the carbonate-based resin is the content of the structural unit containing carbonyl groups derived from a carboxylic acid at both ends.

On the other hand, from the viewpoint of achieving both moist heat resistance of the carbonate-based resin and film strength, the content of the structural unit B1 in the carbonate-based resin based on the amount of substance is preferably 0.1 mol% or more, more preferably 1 mol% or more, even more preferably 2 mol% or more, even more preferably 4 mol% or more, particularly preferably 5 mol% or more. Further, the content of the structural unit B1 is preferably 50 mol% or less, more preferably 37.5 mol% or less, even more preferably 25 mol% or less, even more preferably 20 mol% or less, particularly preferably 15 mol% or less. From the same viewpoint, the content of the structural unit B1 in the carbonate-based resin based on mass is preferably 1 mass% or more, more preferably 2 mass% or more, even more preferably 5 mass% or more, even more preferably 7 mass% or more, particularly preferably 10 mass% or more. Further, the content of the structural unit B1 is preferably 95 mass% or less, more preferably 80 mass% or less, even more preferably 60 mass% or less, even more preferably 50 mass% or less, particularly preferably 40 mass% or less.

From the viewpoint that optical properties such as translucency and characteristics such as weatherability, moldability, and heat resistance can be improved, the carbonate-based resin preferably further contains a structural unit derived from at least one compound selected from the group consisting of isosorbide, isomannide, and isoidide. Hereinafter, the structural unit derived from at least one compound selected from the group consisting of isosorbide, isomannide, and isoidide is referred to as a "structural unit D1" as appropriate. The structural unit D1 is represented by the following formula (D1).

[Formula 64]

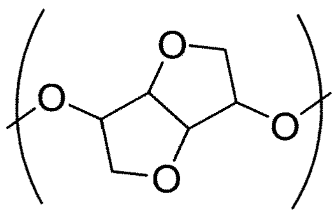

(D1)

When having the structural unit D1, the carbonate-based resin is a copolymeric polycarbonate having at least the structural unit B1 and the structural unit D1. Further, from the viewpoint that isosorbide can be obtained by dehydration condensation of sorbitol produced from various starches that are present in abundance as plant-derived resources and are easily available and the viewpoint of carbon neutral, the carbonate-based resin more preferably has a structural unit derived from isosorbide.

From the viewpoint of improving heat resistance, the content of the structural unit D1 in the carbonate-based resin is preferably 5 mol% or more, more preferably 10 mol% or more, even more preferably 20 mol% or more, even more preferably 30 mol% or more, particularly preferably 40 mol% or more and is preferably 49 mol% or less with respect to the total molar quantity of all the structural units based on all the dihydroxy compounds, all the dicarboxylic acid compounds, and all the carbonates in the carbonate-based resin. From the same viewpoint, the content of the structural unit D1 in the carbonate-based resin is preferably 5 mass% or more, more preferably 20 mass% or more, even more preferably 40 mass% or more, even more preferably 60 mass% or more, particularly preferably 80 mass% or more and is preferably 99 mass% or less with respect to the total mass of the carbonate-based resin based on all the dihydroxy compounds, all the dicarboxylic acid compounds, and all the carbonates in the carbonate-based resin. From the viewpoint of reducing a water absorption rate to achieve a balance with mechanical strength and optical properties, the content of the structural unit D1 in the carbonate-based resin is preferably 5 mol% or more, more preferably 10 mol% or more, even more preferably 15 mol% or more, even more preferably 20 mol% or more, particularly preferably 30 mol% or more, and is preferably 49 mol% or less, more preferably 47 mol% or less, even more preferably 45 mol% or less, even more preferably 42 mol% or less, particularly preferably 40 mol% or less with respect to the total molar quantity of all the structural units based on all the dihydroxy compounds, all the dicarboxylic acid compounds, and all the carbonates in the carbonate-based resin. From the same viewpoint, the content of the structural unit D1 in the carbonate-based resin is preferably 5 mass% or more, more preferably 20 mass% or more, even more preferably 30 mass% or more, even more preferably 37.5 mass% or more, particularly preferably 42.5 mass% or more with respect to the total mass of the carbonate-based resin based on all the dihydroxy compounds, all the dicarboxylic acid compounds, and all the carbonates in the carbonate-based resin. Further, the content of the structural unit D1 in the carbonate-based resin is preferably 95 mass% or less, more preferably 90 mass% or less, even more preferably 80 mass% or less, even more preferably 70 mass% or less, particularly preferably 65 mass% or less.

From the viewpoint that optical properties such as translucency and characteristics such as mechanical strength

and flowability during melting can be improved, the carbonate-based resin preferably contains a structural unit derived from an aliphatic dihydroxy compound other than the structural unit B1 and the structural unit D1. Hereinafter, the structural unit derived from an aliphatic dihydroxy compound other than the structural unit B1 and the structural unit D1 is referred to as a "structural unit D2", and a structural unit derived from an alicyclic dihydroxy compound is referred to as a "structural unit D2'" as appropriate. It is noted that the structural units D2 and D2' are concepts excluding not only the structural unit B1 and the structural unit D1 but also structural units D3 to D7 described later. When having the structural unit D2 and/or the structural unit D2', the carbonate-based resin is a copolymeric polycarbonate having at least the structural unit B1 and the structural unit D2 and/or the structural unit D2'. Examples of the aliphatic dihydroxy compound include a linear aliphatic dihydroxy compound and a branched aliphatic dihydroxy compound, and an alicyclic dihydroxy compound is not included.

Examples of the linear aliphatic dihydroxy compound include ethylene glycol, 1,3-propanediol, 1,2-propanediol, 1,4-butanediol, 1,3-butanediol, 1,2-butanediol, 1,5-pentanediol, 1,6-hexanediol, 1,7-pentanediol, 1,8-octanediol, 1,9-nonanediol, 1,10-decanediol, 1,12-dodecanediol, diethylene glycol, triethylene glycol, tetraethylene glycol, polyethylene glycol, dipropylene glycol, tripropylene glycol, tetrapropylene glycol, polypropylene glycol, polytrimethylene glycol, polytetramethylene glycol, and polydecamethylene glycol.

Examples of the dihydroxy compound of a branched aliphatic hydrocarbon include neopentyl glycol and hexylene glycol.

From the viewpoint of imparting flexibility to the carbonate-based resin to improve toughness of the resin, the dihydroxy compound constituting the structural unit D2 is particularly preferably a linear aliphatic dihydroxy compound having primary hydroxy groups. Examples of the linear aliphatic dihydroxy compound having primary hydroxy groups include ethylene glycol, 1,3-propanediol, 1,4-butanediol, 1,6-hexanediol, 1,10-decanediol, and 1,12-dodecanediol.

From the viewpoint of improving characteristics such as toughness, the content of the structural unit D2 in the carbonate-based resin is preferably 5 mol% or more, more preferably 10 mol% or more, even more preferably 20 mol% or more, even more preferably 30 mol% or more, particularly preferably 40 mol% or more and is preferably 49 mol% or less with respect to the total molar quantity of all the structural units based on all the dihydroxy compounds, all the dicarboxylic acid compounds, and all the carbonates in the carbonate-based resin.

From the same viewpoint, the content of the structural unit D2 in the carbonate-based resin is preferably 5 mass% or more, more preferably 15 mass% or more, even more preferably 30 mass% or more, even more preferably 50 mass% or more, particularly preferably 70 mass% or more with respect to the total mass of the carbonate-based resin based on all the dihydroxy compounds, all the dicarboxylic acid compounds, and all the carbonates in the carbonate-based resin. Further, the content of the structural unit D2 in the carbonate-based resin is preferably 99 mass% or less.

From the viewpoint of achieving both heat resistance and mechanical strength, the content of the structural unit D2 in the carbonate-based resin is preferably 0.1 mol% or more, more preferably 1 mol% or more, even more preferably 2 mol% or more, even more preferably 4 mol% or more, particularly preferably 15 mol% or more with respect to the total molar quantity of all the structural units based on all the dihydroxy compounds, all the dicarboxylic acid compounds, and all the carbonates in the carbonate-based resin. Further, the content of the structural unit D2 in the carbonate-based resin is preferably 49 mol% or less, more preferably 40 mol% or less, even more preferably 30 mol% or less, even more preferably 20 mol% or less, particularly preferably 15 mol% or less.

From the same viewpoint, the content of the structural unit D2 in the carbonate-based resin is preferably 0.1 mass% or more, more preferably 1 mass% or more, even more preferably 2 mass% or more, even more preferably 4 mass% or more, particularly preferably 5 mass% or more and is preferably 99 mass% or less, more preferably 80 mass% or less, even more preferably 60 mass% or less, even more preferably 40 mass% or less, particularly preferably 30 mass% or less with respect to the total mass of the carbonate-based resin based on all the dihydroxy compounds, all the dicarboxylic acid compounds, and all the carbonates in the carbonate-based resin.

Examples of the alicyclic dihydroxy compound include 1,2-cyclohexanediol, 1,2-cyclohexanedimethanol, 1,3-cyclohexanedimethanol, 1,4-cyclohexanedimethanol, tricyclodecanedimethanol, pentacyclopentadecanedimethanol, 2,6-decalindimethanol, 1,5-decalindimethanol, 2,3-decalindimethanol, 2,3-norbornanedimethanol, 2,5-norbornanedimethanol, 1,3-adamantanedimethanol , and a dihydroxy compound derived from a terpene compound such as limonene.

From the viewpoint of imparting flexibility to the carbonate-based resin to improve toughness of the resin, the dihydroxy compound constituting the structural unit D2' is preferably 1,4-cyclohexanedimethanol, tricyclodecanedimethanol, or the like.

From the viewpoint of improving characteristics such as toughness, the content of the structural unit D2' in the carbonate-based resin is preferably 5 mol% or more, more preferably 10 mol% or more, even more preferably 20 mol% or more, even more preferably 30 mol% or more, particularly preferably 40 mol% or more and is preferably 49 mol% or less with respect to the total molar quantity of all the structural units based on all the dihydroxy compounds, all the dicarboxylic acid compounds, and all the carbonates in the carbonate-based resin.

From the same viewpoint, the content of the structural unit D2' in the carbonate-based resin is preferably 5 mass% or more, more preferably 15 mass% or more, even more preferably 30 mass% or more, even more preferably 50 mass% or more, particularly preferably 70 mass% or more and is preferably 99 mass% or less with respect to the total mass

of the carbonate-based resin based on all the dihydroxy compounds, all the dicarboxylic acid compounds, and all the carbonates in the carbonate-based resin.

From the viewpoint of achieving both heat resistance and mechanical strength, the content of the structural unit D2' in the carbonate-based resin is preferably 0.1 mol% or more, more preferably 1 mol% or more, even more preferably 2 mol% or more, even more preferably 4 mol% or more, particularly preferably 5 mol% or more and is preferably 49 mol% or less, more preferably 40 mol% or less, even more preferably 30 mol% or less, even more preferably 20 mol% or less, particularly preferably 15 mol% or less with respect to the total molar quantity of all the structural units based on all the dihydroxy compounds, all the dicarboxylic acid compounds, and all the carbonates in the carbonate-based resin. From the same viewpoint, the content of the structural unit D2' in the carbonate-based resin is preferably 0.1 mass% or more, more preferably 1 mass% or more, even more preferably 2 mass% or more, even more preferably 4 mass% or more, particularly preferably 5 mass% or more and is preferably 99 mass% or less, more preferably 80 mass% or less, even more preferably 60 mass% or less, even more preferably 40 mass% or less, particularly preferably 30 mass% or less with respect to the total mass of the carbonate-based resin based on all the dihydroxy compounds, all the dicarboxylic acid compounds, and all the carbonates in the carbonate-based resin.

The carbonate-based resin may further contain a structural unit derived from a dihydroxy compound represented by the following formula (D3). In this case, the glass transition temperature of the carbonate-based resin can be controlled to fall within a preferred range, which may make it easy to subject the resin to melt molding and film formation. Further, in this case, an optical property (specifically, low photoelastic coefficient) may be imparted to the resin. The carbonate-based resin may contain one or two or more structural units represented by the formula (D3). The structural unit represented by the formula (D3) is referred to as a "structural unit D3" as appropriate.

[Formula 65]

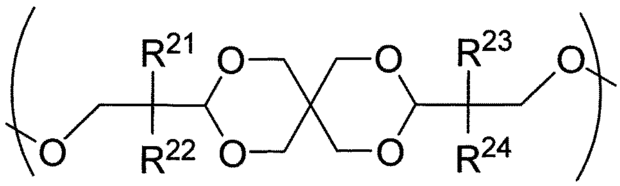

(D3)

In the formula (D3), $R^{21}$ to $R^{24}$ are each independently a hydrogen atom, a substituted or unsubstituted alkyl group having 1 to 20 carbon atoms, a substituted or unsubstituted cycloalkyl group having 5 to 20 carbon atoms, or a substituted or unsubstituted aryl group having 6 to 20 carbon atoms.

An example of the dihydroxy compound represented by the formula (D3) includes a dihydroxy compound described in paragraph [0024] in WO2017/159525.

From the viewpoint of reducing photoelastic coefficient and obtaining a carbonate-based resin having high surface hardness, the content of the structural unit D3 in the carbonate-based resin is preferably 5 mol% or more, more preferably 15 mol% or more, even more preferably 22.5 mol% or more, even more preferably 32.5 mol% or more, particularly preferably 40 mol% or more and is preferably 49 mol% or less with respect to the total molar quantity of all the structural units based on all the dihydroxy compounds, all the dicarboxylic acid compounds, and all the carbonates in the carbonate-based resin. From the same viewpoint, the content of the structural unit D3 in the carbonate-based resin is preferably 10 mass% or more, more preferably 30 mass% or more, even more preferably 45 mass% or more, even more preferably 65 mass% or more, particularly preferably 80 mass% or more and is preferably 99 mass% or less with respect to the total mass of the carbonate-based resin based on all the dihydroxy compounds, all the dicarboxylic acid compounds, and all the carbonates in the carbonate-based resin.

On the other hand, from the viewpoint of adjusting glass transition temperature and mechanical strength to fall within their respective preferred ranges to improve melt-moldability and film formability of the carbonate-based resin, the content of the structural unit A5 is preferably 5 mol% or more, more preferably 10 mol% or more, even more preferably 15 mol% or more, even more preferably 17.5 mol% or more, particularly preferably 20 mol% or more and is preferably 45 mol% or less, more preferably 40 mol% or less, even more preferably 35 mol% or less, particularly preferably 30 mol% or less, most preferably 27.5 mol% or less with respect to the total molar quantity of all the structural units based on all the dihydroxy compounds, all the dicarboxylic acid compounds, and all the carbonates in the carbonate-based resin. From the same viewpoint, the content of the structural unit A5 is preferably 10 mass% or more, more preferably 20 mass% or more, even more preferably 30 mass% or more, even more preferably 35 mass% or more, particularly preferably 40 mass% or more with respect to the total mass of the carbonate-based resin based on all the dihydroxy compounds, all the dicarboxylic acid compounds, and all the carbonates in the carbonate-based resin. Further, the content of the structural unit A5 is preferably 90 mass% or less, more preferably 80 mass% or less, even more preferably 70 mass% or less, even more preferably 60 mass% or less, particularly preferably 55 mass% or less.

The carbonate-based resin may further contain a structural unit represented by the following formula (D4) and/or a structural unit represented by the following formula (D5). In this case, the carbonate-based resin can have a reverse wavelength dispersion property, and is therefore suitable for optical films such as retardation films. The structural unit represented by the formula (D4) is referred to as a "structural unit D4" as appropriate, and the structural unit represented by the formula (D5) is referred to as a "structural unit D5" as appropriate. When having the structural unit D5, the carbonate-based resin is a carbonate-based resin having a carbonate bond and an ester bond, that is, a polyester carbonate.

[Formula 66]

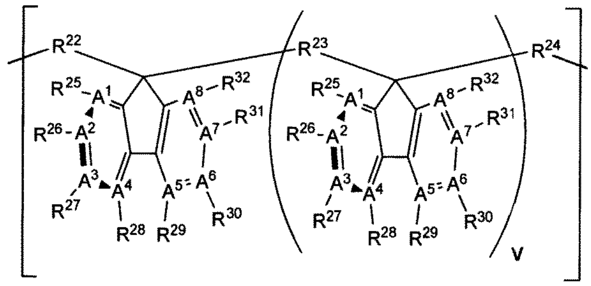

(D4)

[Formula 67]

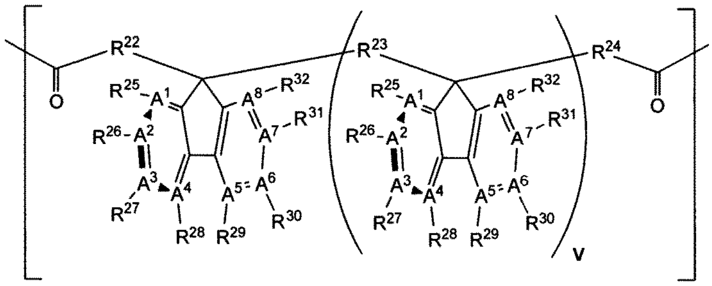

(D5)

The structural unit represented by the formula (D4) is referred to as a "first structural unit D4" as appropriate. Similarly to the structural unit (B1) described above, when having the structural unit represented by the formula (D4), the carbonate-based resin can also be regarded to have the structural unit in which -O- is bonded to each of $R^{22}$ and $R^{24}$. Such a structural unit is referred to as a "second structural unit D4" as appropriate.

In the formula (D4) and the formula (D5), $A^1$ to $A^8$ are each independently =CH- or =N-. $R^{22}$, $R^{23}$, and $R^{24}$ are each independently a direct bond or a group formed by linking two or more groups selected from the group consisting of an optionally-substituted alkylene group having 1 to 10 carbon atoms, an optionally-substituted arylene group having 4 to 10 carbon atoms, an optionally-substituted aralkylene group having 6 to 12 carbon atoms, an optionally-substituted alkylene group having 1 to 10 carbon atoms, and an optionally-substituted arylene group having 4 to 10 carbon atoms by an oxygen atom, an optionally-substituted nitrogen atom, or a carbonyl group.

In the formula (D4) and the formula (D5), $R^{25}$ to $R^{32}$ are each independently a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom, a nitro group, or a cyano group. At least two adjacent groups out of $R^{25}$ to $R^{32}$ may be bonded to each other to form a ring. v is an integer of 0 to 5.

If the content of the structural unit (D4) and the structural unit (D5) in the carbonate-based resin is excessively high, the photoelastic coefficient and reliability may deteriorate and a film formed from the carbonate-based resin cannot have high birefringence even when stretched. Further, if the ratio of the oligofluorene structural unit occupying the carbonate-based resin is excessively high, the degree of freedom in molecular design reduces so that it is difficult to modify the resin if necessary. On the other hand, even if a desired reverse wavelength dispersion property is obtained by a very small amount of the oligofluorene structural unit, in this case, the optical property sensitively changes when the content of an oligofluorene even slightly varies. Therefore, it is difficult to produce a carbonate-based resin whose various

characteristics fall within certain ranges.

In regard to $R^{22}$, $R^{23}$, and $R^{24}$ in the formula (D4) and the formula (D5), specific examples of the "arylene group having 4 to 10 carbon atoms" in the "optionally-substituted arylene group having 4 to 10 carbon atoms" include, but are not limited to: a phenylene group such as a 1,2-phenylene group, a 1,3-phenylene group, or a 1,4-phenylene group; a naphthylene group such as a 1,5-naphthylene group or a 2,6-naphthylene group; and a heteroarylene group such as a 2,5-pyridylene group or a 2,4-furylene group.

In regard to $R^{22}$, $R^{23}$, and $R^{24}$ in the formula (D4) and the formula (D5), an example of the "aralkylene group having 6 to 10 carbon atoms" in the "optionally-substituted aralkylene group having 6 to 12 carbon atoms" includes a group formed from an aromatic ring structure and two linear or branched alkylene groups bonded thereto at any two respective positions. The aromatic ring structure may be a hydrocarbon ring structure such as a benzene ring or a naphthalene ring or a heterocyclic structure such as a furan ring or a pyridine ring. Specific examples of the aralkylene group having 6 to 10 carbon atoms include, but are not limited to, those shown in the following group [G].

[Formula 68]

[G]

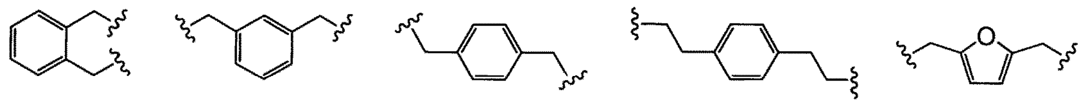

From the viewpoint that synthesis can easily be performed and the viewpoint that raw materials are inexpensively available, the structural unit D4 and the structural unit D5 are preferably represented by the following formula (D4-1) and formula (D5-1), respectively.

[Formula 69]

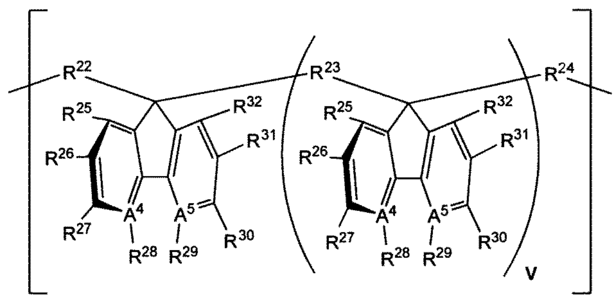

(D4-1)

[Formula 70]

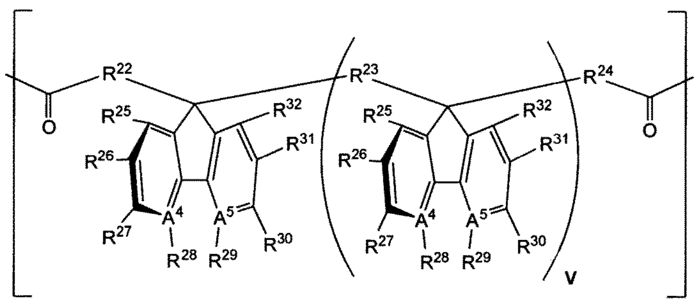

(D5-1)

In the formula (D4-1) and the formula (D5-1), $A^4$ and $A^5$ are each independently =CH- or =N-. $R^{22}$, $R^{23}$, and $R^{24}$ in the formula (D4-1) and the formula (D5-1) are respectively the same as $R^{22}$, $R^{23}$, and $R^{24}$ in the formula (D4) and the formula (D5). $R^{25}$ to $R^{32}$ in the formula (D4-1) and the formula (D5-1) are respectively the same as $R^{25}$ to $R^{32}$ in the formula (D4) and the formula (D5). v is an integer of 0 to 2.

$R^{22}$, $R^{23}$, and $R^{24}$ in the formula (D4-1) and the formula (D5-1) are preferably each independently an optionally-substituted alkylene group having 1 to 10 carbon atoms, more preferably an alkylene group having 1 to 3 carbon atoms.

From the viewpoint that synthesis can easily be performed and the viewpoint that raw materials are inexpensively available, the structural unit D4 and the structural unit D5 are preferably represented by the following formula (D4-2) and formula (D5-2), respectively.

[Formula 71]

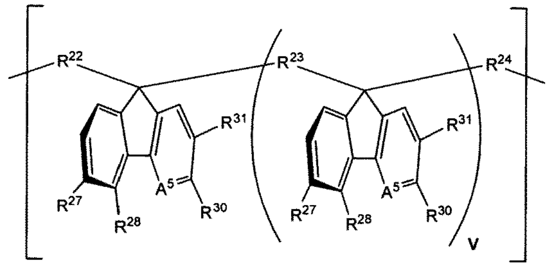

(D4-2)

[Formula 72]

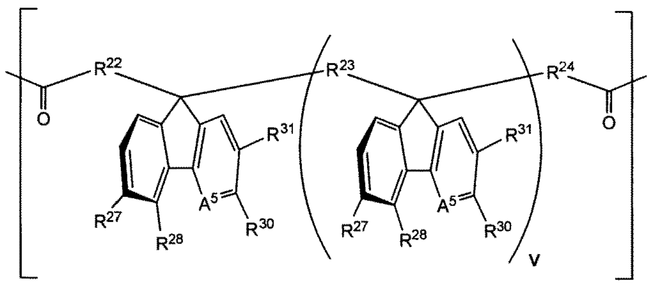

(D5-2)

In the formula (D4-2) and the formula (D5-2), $A^5$ is =CH- or =N-. $R^{22}$, $R^{23}$, and $R^{24}$ in the formula (D4-2) and the formula (D5-2) are respectively the same as $R^{22}$, $R^{23}$, and $R^{24}$ in the formula (D4) and the formula (D5). $R^{25}$ to $R^{32}$ in the formula (D4-2) and the formula (D5-2) are respectively the same as $R^{25}$ to $R^{32}$ in the formula (D4) and the formula (D5). v is an integer of 0 to 2.

From the viewpoint that synthesis can easily be performed and the viewpoint that raw materials are inexpensively available, the structural unit D4 and the structural unit D5 are preferably represented by the following formula (D4-3) and formula (D5-3), respectively.

[Formula 73]

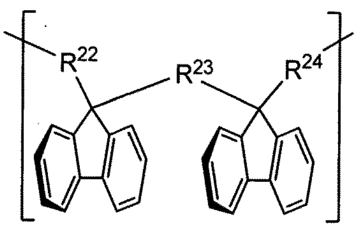

(D4-3)

[Formula 74]

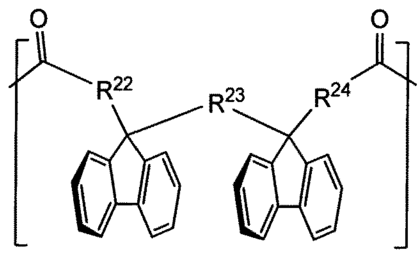

(D5-3)

$R^{22}$, $R^{23}$, and $R^{24}$ in the formula (D4-3) and the formula (D5-3) are each independently a direct bond, a methylene group, or an ethylene group.

From the viewpoint that synthesis can easily be performed and the viewpoint that a reverse wavelength dispersion property improves, the structural unit D4 and the structural unit D5 are more preferably represented by the following formula (D4-4) and formula (D5-4), respectively.

[Formula 75]

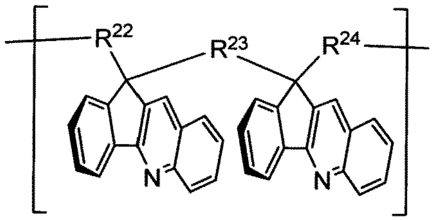

(D4-4)

[Formula 76]

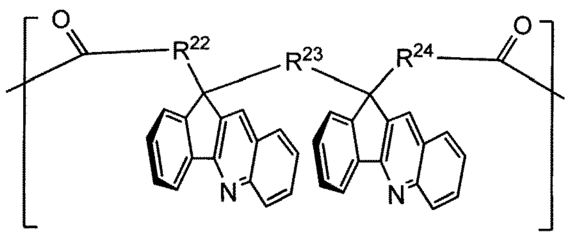

(D5-4)

$R^{22}$, $R^{23}$, and $R^{24}$ in the formula (D4-4) and the formula (D5-4) are each independently a direct bond, a methylene group, or an ethylene group.

From the viewpoint that a reverse wavelength dispersion property improves, the structural unit D4 and the structural unit D5 are even more preferably represented by the following formula (D4-5) and formula (D5-5), respectively.

[Formula 77]

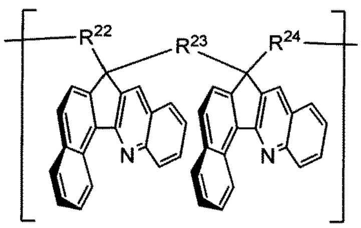

(D4-5)

[Formula 78]

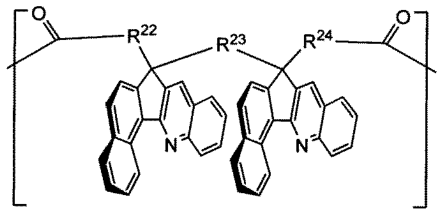

(D5-5)

$R^{22}$, $R^{23}$, and $R^{24}$ in the formula (D4-5) and the formula (D5-5) are each independently a direct bond, a methylene group, or an ethylene group.

From the viewpoint that raw materials are inexpensively available, specifically, the structural unit D4 or the structural unit D5 is preferably at least one selected from the group consisting of structures shown below.

[Formula 79]

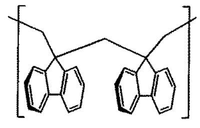 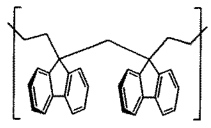 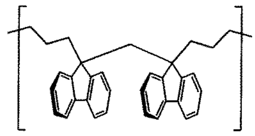

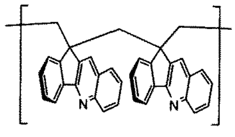 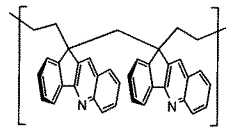 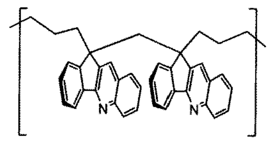

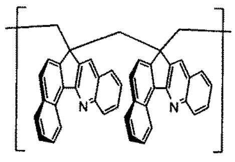 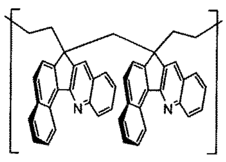 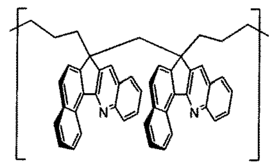

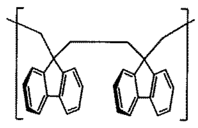 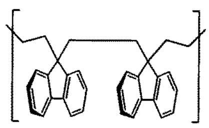 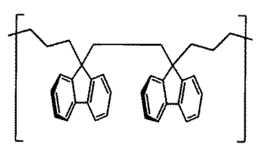

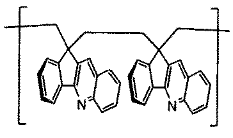 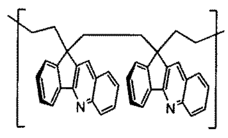 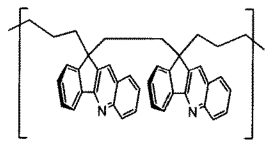

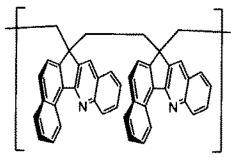 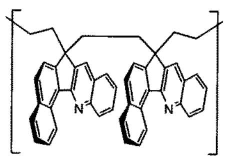 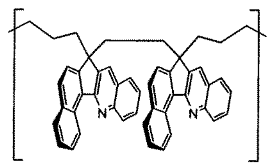

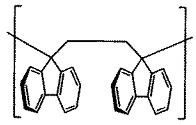 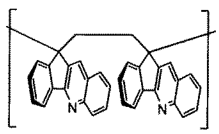 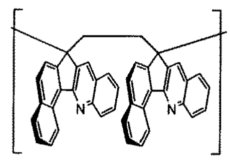

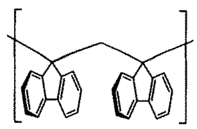 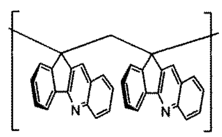 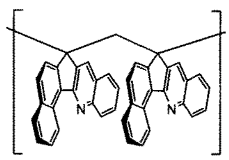

[Formula 80]

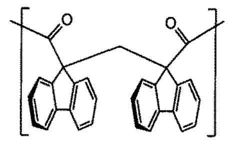
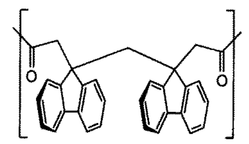
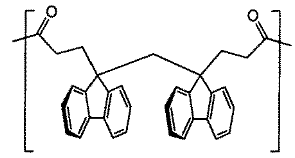
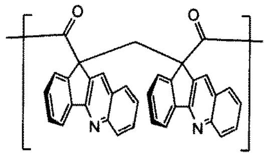
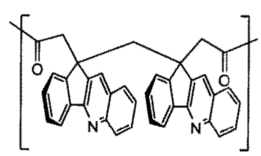
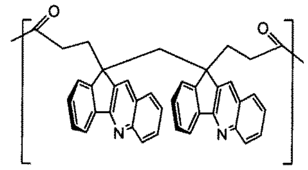
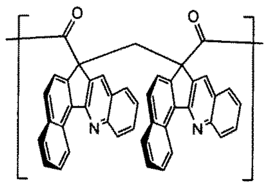
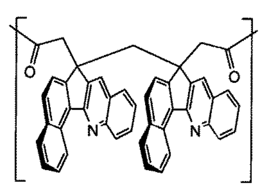
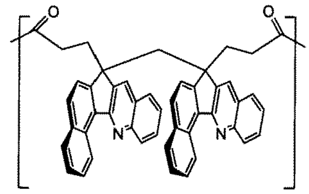
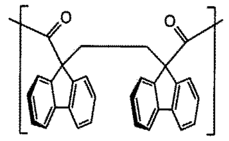
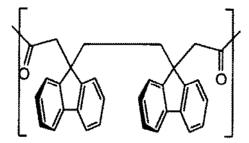
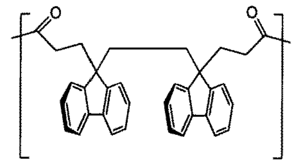
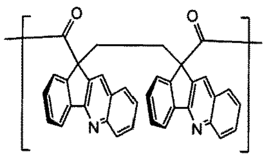
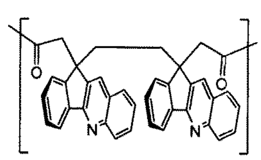
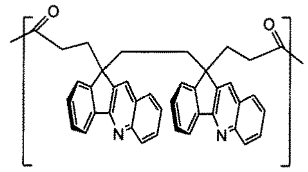
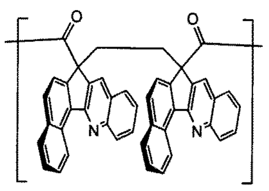
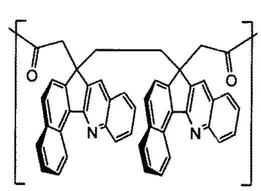
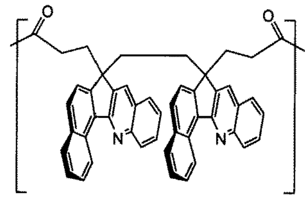

Further, structures shown below have a rigid skeleton due to dense introduction of aromatic rings and therefore exhibit low photoelastic coefficient. The carbonate-based resin containing a structural unit having low photoelastic coefficient is preferred from the viewpoint of moldability and reliability because a change in phase difference due to stress is small. That is, from the viewpoint of moldability and reliability on a phase difference change, specifically, the structural unit D4 or the structural D5 is preferably at least one selected from the group consisting of structures shown below.

[Formula 81]

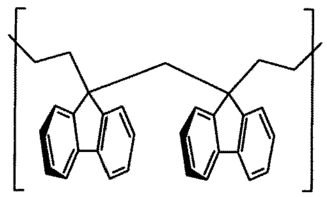

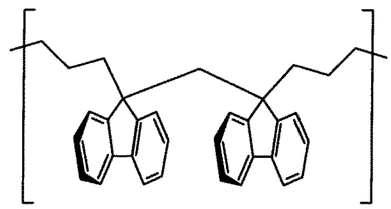

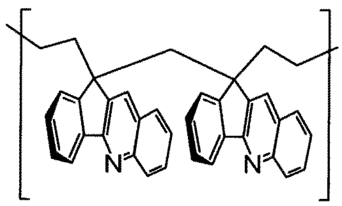

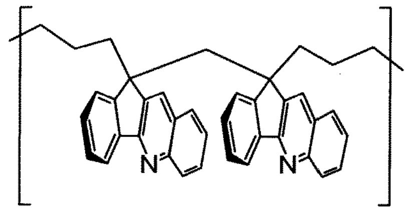

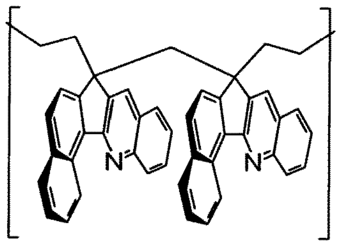

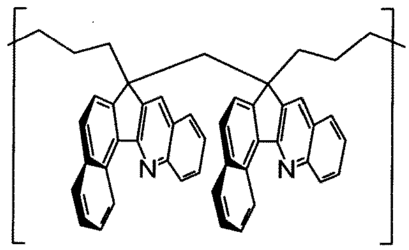

[Formula 82]

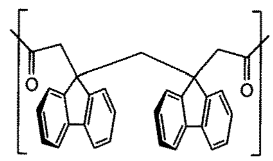

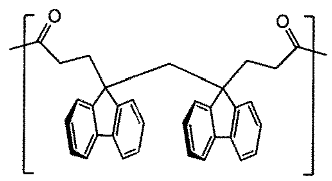

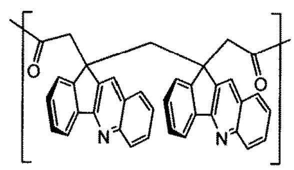

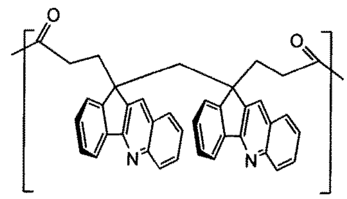

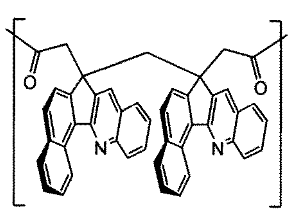

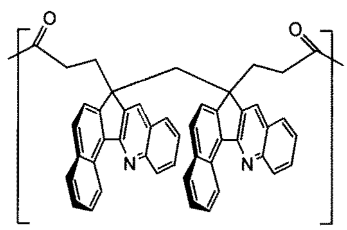

From the viewpoint that inexpensive synthesis and low water absorption are both achieved and wavelength dispersion can be adjusted to be more preferred, the structural unit D5 is particularly preferably a structure represented by the following formula (D5-5).

[Formula 83]

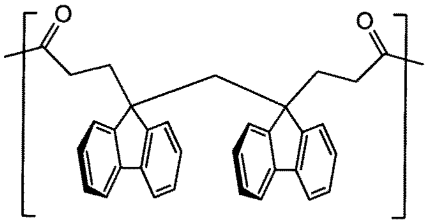

(D5-5)

The carbonate-based resin having the structural unit D4 and/or the structural unit D5 can be produced by, for example, a method such as polymerization of a monomer represented by the following formula (j).

[Formula 84]

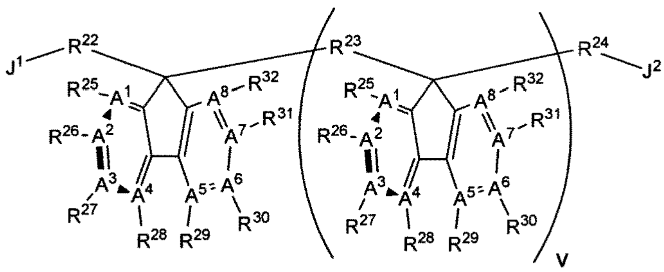

(j)

In the formula (j), $A^1$ to $A^8$, $R^{22}$ to $R^{32}$, v are respectively the same as $A^1$ to $A^8$, $R^{22}$ to $R^{32}$, and v in the formula (D4). $J^1$ and $J^2$ are each independently a polymerization reactive group. Examples of $J^1$ and $J^2$ include a hydroxy group and a hydroxy group-containing group such as a hydroxyalkyl group. $J^1$ and $J^2$ may be the same or different from each other. $J^1$ and $J^2$ are preferably the same because the monomer represented by the formula (j) tends to be produced in a short process.

The monomer represented by the formula (j) can be used as a raw material of a polymer having a divalent oligofluorene as a repeating unit. The polymerization reactive group is preferably present only at two positions, $J^1$ and $J^2$, and it is preferred that $R^{25}$ to $R^{32}$ do not have a substituent that functions as a polymerization reactive group under polymerization conditions for a polycarbonate.

$J^1$ and $J^2$ in the formula (j) are preferably hydroxy groups. The monomer in which $J^1$ and $J^2$ are hydroxy groups can be used for production of a polycarbonate-based resin having excellent optical performance. The monomer in which $J^1$ and $J^2$ are hydroxy groups is represented by the following formula (j1).

[Formula 85]

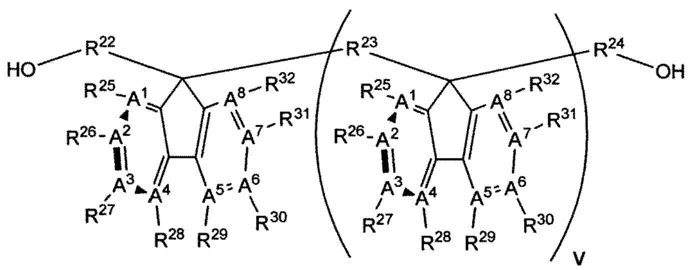

(j1)

Further, $J^1$ and $J^2$ in the formula (j) are preferably ester groups. The monomer in which $J^1$ and $J^2$ are ester groups can be used for production of a polyester carbonate having excellent optical performance. From the viewpoint of easy introduction using industrially-available methyl acrylate, ethyl acrylate, or methyl methacrylate, the ester group is preferably a 2-(methoxycarbonyl)ethyl group, a 2-(ethoxycarbonyl)ethyl group, or a 2-(methoxycarbonyl)propyl group.

From the viewpoint that a transesterification reaction easily proceeds due to an improvement in the activity of ester groups and therefore a polyester carbonate can be synthesized in one step by reacting a diester compound, a dihydroxy compound, and a diester carbonate under the same conditions, the ester group is preferably a phenoxycarbonylalkyl group. Particularly, a 2-(phenoxycarbonyl)methyl group, a 2-(phenoxycarbonyl)ethyl group, and a 2-(phenoxycarbonyl)propyl group are particularly preferred because these groups can be introduced by a method using phenyl 2-bromoacetate, phenyl acrylate, and phenyl methacrylate or a method based on transesterification from a 2-bromoacetic acid ester, a 2-chloroacetic acid ester, a 2-iodoacetic acid ester, an acrylic acid ester, and a methacrylic acid ester. The monomer in which $J^1$ and $J^2$ are ester groups is represented by, for example, the following formula (j2).

[Formula 86]

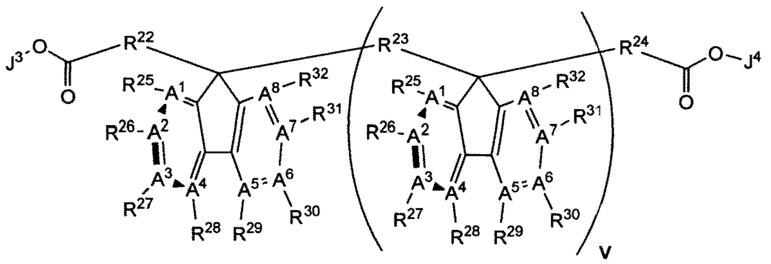

(j2)

In the formula (j2), $J^3$ and $J^4$ are each independently an organic substituent having 1 to 10 carbon atoms or a halogen atom.

Specific examples of the organic substituent having 1 to 10 carbon atoms represented by $J^3$ or $J^4$ include, but are not limited to: linear alkyloxy groups such as a methoxy group, an ethoxy group, a n-propoxy group, a n-butoxy group, a n-pentyloxy group, a n-hexyloxy group, and a n-decyloxy group; alkyloxy groups having a branched chain, such as an isopropyloxy group, a 2-methylpropyloxy group, a 2,2-dimethylpropyloxy group, and a 2-ethylhexyloxy group; cyclic alkyloxy groups such as a cyclopropyloxy group, a cyclopentyloxy group, a cyclohexyloxy group, and a cyclooctyloxy group; aryloxy groups such as a phenoxy group, a 1-naphthyloxy group, and a 2-naphthyloxy group; heteroaryl groups containing a 1-imidazoyl group; heteroaryloxy groups such as a 2-pyridyloxy group and a 2-furyloxy group; and aralkyloxy groups such as a benzyloxy group, a 2-phenylethoxy group, and a p-methoxybenzyloxy group. Specific examples of the halogen atom include, but are not limited to, a chlorine atom and a bromine atom.

From the viewpoint that a polyester carbonate can efficiently be synthesized by removing a low-boiling alcohol generated by transesterification with a dihydroxy compound, $J^3$ and $J^4$ are preferably methyl groups or ethyl groups. From the viewpoint that a transesterification reaction easily proceeds and therefore a polyester carbonate as a preferred polymer can be synthesized in one step by adding a diester compound, a dihydroxy compound, and a diester carbonate to a reactor at the same time, $J^3$ and $J^4$ are preferably aryl groups. Particularly, $J^3$ and $J^4$ are particularly preferably phenyl groups because a phenyl group has a low molecular weight and can be distilled away as phenol after synthesis of a polyester carbonate. It is noted that when the compound having aryl groups as $J^3$ and $J^4$ is used in a method for producing a polycarbonate resin described later, a diaryl carbonate described later is preferably used as a diester carbonate from the viewpoint of reactivity during polymerization, and it is more preferred that the aryl groups as $J^3$ and $J^4$ and aryl groups in the diaryl carbonate are the same from the viewpoint that a by-product can easily be removed. From the viewpoint that polymerization reactivity is excellent and a carbonate-based resin can be obtained by polymerization using a relatively simple equipment, such as solution polymerization or interfacial polymerization, $J^3$ and $J^4$ are preferably acid chloride-containing groups, and acid chloride or acid bromide is more preferred because it can industrially inexpensively be produced.

Specific examples of the monomer represented by the formula (j) include compounds shown below.

[Formula 87]

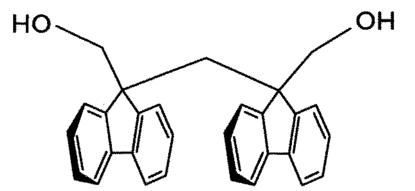
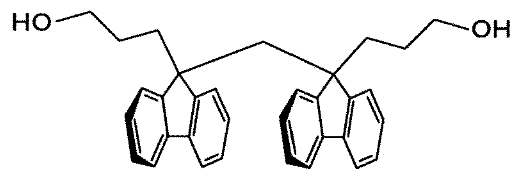
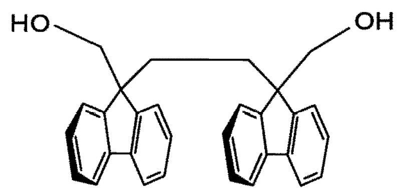
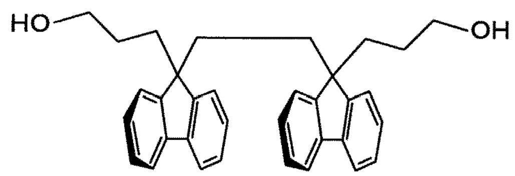
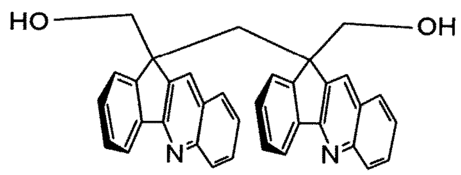
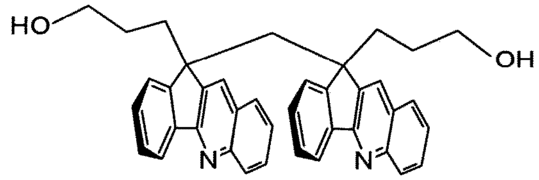
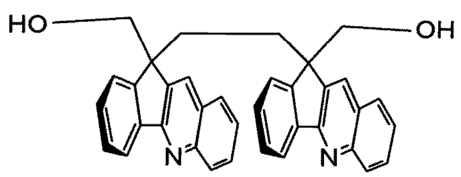
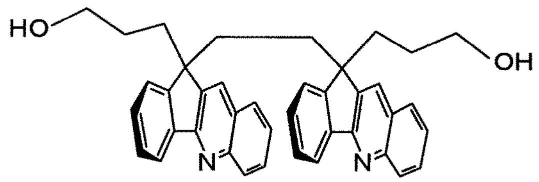
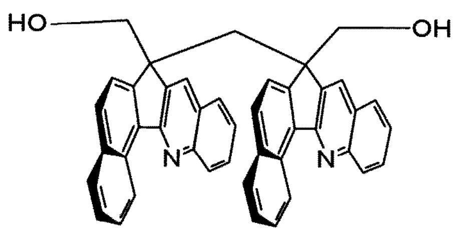
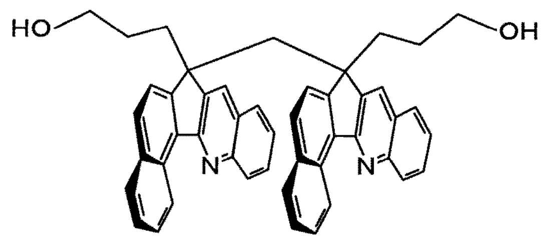
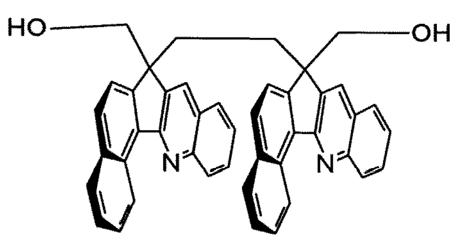
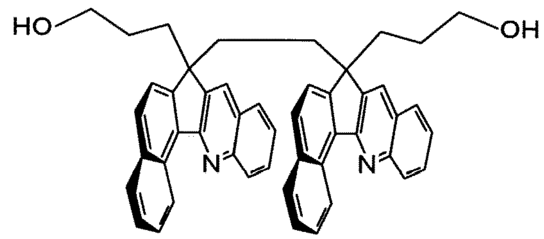

[Formula 88]

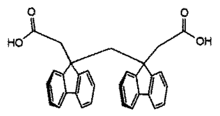
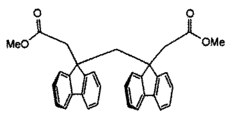
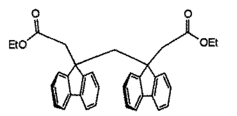
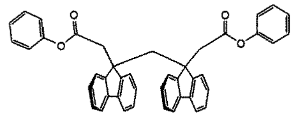

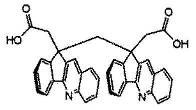
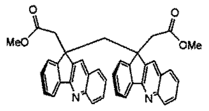
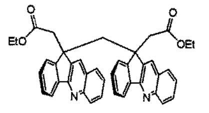
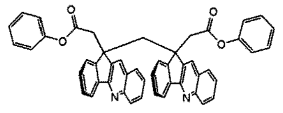

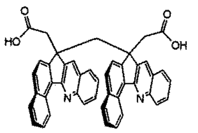
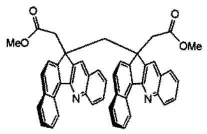
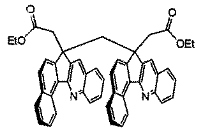
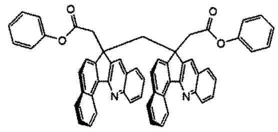

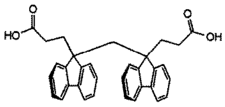
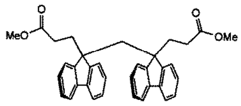
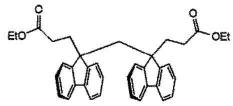
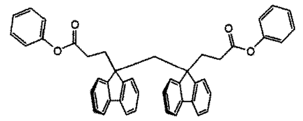

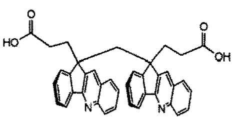
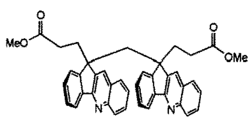
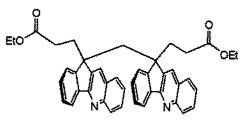
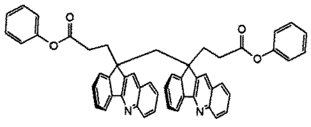

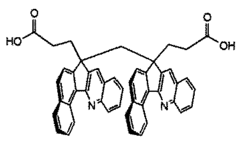
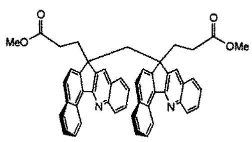
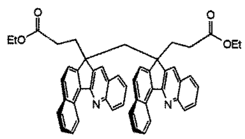
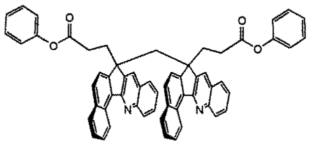

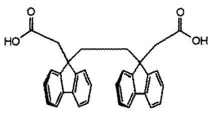 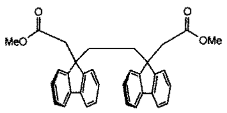 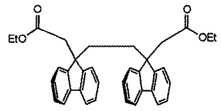 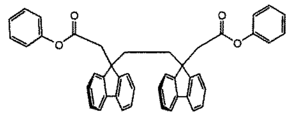

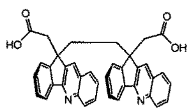 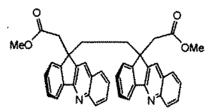 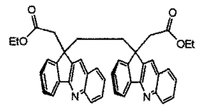 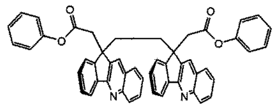

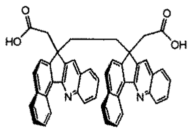 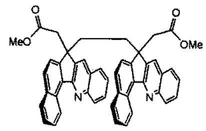 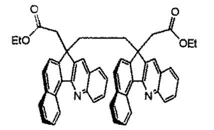 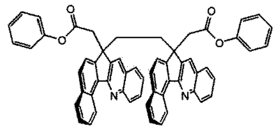

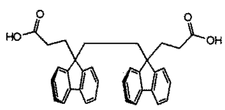 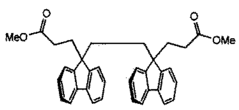 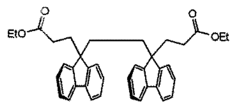 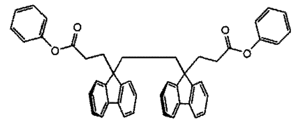

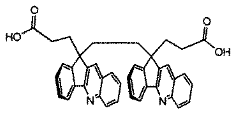 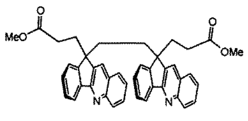 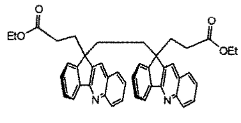 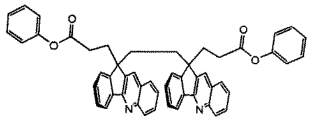

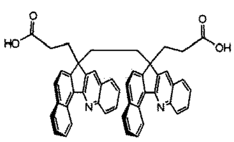 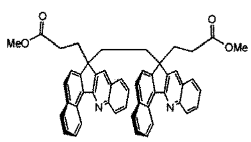 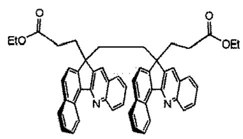 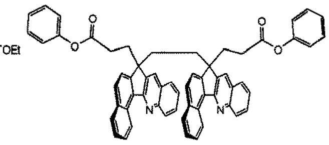

[Formula 89]

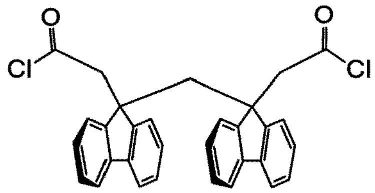
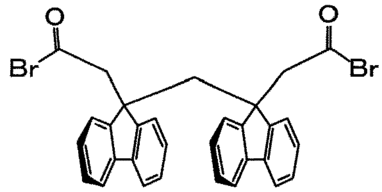

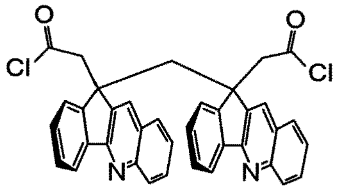
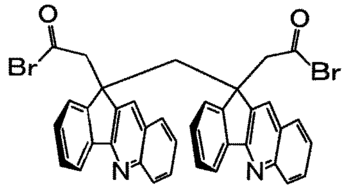

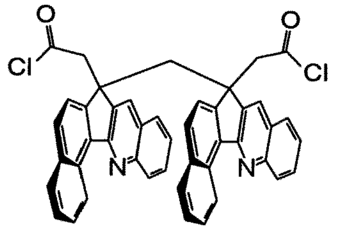
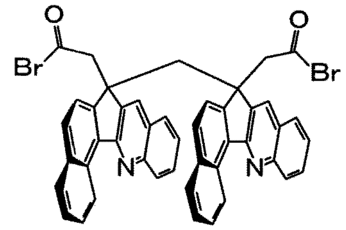

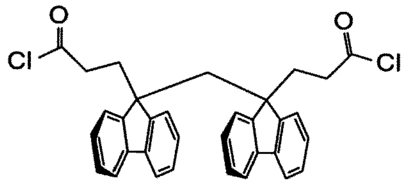
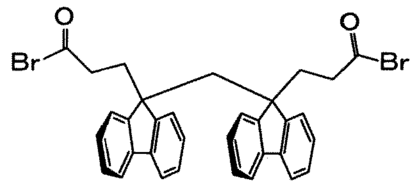

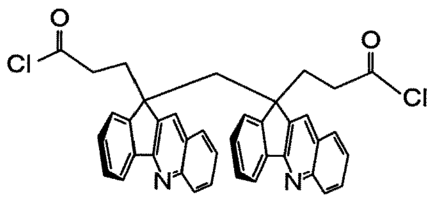
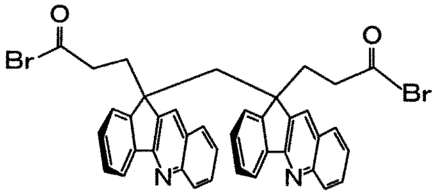

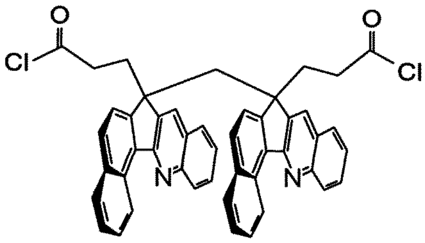
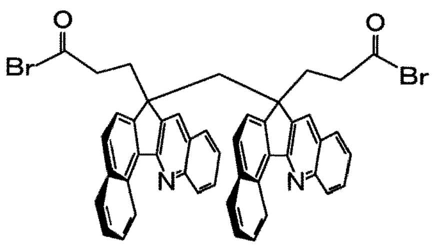

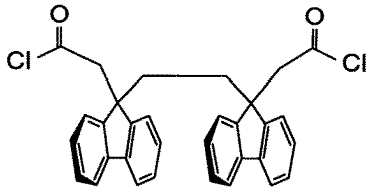
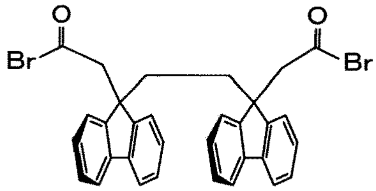

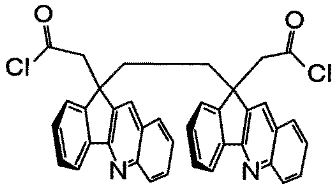
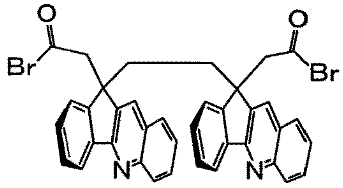

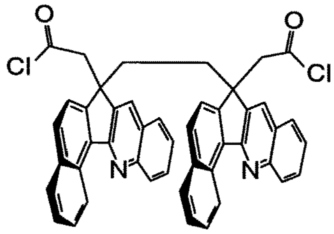
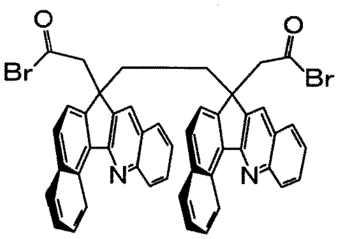

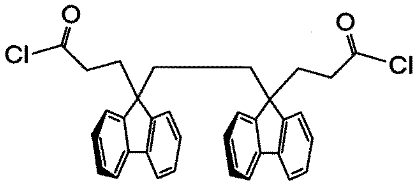
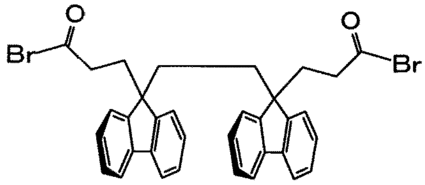

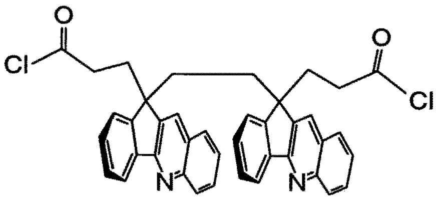
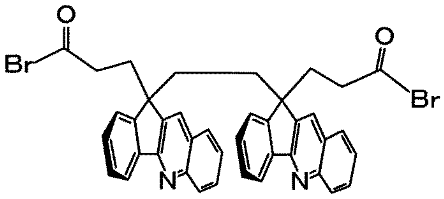

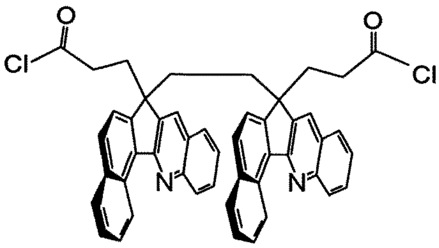
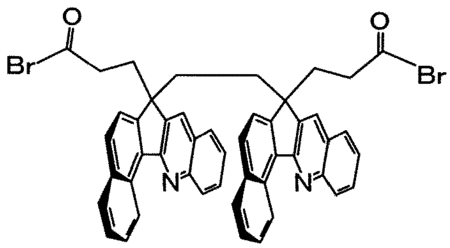

When the carbonate-based resin contains the structural unit D4 (specifically, the second structural unit D4) and/or the structural unit D5, from the viewpoint of improving optical properties, the viewpoint of allowing an optical film formed from the carbonate-based resin to have a preferred wavelength dispersion property, and the viewpoint of adjusting a birefringence to fall within a preferred range, the content of the structural unit D4 and the content of the structural unit D5 in the carbonate-based resin in terms of the amount of substance are each preferably 0.1 mol% or more, more preferably 1 mol% or more, even more preferably 3 mol% or more, even more preferably 4 mol% or more, particularly preferably 5 mol% or more and are each preferably 45 mol% or less, more preferably 35 mol% or less, even more preferably 25 mol% or less, even more preferably 17.5 mol% or less, particularly preferably 12.5 mol% or less. From the same viewpoint, the content of the structural unit D4 and the content of the structural unit D5 in the carbonate-based resin in terms of mass are each

preferably 2 mass% or more, more preferably 5 mass% or more, even more preferably 7.5 mass% or more, even more preferably 10 mass% or more, particularly preferably 15 mass% or more and are each preferably 90 mass% or less, more preferably 70 mass% or less, even more preferably 50 mass% or less, even more preferably 35 mass% or less, particularly preferably 25 mass% or less.

The carbonate-based resin may further contain a structural unit represented by the following formula (D6). In this case, the carbonate-based resin exhibits excellent optical properties. The carbonate-based resin may contain one or two or more structural units represented by the formula (D6). The structural unit represented by the formula (D6) is referred to as a "structural unit D6" as appropriate.

[Formula 90]

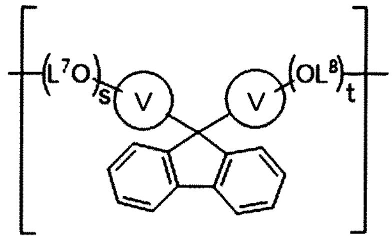

(D6)

In the formula (D6), V is an optionally-substituted arylene group, a substituent of V is a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom, a nitro group, or a cyano group, L7 and L8 are each independently an optionally-substituted alkylene group having 1 to 10 carbon atoms, an optionally-substituted arylene group having 4 to 10 carbon atoms, or an optionally-substituted aralkylene group having 6 to 12 carbon atoms, s is an integer of 0 to 4, and t is an integer of 0 to 4.

In the formula (D6), s and t are each independently an integer of 0 to 4, but from the viewpoint of heat resistance, s and t are preferably each independently an integer of 0 to 3, more preferably an integer of 0 to 2. From the viewpoint of adjusting glass transition temperature to achieve excellent molding processability and the viewpoint that a monomer material is inexpensively synthesized, s and t are particularly preferably each independently 0 or 1.

Further, from the viewpoint of ease of synthesis of a monomer material, the structural unit D6 is preferably represented by the following formula (D6-1).

[Formula 91]

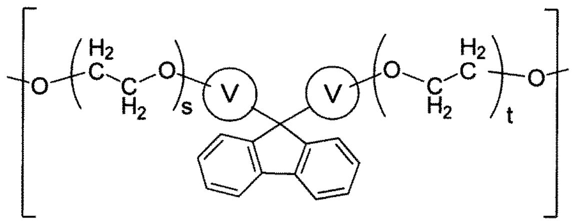

(D6-1)

In the formula (D6-1), V is an optionally-substituted phenylene or naphthylene group, a substituent of V is a hydrogen atom, an alkyl group having 1 to 4 carbon atoms, or an optionally-substituted aryl group having 6 to 20 carbon atoms, s is 0 or 1, and t is 0 or 1.

From the viewpoint of ease of synthesis of a monomer material and the viewpoint of adjustment to a preferred optical property (specifically, a preferred wavelength dispersion property), the structural unit D6 is preferably represented by the following formula (D6-2).

[Formula 92]

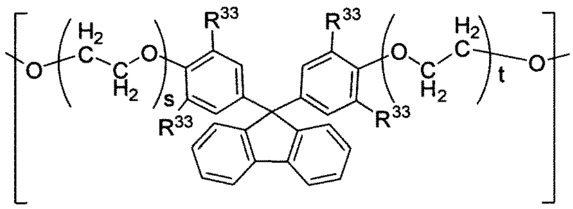

(D6-2)

In the formula (D6-2), $R^{33}$ is a hydrogen atom, a methyl group, or a phenyl group, s is 0 or 1, and t is 0 or 1.

Specific examples of the structural unit D6 include structural units shown below.

[Formula 93]

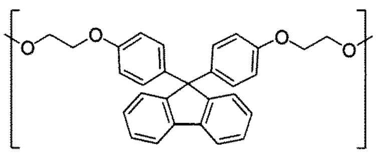

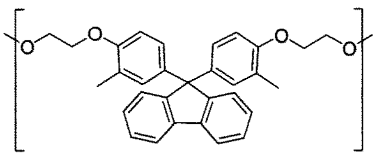

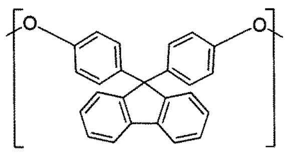

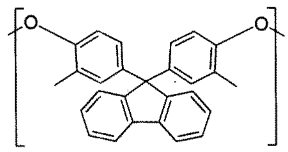

The carbonate-based resin having the structural unit D6 can be produced by, for example, a method such as polymerization of a monomer represented by the following formula (i).

[Formula 94]

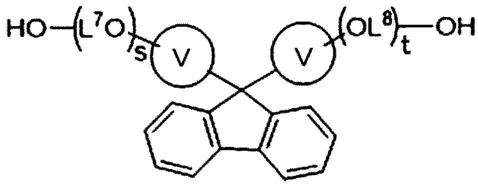

(i)

In the formula (i), V, $L^7$, $L^8$, s, and t are respectively the same as V, $L^7$, $L^8$, s, and t in the formula (D6). Specific examples of the monomer represented by the formula (i) include monomers shown below.

[Formula 95]

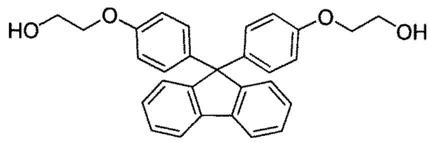 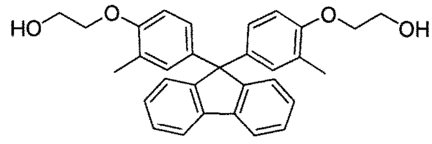

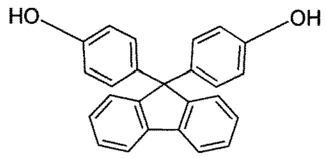 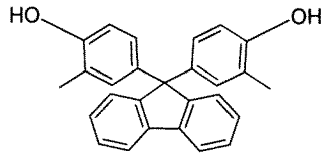

When the carbonate-based resin contains a structural unit derived from the structural unit D6, from the viewpoint of allowing an optical film using the carbonate-based resin to have a preferred wavelength dispersion property, the content of the structural unit is preferably 40 mass% or more, more preferably 45 mass% or more, even more preferably 50 mass% or more, even more preferably 55 mass% or more, particularly preferably 60 mass% or more with respect to the total mass of the carbonate-based resin based on all the dihydroxy compounds, all the dicarboxylic acid compounds, and all the carbonates in the carbonate-based resin.

If the content of the structural unit is excessively small, there is a case where an optical film using the carbonate-based resin does not have a preferred wavelength dispersion property. On the other hand, if the content of the structural unit is excessively large, there is a case where an optical film using the carbonate-based resin does not have a preferred optical property because a ratio between a phase difference as measured at a wavelength of 450 nm and a phase difference as measured at a wavelength of 550 nm is excessively large. Therefore, the content of the structural unit is preferably 95 mass% or less, more preferably 90 mass% or less, even more preferably 85 mass% or less, particularly preferably 80 mass% or less with respect to the total mass of the carbonate-based resin based on all the dihydroxy compounds, all the dicarboxylic acid compounds, and all the carbonates in the carbonate-based resin. It is noted that the phase difference ratio of the optical film refers to a ratio between the phase difference of the optical film as measured at a wavelength of 450 nm, $\lambda(450)$ and the phase difference of the optical film as measured at a wavelength of 550 nm, $\lambda(550)$ ($\lambda(450)/\lambda(550)$).

The carbonate-based resin can further contain a structural unit represented by the following formula (D7). In this case, the carbonate-based resin can have improved toughness and heat resistance and absorb less water. Further, a raw material of the structural unit represented by the formula (D7) is inexpensively available. The structural unit represented by the formula (D7) is referred to as a "structural unit D7" as appropriate.

[Formula 96]

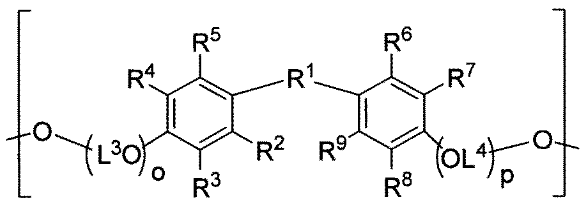

(D7)

In the formula (D7), $R^1$ is a direct bond, an oxygen atom, or an optionally-substituted alkylene group having 1 to 40 carbon atoms. $R^2$ to $R^9$ are each independently a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom having a substituent, a nitro group having a substituent, or a cyano group having a substituent. $L^3$ and $L^4$ are each independently an optionally-substituted alkylene group having 1 to 10 carbon atoms, an optionally-substituted arylene group having 4 to 10 carbon atoms, or an optionally-substituted aralkylene group having 6 to 12 carbon atoms. o is an integer of 0 to 4 and p is an integer of 0 to 4.

From the viewpoint of heat resistance and ease of synthesis, $L^3$ and $L^4$ in the formula (D7) are preferably each independently an optionally-substituted alkylene group having 1 to 4 carbon atoms, more preferably an ethylene group. From the viewpoint of heat resistance and ease of synthesis, o and p in the formula (D7) are preferably each independently 0 or 1.

From the viewpoint of ease of synthesis, the structural unit D7 is preferably represented by the following formula (D7-1).

[Formula 97]

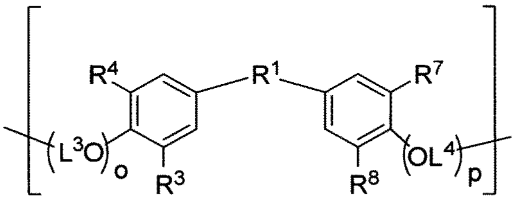

(D7-1)

In the formula (D7-1), $R^1$ is a direct bond, an oxygen atom, or an optionally-substituted alkylene group having 1 to 40 carbon atoms. $R^3$, $R^4$, $R^7$, and $R^8$ are each independently a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom, a nitro group, or a cyano group, $L^3$ and $L^4$ are each independently an optionally-substituted alkylene group having 1 to 10 carbon atoms, o is 0 or 1, and p is 0 or 1.

From the viewpoint of ease of synthesis due to a symmetric structure, the structural unit D7 is preferably represented by the following formula (D7-2).

[Formula 98]

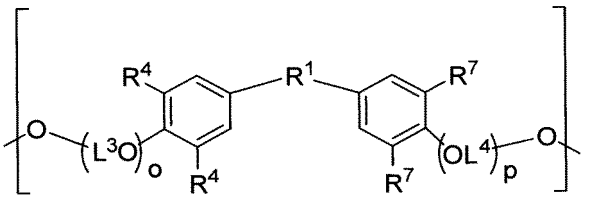

(D7-2)

In the formula (D7-2), $R^1$ is a direct bond, an oxygen atom, or an optionally-substituted alkylene group having 1 to 40 carbon atoms. $R^4$ and $R^7$ are each independently a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom having a substituent, a nitro group having a substituent, or a cyano group having a substituent. $L^3$ and $L^4$ are each independently an optionally-substituted alkylene group having 1 to 10 carbon atoms. o is 0 or 1 and p is 0 or 1.

From the viewpoint that raw materials are inexpensively available and the viewpoint of improving heat resistance, the structural unit D7 is preferably represented by the following formula (D7-3).

[Formula 99]

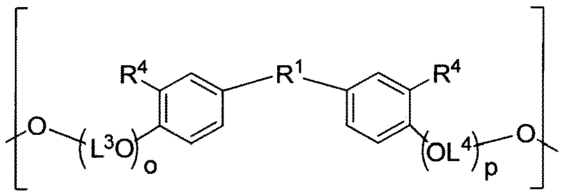

(D7-3)

In the formula (D7-3), $R^1$ is a direct bond, an oxygen atom, or an optionally-substituted alkylene group having 1 to 40 carbon atoms. $R^4$ is a hydrogen atom or an optionally-substituted alkyl group having 1 to 4 carbon atoms, $L^3$ and $L^4$ are each independently an optionally-substituted alkylene group having 1 to 10 carbon atoms. o is 0 or 1 and p is 0 or 1.

When the alkylene group as $L^3$ or $L^4$ in the formula (D7-3) is long, heat resistance tends to reduce. Therefore, it is preferred that o and p are 0 or o or p is 1 and $L^3$ or $L^4$ is an alkylene group having 1 to 2 carbon atoms. From the viewpoint of ease of synthesis, the number of carbon atoms of the alkylene group is more preferably 2.

From the viewpoint of high heat resistance, $R^1$ in the formula (D7-3) is preferably a direct bond or an oxygen atom. From the viewpoint of ease of synthesis and adjustment to high toughness, $R^1$ in the formula (D7-3) is preferably a methylene group, an alkyl methylene group having 2 to 40 carbon atoms, or a dialkyl methylene group having 3 to 40 carbon atoms, more preferably an alkyl methylene group having 2 to 40 carbon atoms.

From the viewpoint of heat resistance, $R^1$ in the formula (D7-3) more preferably has a methylene group. From the viewpoint of ease of synthesis and improvement in toughness, $R^1$ in the formula (D7-3) is more preferably an alkyl methylene group having 2 to 40 carbon atoms. From the viewpoint that a raw material is inexpensively available, the number of carbon atoms of the alkyl methylene group is preferably 2 to 4. From the viewpoint of further improving toughness, the number of carbon atoms of the alkyl methylene group is preferably 3 or more, more preferably 10 or more, even more preferably 12 or more. From the viewpoint of further improving heat resistance, the number of carbon atoms of the alkyl methylene group is preferably 40 or less, more preferably 30 or less, even more preferably 20 or less. From the viewpoint of balance between improvement in toughness and heat resistance, the number of carbon atoms of the alkyl methylene group is preferably 7 to 15.

From the viewpoint of heat stability, $R^1$ in the formula (D7-3) is preferably a dialkyl methylene group having 3 to 40 carbon atoms. From the viewpoint of toughness, the number of carbon atoms of the dialkyl methylene group is preferably 5 or more, more preferably 10 or more, even more preferably 20 or more. From the viewpoint of heat resistance, the number of carbon atoms of the dialkyl methylene group is preferably 40 or less, more preferably 30 or less, even more preferably 20 or less. From the viewpoint of ease of synthesis, the number of carbon atoms of the dialkyl methylene group is preferably 3 to 10.

From the viewpoint of heat resistance, $R^4$ in the formula (D7-3) is preferably a hydrogen atom. Further, from the viewpoint that a raw material is inexpensively available and the viewpoint of toughness, $R^4$ in the formula (D7-3) is preferably a methyl group.

From the viewpoint of high heat resistance, o and p in the formula (D7-3) are preferably 0. Further, from the viewpoint of toughness, o and p in the formula (D7-3) are preferably 1.

A specific example of the structural unit D7 is any one of structures shown below.

[Formula 100]

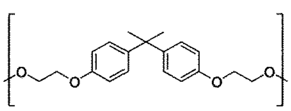
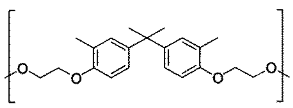
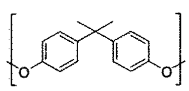
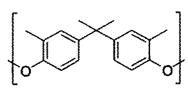

The carbonate-based resin having the structural unit D7 is produced by, for example, polymerization of a monomer represented by the following formula (g).

[Formula 101]

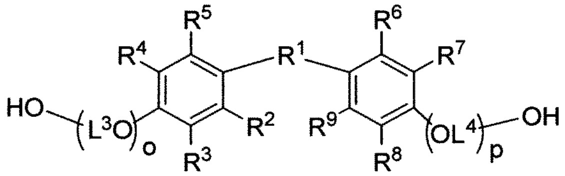

(g)

$R^1$ to $R^9$, $L^3$, $L^4$, o, and p in the formula (g) are respectively the same as $R^1$ to $R^9$, $L^3$, $L^4$, o, and p in the formula (D7). Specific examples of the monomer represented by the formula (g) include monomers shown below.

[Formula 102]

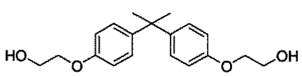 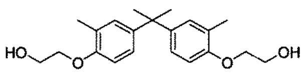 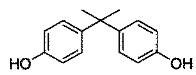 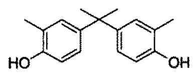

From the viewpoint of improving moist heat resistance and toughness of the carbonate-based resin, the content of the structural unit D7 in the carbonate-based resin in terms of mass is preferably 5 mass% or more, more preferably 10 mass% or more, even more preferably 15 mass% or more, even more preferably 25 mass% or more, particularly preferably 35 mass% or more. Further, from the viewpoint of improving weatherability, the content of the structural unit D7 in the carbonate-based resin is preferably 95 mass% or less, more preferably 80 mass% or less, even more preferably 65 mass% or less, even more preferably 55 mass% or less, particularly preferably 45 mass% or less with respect to the total mass of the carbonate-based resin based on all the dihydroxy compounds, all the dicarboxylic acid compounds, and all the carbonates constituting the carbonate-based resin.

[Method for producing carbonate-based resin having carbonate bond]

When the carbonate-based resin is a polycarbonate resin, the polycarbonate resin is produced by a polymerization method generally used. Specific examples of such a polymerization method include solution polymerization using phosgene and melt polymerization involving a reaction between a monomer material and a diester carbonate. Either of these methods may be used, but melt polymerization is preferred in which a raw material monomer (specifically, a dihydroxy compound) is reacted with a diester carbonate less toxic to the environment in the presence of a polymerization catalyst. It is noted that in the polymerization method to produce a polyester carbonate resin as the carbonate-based resin, a transesterification catalyst, polymerization conditions, and an additive or the like used in a polymerization step may be the same as those used in a polymerization method for a polycarbonate resin described later.
As the diester carbonate, one represented by the following formula (o) can be used.

[Formula 103]

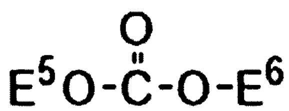

(o)

In the formula (o), $E^5$ and $E^6$ are each independently an optionally-substituted aliphatic group having 1 to 18 carbon atoms or an optionally-substituted aromatic group having 6 to 12 carbon atoms.
Examples of the diester carbonate represented by the formula (o) include diphenyl carbonate and a substituted diphenyl carbonate typified by ditolyl carbonate. Other examples of the diester carbonate represented by the formula (o) include dimethyl carbonate, diethyl carbonate, and di-t-butyl carbonate. The diester carbonate represented by the formula (o) is preferably diphenyl carbonate or a substituted diphenyl carbonate. As the diester carbonate, one of these compounds may be used or two or more of these compounds may be used.
The diester carbonate may be substituted by a dicarboxylic acid or a dicarboxylic acid ester. The amount of the substituted diester carbonate is preferably 50 mol% or less, more preferably 30 mol% or less with respect to the amount of the all the diester carbonates. Examples of the dicarboxylic acid and the dicarboxylic acid ester include terephthalic acid, isophthalic acid, diphenyl terephthalate, and diphenyl isophthalate. A dicarboxylic acid compound as a raw material for introducing the above-described structural unit (D4) or (D5) may also be used. When the diester carbonate is substituted

by such a dicarboxylic acid or dicarboxylic acid ester, a polyester carbonate resin is obtained.

The amount of the diester carbonate used with respect to the amount of all the dihydroxy compounds used in the reaction is preferably 0.90 to 1.10, more preferably 0.96 to 1.04 in terms of mole ratio. If the amount of the diester carbonate used is smaller than 0.90, the amount of terminal OH groups of the polycarbonate resin may increase so that the heat stability of the polymer may deteriorate or a desired high-molecular-weight polymer cannot be obtained. On the other hand, if the amount of the diester carbonate used exceeds 1.10, there is a possibility that the speed of a transesterification reaction decreases under the same polymerization conditions or it is difficult to produce a polycarbonate resin having a desired molecular weight, and in addition to that, the amount of the diester carbonate remaining in the polycarbonate resin increases. The remaining diester carbonate becomes a cause of odor during molding or odor of a molded article.

As the raw material dihydroxy compound, a dihydroxy compound for introducing the structural unit B1 and a dihydroxy compound added if necessary to introduce another structural unit are used. As described above, the blending ratio between these dihydroxy compounds can appropriately be adjusted to achieve desired physical properties and characteristics.

As the polymerization catalyst (transesterification catalyst) in melt polymerization, an alkali metal compound and/or an alkaline-earth metal compound are/is used. A basic compound may also supplementarily be used together with the alkali metal compound and/or the alkaline-earth metal compound, and examples of such a basic compound include a basic boron compound, a basic phosphorus compound, a basic ammonium compound, and an amine-based compound. Preferably, only the alkali metal compound and/or the alkaline-earth metal compound are/is used.

Examples of the alkali metal compound used as the polymerization catalyst include sodium hydroxide, potassium hydroxide, lithium hydroxide, cesium hydroxide, sodium hydrogen carbonate, potassium hydrogen carbonate, lithium hydrogen carbonate, cesium hydrogen carbonate, sodium carbonate, potassium carbonate, lithium carbonate, cesium carbonate, sodium acetate, potassium acetate, lithium acetate, cesium acetate, sodium stearate, potassium stearate, lithium stearate, cesium stearate, sodium boron hydride, potassium boron hydride, lithium boron hydride, cesium boron hydride, sodium phenylboron, potassium phenylboron, lithium phenylboron, cesium phenylboron, sodium benzoate, potassium benzoate, lithium benzoate, cesium benzoate, disodium hydrogen phosphate, dipotassium hydrogen phosphate, dilithium hydrogen phosphate, dicesium hydrogen phosphate, disodium phenyl phosphate, dipotassium phenyl phosphate, dilithium phenyl phosphate, and dicesium phenyl phosphate. Other examples of the alkali metal compound used as the polymerization catalyst include: an alcoholate of sodium, potassium, lithium, or cesium; a disodium salt, a dipotassium salt, a dilithium salt, or a dicesium salt of phenolate; and a disodium salt, a dipotassium salt, a dilithium salt, or a dicesium salt of bisphenol A.

Examples of the alkaline-earth metal compound include potassium hydroxide, barium hydroxide, magnesium hydroxide, strontium hydroxide, calcium hydrogen carbonate, barium hydrogen carbonate, magnesium hydrogen carbonate, strontium hydrogen carbonate, calcium carbonate, barium carbonate, magnesium carbonate, strontium carbonate, calcium acetate, barium acetate, magnesium acetate, strontium acetate, calcium stearate, barium stearate, magnesium stearate, and strontium stearate.

As the alkali metal compound and/or the alkaline earth metal compound, one or two or more of these compounds can be used.

Specific examples of the basic boron compound include metallic salts of tetramethylboron, tetraethylboron, tetrapropylboron, tetrabutylboron, trimethylethylboron, trimethylbenzylboron, trimethylphenylboron, triethylmethylboron, triethylbenzylboron, triethylphenylboron, tributylbenzylboron, tributylphenylboron, tetraphenylboron, benzyltriphenylboron, methyltriphenylboron, and butyltriphenylboron. Examples of the metallic salts include a sodium salt, a potassium salt, a lithium salt, a calcium salt, a barium salt, a magnesium salt, and a strontium salt.

Examples of the basic phosphorus compound include triethylphosphine, tri-n-propylphosphine, triisopropylphosphine, tri-n-butylphosphine, triphenylphosphine, tributylphosphine, and a quaternary phosphonium salt.

Examples of the basic ammonium compound include tetramethylammonium hydroxide, tetraethylammonium hydroxide, tetrapropylammonium hydroxide, tetrabutylammonium hydroxide, trimethylethylammonium hydroxide, trimethylbenzylammonium hydroxide, trimethylphenylammonium hydroxide, triethylmethylammonium hydroxide, triethylbenzylammonium hydroxide, triethylphenylammonium hydroxide, tributylbenzylammonium hydroxide, tributylphenylammonium hydroxide, tetraphenylammonium hydroxide, benzyltriphenylammonium hydroxide, methyltriphenylammonium hydroxide, and butyltriphenylammonium hydroxide.

Examples of the amine-based compound include 4-aminopyridine, 2-aminopyridine, N,N-dimethyl-4-aminopyridine, 4-diethylaminopyridine, 2-hydroxypyridine, 2-methoxypyridine, 4-methoxypyridine, 2-dimethylaminoimidazole, 2-methoxyimidazole, imidazole, 2-mercaptoimidazole, 2-methylimidazole, and aminoquinoline.

As the basic compound, one or two or more of these compounds can be used.

When the alkali metal compound and/or the alkaline-earth metal compound are/is used as the polymerization catalyst, the amount of the polymerization catalyst used per mole of all the dihydroxy compounds used in the reaction is usually 0.1 to 500 μmol, preferably 0.5 to 300 μmol, more preferably 1 to 250 μmol in terms of the amount of metal. If the amount of the polymerization catalyst used is too small, there is a possibility that polymerization activity necessary to

produce a polycarbonate resin having a desired molecular weight cannot be obtained. On the other hand, if the amount of the polymerization catalyst used is too large, there is a possibilty that the hue of the polycarbonate resin deteriorates, flowability reduces due to the generation of a by-product, or a large amount of gel is generated. As a result, it is difficult to produce a polycarbonate resin having desired quality.

Each of the raw material dihydroxy compounds used to produce the polycarbonate resin may be supplied as a solid, or may be supplied in a melt state by heating, or may be supplied as an aqueous solution when soluble in water. When the raw material dihydroxy compound is supplied in a melt state or as an aqueous solution, there is an advantage that weighing and transportation in industrial production can become easy.

The method in which a raw material dihydroxy compound is reacted with a diester carbonate in the presence of a polymerization catalyst is usually performed in a multistage process having two or more stages. Specifically, the reaction of the first stage is performed at a temperature of 140 to 220°C, preferably 150 to 200°C for 0.1 to 10 hours, preferably 0.5 to 3 hours. In and after the second stage, the reaction is performed by increasing the reaction temperature and gradually reducing the pressure as compared to the first stage while generated phenol is discharged to the outside of the reaction system. Finally, a polycondensation reaction is performed at a pressure in the reaction system of 200 Pa or less and a temperature in the range of 210 to 280°C.

Pressure reduction in the polycondensation reaction is preferably performed by controlling a balance between the temperature and the pressure in the reaction system. Particularly, if either of the temperature and the pressure is too quickly changed, there is a possibility that an unreacted monomer distills so that the degree of polymerization decreases due to an inappropriate mole ratio between the dihydroxy compound and the diester carbonate.

Specifically, when, for example, a dihydroxy compound represented by the formula (P1) such as a compound represented by the formula(P24),isosorbide, and 1,4-cyclohexanedimethanol are used as the dihydroxy compounds and the ratio of 1,4-cyclohexanedimethanol used to all the dihydroxy compounds in terms of a mole ratio is 50 mol% or more, 1,4-cyclohexanedimethanol is likely to distill as a monomer. Therefore, the polycondensation reaction is preferably performed by increasing the temperature at a temperature rise rate of 40°C/h or less until the pressure in the reaction system is reduced to about 13 kPa, then increasing the temperature at a temperature rise rate of 40°C/h or less at a pressure of up to about 6.67 kPa, and finally keeping the temperature at 200 to 250°C at a pressure of 200 Pa or less. In this case, a polycarbonate resin having a sufficiently high polymerization degree is obtained.

When the ratio of 1,4-cyclohexanedimethanol used to all the dihydroxy compounds in terms of mole ratio is less than 50 mol%, a sudden viscosity increase is likely to occur, and when the ratio is 30 mol% or less, a sudden viscosity increase is more likely to occur. Therefore, the polycondensation reaction is preferably performed by increasing the temperature at a temperature rise rate of 40°C/h or less until the pressure in the reaction system is reduced to about 13 kPa, then increasing the temperature at a temperature rise rate of 40°C/h or more at a pressure of up to about 6.67 kPa, and finally keeping the temperature at 220 to 290°C at a reduced pressure of 200 Pa or less. In this case, a polycarbonate resin having a sufficiently high polymerization degree is obtained. It is noted that the temperature rise at a pressure of up to about 6.67 kPa is more preferably performed at a temperature rise rate of 50°C/h.

The reaction may be performed either in batch or continuous manner. Alternatively, the reaction may be performed by a combination of batch and continuous operations.

When the polycarbonate resin is produced by melt polymerization, a phosphate compound, a phosphite compound, a metallic salt of phosphoric acid, or a metallic salt of phosphorous acid may be added during polymerization for the purpose of preventing coloration.

Preferred examples of the phosphate compound include trialkyl phosphates such as trimethyl phosphate and triethyl phosphate. As the phosphate compound, one or two or more of these compounds can be used. The amount of the phosphate compound added is preferably 0.0001 mol% or more and 0.005 mol% or less, more preferably 0.0003 mol% or more and 0.003 mol% or less with respect to the amount of all the dihydroxy compounds used in the reaction. If the amount of the phosphate compound added is smaller than the above lower limit, the effect of preventing coloration is small. On the other hand, if the amount of the phosphate compound added is larger than the above upper limit, an increase in haze may be caused, the addition may promote coloration by the opposite effect, or heat resistance may reduce.

As the phosphite compound, the following compounds used as a heat stabilizer can be used. Specific examples of the phosphite compound include trimethyl phosphite, triethyl phosphite, trisnonylphenyl phosphite, trimethyl phosphate, tris(2,4-di-tert-butylphenyl) phosphite, bis(2,4-di-tert-butylphenyl) pentaerythritol diphosphite. As the phosphite compound, one or two or more of these compounds are used.

The amount of the phosphite compound added is preferably 0.0001 mol% or more and 0.005 mol% or less, more preferably 0.0003 mol% or more and 0.003 mol% or less with respect to the amount of all the dihydroxy compounds used in the reaction. If the amount of the phosphite compound added is smaller than the above lower limit, the effect of preventing coloration is small. On the other hand, if the amount of the phosphite compound added is larger than the above upper limit, an increase in haze may be caused, the addition may promote coloration by the opposite effect, or heat resistance may reduce.

The phosphate compound and a metallic salt thereof or the phosphite compound and a metallic salt thereof may

be used in combination. In this case, the total amount of these compound and metallic salt added is preferably 0.0001 mol% or more and 0.005 mol% or less, more preferably 0.0003 mol% or more and 0.003 mol% or less with respect to the amount of all the dihydroxy compounds. If the total amount added is smaller than the above lower limit, the effect of preventing coloration is small. On the other hand, if the total amount added is larger than the above upper limit, an increase in haze may be caused, the addition may promote coloration by the opposite effect, or heat resistance may reduce.

It is noted that the metallic salt of the phosphate compound and the metallic salt of the phosphite compound are preferably alkali metal salts or zinc salts, more preferably zinc salts. Among zinc phosphates, a zinc long-chain alkylphosphate is preferred.

Further, the polycarbonate resin may contain a heat stabilizer to prevent a reduction in molecular weight or deterioration of hue during molding or the like.

Examples of such a heat stabilizer include phosphorous acid, phosphoric acid, phosphonous acid, phosphonic acid, and esters thereof. Specific examples thereof include triphenyl phosphite, tris(nonylphenyl) phosphite, tris(2,4-di-tert-butylphenyl) phosphite, tridecyl phosphite, trioctyl phosphite, trioctadecyl phosphite, didecylmonophenyl phosphite, dioctylmonophenyl phosphite, diisopropylmonophenyl phosphite, monobutyldiphenyl phosphite, monodecyldiphenyl phosphite, monooctyldiphenyl phosphite, bis(2,6-di-tert-butyl-4-methylphenyl)pentaerythritol diphosphite, 2,2-methylenebis(4,6-di-tert-butylphenyl)octyl phosphite, bis(nonylphenyl) pentaerythritol diphosphite, bis(2,4-di-tert-butylphenyl) pentaerythritol diphosphite, distearylpentaerythritol diphosphite, tributyl phosphate, triethyl phosphate, trimethyl phosphate, triphenyl phosphate, diphenyl monoorthoxenyl phosphate, dibutyl phosphate, dioctyl phosphate, diisopropyl phosphate, tetrakis(2,4-di-tert-butylphenyl) 4,4'-biphenylene diphosphinate, dimethyl benzenephosphonate, diethyl benzenephosphonate, and dipropyl benzenephosphonate.

Among them, trisnonylphenyl phosphite, trimethyl phosphate, tris(2,4-di-tert-butylphenyl) phosphite, bis(2,4-di-tert-butylphenyl) pentaerythritol diphosphite, bis(2,6-di-tert-butyl-4-methylphenyl) pentaerythritol diphosphite, and dimethyl benzenephosphonate are preferably used.

As the heat stabilizer, one of these compounds may be used or two or more of these compounds may be used.

The heat stabilizer may additionally be added after added during melt polymerization.

That is, after a polycarbonate resin is obtained by adding an appropriate amount of a phosphite compound or a phosphate compound, the heat stabilizer such as a phosphite compound may additionally be added. In this case, deterioration of hue of the polycarbonate resin can further be prevented.

The amount of the heat stabilizer added is preferably 0.0001 to 1 part by mass, more preferably 0.0005 to 0.5 parts by mass, even more preferably 0.001 to 0.2 parts by mass per 100 parts by mass of the polycarbonate resin.

The polycarbonate resin or a polycarbonate resin composition obtained by adding an additive or the like thereto may directly be molded or may be molded after once pelletized by a melt extruder. The molding is performed by a commonly-known method such as injection molding, extrusion molding, or compression molding.

In order to obtain stable releasability and various physical properties by increasing miscibility of the polycarbonate resin, a single-screw extruder or a twin-screw extruder is preferably used for melt extrusion. In this case, the use of a solvent or the like can be avoided, and therefore an environmental load can be reduced and productivity can further be improved.

A melt kneading temperature depends on the glass transition temperature of the polycarbonate resin. For example, when the glass transition temperature of the polycarbonate resin is lower than 90°C, the melt kneading temperature of the extruder is usually 130°C to 250°C, preferably 150°C to 240°C. If the melt kneading temperature is lower than 130°C, a heavy load may be imposed on the extruder due to an increase in the melt viscosity of the polycarbonate resin so that productivity may reduce. On the other hand, if the melt kneading temperature is higher than 250°C, the melt viscosity of the polycarbonate resin decreases, which makes it difficult to obtain pellets. As a result, productivity may reduce.

On the other hand, when the glass transition temperature of the polycarbonate resin is 90°C or higher, the melt kneading temperature is usually 200°C to 300°C, preferably 220°C to 260°C. If the melt kneading temperature is lower than 200°C, a heavy load may be imposed on the extruder due to an increase in the melt viscosity of the polycarbonate resin so that productivity may reduce. On the other hand, if the melt kneading temperature is higher than 300°C, the polycarbonate resin is likely to deteriorate. Specifically, there is a possibility that yellowing of the polycarbonate resin occurs or the strength of the polycarbonate resin deteriorates due to a reduction in molecular weight.

When the extruder is used, a filter is preferably disposed to prevent burning of the polycarbonate resin or entry of foreign matter during extrusion. The opening of the filter (specifically, the size of foreign matter to be removed) depends on the intended use, physical properties, and characteristics of the polycarbonate resin, but is preferably 100 μm or less. Particularly, when it is necessary to avoid the entry of foreign matter, the opening of the filter is more preferably 40 μm or less, even more preferably 10 μm or less.

The extrusion of the polycarbonate resin is preferably performed in a clean room. In this case, it is possible to prevent the entry of foreign matter into the polycarbonate resin after extrusion.

Further, when the extruded polycarbonate resin is formed into chips by cooling, the cooling is preferably air

cooling, water cooling, or the like. In the case of air cooling, air is preferably passed through a hepafilter or the like before use to remove foreign matter. In this case, it is possible to prevent reattachment of foreign matter contained in air. In the case of water cooling, water is preferably passed through an ion-exchange resin or the like to remove metallic components and then through a filter to remove foreign matter before use. The opening of the filter may appropriately be selected, but is preferably 0.45 to 10 μm.

[Physical properties of carbonate-based resin having carbonate bond]

The physical properties and characteristics of the carbonate-based resin can be adjusted depending on its desired purpose of use.

<Glass transition temperature>

From the viewpoint of enhancing heat resistance, the glass transition temperature of the carbonate-based resin is preferably 100°C or higher, more preferably 105°C or higher, even more preferably 110°C or higher, even more preferably 115°C or higher, particularly preferably 125°C or higher. From the viewpoint of increasing the glass transition temperature to enhance heat resistance, it is preferred that at least one of the ends of the carbonate-based resin is not a hydroxy group. On the other hand, if the glass transition temperature is too high, there is a possibility that the carbonate-based resin deteriorates due to shear heating during extrusion. Further, in this case, there is a possibility that the carbonate-based resin deteriorates due to an excessive increase in melt viscosity during filtration through a filter. Therefore, the glass transition temperature Tg is preferably 260°C or lower, more preferably 230°C or lower, even more preferably 200°C or lower, particularly preferably 180°C or lower. The glass transition temperature of the carbonate-based resin is adjusted to fall within the above range by adjusting the kind of monomer used for production, the blending ratio thereof, the polymerization temperature, and the amount of an additive added.

The glass transition temperature is measured as a temperature at which glass transition of the carbonate-based resin starts. Specifically, the glass transition temperature of the carbonate-based resin is measured by, for example, a method described in Examples.

<Saturated water absorption rate>

If the saturated water absorption rate of the carbonate-based resin is high, there is a possibility that the physical properties of the resin change under high humidity so that the reliability of a molded article reduces. Therefore, the saturated water absorption rate of the carbonate-based resin is preferably 4 wt% or less, more preferably 3.5 wt% or less, even more preferably 3 wt% or less, even more preferably 2.8 wt% or less, particularly preferably 2.5 wt% or less. It is noted that the saturated water absorption rate of the carbonate-based resin is measured by, for example, a method described in Examples. The saturated water absorption rate of the carbonate-based resin is adjusted to fall within the above range by adjusting the kind of monomer used for production, the blending ratio thereof, the polymerization temperature, and the amount of an additive added.

<Boiling water test>

The moist heat resistance of the carbonate-based resin can be evaluated by determining whether or not the carbonate-based resin is deformed by a boiling water test. From the viewpoint of excellent moist heat stability, it is preferred that the carbonate-based resin is not deformed by a boiling water test.

<Reduced viscosity>

The polymerization degree (specifically, molecular weight) of the carbonate-based resin can be expressed by a reduced viscosity when increased to a certain level or higher. Except for polycarbonate diols used even when having a relatively low polymerization degree (molecular weight), the polymerization degree (specifically, molecular weight) of the carbonate-based resin is determined by measuring a reduced viscosity. The reduced viscosity is measured in the following manner. First, a sample is prepared by precisely adjusting the concentration of the carbonate-based resin to 1.00 g/dl with the use of a mixed solvent obtained by mixing phenol and 1,1,2,2-tetrachloroethane in a weight ratio of 1:1. Then, the reduced viscosity of the sample is measured at a temperature of 30.0°C ± 0.1°C.

If the reduced viscosity of the carbonate-based resin is too low, there is a possibility that the characteristics of a molded article obtained after molding, such as heat resistance, chemical resistance, abrasion resistance, and mechanical strength, deteriorate. Therefore, the reduced viscosity of the carbonate-based resin is preferably 0.20 dL/g or more, more preferably 0.30 dL/g or more. On the other hand, if the reduced viscosity is too high, there is a possibility that flowability of

the resin during molding reduces so that productivity and moldability reduce or a molded article is highly distorted. Therefore, the reduced viscosity is preferably 1.50 dL/g or less, more preferably 1.20 dL/g or less, even more preferably 1.00 dL/g or less, even more preferably 0.90 dL/g or less. It is noted that the reduced viscosity of the carbonate-based resin is measured by, for example, a method described in Examples. The reduced viscosity of the carbonate-based resin is adjusted to fall within the above range by adjusting the kind of monomer used for production, the blending ratio thereof, the polymerization temperature, and the amount of an additive added.

<Folding test>

The toughness of the carbonate-based resin is evaluated by, for example, a folding test. When the carbonate-based resin is broken by this test, a molded article may be brittle. It is noted that more specifically, the folding test is performed by a method described in Examples.

<Charpy impact test>

Further, the toughness of the carbonate-based resin is evaluated by, for example, a Charpy impact test. If the Charpy impact strength is low, a molded article may be brittle. Therefore, the value of the Charpy impact test as measured at room temperature (23°C) in accordance with ISO179 (2000) using a specimen having a notch top radius of 0.25R is preferably 1 $kJ/m^2$ or more, more preferably 2 $kJ/m^2$ or more, even more preferably 3 $kJ/m^2$ or more. The value of the Charpy impact test as measured in the same manner except that the test is performed at a low temperature (-20°C) is preferably 1 $kJ/m^2$ or more, more preferably 2 $kJ/m^2$ or more, even more preferably 3 $kJ/m^2$ or more. It is most preferred that the value of the Charpy impact test as measured at room temperature (23°C) and the value of the Charpy impact test as measured at a low temperature (-20°C) are both 3 $kJ/m^2$ or more. It is noted that more specifically, the Charpy impact test is performed by a method described in Examples. The Charpy impact strength of the carbonate-based resin is adjusted to fall within the above range by adjusting the kind of monomer used for production, the blending ratio thereof, the polymerization temperature, and the amount of an additive added.

<Pencil hardness>

If the hardness of the carbonate-based resin is low, a molded article is easily scratched. The pencil hardness of the carbonate-based resin is preferably B or higher, more preferably F or higher. From the viewpoint of further enhancing the scratch resistance of a molded article, the pencil hardness of the carbonate-based resin is even more preferably H or higher. The pencil hardness is measured by a pencil hardness test. It is noted that more specifically, the pencil hardness is measured by a method described in Examples. The pencil hardness of the carbonate-based resin is adjusted to fall within the above range by adjusting the kind of monomer used for production, the blending ratio thereof, the polymerization temperature, and the amount of an additive added.

<Photoelastic coefficient>

If the photoelastic coefficient of the carbonate-based resin is high, a phase difference is generated by stress, which reduces optical reliability. The photoelastic coefficient of the carbonate-based resin is preferably $18 \times 10^{-12}$ $Pa^{-1}$ or less, more preferably $15 \times 10^{-12}$ $Pa^{-1}$ or less, even more preferably, $12 \times 10^{-12}$ $Pa^{-1}$ or less, even more preferably $9 \times 10^{-12}$ $Pa^{-1}$ or less, particularly preferably $4 \times 10^{-12}$ $Pa^{-1}$ or less. Further, from the viewpoint that a resin having a negative photoelastic coefficient can reduce the photoelastic coefficient of another resin by blending, the photoelastic coefficient is preferably negative. It is noted that specifically, the photoelastic coefficient is measured by a method described in Examples.

<5% thermal weight loss temperature (Td5)>

When the 5% thermal weight loss temperature of the carbonate-based resin is high, the carbonate-based resin is less likely to be thermally decomposed. The 5% thermal weight loss temperature of the carbonate-based resin is preferably 200°C or higher, more preferably 250°C or higher, particularly preferably 300°C or higher. It is noted that specifically, the 5% thermal weight loss temperature is measured by a method described in Examples.

[Resin composition]

Various additives may be added to the carbonate-based resin to obtain a resin composition.
An antioxidant may be added to the carbonate-based resin. In this case, oxidation of the carbonate-based resin

can be prevented.

Examples of the antioxidant include pentaerythritol tetrakis(3-mercaptopropionate), pentaerythritol tetrakis(3-laurylthiopropionate), glycerol-3-stearylthiopropionate, triethylene glycol-bis[3-(3-tert-butyl-5-methyl-4-hydroxyphenyl) propionate], 1,6-hexanediol-bis[3-(3,5-di-tert-butyl-4-hydroxyphenyl)propionate], pentaerythritol-tetrakis[3-(3,5-di-tert-butyl-4-hydroxyphenyl)propionate], octadecyl-3-(3,5-di-tert-butyl-4-hydroxyphenyl)propionate, 1,3,5-trimethyl-2,4,6-tris(3,5-di-tert-butyl-4-hydroxybenzyl)benzene, N,N-hexamethylenebis(3,5-di-tert-butyl-4-hydroxy-hydrocinnamide), diethyl 3,5-di-tert-butyl-4-hydroxy-benzylphosphonate, tris(3,5-di-tert-butyl-4-hydroxybenzyl) isocyanurate, tetrakis(2,4-di-tert-butylphenyl) 4,4'-biphenylene diphosphinate, and 3,9-bis{1,1-dimethyl-2-[β-(3-tert-butyl-4-hydroxy-5-methylphenyl)propionyloxy]ethyl}-2,4,8,10-tetraoxaspiro(5,5)undecane. As the antioxidant, one or two or more of these compounds can be used.

The amount of the antioxidant added is preferably 0.0001 to 0.5 parts by mass per 100 parts by mass of the carbonate-based resin.

Further, a releasing agent may be added to the carbonate-based resin. In this case, releasability from a mold in melt molding is improved.

Examples of the releasing agent include a higher fatty acid ester of a monohydric or polyhydric alcohol, a higher fatty acid, paraffin wax, bees wax, olefin-based wax, olefin-based wax containing a carboxyl group and/or a carboxylic anhydride group, silicone oil, and an organopolysiloxane.

As the higher fatty acid ester, a partial or total ester of a monohydric or polyhydric alcohol having 1 to 20 carbon atoms and a saturated fatty acid having 10 to 30 carbon atoms is preferred. Examples of the partial or total ester of a monohydric or polyhydric alcohol and a saturated fatty acid include monoglyceride stearate, diglyceride stearate, triglyceride stearate, monosorbitate stearate, stearyl stearate, monoglyceride behenate, behenyl behenate, pentaerythritol monostearate, pentaerythritol tetrastearate, pentaerythritol tetrapelargonate, propylene glycol monostearate, stearyl stearate, palmityl palmitate, butyl stearate, methyl laurate, isopropyl palmitate, biphenyl biphenate, sorbitan monostearate, and 2-ethylhexyl stearate.

Among them, monoglyceride stearate, triglyceride stearate, pentaerythritol tetrastearate, and behenyl behenate are preferably used.

As the higher fatty acid, a saturated fatty acid having 10 to 30 carbon atoms is preferred. Examples of such a fatty acid include myristic acid, lauric acid, palmitic acid, stearic acid, and behenic acid.

As the releasing agent, one of these compounds may be used or two or more of these compounds may be used.

The amount of the releasing agent added is preferably 0.01 to 5 parts by mass per 100 parts by mass of the carbonate-based resin.

Further, a light stabilizer may be added to the carbonate-based resin.

Examples of the light stabilizer include 2-(2'-hydroxy-5'-tert-octylphenyl)benzotriazole, 2-(3-tert-butyl-5-methyl-2-hydroxyphenyl)-5-chlorobenzotriazole, 2-(5-methyl-2-hydroxyphenyl)benzotriazole, 2-[2-hydroxy-3,5-bis(α,α-dimethylbenzyl)phenyl]-2H-benzotriazole, 2,2'-methylenebis(4-cumyl-6-benzotriazolephenyl), and 2,2'-p-phenylenebis(1,3-benzoxazin-4-one).

As the heat stabilizer, one of these compounds may be used or two or more of these compounds may be used.

The amount of the light stabilizer added is preferably 0.01 to 2 parts by mass per 100 parts by mass of the carbonate-based resin.

Further, a bluing agent may be added to the carbonate-based resin to cancel out yellow tint. As the bluing agent, any bluing agent can be used without any problem as long as it can be used for carbonate-based resins. From the viewpoint of availability, an anthraquinone-based dye is preferred.

Specific typical examples of the bluing agent include, common name, Solvent Violet 13 [CA. No. (color index No.) 60725], common name, Solvent Violet 31 [CA. No. 68210], common name, Solvent Violet 33 [CA. No. 60725], common name, Solvent Blue 94 [CA. No. 61500], common name, Solvent Violet 36 [CA. No. 68210], common name, Solvent blue 97 ["Macrolex Violet RR" manufactured by Bayer AG], and common name, Solvent Blue 45 [CA. No. 61110].

These bluing agents may be used singly or in combination of two or more of them.

The amount of the bluing agent added is usually $0.1 \times 10^{-4}$ to $2 \times 10^{-4}$ parts by mass per 100 parts by mass of the carbonate-based resin.

The carbonate-based resin and the above-described various additives are mixed by, for example, a method using a tumbler, a V-type blender, a SUPERMIXER, a Nauta mixer, a Banbury mixer, a kneading roll, or an extruder. Another mixing method is a solution blending method in which components are mixed in a state where they are dissolved in a common good solvent such as methylene chloride. The mixing method is not particularly limited, and any mixing method may be used as long as it is a polymer blending method usually used.

The carbonate-based resin may be blended with another thermoplastic resin. From the viewpoint of excellent optical performance and the viewpoint that injection molding tends to become possible, the carbonate-based resin is preferably blended with another thermoplastic resin. Specific examples of another thermoplastic resin allowed to coexist with the carbonate-based resin by blending include a polycondensation-type polymer, an olefin-based polymer, and an

addition-polymerization-type polymer, and a polycondensation-type polymer is preferred.

Specific examples of the olefin-based resin include polyethylene and polypropylene, and specific examples of the polycondensation-type polymer include a polyester, a polyamide, a polyester carbonate, and a polyimide.

The carbonate-based resin and another thermoplastic resin are preferably compatible with each other. In this case, when the resin composition or a molded article thereof has excellent transparency, it is possible to prevent a reduction in such transparency.

The resin composition obtained by blending is a product in which a polymer having at least the structural unit B1 (i.e., the carbonate-based resin) and another polymer different from this polymer (i.e., the above-described another thermoplastic resin) coexist as polymers. In this case, the resin composition may contain the structural unit B1 in, for example, any mole fraction. From the viewpoint of improving surface hardness or reducing photoelastic coefficient, the content of the structural unit B1 of the carbonate-based resin with respect to the total amount of the thermoplastic resins contained in the resin composition (specifically, the total amount of the carbonate-based resin having the structural unit B1 and the another thermoplastic resin) in terms of the amount of substance is preferably 1 mol% or more, more preferably 5 mol% or more, even more preferably 15 mol% or more, even more preferably 25 mol% or more, particularly preferably 37.5 mol% or more. From the same viewpoint, the content of the structural unit B1 of the carbonate-based resin with respect to the total amount of the thermoplastic resins contained in the resin composition in terms of mass is preferably 1 mass% or more, more preferably 10 mass% or more, even more preferably 40 mass% or more, even more preferably 60 mass% or more, particularly preferably 75 mass% or more.

On the other hand, from the viewpoint of achieving both moist heat resistance and film strength, the content of the structural unit B1 of the carbonate-based resin with respect to the total amount of the thermoplastic resins contained in the resin composition in terms of the amount of substance is preferably 0.1 mol% or more, more preferably 1 mol% or more, even more preferably 2 mol% or more, even more preferably 4 mol% or more, particularly preferably 5 mol% or more. Further, the content of the structural unit B1 of the carbonate-based resin with respect to the total amount of the thermoplastic resins contained in the resin composition in terms of the amount of substance is preferably 50 mol% or less, more preferably 37.5 mol% or less, even more preferably 25 mol% or less, even more preferably 20 mol% or less, particularly preferably 15 mol% or less. From the same viewpoint, the content of the structural unit B1 of the carbonate-based resin with respect to the total amount of the thermoplastic resins contained in the resin composition in terms of mass is preferably 1 mass% or more, more preferably 2 mass% or more, even more preferably 5 mass% or more, even more preferably 7 mass% or more, particularly preferably 10 mass% or more. Further, the content of the structural unit B1 of the carbonate-based resin with respect to the total amount of the thermoplastic resins contained in the resin composition in terms of mass is preferably 95 mass% or less, more preferably 80 mass% or less, even more preferably 60 mass% or less, even more preferably 50 mass% or less, particularly preferably 40 mass% or less.

The carbonate-based resin may be kneaded with a synthetic resin, a biodegradable resin, rubber, or the like to obtain a resin composition such as a polymer alloy. As the synthetic resin, the biodegradable resin, or the rubber, at least one resin or rubber can be used. Examples of the synthetic resin to be used include an aromatic polycarbonate, an aromatic polyester, an aliphatic polyester, a polyamide, polystyrene, a polyolefin, an acrylic, an amorphous polyolefin, an acrylonitrile-butadiene-styrene copolymer (i.e., ABS), and an acrylonitrile-styrene copolymer (i.e., AS, SAN). Examples of the biodegradable resin to be used include polylactic acid and polybutylene succinate.

In addition to the above-described another resin, additives such as a nucleating agent, a flame retarder, a flame retardant aid, an inorganic filler, an impact modifier, a hydrolysis inhibitor, a foaming agent, and a dye or pigment may be added to the carbonate-based resin to produce a resin composition. As such additives, those generally used for thermoplastic resin compositions are used.

[Method for molding thermoplastic resin having carbonate bond]

The carbonate-based resin or the resin composition containing the same is molded by a usually-known method such as injection molding, extrusion molding, or compression molding. A molded article is obtained by molding. In this way, it is possible to obtain a molded article excellent in characteristics such as heat resistance, transparency, light resistance, weatherability, and mechanical strength.

[Intended uses of thermoplastic resin having carbonate bond]

The carbonate-based resin is suitably used for films, sheets, bottles, various structural materials, optical members, etc. The carbonate-based resin containing at least the structural unit B1 is excellent in mechanical strength and heat moist resistance, and is therefore suitably used for films, sheets, bottles, materials in the field of containers, various structural materials, automobile parts, glass substitutes, and injection molding materials required to have flexibility, surface hardness, and moist heat resistance. Further, the carbonate-based resin containing at least the structural unit B1 has low photoelastic coefficient, and is therefore considered to have excellent optical reliability. For this reason, such a

carbonate-based resin is suitably used also optical films used for retardation films, diffusion sheets, polarizing films, and the like used in liquid crystal devices and organic EL devices, optical disks, binders for fixing a pigment and a charge transfer agent, and lenses used in cameras, finders, CCDs, and CMOSs.

[Reasons why effects are produced]

The reasons why the present invention produces effects are not still clear, but can be expected as follows. As described above, the carbonate-based resin containing the structural unit B1 is a novel carbonate-based resin having a specific fused-ring skeleton. Such a carbonate-based resin has a cyclobutane skeleton having a non-planar structure and two carbon ring structures sandwiching this cyclobutane skeleton.

A fused-ring structure containing a cyclobutane structure has a larger strain as compared to a fused-ring structure containing a cyclopentane structure or a cyclohexane structure and is a very rigid skeleton, and therefore a resulting resin has high heat resistance and surface hardness and causes little local structural change even when subjected to stress, which produces an excellent effect that photoelastic coefficient as one of important optical properties is low. On the other hand, a material having high heat resistance due to its rigid structure generally tends to be brittle due to its high yield stress. However, surprisingly, a carbonate-based resin having excellent toughness was obtained despite that its fused-ring structure containing a cyclobutane structure was a rigid skeleton.

It is generally known that brittle fracture of a resin is less likely to occur when the number of entanglements of the main chain per unit volume is larger, and a polymer having a bulky substituent in its side chain, such as polystyrene, has a short main chain length per molecular weight and therefore brittle fracture is likely to occur due to a reduction in the number of entanglements of the main chain per unit volume. The study by the present inventors revealed that for the same reason, the above-described carbonate-based resin using DMNDM and having a bulky substituent in its side chain had high heat resistance but was very brittle. On the other hand, the carbonate-based resin having a fused ring exhibits high heat resistance and surface hardness while having toughness because the cyclobutane structure occupies a small volume in the resin and therefore the number of entanglements of the main chain is not reduced.

[II] Next, the polymerizable alicyclic compound will be described.

[Polymerizable alicyclic compound]

The polymerizable alicyclic compound is represented by the following formula (P1), and is used as, for example, a monomer for producing a thermoplastic resin having a carbonate bond. Examples of such a thermoplastic resin include a polycarbonate resin and a polyester carbonate resin. It is noted that the thermoplastic resin having a carbonate bond can be referred to as, for example, a "carbonate-based resin". Although described later in detail, the thermoplastic resin has at least a structural unit derived from a polymerizable alicyclic compound represented by the formula (P1). As shown in the formula (P1), the polymerizable alicyclic compound has a fused ring.

[Formula 104]

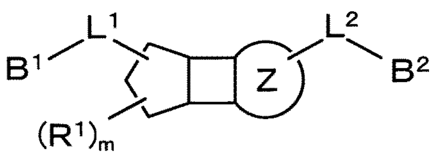

(P1)

In the polymerizable alicyclic compound represented by the formula (P1), cyclopentane (specifically, a cyclopentane skeleton) and a ring Z (specifically, an alicyclic hydrocarbon skeleton) sandwich cyclobutane (specifically, a cyclobutane skeleton) and each form a fused ring with the cyclobutane (specifically, the cyclobutane skeleton). In other words, the cyclopentane and the ring Z sandwich the cyclobutane and are fused to the cyclobutane.

In the formula (P1), the ring Z is an alicyclic carbon ring having 6 to 15 carbon atoms. The alicyclic carbon ring means a cyclic skeleton of an alicyclic compound and is a concept including not only a ring formed from carbon atoms but also a hetero ring. Since the number of carbon atoms of the alicyclic carbon ring is 6 or more, the total length of the polymerizable alicyclic compound is long to some extent. Therefore, when the polymerizable alicyclic compound is used as a monomer, it is possible to increase the bond distance between structural units. As a result, a thermoplastic resin having excellent mechanical properties such as toughness can be produced. Further, since the number of carbon atoms of the alicyclic carbon ring is 6 or more, it is possible to obtain the effect that a low water-absorption resin having excellent heat resistance can be obtained. From the viewpoint of enhancing such an effect, the number of carbon atoms of the alicyclic

carbon ring is preferably 7 or more, more preferably 9 or more. It is noted that the number of carbon atoms of the alicyclic carbon ring specifically represents the number of atoms circularly linked in the alicyclic carbon ring, and in the case of a heterocycle, the number of carbon atoms includes not only the number of carbon atoms but also the number of atoms other than carbon (specifically, hetero atoms).

Further, since the number of carbon atoms of the alicyclic carbon ring of the ring Z is 15 or less, it is possible to obtain the effect that the number of rigid bonds is reduced so that the resin can achieve both heat resistance and toughness. From the viewpoint of enhancing such an effect, the number of carbon atoms of the alicyclic carbon ring is preferably 12 or less, more preferably 10 or less.

The alicyclic carbon ring of the ring Z may have one or two or more substituents. Examples of the substituent that the alicyclic carbon ring of the ring Z may have include a hydrocarbon group having 1 to 14 carbon atoms, an acyl group having 1 to 10 carbon atoms, an alkoxy group having 1 to 10 carbon atoms, an aryloxy group having 3 to 14 carbon atoms, an acyloxy group having 1 to 10 carbon atoms, a silyl group, a sulfinyl group, a sulfo group, an alkylthio group, an arylthio group, an amino group, a halogen atom, a nitro group, and a cyano group.

Specific examples of the hydrocarbon group having 1 to 14 carbon atoms include an alkyl group having 1 to 12 carbon groups, an alkenyl group having 2 to 10 carbon atoms, an alkynyl group having 2 to 10 carbon atoms, and an aryl group having 3 to 14 carbon atoms.

Examples of the alkyl group having 1 to 12 carbon atoms include a methyl group, an ethyl group, a propyl group, an isopropyl group, and a butyl group.

Examples of the alkenyl group having 2 to 10 carbon atoms include a vinyl group, an allyl group, and a butenyl group.

Examples of the alkynyl group having 2 to 10 carbon atoms include an acetylene group and a propynyl group.

Examples of the aryl group having 3 to 14 carbon atoms include a phenyl group, a tolyl group, and a naphthyl group. Examples of the acyl group having 1 to 10 carbon atoms include an acetyl group and the like.

Examples of the alkoxy group having 1 to 10 carbon atoms include a methoxy group, an ethoxy group, and a propoxy group.

Examples of the aryloxy group having 3 to 14 carbon atoms include a phenoxy group and the like.

Examples of the acyloxy group having 1 to 10 carbon atoms include a methoxyacetyl group and a phenoxyacetyl group.

Examples of the silyl group include a trimethylsilyl group and the like.

Examples of the sulfo group include a sulfo group, a methylsulfonyl group, and an ethylsulfonyl group.

Examples of the sulfinyl group include a methyl sulfinyl group and an ethyl sulfinyl group.

Examples of the alkylthio group include a methylthio group and an ethylthio group.

Examples of the arylthio group include a phenylthio group and the like.

Examples of the amino group include an amino group and a dimethylamino group.

The substituent that the ring Z may have, such as the hydrocarbon group having 1 to 14 carbon atoms, may further have at least one substituent, and examples of such a substituent include a halogen atom, a nitro group, and a cyano group.

The upper limit of the number of substituents is determined by the structure of the ring Z. From the viewpoint that raw materials for synthesizing the polymerizable alicyclic compound are easily available, the viewpoint that the polymerizable alicyclic compound is easily synthesized, and the viewpoint that there is a possibility that an increase in the number of substituents causes a reduction in heat resistance due to a reduction in the ratio of a rigid skeleton in the skeleton of the compound, the number of substituents is preferably 0 or more and 4 or less, more preferably 0 or more and 2 or less, even more preferably 0 or 1.

From the viewpoint of increasing the ratio of a rigid skeleton in the skeleton of the compound to obtain a resin having excellent heat resistance, the alicyclic carbon ring of the ring Z preferably has no substituent and is constituted from carbon atoms bonded to hydrogen atoms, or preferably has, as a substituent, a hydrocarbon group having 1 to 14 carbon atoms. From the viewpoint of improving toughness of a resulting resin and obtaining a resin having low melt viscosity and excellent flowability during molding, the substituent is preferably an alkyl group having 5 to 12 carbon atoms. Further, from the viewpoint of improving heat resistance of a resulting resin, the substituent is preferably an alkyl group having 1 to 4 carbon atoms. From the viewpoint of further improving heat resistance and ease of synthesis of the thermoplastic resin, the substituent is preferably an alkyl group having 1 to 2 carbon atoms, more preferably an alkyl group having 1 carbon atom (specifically, a methyl group). Further, from the viewpoint that heat resistance of the thermoplastic resin such as a polycarbonate is further improved and raw materials are inexpensively available, it is preferred that the alicyclic carbon ring in the ring Z has one methyl group as a substituent and other carbon atoms constituting the alicyclic carbon ring (i.e., carbon atoms other than a carbon atom bonded to the substituent) are bonded to hydrogen atoms without being bonded to substituents. Further, from the viewpoint that the thermoplastic resin can achieve both excellent heat resistance and toughness and raw materials are inexpensively available, the alicyclic carbon ring of the ring Z preferably has no substituent. However, "-$L^2$-$B^2$" in the formula (P1) is a polymerizable reactive group and is not regarded as a substituent.

From the viewpoint of improving heat resistance of the thermoplastic resin and reactivity of the compound during polymerization, the substituent that the alicyclic carbon ring of the ring Z has is preferably an alkoxy group having 1 to 10 carbon atoms, and from the same viewpoint, the substituent is more preferably an alkoxy group having 1 to 3 carbon atoms, and from the viewpoint of more excellent heat resistance, the substituent is even more preferably a methoxy group.

When a dihydroxy compound is used for polymerization and a hydrogen atom on the β carbon atom relative to a hydroxy group is substituted by a substituent such as an alkyl group, it is possible to prevent elimination of the hydroxy group from a monomer in the production of the resin. This makes it possible to further increase a polymerization temperature to obtain a resin having a larger molecular weight. Therefore, when $L^2$ is, for example, a methylene group, the carbon atom of the ring Z bonded to $L^2$ is preferably further bonded to a substituent.

The alicyclic carbon ring of the ring Z may contain a hetero atom. Examples of the hetero atom include an oxygen atom, a sulfur atom, a nitrogen atom, and a halogen atom. From the viewpoint of imparting polarity to the polymerizable alicyclic compound represented by the formula (P1), the hetero atom is preferably an oxygen atom and/or a sulfur atom. In this case, polarity is imparted also to a structural unit derived from the polymerizable alicyclic compound and constituting the thermoplastic resin. Therefore, for example, when the thermoplastic resin and a dissimilar material resin are mixed, it is possible to improve affinity between the thermoplastic resin and the dissimilar material resin and compatibility between the dissimilar material resin and the thermoplastic resin in blending. The structural unit derived from the polymerizable alicyclic compound represented by the formula (P1) is referred to as a "structural unit B0" as appropriate. The alicyclic carbon ring may contain a hetero atom as, for example, the functional group described above. Further, the alicyclic carbon ring may contain a hetero atom in a cross-linked structure described later. From the viewpoint that the polymerizable alicyclic compound can easily be synthesized, the viewpoint that a condensation polymerization reaction is less likely to be inhibited in production of the thermoplastic resin, and the viewpoint that the polarity of a resulting resin can be reduced to reduce a water absorption rate, the ring Z preferably contains no hetero atom.

The alicyclic carbon ring of the ring Z preferably has at least one carbon ring constituted from 4 or more carbon atoms. In this case, the effect that excellent heat resistance is achieved by a rigid skeleton is obtained.

In this specification, the alicyclic carbon ring is a concept that includes one having a fused ring. The alicyclic carbon ring of the ring Z may have a fused ring. In this case, as described above, the number of carbon atoms of the alicyclic carbon ring containing a fused ring is 6 to 15. When the alicyclic carbon ring contains a fused ring, it is possible to increase the bond distance between monomer units constituting the thermoplastic resin (specifically, the bond distance between the structural units B1). This makes it possible to improve mechanical properties, such as toughness, of the thermoplastic resin.

The alicyclic carbon ring preferably has, like a norbornane skeleton or the like, a structure having a ring formed by bonding a hydrocarbon group having 5 or less carbon atoms and bonded to a carbon atom constituting the alicyclic carbon ring to another carbon atom constituting the alicyclic carbon ring. That is, the alicyclic carbon ring preferably has a structure formed by cross-linking a hydrocarbon group having 5 or less carbon atoms to an alicyclic hydrocarbon ring skeleton. In this case, it is possible to obtain the effect that excellent heat resistance and pencil hardness are achieved by a rigid skeleton. From the viewpoint of improving such an effect, the number of carbon atoms of the hydrocarbon group of the cross-linked structure is more preferably 3 or less, even more preferably 2 or less.

Further, the alicyclic carbon ring preferably has, like oxabicyclo[2.2.1]heptane or the like, a structure having a ring formed by bonding an oxygen atom bonded to a carbon atom constituting the alicyclic carbon ring to another carbon atom constituting the alicyclic carbon ring. That is, the alicyclic carbon ring preferably has a structure formed by cross-linking an oxygen atom to an alicyclic hydrocarbon skeleton (i.e., a cyclic ether bond). In this case, it is possible to obtain the effect that compatibility with a dissimilar polymer improves due to high polarity of the hetero atom. Further, the alicyclic carbon ring of the ring Z preferably has, like thiabicyclo [2.2.1]heptane or the like, a structure formed by cross-linking a sulfur atom to an alicyclic hydrocarbon skeleton (i.e., a cyclic thioether bond). In this case, it is possible to obtain the effect that compatibility with a dissimilar polymer improves due to high polarity of the hetero atom.

In the formula (P1), $L^1$ and $L^2$ are each independently a direct bond or a divalent hydrocarbon group having 1 to 5 carbon atoms. The hydrocarbon group is, for example, an alkylene group, and may have a substituent having 1 to 5 carbon atoms. Examples of the substituent include a methyl group, an ethyl group, a propyl group, a butyl group, and an isopropyl group.

From the viewpoint that the polymerizable alicyclic compound can easily be synthesized, the viewpoint that a condensation polymerization reaction is less likely to be inhibited in production of the thermoplastic resin, and the viewpoint that mobility improves and heat resistance reduces due to the presence of the substituent, it is preferred that $L^1$ and $L^2$ have no substituent. Further, from the viewpoint of further improving toughness and molding processability (specifically, melt flowability during molding) of the thermoplastic resin, the number of carbon atoms of each of the hydrocarbon groups of $L^1$ and $L^2$ is preferably 3 to 5. On the other hand, from the viewpoint that the thermoplastic resin has further improved heat resistance due to the formation of a rigid skeleton while having toughness and molding processability (specifically, melt flowability during molding) further improves, the number of carbon atoms of each of the hydrocarbon groups of $L^1$ and $L^2$ is preferably 1 to 2. Further, from the viewpoint that raw materials for synthesizing a

monomer in production of the thermoplastic resin are inexpensive, $L^1$ and $L^2$ are preferably methylene groups. A methylene group is also referred to as a methanediyl group.

The bonding site of $L^1$ in the formula (P1) is not limited as long as $L^1$ is bonded to a carbon atom forming the cyclopentane skeleton, and $L^1$ may be bonded to a carbon atom in a site where the cyclopentane and the cyclobutane are fused. Similarly, the bonding site of $L^2$ in the formula (P1) is not limited as long as $L^2$ is bonded to a carbon atom forming the alicyclic hydrocarbon skeleton of the ring Z, and $L^2$ may be bonded to a carbon atom in a site where the alicyclic carbon ring of the ring Z and the cyclobutane are fused, or, when the alicyclic carbon ring itself of the ring Z has a fused ring, may be bonded to a ring fusion site in the alicyclic carbon ring.

$B^1$ and $B^2$ are functional groups (specifically, a polymerization reactive group) respectively bonded to $L^1$ and $L^2$. $B^1$ and $B^2$ are hydroxy groups. That is, the polymerizable alicyclic compound is a diol (i.e., a dihydroxy compound) and is used as a monomer for producing a polycarbonate resin or a polyester carbonate resin.

$L^1$ and $L^2$ in the formula (P1) are preferably methylene groups. In this case, "$-L^1-B^1$" and "$-L^2-B^2$" in the formula (P1) are hydroxymethyl groups. In this case, it is possible to obtain the effects that raw materials are inexpensively available, synthesis is easily performed, reactivity during polymerization improves, and a resulting resin has both heat resistance and toughness.

From the viewpoint of improving heat resistance of the thermoplastic resin and reactivity of the compound during polymerization, it is preferred that $L^1$ and $L^2$ in the formula (P1) are direct bonds and $B^1$ and $B^2$ are hydroxy groups. In this case, "$-L^1-B^1$" and "$-L^2-B^2$" in the formula (P1) are hydroxy groups. In this case, it is possible to obtain the effects that reactivity during polymerization improves and a resulting resin has improved heat resistance. When "$-L^1-B^1$" and "$-L^2-B^2$" in the formula (P1) are hydroxy groups, it is more preferred that an alkoxy group is bonded to a carbon atom adjacent to a carbon atom of the carbon ring to which each of $L^1$ and $L^2$ is bonded. In this case, heat resistance of the resin further improves.

$R^1$ in the formula (P1) is a substituent bonded to the cyclopentane (specifically, the cyclopentane skeleton), and m is the number of the substituents. When the number of the substituents $R^1$ is two or more, the substituents may be the same or different from each other. The bonding site of the substituent in the cyclopentane is not limited as long as the substituent is bonded to a carbon atom forming the cyclopentane skeleton, and the substituent may be bonded to a carbon atom in a site where the cyclopentane and the cyclobutane are fused. When a hydrogen atom on the $\beta$ carbon atom relative to a hydroxy group is substituted by an alkyl group, it is possible to prevent elimination of the hydroxy group from the monomer in production of the resin. This makes it possible to further increase a polymerization temperature to obtain a resin having a larger molecular weight. Therefore, when $L^1$ is, for example, a methylene group, a substituent is preferably bonded to a carbon atom constituting the cyclopentane skeleton and bonded to $L^1$. The details of the substituent $R^1$ are the same as those of the above-described substituent of the alicyclic carbon ring of the ring Z.

m in the formula (P1) is an integer of 0 to 4. From the viewpoint that raw materials for synthesizing the polymerizable alicyclic compound are easily available, the viewpoint that the polymerizable alicyclic compound is easily synthesized, and the viewpoint of increasing the ratio of a rigid skeleton in the skeleton of the compound to enhance heat resistance, m is preferably 1 or 0. When m is 0, the cyclopentane has no substituent $R^1$ and has no substituent except for "$-L^1-B^1$" in the formula (P1) as a polymerization reactive group. That is, in this case, hydrogen atoms are bonded to carbon atoms constituting the cyclopentane except for "$-L^1-B^1$".

The ring Z in the formula (P1) preferably has an alicyclic skeleton other than a cyclopentane ring. That is, the polymerizable cyclic compound preferably has a structure in which the cyclopentane ring and the ring Z are asymmetric relative to the cyclobutane ring sandwiched between them. In this case, it is possible to obtain the effect of improving solubility, which is advantageous for synthesis of a monomer or formation of a polymer. Further, it is possible to obtain the effect of capable of reducing the photoelastic coefficient of the carbonate-based resin having the structure represented by the formula (B1).

The polymerizable alicyclic compound is preferably any one selected from among compounds represented by the following formulas (P2) to (P11). In this case, it is possible to easily produce the polymerizable alicyclic compound by a production method described later. Further, in this case, it is possible to obtain the effect that heat resistance, toughness, and low water absorption are achieved.

[Formula 105]

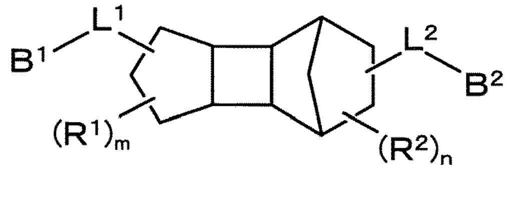

(P2)

[Formula 106]

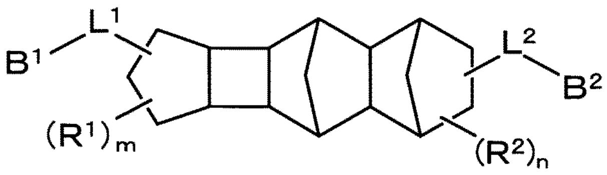

(P3)

[Formula 107]

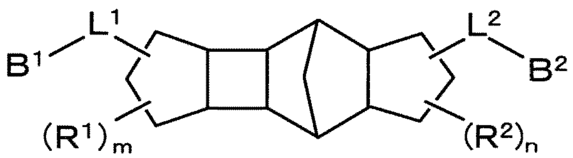

(P4)

[Formula 108]

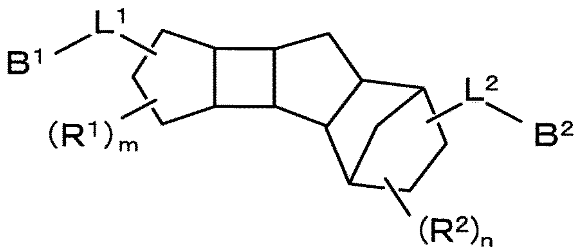

(P5)

[Formula 109]

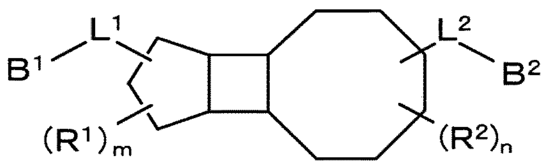

(P6)

[Formula 110]

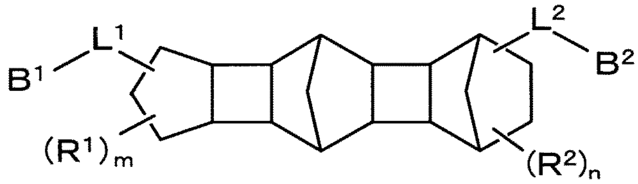

(P7)

[Formula 111]

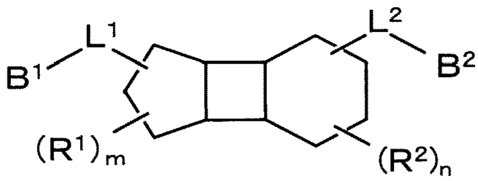

(P8)

[Formula 112]

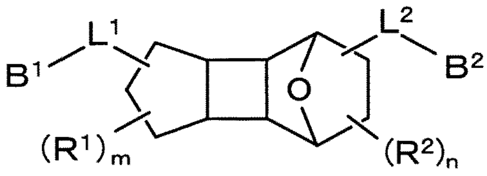

(P9)

[Formula 113]

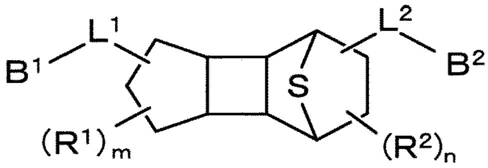

(P10)

[Formula 114]

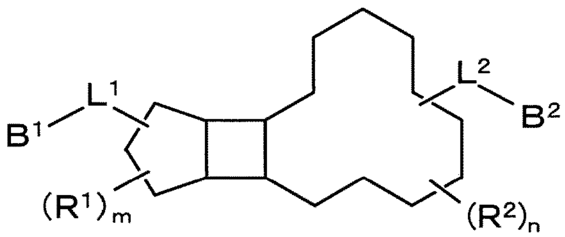

(P11)

Each of the formulas (P2) to (P11) represents the structure of the formula (P1) in which the ring Z fused to the cyclobutane has a specific alicyclic carbon ring skeleton. The details of $R^1$, $L^1$, $L^2$, $B^1$, $B^2$, and m in the formulas (P2) to (P11) are the same as those in the formula (P1). $R^2$ in the formulas (P2) to (P11) is synonymous with the above-described substituent that may be bonded to the alicyclic carbon ring of the ring Z in the formula (P1). n is the number of the substituents $R^2$, the lower limit of n is 0, and the upper limit of n varies depending on the number of carbon atoms of the alicyclic carbon ring corresponding to the ring Z. Specifically, n in the formula (P2) is an integer of 0 to 6. n in the formula (P3) and the formula (P11) is an integer of 0 to 11. n in the formula (P4) and the formula (P5) is an integer of 0 to 9. n in the formula (P6) is an integer of 0 to 7. n in the formula (P7) is an integer of 0 to 13. n in the formulas (P8) to (P10) is an integer of 0 to 5.

When represented by the formula (P2), the polymerizable alicyclic compound has an appropriate rigid skeleton, and therefore it is possible to obtain the effect that minimum heat resistance and high toughness are achieved.

When represented by the formula (P3), the polymerizable alicyclic compound has two rigid norbornene skeletons, and therefore it is possible to obtain the effect that high heat resistance and surface hardness and low photoelastic coefficient are achieved.

When represented by the formula (P4), the polymerizable alicyclic compound has a ring having a cross-linked structure as a rigid skeleton and a ring having no cross-linked structure in good balance and contains many non-polar carbon atoms due to ring fusion, and therefore it is possible to obtain the effect that heat resistance, surface hardness, photoelastic coefficient, and toughness are achieved at the same time.

When represented by the formula (P5), the polymerizable alicyclic compound has a ring having a cross-linked structure as a rigid skeleton and a ring having no cross-linked structure in good balance and contains many non-polar carbon atoms due to ring fusion, and therefore it is possible to obtain the effect that heat resistance, surface hardness, photoelastic coefficient, and toughness are achieved at the same time.

When represented by the formula (P6), the polymerizable alicyclic compound has an appropriate rigid skeleton, and therefore it is possible to obtain the effect that minimum heat resistance and high toughness are achieved.

When represented by the formula (P7), the polymerizable alicyclic compound has two rigid norbornene skeletons, and therefore it is possible to obtain the effect that high heat resistance, high surface hardness, and low photoelastic coefficient are achieved.

When represented by the formula (P8), the polymerizable alicyclic compound has a flexible alicyclic structure, and therefore it is possible to obtain the effect that minimum heat resistance and high toughness are achieved.

When represented by the formula (P9), the polymerizable alicyclic compound has a rigid skeleton and a hetero atom having high polarity, and therefore it is possible to obtain the effect of improving heat resistance and compatibility with a dissimilar polymer in blending.

When represented by the formula (P10), the polymerizable alicyclic compound has a rigid skeleton and a hetero atom having high polarity, and therefore it is possible to obtain the effect of improving heat resistance and compatibility with a dissimilar polymer in blending.

When represented by the formula (P11), the polymerizable alicyclic compound has a flexible alicyclic structure, and therefore it is possible to obtain the effect that minimum heat resistance and high toughness are achieved.

$R^1$ and $R^2$ in the formulas (P2) to (P11) are both preferably hydrocarbon groups having 1 to 14 carbon atoms, more preferably alkyl groups having 1 to 12 carbon atoms, even more preferably alkyl groups having 1 to 5 carbon atoms, particularly preferably methyl groups. In this case, the ratio of a rigid skeleton in the skeleton of the compound increases, and therefore it is possible to obtain the effect that high heat resistance is achieved.

It is preferred that m and n in the formulas (P2) to (P11) are 0 or 1. In this case, the ratio of a rigid skeleton in the skeleton of the compound increases, and therefore it is possible to obtain the effect that high heat resistance is achieved.

Among the compounds of the formulas (P2) to (P11), any one selected from among the compounds of the formulas (P2) to (P7) is preferred. In this case, the compound has a rigid norbornene skeleton and a flexible alicyclic structure, and therefore it is possible to obtain the effect that heat resistance and toughness are both achieved. Further, from the viewpoint that synthesis is easily performed and the viewpoint that heat resistance and toughness are both achieved, the formula (P4) is preferred.

From the viewpoint that synthesis can easily and inexpensively be performed, the polymerizable alicyclic compound is preferably any one selected from a group (I-1) consisting of compounds shown below.

[Formula 115]

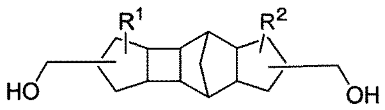

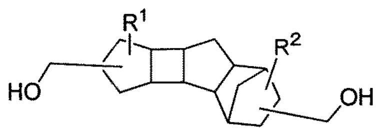

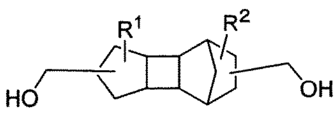

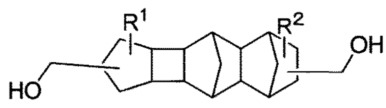

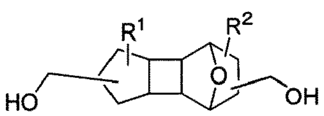

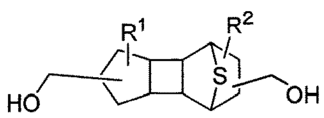

(I−1)

Further, compounds of a group (I-2) shown below have a polar atom (specifically, O in an ether structure or S in a thioether structure), and therefore when any compound selected from the group (I-2) is used as a monomer, polarity is imparted to the thermoplastic resin. Therefore, for example, when the thermoplastic resin is mixed with a dissimilar material resin, it is possible to improve affinity between the thermoplastic resin and the dissimilar material resin and improve adhesiveness at an interface between the dissimilar material resin and the thermoplastic resin. In order to sufficiently obtain such an effect, it is preferred that the dissimilar material resin also has polarity. That is, from the viewpoint that affinity or adhesiveness with the dissimilar material resin can be improved, the polymerizable alicyclic compound is preferably any one selected from the group (I-2).

[Formula 116]

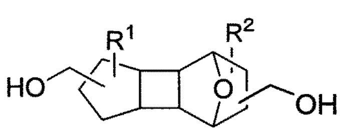
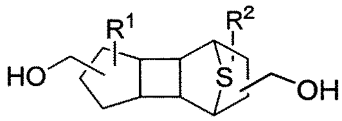

(I-2)

From the viewpoint of obtaining a thermoplastic resin having a low water absorption rate, among the compounds of the group I-1, any one selected from a group (I-3) consisting of compounds shown below is preferred.

[Formula 117]

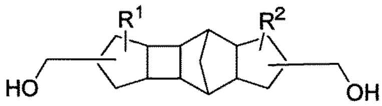
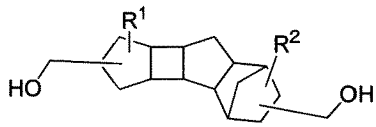
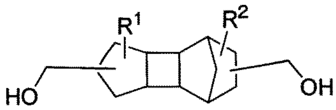
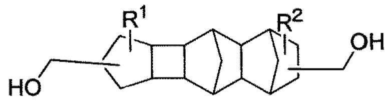

(I-3)

From the viewpoint of obtaining a thermoplastic resin having excellent heat resistance and toughness and the viewpoint that raw materials are inexpensively available, among the compounds of the group (I-1), any one selected from a group (I-4) consisting of compounds shown below is preferred.

[Formula 118]

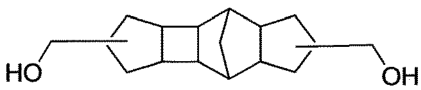
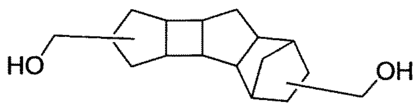
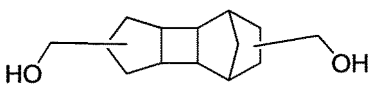
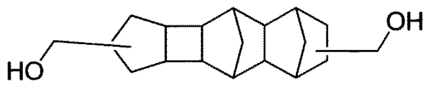

(I-4)

From the viewpoint of obtaining a thermoplastic resin having excellent heat resistance, the formula (P1) is preferably any one selected from a group (Z-1) consisting of compounds shown below.

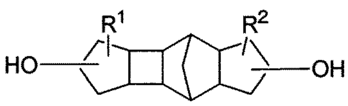
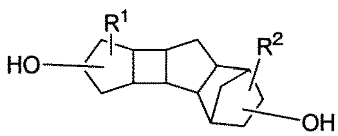
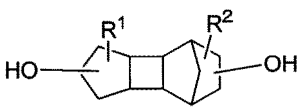
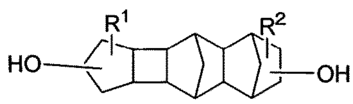

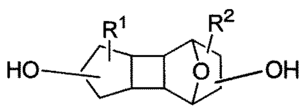 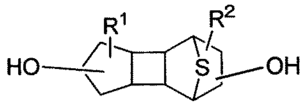 (Z−1)

$R^1$ and $R^2$ are each independently a hydrogen atom, a methyl group, a methoxy group, an ethoxy group, or a propoxy group.

From the viewpoint that heat resistance is achieved and high reactivity is exhibited in polymerization, the formula (P1) is particularly preferably represented by the following formula (Z-2).

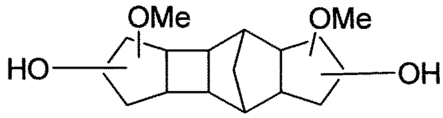 (Z−2)

In the formula (Z-2), OMe is a methoxy group. A carbon atom bonded to the hydroxy group and a carbon atom bonded to the methoxy group are preferably carbon atoms that constitute the same cyclopentane skeleton and are adjacent to each other. That is, in the formula (Z-2), it is preferred that the hydroxy group and the methoxy group are bonded to any two respective carbon atoms adjacent to each other in the cyclopentane skeleton.

<Method for synthesizing polymerizable alicyclic compound>

A method for producing the polymerizable alicyclic compound is not particularly limited. When the polymerizable alicyclic compound represented by the formula (P1) is a dihydroxy compound, that is, when $B^1$ and $B^2$ in the formula (P1) are hydroxy groups, the polymerizable alicyclic compound is produced by, for example, any of production methods A to C shown in Fig. 9 to Fig. 11.

In Fig. 9 to Fig. 11, a compound (M1) is a compound having a cyclopentadiene skeleton (i.e., a cyclopentadiene) and has two double bonds in a five-membered ring alicyclic structure. Details of $R^1$ and m in the compound M1 are the same as those in the formula (P1). A ring Z1 is an alicyclic carbon ring having 6 to 15 carbon atoms and has two double bonds in an alicyclic carbon ring skeleton. This alicyclic carbon ring may have a substituent, may contain a hetero atom, and may have a fused ring. Details of the substituent, the hetero atom, and the fused ring are the same as those described above with reference to the ring Z in the formula (P1).

Further, in Fig. 9 to Fig. 11, a ring Z2 is an alicyclic carbon ring having 6 to 15 carbon atoms and has one double bond in an alicyclic carbon ring skeleton. This alicyclic carbon ring may have a substituent, may contain a hetero atom, and may have a fused ring. Details of the substituent, the hetero atom, and the fused ring are the same as those described above with reference to the ring Z in the formula (P1).

In Fig. 9 to Fig. 11, $M^1$ and $M^2$ are each independently a direct bond or a divalent hydrocarbon group having 1 to 4 carbon atoms. The hydrocarbon group may have a substituent having 1 to 5 carbon atoms. Specifically, the divalent hydrocarbon group is an alkylene group. Details of $L^1$ and $L^2$ are the same as those described above with reference to the formula (P1).

As shown in Fig. 9, a compound (M3) is produced from the compound (M1) having unsaturated bonds and the compound (M2) having unsaturated bonds. Specifically, two unsaturated molecules, one of which has 2m $\pi$ electrons and the other of which has 2n $\pi$ electrons, such as the compound (M1) and the compound (M2), form a metallacycle (not shown) in the presence of a transition metal active species (specifically, a metallic catalyst) due to formation of two metal-carbon bonds and one carbon-carbon bond and cyclization. At this time, the valence of a metallic center formally increases by 2 due to its oxidation state. Then, reductive elimination occurs so that a carbon-carbon bond is formed, and the oxidation state of the transition metal center is reduced and an active species is regenerated. This allows a plurality of reactions, such as multimerization of unsaturated molecules, to proceed. At this time, when Pd or Ni is used, a four-membered ring is formed (see "Organotransition Metal Catalyzed Cycloaddition" written by Itoh Kenji, 2002, vol. 60. No. 1, p. 28 - p. 41). Further, when an Fe catalyst is used, a four-membered ring is formed from two unsaturated molecules (see Jordan M. Hoyt et al., "Iron-catalyzed intermolecular [2+2]cycloadditions of unactivated alkenes", "Science" Vol. 349, Issue 6251, Aug. 28, 2015, p. 960 - p. 963). Further, a four-membered ring is formed by using Ni in an unsaturated structure having a hetero atom at a bridgehead position or another position, such as 1,4-dihydro-epoxynaphthalene (see Daw-Jen Huang et al., "[2+2] Dimerization of norbornadiene and its derivatives in the presence of nickel complexes and zinc metal", "Journal of Organometallic Chemistry", 490, 1995, C1 - C7).

In the production methods shown in Fig. 9 to Fig. 11, the compound (M1) is a cyclic diene compound having a cyclopentadiene skeleton. As the compound (M2), a compound (M5), and a compound (M8), for example, compounds having a distorted unsaturated bond such as a norbornene skeleton can be used. When the compound having a

cyclopentadiene skeleton and the compound having a norbornene skeleton are used as raw materials, reactivity of the raw materials increases so that a four-membered ring structure (specifically, cyclobutane) contained in the formula (P1) is easily formed.

Further, the compound having a distorted unsaturated bond such as a norbornene skeleton forms a four-membered ring by using a photocyclization reaction. In fact, it is known that dicyclopentadiene is dimerized by light irradiation in the presence of a metallic catalyst (see Robert G. Salomon et al., "Copper(I) catalysis in photocycloadditions. I. Norbornene", "Journal of the American Chemical Society", 1974, 96, 4, p. 1137). Therefore, a compound having a four-membered ring can also be produced using a photocyclization reaction. However, the yield of photoreaction is generally low, and photoreaction is not suitable for synthesis of an alicyclic compound having a structure asymmetric with respect to a four-membered ring, such as alicyclic compounds shown in Fig. 9 to Fig. 11 and Fig. 12.

As shown in Fig. 9, the production method A is a method in which a compound (M1) and a compound (M2) are used as starting materials of a cyclization reaction. In the production method A, a cyclization reaction between the compound (M1) and the compound (M2) occurs so that a compound (M3) is obtained. Then, an aldehyde is added to the compound (M3) by an oxo reaction to obtain a compound (M4). Then, the compound (M4) is subjected to a hydrogenation reaction (i.e., a reduction reaction) to obtain a compound (P1-1).

In the production method A, the cyclization reaction between the compound (M1) and the compound (M2) uses, as a transition metal catalyst, $Pd(dba)_2$, $Pd(acac)_2$, $Pd(PPh_3)_4$, $PdCl_2(PPh_3)_2$, $PdCl_2(dppp)$, $PdCl_2(H_2NCH_2CH_2NH_2)$, $NiCl_2$, $NiBr_2$, $NiCl_2(PPh_3)_2$, or $Ni(Cod)_2$. As a ligand, $P(p\text{-tolyl})_3$, $PPh_3$, $P[(p\text{-MeO})Ph]_3$, or $P(Cy)_3$ is used. However, a ligand may not be used. As a reducing agent, for example, zinc can be used. A reaction temperature is, for example, 60 to 200°C. As a solvent, acetonitrile, tetrahydrofuran (THF), toluene, xylene, trimethylbenzene, tetralin, decalin, or the like is used. When a low-boiling solvent is used, pressure may be applied. For example, the reaction is performed under a pressure condition of 0.5 MPa to 10 MPa. During the reaction, for the purpose of adjusting pH, acetic acid, propionic acid, 4-methoxybenzoic acid, benzoic acid, formic acid, chloroacetic acid, trifluoroacetic acid, pyridinium p-toluenesulfonate (PPTS), tosic acid, methanesulfonic acid, or the like is used.

The compound (M4) shown in Fig. 9, in which $M^1$ and $M^2$ are direct bonds, is obtained from the compound (M3) by, for example, an oxo reaction. A reaction temperature is, for example, 20 to 200°C. A rhodium compound used in this step may be in any form as long as it forms a complex with an organophosphorus compound and exhibits hydroformylation activity in the presence of hydrogen and carbon monoxide, and may be in the form of a precursor. That is, a catalyst precursor such as $Rh(acac)(CO)_2$, $Rh_2O_3$, $Rh_4(CO)_{12}$, $Rh_6(CO)_{16}$, or $Rh(NO_3)_3$ may be introduced into a reaction mixture together with an organophosphorus compound to form a rhodium hydride carbonyl phosphorus complex having catalytic activity in a reaction container, or a rhodium hydride carbonyl phosphorus complex catalyst previously prepared may be introduced into a reaction container. A amount of the rhodium catalyst in terms of the amount of rhodium metal is preferably 50 to 50000 ppm, more preferably 50 to 2000 ppm with respect to the amount of the compound (M3) as a raw material. The mole ratio between the ligand and the rhodium metal (ligand/rhodium) is in the range of 1 to 50. Examples of the organophosphorus compound include a phosphine represented by $P(-R_1)(-R_2)(-R_3)$ and a phosphite represented by $P(-OR_1)(-OR_2)(-OR_3)$. Specific examples of $R_1$, $R_2$, and $R_3$ include an optionally-substituted alkyl group having 1 to 12 carbon atoms, an optionally-substituted alicyclic alkyl group having 1 to 12 carbon atoms, and an optionally-substituted aryl group having 1 to 12 carbon atoms. Preferred specific examples of the phosphite used in this step include tris(2-t-butylphenyl) phosphite, tris(3-methyl-6-t-butylphenyl) phosphite, tris(3-methoxy-6-t-butylphenyl) phosphite, tris(2,4-di-t-butylphenyl) phosphite, and di(2-t-butylphenyl)t-butyl phosphite. The organophosphorus compound is not limited to these phosphine and phosphite. Further, these phosphites may be used singly or in combination of two or more of them. As a solvent, methylcyclohexane, cyclohexane, toluene, xylene, trimethylbenzene, an aliphatic hydrocarbon having 5 or more carbon atoms, ethylbenzene, propanol, butanol, or the like is used. A pressure condition during the reaction is, for example, 1 MPa to 15 MPa. The mole ratio between hydrogen and carbon monoxide in a hydrogen/carbon monoxide mixed gas used in the reaction is preferably in the range of 0.2 to 5.0 in terms of an introduced gas composition (hydrogen/carbon monoxide). The synthesis of (M4) containing aldehyde groups is not limited to the oxo reaction, and may be performed by another known reaction.

The compound (M4) can be converted to a compound (P1-1) by a reduction reaction. Examples of a transition metal catalyst for hydrogen reduction to be used include metallic catalysts such as the above-described Rh catalysts preferably used in the oxo reaction, known metals belonging to Group VIII of the Periodic Table and having hydrogen reduction ability, such as nickel, cobalt, ruthenium, palladium, and platinum, copper chromite, and copper-zinc. These metallic catalysts may be used as an elemental metal or a metal oxide, or may be used in the form of an elemental metal or a metal oxide supported by an inorganic carrier such as silica, alumina, diatomite, or carbon, or may be used in the form of a metallic complex. From the viewpoint of a hydrogen reduction reaction speed and catalyst separation after reaction, among these hydrogenation catalysts, a Rh catalyst, raney nickel, nickel/diatomite, copper chromite, ruthenium/carbon, or a ruthenium/alumina catalyst is particularly suitably used. As a solvent, methylcyclohexane, cyclohexane, toluene, xylene, trimethylbenzene, an aliphatic hydrocarbon having 5 or more carbon atoms, ethylbenzene, propanol, butanol, or the like is used. A pressure condition during the reaction is, for example, 1 MPa to 15 MPa. As a ligand, the above-described ligand

preferably used in the oxo reaction may be used, or a ligand may not be used. The mole ratio between hydrogen and carbon monoxide in a carbon monoxide mixed gas is preferably in the range of 0.2 to 5.0 in terms of an introduced gas composition (hydrogen/carbon monoxide), or only hydrogen gas is preferably used. It is noted that after the oxo reaction, hydrogen reduction may be performed without performing quenching. In this case, the same catalyst as that used in the oxo reaction step is added, or a preferred catalyst is separately added. The synthesis of (P1-1) from (M4) containing aldehyde groups is not limited to the hydrogen reduction reaction, and may be performed by another known reaction. An example of another known reaction includes a reduction reaction using a reducing agent such as sodium boron hydride. The reducing agent such as sodium boron hydride is used together with the above-described solvent used in the reduction step or an alcohol solvent such as methanol or ethanol.

Abbreviations for ligands are as follows.

dba: dibenzylideneacetone
acac: acetylacetonate
PPh: phenylphosphine
dppp: diphenylphosphinopropane
tolyl: methylphenyl group
Cy: cyclohexyl group
Me: methyl group
Ph: phenyl group

As shown in Fig. 10, the production method B is a method in which a compound (M1) and a compound (M5) are used as starting materials of a cyclization reaction. Unlike the compound (M2) in the production method A, the compound (M5) in the production method B has only one unsaturated bond, and therefore excessive cyclization is prevented in the production method B. In this point, the production method B is preferably performed. A compound (M6) is obtained by a cyclization reaction between the compound (M1) and the compound (M5). Then, an aldehyde is added to the compound (M6) by, for example, an oxo reaction to obtain a compound (M7). Then, the compound (M7) is subjected to a hydrogenation reaction to obtain a compound (P1-1). The reaction conditions of the cyclization reaction, the oxo reaction, and the hydrogenation reaction are the same as those in the production method A.

As shown in Fig. 11, the production method C is a method in which a compound (M1) and a compound (M8) are used as starting materials of a cyclization reaction. Unlike the compound (M2) in the production method A, the compound (M8) in the production method C has only one unsaturated bond, and therefore excessive cyclization is prevented in the production method C. In this point, the production method C is preferably performed. A compound (M9) is obtained by a cyclization reaction between the compound (M1) and the compound (M8). Then, an aldehyde is added to the compound (M9) by, for example, an oxo reaction to obtain a compound (M10). Then, the compound (M10) is subjected to a hydrogenation reaction to obtain a compound (P1-1). The reaction conditions of the cyclization reaction, the oxo reaction, and the hydrogenation reaction are the same as those in the production method A.

It is noted that in Fig. 9 to Fig. 11, substituents that the ring Z1, the ring Z2, and the ring Z may have are not shown, but a compound (P1-1) having a substituent bonded to the alicyclic carbon ring of the ring(Z) can be produced by, for example, using a compound having a substituent as the compound (M2), the compound (M5), or the compound (M8).

Dihydroxy compounds represented by, for example, the following formulas (P21) to (P34) can be obtained by the above-described production methods A to C.

[Formula 119]

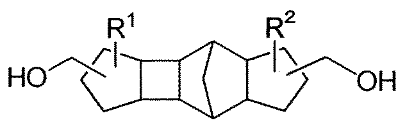

(P21)

[Formula 120]

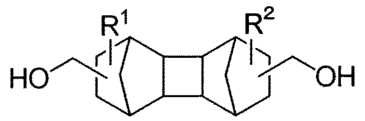

(P22)

[Formula 121]

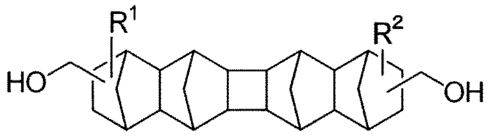

(P23)

[Formula 122]

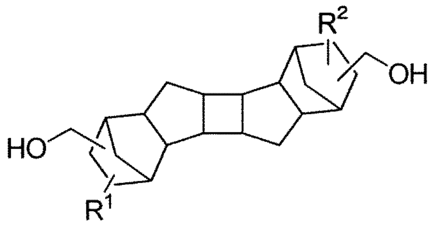

(P24)

[Formula 123]

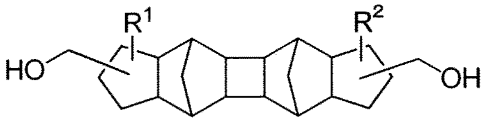

(P25)

[Formula 124]

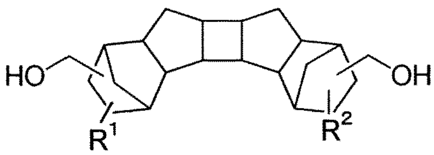

(P26)

[Formula 125]

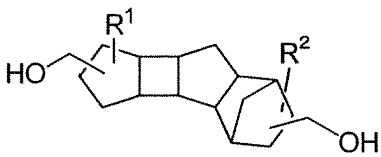

(P27)

[Formula 126]

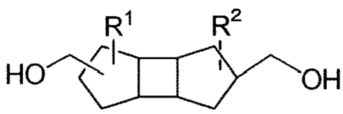

(P28)

[Formula 127]

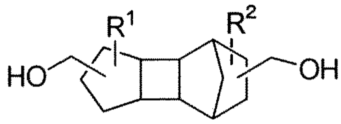

(P29)

[Formula 128]

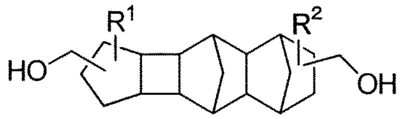

(P30)

[Formula 129]

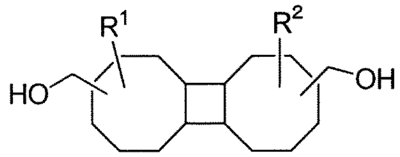

(P31)

[Formula 130]

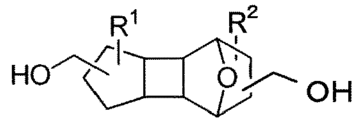

(P32)

[Formula 131]

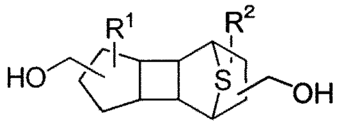

(P33)

[Formula 132]

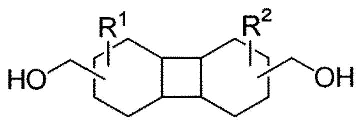

(P34)

As a specific example of the compound represented by the formula (P1), a dihydroxy compound represented by a formula (P4-1) shown in Fig. 12 (hereinafter, referred to as a compound (P4-1)) can be mentioned as appropriate. As shown in Fig. 12, the dihydroxy compound represented by the formula (P4-1) is obtained by, for example, the production method A. Specifically, a compound (N3) and a compound (N4) are obtained by a cyclization reaction between a compound (N1) and a compound (N2). It is noted that also when only (N1) is used as a starting material without using (N2) in this step, (N2) is generated due to the occurrence of cracking in the reaction, and as a result, (N3) and (N4) can be obtained by a cyclization reaction between the compound (N1) and the compound (N2). Further, also when only (N2) is used as a starting material without using (N1) in this step, (N1) is generated due to the occurrence of dimerization of (N2) in the reaction, and as a result, (N3) and (N4) can be obtained by a cyclization reaction between the compound (N1) and the compound (N2) (see Kyung-sun Son, "Selective synthesis of tricyclopentadiene from dicyclopentadiene with homogeneous Pd catalysts", "Applied Organometallic Chemistry", 2014, Vol. 28, p.151-155). Then, a compound (N5) is obtained from the compound (N3) and the compound (N4) by an oxo reaction, and a compound (P4-1) is obtained by a reduction reaction of the compound (N5). It is noted that Fig. 12 shows an example using a compound not having a substituent $R^1$ and a compound not having a substituent $R^2$, but as described above, a dihydroxy compound having substituents can be obtained by, for example, using compounds having a substituent as the compound (N1) and the compound (N2).

Fig. 13 shows starting materials used for synthesis of compounds (P2) to (P5), a compound (P9), and a compound (P10) as examples of the compound (P1). In Fig. 13, $R^1$, $R^2$, $B^1$, $B^2$, $L^1$, and $L^2$ are the same as those in the

formula (P1), and $L^1$ and $L^2$ are, for example, methylene groups and $B^1$ and $B^2$ are hydroxy groups.

The compound (P4) shown in Fig. 13 is produced by the production method A in which only a compound (Q1) or a compound (Q2) is used as a starting material or the compound (Q1) and the compound (Q2) are used as starting materials. The compound (P5) is produced by the production method B or C in which a compound (Q1) and a compound (Q3) are/is used as raw materials. The compound (P2) is produced by the production method B or C in which a compound (Q1) and a compound (Q4) are used as raw materials. The compound (P3) is produced by the production method B or C in which a compound (Q1) and a compound (Q5) are used as raw materials. The compound (P9) is produced by the production method B or C in which a compound (Q1) and a compound (Q6) are used as raw materials. The compound (P10) is produced by the production method B or C in which a compound (Q1) and a compound (Q7) are used as raw materials. Other compounds represented by the formula (P1) can be produced by, for example, the production methods A to C by appropriately selecting starting materials.

Further, when the compound (M3) is epoxidized and subjected to ring opening using a solvent such as an alcohol, a compound (W2) or (W3) are obtained in which $L^1$ and $L^2$ are direct bonds and hydroxy groups are contained. Preferred examples of an oxidizing agent used for epoxidization include m-chlorobenzoic acid (mCPBA), hydrogen peroxide, and peracetic acid, and examples of the alcohol used for ring opening include methanol, ethanol, and propanol.

[Formula 133]

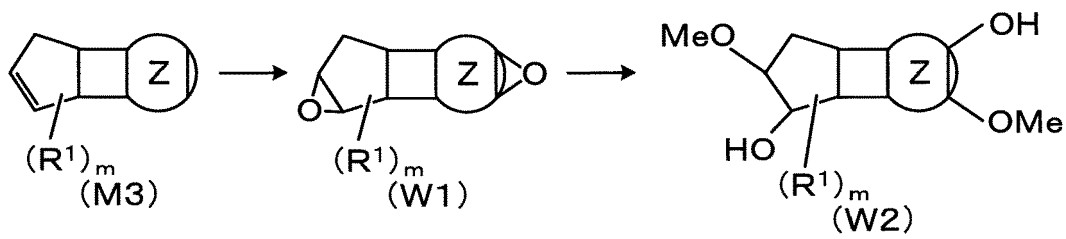

(M3) (W1) (W2)

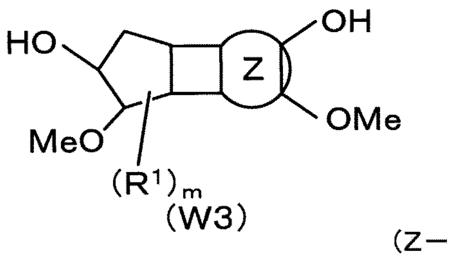

(W3)

(Z−3)

Details of $R^1$ and m in the Formula (Z-3) are the same as those in the formula (P1). In the formula (Z-3), one of two epoxy groups of a compound (W1) is on the cyclopentane ring, and the other epoxy group is on the ring Z. Further, in the compound (W2), a carbon atom bonded to the hydroxy group and a carbon atom bonded to the methoxy group are carbon atoms adjacent to each other in the same alicyclic carbocyclic skeleton. That is, in the formula (Z-3), the hydroxy group and the methoxy group of the compound (W2) are bonded to any two respective carbon atoms adjacent to each other in the alicyclic carbocyclic skeleton.

[Intended uses of polymerizable alicyclic compound]

The polymerizable alicyclic compound is used as a monomer for producing a thermoplastic resin such as a polycarbonate resin or a polyester carbonate resin.

When the thermoplastic resin such as the polycarbonate resin or the polyester carbonate resin is produced using the polymerizable alicyclic compound, the 5% thermal weight loss temperature (Td5) of the polymerizable alicyclic compound is preferably high from the viewpoint of adjusting a quantitative ratio. Specifically, if the 5% thermal weight loss temperature (Td5) of the polymerizable alicyclic compound is 150°C or lower, the polymerizable alicyclic compound is thermally decomposed, which is not preferred in that there is a problem in quantitativeness. The Td5 of the polymerizable alicyclic compound is preferably 170°C or higher, more preferably 200°C or higher, even more preferably 210°C or higher, particularly preferably 220°C or higher.

<Structure of thermoplastic resin>

A thermoplastic resin obtained using the polymerizable alicyclic compound represented by the formula (P1) contains a repeating structural unit represented by, for example, the following formula (X) in its molecule.

[Formula 134]

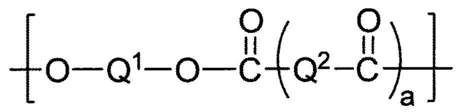

(X)

In the formula (X), $Q^1$ is a structural unit based on a dihydroxy compound, $Q^2$ is a structural unit based on a dicarboxylic acid compound, and a is 0 or 0 and 1. Specifically, when a in the formula (X) is 0, the thermoplastic resin having a structural unit represented by the formula (X) is a polycarbonate resin, and when a is 0 and 1, specifically when the thermoplastic resin contains a repeating structural unit represented by the formula (X) in which a = 0 and a repeating structural unit represented by the formula (X) in which a = 1, the thermoplastic resin is a polyester carbonate resin.
The "structural unit based on a dihydroxy compound" is a moiety obtained by removing two functional groups (hydroxy groups) from a dihydroxy compound. The "structural unit based on a dicarboxylic acid compound" is a moiety obtained by removing two functional groups from a dicarboxylic acid compound that is a dicarboxylic acid or a derivative thereof (specifically, an ester, a halide, or the like). The functional group of the dicarboxylic acid compound is typically represented as $-C(=O)-X^1$. $X^1$ is OH, $OX^2$, or a halogen atom. $X^2$ is a hydrocarbon group such as an alkyl group having 1 to 10 carbon atoms or a phenyl group. As at least one of $Q^1$ and $Q^2$ in the formula (X), a structural unit A1 derived from the above-described polymerizable alicyclic compound represented by the formula (P1) is contained.
The thermoplastic resin having the structure represented by the formula (X) contains, as the structural unit A1 derived from the polymerizable alicyclic compound represented by the formula (P1), a structural unit derived from a dihydroxy compound represented by the formula (P1) in which $B^1$ and $B^2$ are hydroxy groups. Further, the thermoplastic resin may contain the structural unit A1 derived from a dihydroxy compound represented by the formula (P1) and a structural unit derived from a dihydroxy compound other than the dihydroxy compound represented by the formula (P1). In this case, $Q^1$ in the formula (X) contains the structural unit A1 and another structural unit.
Further, when the thermoplastic resin having the structure represented by the formula (X) contains, as the structural unit A1 derived from the polymerizable alicyclic compound represented by the formula (P1), a structural unit derived from a dihydroxy compound represented by the formula (P1) and a in the formula (X) is 1, $Q^2$ may either be the structural unit A1 or another structural unit other than the structural unit A1.

(Structural unit A1)

The structural unit A1 is a structural unit derived from the polymerizable alicyclic compound represented by the formula (P1), and is represented by the following formula (B1).

[Formula 135]

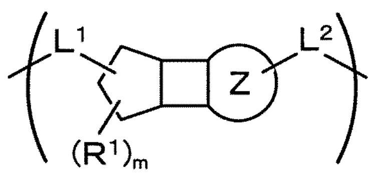

(B1)

The formula (B1) represents a structure obtained by eliminating $B^1$ and $B^2$ from the polymerizable alicyclic compound represented by the formula (P1). Details of a ring Z, $L^1$, $L^2$, $R^1$, and m in the formula (B1) are the same as those in the formula (P1).

(Other structures)

The thermoplastic resin may contain another structural unit other than the structural unit A1. Such a structural unit is not particularly limited, and examples thereof include a structural unit A2, a structural unit A3, a structural unit A4, a

structural unit A5, a structural unit A6, a structural unit A7, and a structural unit A8 described later. That is, the polymerizable alicyclic compound represented by the formula (P1) can be used for producing not only a homopolymer but also a copolymer. When a copolymer is produced using the polymerizable alicyclic compound, characteristics derived from a monomer other than the polymerizable alicyclic compound are imparted to the thermoplastic resin such as a polycarbonate resin or a polyester carbonate resin.

From the viewpoint of improving surface hardness of a resulting resin or reducing photoelastic coefficient of a resulting resin, the content of the structural unit A1 is preferably 1 mol% or more, more preferably 5 mol% or more, even more preferably 15 mol% or more, even more preferably 25 mol% or more, particularly preferably 37.5 mol% or more with respect to the total molar quantity of all the structural units based on all the dihydroxy compounds, all the dicarboxylic acid compounds, and all the carbonates in the thermoplastic resin. The maximum content of the structural unit A1 is 50 mol%.

From the same viewpoint, the content of the structural unit A1 is preferably 1 mass% or more, more preferably 10 mass% or more, even more preferably 40 mass% or more, even more preferably 55 mass% or more, particularly preferably 75 mass% or more with respect to the total mass of the thermoplastic resin based on all the dihydroxy compounds, all the dicarboxylic acid compounds, and all the carbonates in the thermoplastic resin.

On the other hand, from the viewpoint of achieving both moist heat resistance and film strength of a resulting resin, the content of the structural unit A1 is preferably 0.1 mol% or more, more preferably 1 mol% or more, even more preferably 2 mol% or more, even more preferably 4 mol% or more, particularly preferably 5 mol% or more with respect to the total molar quantity of all the structural units based on all the dihydroxy compounds, all the dicarboxylic acid compounds, and all the carbonates in the thermoplastic resin. Further, the content of the structural unit A1 is preferably 50 mol% or less, more preferably 37.5 mol% or less, even more preferably 25 mol% or less, even more preferably 20 mol% or less, particularly preferably 15 mol% or less.

From the same viewpoint, the content of the structural unit A1 is preferably 1 mass% or more, more preferably 2 mass% or more, even more preferably 5 mass% or more, even more preferably 7 mass% or more, particularly preferably 10 mass% or more with respect to the total mass of the thermoplastic resin based on all the dihydroxy compounds, all the dicarboxylic acid compounds, and all the carbonates in the thermoplastic resin. Further, the content of the structural unit A1 is preferably 95 mass% or less, more preferably 80 mass% or less, even more preferably 60 mass% or less, even more preferably 50 mass% or less, particularly preferably 40 mass% or less.

From the viewpoint that optical properties such as transparency and characteristics such as weatherability, moldability, and heat resistance can be improved, the thermoplastic resin such as a polycarbonate resin preferably further contains a structural unit derived from at least one compound selected from the group consisting of isosorbide, isomannide, and isoidide. The structural unit derived from at least one compound selected from the group consisting of isosorbide, isomannide, and isoidide is hereinafter referred to as a "structural unit A2" as appropriate. The structural unit A2 is represented by the following formula (A2).

[Formula 136]

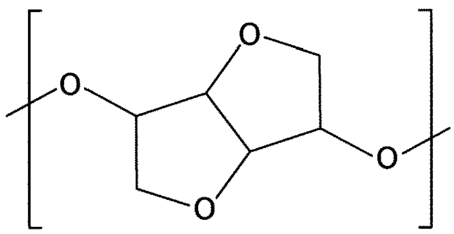

(A2)

When having the structural unit A2, the thermoplastic resin is a copolymer containing at least the structural unit A1 and the structural unit A2. Further, from the viewpoint that isosorbide can be obtained by dehydration condensation of sorbitol produced from various starches that are present in abundance as plant-derived resources and are easily available and the viewpoint of carbon neutral, the thermoplastic resin more preferably contains a structural unit derived from isosorbide.

From the viewpoint of improving heat resistance, the content of the structural unit A2 in the thermoplastic resin having a carbonate bond is preferably 5 mol% or more, more preferably 10 mol% or more, even more preferably 20 mol% or more, even more preferably 30 mol% or more, particularly preferably 40 mol% or more and is preferably 49 mol% or less with respect to the total molar quantity of all the structural units based on all the dihydroxy compounds, all the dicarboxylic acid compounds, and all the carbonates in the thermoplastic resin.

From the same viewpoint, the content of the structural unit A2 is preferably 5 mass% or more, more preferably 20 mass% or more, even more preferably 40 mass% or more, even more preferably 60 mass% or more, particularly preferably 80 mass% or more and is preferably 99 mass% or less with respect to the total mass of the thermoplastic resin

based on all the dihydroxy compounds, all the dicarboxylic acid compounds, and all the carbonates in the thermoplastic resin.

From the viewpoint of reducing a water absorption rate to achieve a balance with mechanical strength and optical properties, the content of the structural unit A2 in the thermoplastic resin having a carbonate bond is preferably 5 mol% or more, more preferably 10 mol% or more, even more preferably 15 mol% or more, even more preferably 20 mol% or more, particularly preferably 30 mol% or more and is preferably 49 mol% or less, more preferably 47 mol% or less, even more preferably 45 mol% or less, even more preferably 42 mol% or less, particularly preferably 40 mol% or less with respect to the total molar quantity of all the structural units based on all the dihydroxy compounds, all the carboxylic acid compounds, and all the carbonates in the thermoplastic resin. From the same viewpoint, the content of the structural unit A2 is preferably 5 mass% or more, more preferably 20 mass% or more, even more preferably 30 mass% or more, even more preferably 37.5 mass% or more, particularly preferably 42.5 mass% or more with respect to the total mass of the thermoplastic resin based on all the dihydroxy compounds, all the dicarboxylic acid compounds, and all the carbonates in the thermoplastic resin. Further, the content of the structural unit A2 is preferably 95 mass% or less, more preferably 90 mass% or less, even more preferably 80 mass% or less, even more preferably 70 mass% or less, particularly preferably 65 mass% or less.

From the viewpoint that optical properties such as transparency and characteristics such as mechanical strength and melt flowability can be improved, the polycarbonate resin preferably contains a structural unit derived from an aliphatic dihydroxy compound other than the structural unit A1 and the structural unit A2. The structural unit derived from an aliphatic dihydroxy compound other than the structural unit A1 and the structural unit A2 is hereinafter referred to as a "structural unit A3", and a structural unit derived from an alicyclic dihydroxy compound is referred to as a "structural unit A3'" as appropriate. It is noted that the structural unit A3 or the structural unit A3' is a concept excluding not only the structural unit A1 and the structural unit A2 but also structural units A4 to A8 described later. When having the structural unit A3 or the structural unit A3', the polycarbonate resin is a copolymeric polycarbonate having at least the structural unit A1 and the structural unit A3 or the structural unit A3'. Examples of the aliphatic dihydroxy compound include a linear aliphatic dihydroxy compound and a branched aliphatic dihydroxy compound, and an alicyclic dihydroxy compound is not regarded as the aliphatic dihydroxy compound.

Examples of the linear aliphatic dihydroxy compound include ethylene glycol, 1,3-propanediol, 1,2-propanediol, 1,4-butanediol, 1,3-butanediol, 1,2-butanediol, 1,5-heptanediol, 1,6-hexanediol, 1,10-decanediol, 1,12-dodecanediol, diethylene glycol, triethylene glycol, tetraethylene glycol, polyethylene glycol, dipropylene glycol, tripropylene glycol, tetrapropylene glycol, polypropylene glycol, polytrimethylene glycol, polytetramethylene glycol, and polydecamethylene glycol.

Examples of the dihydroxy compound of a branched aliphatic hydrocarbon include neopentyl glycol and hexylene glycol.

From the viewpoint of imparting flexibility to the polycarbonate resin and improving toughness of the polycarbonate resin, the dihydroxy compound constituting the structural unit A3 is particularly preferably a linear aliphatic dihydroxy compound having primary hydroxy groups. Examples of the linear aliphatic dihydroxy compound having primary hydroxy groups include ethylene glycol, 1,3-propanediol, 1,4-butanediol, 1,6-hexanediol, 1,10-decanediol, and 1,12-dodecanediol.

From the viewpoint of improving characteristics such as toughness, the content of the structural unit A3 in the polycarbonate resin is preferably 5 mol% or more, more preferably 10 mol% or more, even more preferably 20 mol% or more, even more preferably 30 mol% or more, particularly preferably 40 mol% or more and is preferably 49 mol% or less with respect to the total molar quantity of all the structural units based on all the dihydroxy compounds, all the dicarboxylic acid compounds, and all the carbonates in the thermoplastic resin.

From the same viewpoint, the content of the structural unit A3 is preferably 0.1 mass% or more, more preferably 1 mass% or more, even more preferably 2 mass% or more, even more preferably 4 mass% or more, particularly preferably 5 mass% or more and is preferably 99 mass% or less with respect to the total mass of the thermoplastic resin based on all the dihydroxy compounds, all the dicarboxylic acid compounds, and all the carbonates in the thermoplastic resin.

From the viewpoint of achieving both heat resistance and mechanical strength, the content of the structural unit A3 in the polycarbonate resin is preferably 49 mol% or less, more preferably 40 mol% or less, even more preferably 30 mol% or less, even more preferably 20 mol% or less, particularly preferably 15 mol% or less
with respect to the total molar quantity of all the structural units based on all the dihydroxy compounds, all the dicarboxylic acid compounds, and all the carbonates in the thermoplastic resin. From the same viewpoint, the content of the structural unit A3 is preferably 0.1 mass% or more, more preferably 1 mass% or more, even more preferably 2 mass% or more, even more preferably 4 mass% or more, particularly preferably 5 mass% or more and is preferably 99 mass% or less, more preferably 80 mass% or less, even more preferably 60 mass% or less, even more preferably 40 mass% or less, particularly preferably 30 mass% or less with respect to the total mass of the thermoplastic resin based on all the dihydroxy compounds, all the dicarboxylic acid compounds, and all the carbonates in the thermoplastic resin.

Examples of the alicyclic dihydroxy compound include 1,2-cyclohexanediol, 1,2-cyclohexanedimethanol, 1,3-cyclohexanedimethanol, 1,4-cyclohexanedimethanol, tricyclodecanedimethanol, pentacyclopentadecanedimethanol, 2,6-decalindimethanol, 1,5-decalindimethanol, 2,3-decalindimethanol, 2,3-norbornanedimethanol, 2,5-norbornanedimethanol, 1,3-adamantanedimethanol , and a dihydroxy compound derived from a terpene compound such as limonene.

From the viewpoint of imparting flexibility to the polycarbonate resin and improving toughness of the polycarbonate resin, the dihydroxy compound constituting the structural unit A3' is preferably 1,4-cyclohexanedimethanol, tricyclodecanedimethanol, or the like.

From the viewpoint of improving characteristics such as toughness, the content of the structural unit A3' in the polycarbonate resin is preferably 5 mol% or more, more preferably 10 mol% or more, even more preferably 20 mol% or more, even more preferably 30 mol% or more, particularly preferably 40 mol% or more and is preferably 49 mol% or less with respect to the total molar quantity of all the structural units based on all the dihydroxy compounds, all the dicarboxylic acid compounds, and all the carbonates in the thermoplastic resin.

From the same viewpoint, the content of the structural unit A3' is preferably 0.1 mass% or more, more preferably 1 mass% or more, even more preferably 2 mass% or more, even more preferably 4 mass% or more, particularly preferably 5 mass% or more and is preferably 99 mass% or less with respect to the total mass of the thermoplastic resin based on all the dihydroxy compounds, all the dicarboxylic acid compounds, and all the carbonates in the thermoplastic resin.

From the viewpoint of achieving both heat resistance and mechanical strength, the content of the structural unit A3' in the polycarbonate resin is preferably 49 mol% or less, more preferably 40 mol% or less, even more preferably 30 mol% or less, even more preferably 20 mol% or less, particularly preferably 15 mol% or less
with respect to the total molar quantity of all the structural units based on all the dihydroxy compounds, all the dicarboxylic acid compounds, and all the carbonates in the thermoplastic resin. From the same viewpoint, the content of the structural unit A3' is preferably 0.1 mass% or more, more preferably 1 mass% or more, even more preferably 2 mass% or more, even more preferably 4 mass% or more, particularly preferably 5 mass% or more and is preferably 99 mass% or less, more preferably 80 mass% or less, even more preferably 60 mass% or less, even more preferably 40 mass% or less, particularly preferably 30 mass% or less with respect to the total mass of the thermoplastic resin based on all the dihydroxy compounds, all the dicarboxylic acid compounds, and all the carbonates in the thermoplastic resin.

The thermoplastic resin may further contain a structural unit derived from a dihydroxy compound represented by the following formula (A5). In this case, the glass transition temperature of the thermoplastic resin can be controlled to fall within a preferred range, which may make it easy to subject the thermoplastic resin to melt molding and film formation Further, in this case, optical properties (specifically, low photoelastic coefficient and transparency) can be imparted to the thermoplastic resin. The thermoplastic resin may contain one or two or more structural units represented by the formula (A5). The structural unit represented by the formula (A5) is referred to as a "structural unit A5" as appropriate.

[Formula 137]

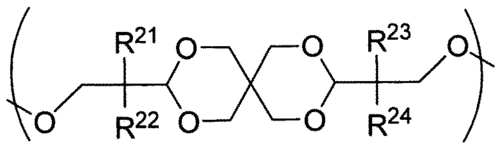

(A5)

In the formula (A5), $R^{21}$ to $R^{24}$ are each independently a hydrogen atom, a substituted or unsubstituted alkyl group having 1 to 20 carbon atoms, a substituted or unsubstituted cycloalkyl group having 5 to 20 carbon atoms, or a substituted or unsubstituted aryl group having 6 to 20 carbon atoms.

An example of the dihydroxy compound represented by the formula (A5) includes a dihydroxy compound described in paragraph [0024] in WO2017/159525.

From the viewpoint of reducing photoelastic coefficient and obtaining a thermoplastic resin having high surface hardness, the content of the structural unit A5 is preferably 5 mol% or more, more preferably 15 mol% or more, even more preferably 22.5 mol% or more, even more preferably 32.5 mol% or more, particularly preferably 40 mol% or more and is preferably 49 mol% or less with respect to the total molar quantity of all the structural units based on all the dihydroxy compounds, all the dicarboxylic acid compounds, and all the carbonates in the thermoplastic resin having a carbonate bond. From the same viewpoint, the content of the structural unit A5 is preferably 5 mass% or more, more preferably 15 mass% or more, even more preferably 22.5 mass% or more, even more preferably 32.5 mass% or more, particularly preferably 40 mass% or more with respect to the total mass of the thermoplastic resin based on all the dihydroxy compounds, all the dicarboxylic acid compounds, and all the carbonates in the thermoplastic resin.

On the other hand, from the viewpoint of adjusting glass transition temperature and mechanical strength to fall within their respective preferred ranges to improve melt-moldability and film formability of the thermoplastic resin having a carbonate bond, the content of the structural unit A5 is preferably 5 mol% or more, more preferably 10 mol% or more, even

more preferably 15 mol% or more, even more preferably 17.5 mol% or more, particularly preferably 20 mol% or more and is preferably 45 mol% or less, more preferably 40 mol% or less, even more preferably 35 mol% or less, particularly preferably 30 mol% or less, most preferably 27.5 mol% or less with respect to the total molar quantity of all the structural units based on all the dihydroxy compounds, all the dicarboxylic acid compounds, and all the carbonates in the thermoplastic resin. From the same viewpoint, the content of the structural unit A5 is preferably 10 mass% or more, more preferably 20 mass% or more, even more preferably 30 mass% or more, even more preferably 35 mass% or more, particularly preferably 40 mass% or more with respect to the total mass of the thermoplastic resin based on all the dihydroxy compounds, all the dicarboxylic acid compounds, and all the carbonates in the thermoplastic resin.

The thermoplastic resin may further contain a structural unit represented by the following formula (A6) and/or a structural unit represented by the following formula (A7). In this case, the thermoplastic resin can have a reverse wavelength dispersion property and is therefore suitable for optical films such as retardation films. The structural unit represented by the formula (A6) is referred to as a "structural unit A6" as appropriate, and the structural unit represented by the formula (A7) is referred to as a "structural unit A7" as appropriate.

[Formula 138]

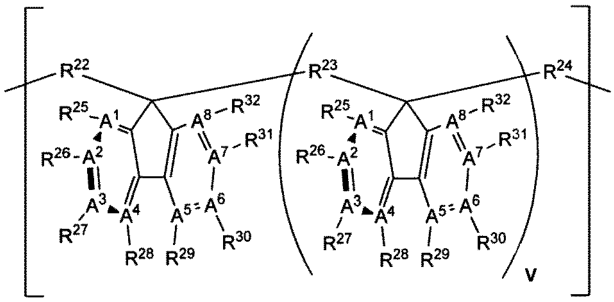

(A6)

[Formula 139]

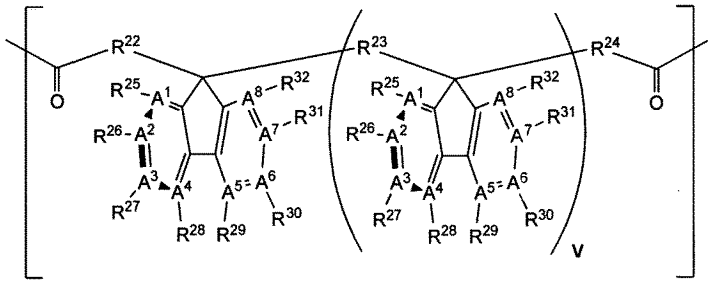

(A7)

In the formula (A6) and the formula (A7), $A^1$ to $A^8$ are each independently =CH- or =N-. $R^{22}$, $R^{23}$, and $R^{24}$ are each independently a direct bond or a group formed by linking two or more groups selected from the group consisting of an optionally-substituted alkylene group having 1 to 10 carbon atoms, an optionally-substituted arylene group having 4 to 10 carbon atoms, an optionally-substituted aralkylene group having 6 to 12 carbon atoms, an optionally-substituted alkylene group having 1 to 10 carbon atoms, and an optionally-substituted arylene group having 4 to 10 carbon atoms by an oxygen atom, an optionally-substituted nitrogen atom, or a carbonyl group.

In the formula (A6) or the formula A7, $R^{25}$ to $R^{32}$ are each independently a hydrogen atom, an optionally substituted alkyl group having 1 to 10 carbon atoms, an optionally substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom, a nitro group, or a cyano group. At least two adjacent groups out of $R^{25}$ to $R^{32}$ may be bonded to each other to form a ring. v is an integer of 0 to 5.

If the content of the structural unit (A6) and the structural unit (A7) in the thermoplastic resin is excessively high, there is a possibility that photoelastic coefficient and reliability deteriorate and a film formed from the thermoplastic resin cannot have high birefringence even when stretched. Further, if the ratio of the oligofluorene structural unit occupying the

thermoplastic resin is excessively high, the degree of freedom in molecular design reduces so that it is difficult to modify the resin if necessary. On the other hand, even if a desired reverse wavelength dispersion property is obtained by a very small amount of the oligofluorene structural unit, in this case, the optical property sensitively changes when the content of an oligofluorene even slightly varies. Therefore, it is difficult to produce a thermoplastic resin whose various properties fall within certain ranges.

In regard to $R^{22}$, $R^{23}$,and $R^{24}$ in the formula (A6) and the formula (A7), specific examples of the "arylene group having 4 to 10 carbon atoms" in the "optionally-substituted arylene group having 4 to 10 carbon atoms" include, but are not limited to: a phenylene group such as a 1,2-phenylene group, a 1,3-phenylene group, or a 1,4-phenylene group; a naphthylene group such as a 1,5-naphthylene group or a 2,6-naphthylene group; and a heteroarylene group such as a 2,5-pyridylene group or a 2,4-furylene group.

In regard to $R^{22}$, $R^{23}$, and $R^{24}$ in the formula (A6) and the formula (A7), an example of the "aralkylene group having 6 to 10 carbon atoms" in the "optionally-substituted aralkylene group having 6 to 12 carbon atoms" includes a group formed from an aromatic ring structure and two linear or branched alkylene groups bonded thereto at any two respective positions. The aromatic ring structure may be a hydrocarbon ring structure such as a benzene ring or a naphthalene ring or a heterocyclic structure such as a furan ring or a pyridine ring. Specific examples of the aralkylene group having 6 to 10 carbon atoms include, but are not limited to, those shown in the following group [G].

[Formula 140]

[G]

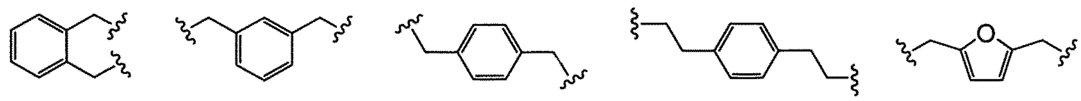

From the viewpoint that synthesis can easily be performed and the viewpoint that raw materials are inexpensively available, the structural unit A6 is preferably represented by the following formula (A6-1). From the same viewpoint, the structural unit (A7) is preferably represented by the following formula (A7-1).

[Formula 141]

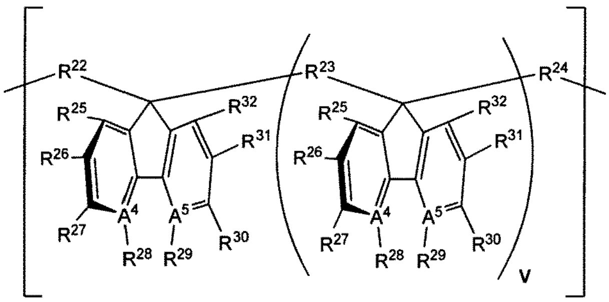

(A6－1)

[Formula 142]

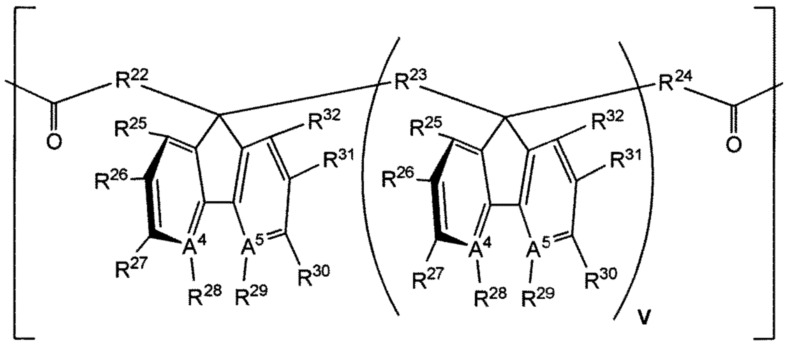

(A7－1)

In the formula (A6-1) and the formula (A7-1), $A^4$ and $A^5$ are each independently =CH- or =N-. $R^{22}$, $R^{23}$, and $R^{24}$ in the formula (A6-1) and the formula (A7-1) are respectively the same as $R^{22}$, $R^{23}$, and $R^{24}$ in the formula (A6) and the

formula (A7). $R^{25}$ to $R^{32}$ in the formula (A6-1) and the formula (A7-1) are respectively the same as $R^{25}$ to $R^{32}$ in the formula (A6) and the formula (A7). v is an integer of 0 to 2.

$R^{22}$, $R^{23}$, and $R^{24}$ in the formula (A6-1) and the formula (A7-1) are preferably each independently an optionally-substituted alkylene group having 1 to 10 carbon atoms, more preferably an alkylene group having 1 to 3 carbon atoms.

From the viewpoint that synthesis can easily be performed and the viewpoint that raw materials are inexpensively available, the structural unit A6 is preferably represented by the following formula (A6-2) and the structural unit A7 is preferably represented by the following formula (A7-2).

[Formula 143]

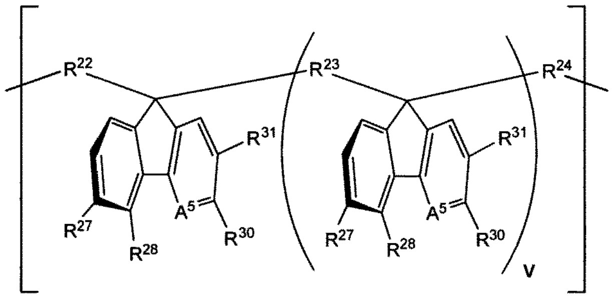

(A6−2)

[Formula 144]

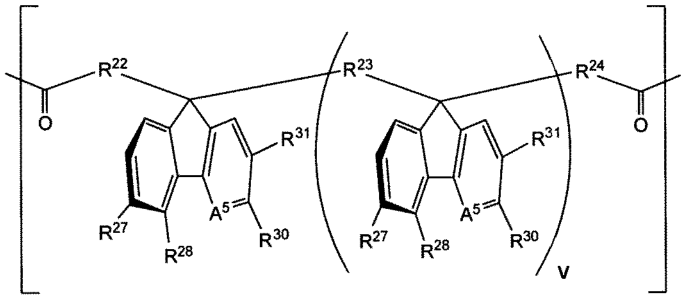

(A7−2)

In the formula (A6-2) and the formula (A7-2), $A^5$ is =CH- or =N-. $R^{22}$, $R^{23}$, and $R^{24}$ in the formula (A6-2) and the formula (A7-2) are respectively the same as $R^{22}$, $R^{23}$, and $R^{24}$ in the formula (A6) and the formula (A7). $R^{25}$ to $R^{32}$ in the formula (A6-2) and the formula (A7-2) are respectively the same as $R^{25}$ to $R^{32}$ in the formula (A6) and the formula (A7). v is an integer of 0 to 2.

From the viewpoint that synthesis can easily be performed and the viewpoint that raw materials are inexpensively available, the structural unit (A6) is preferably represented by the following formula (A6-3) and the structural unit (A7) is preferably represented by the following formula (A7-3).

[Formula 145]

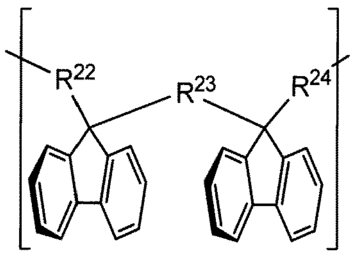

(A6−3)

[Formula 146]

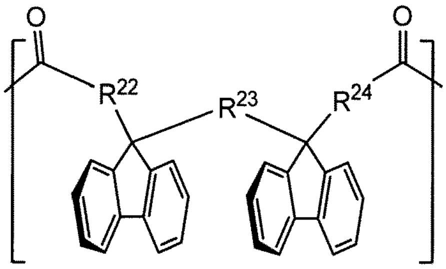

(A7−3)

$R^{22}$, $R^{23}$, and $R^{24}$ in the formula (A6-3) and the formula (A7-3) are each independently a direct bond, a methylene group, or an ethylene group.

From the viewpoint that synthesis can easily be performed and the viewpoint that a reverse wavelength dispersion property improves, the structural unit (A6) is more preferably represented by the following formula (A6-4) and the structural unit (A7) is more preferably represented by the following formula (A7-4).

[Formula 147]

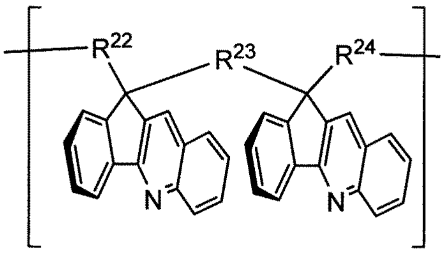

(A6−4)

[Formula 148]

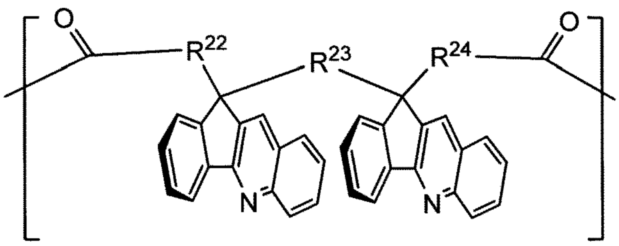

(A7−4)

$R^{22}$, $R^{23}$, and $R^{24}$ in the formula (A6-4) and the formula (A7-4) are each independently a direct bond, a methylene group, or an ethylene group.

From the viewpoint that a reverse wavelength dispersion property improves, the structural unit (A6) is more preferably represented by the following formula (A6-5) and the structural unit (A7) is more preferably represented by the following formula (A7-5).

[Formula 149]

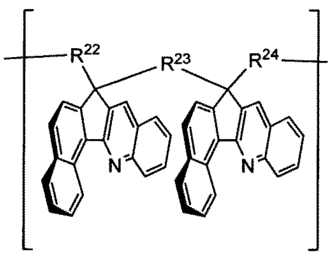

(A6−5)

[Formula 150]

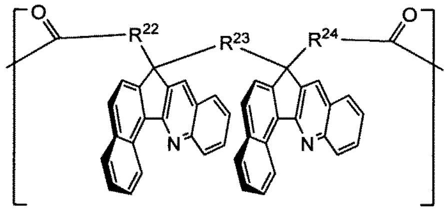

(A7-5)

$R^{22}$, $R^{23}$, and $R^{24}$ in the formula (A6-5) and the formula (A7-5) are each independently a direct bond, a methylene group, or an ethylene group.

From the viewpoint that raw materials are inexpensively available, more specifically, the structural unit (A6) is preferably at least one selected from the group consisting of structures shown below.

[Formula 151]

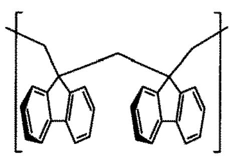 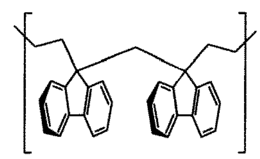 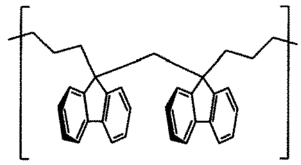

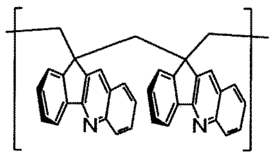 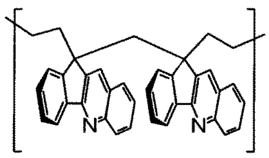 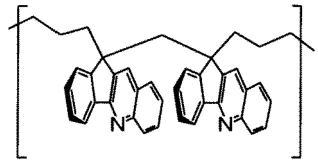

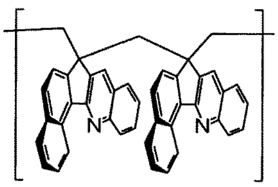 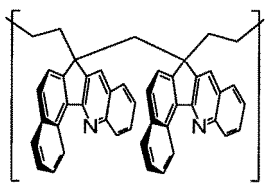 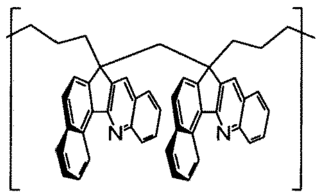

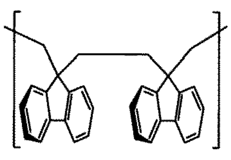 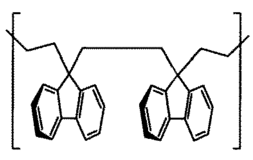 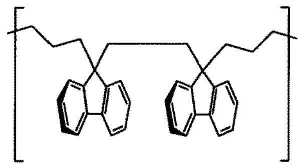

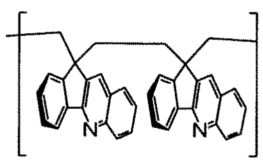 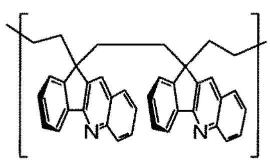 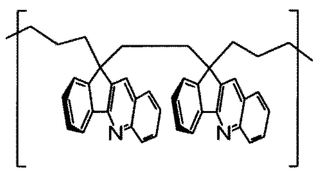

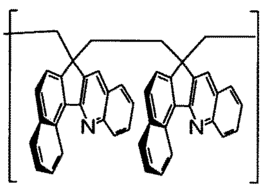 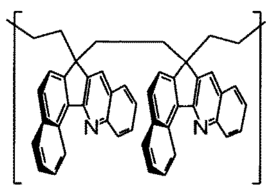 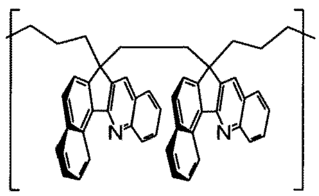

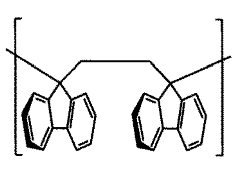 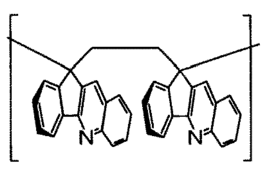 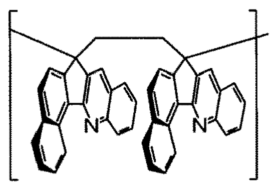

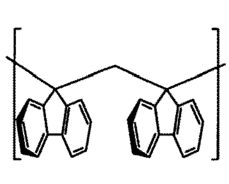
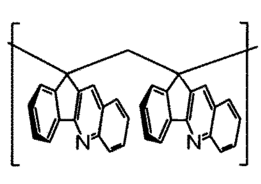
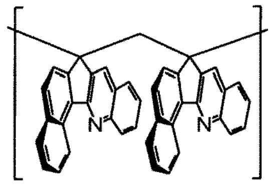
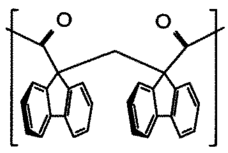
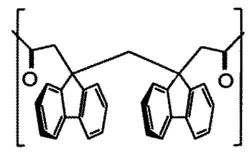
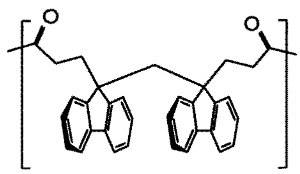
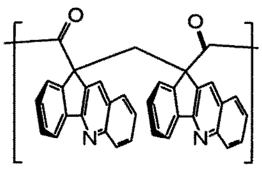
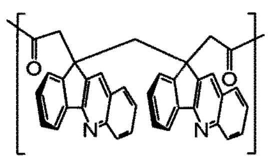
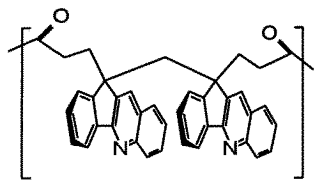
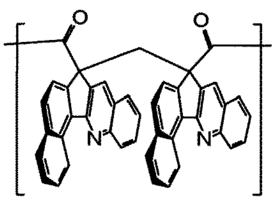
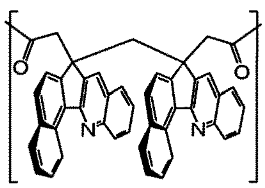
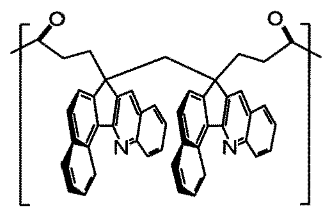
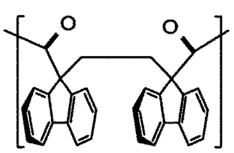
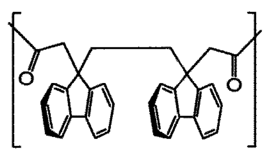
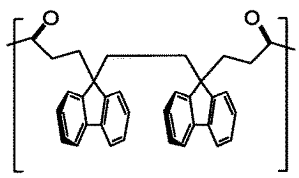
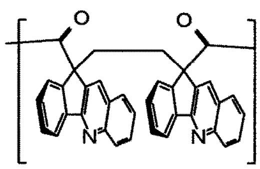
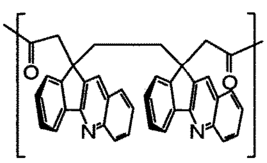
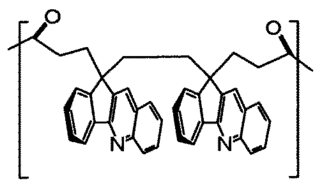
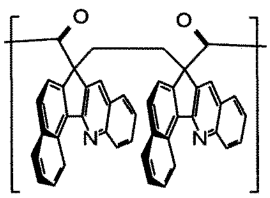
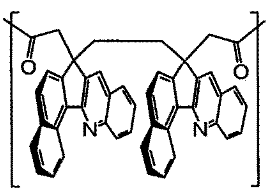
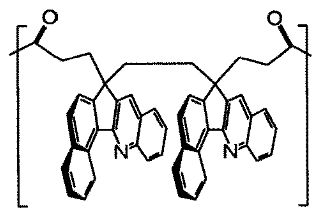

Further, structures shown below have a rigid skeleton due to dense introduction of aromatic rings and therefore exhibit low photoelastic coefficient. The thermoplastic resin containing a structural unit having low photoelastic coefficient is preferred from the viewpoint of moldability and reliability because a change in phase difference due to stress is small. That is, from the viewpoint of moldability and reliability on a phase difference change, specifically, the structural unit (A6) or the structural unit (A7) is preferably at least one selected from the group consisting of structures shown below.

[Formula 152]

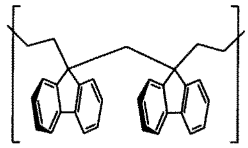

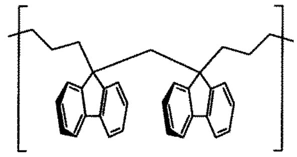

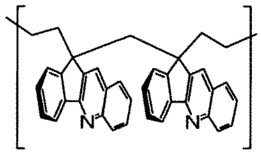

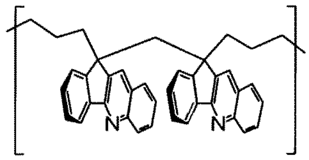

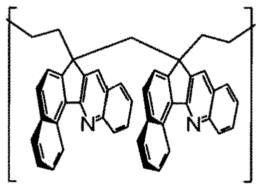

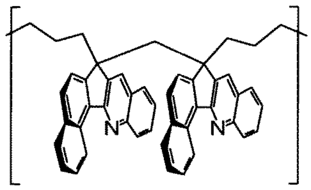

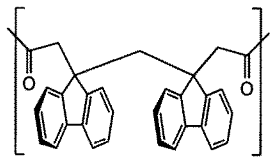

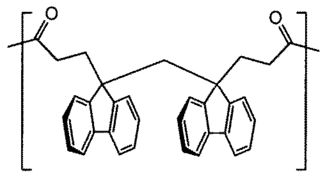

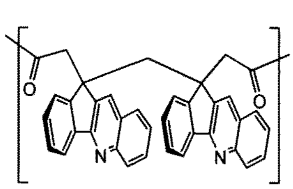

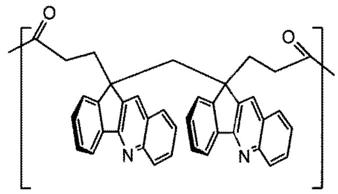

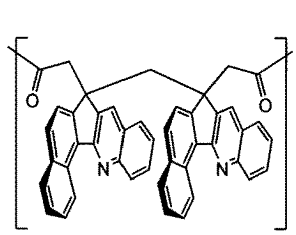

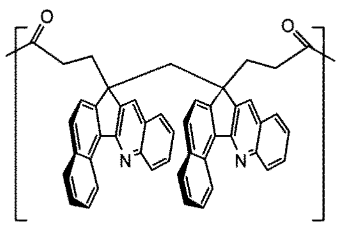

From the viewpoint that both inexpensive synthesis and low water absorption are achieved and wavelength dispersion can be adjusted to be more preferred, the structural unit A7 is particularly preferably a structure represented by the following formula (A7-5).

[Formula 153]

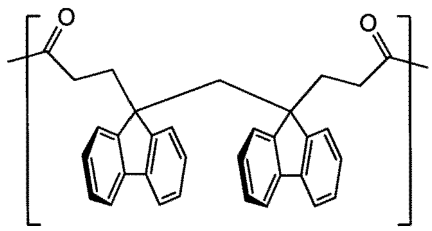

(A7-5)

The thermoplastic resin having the structural unit A6 and/or the structural unit A7 can be produced by, for example, a method such as polymerization of a monomer represented by the following formula (j).

[Formula 154]

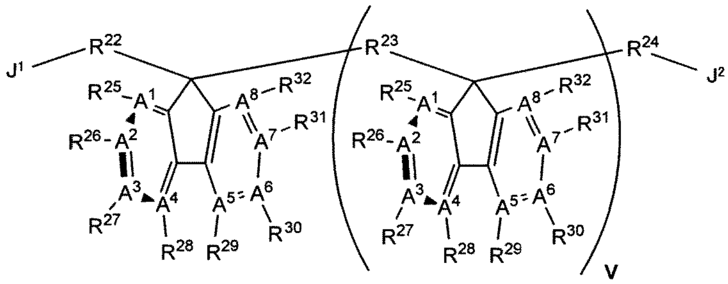

(j)

In the formula (j), $A^1$ to $A^8$, $R^{22}$ to $R^{32}$, and v are respectively the same as $A^1$ to $A^8$, $R^{22}$ to $R^{32}$, and v in the formula (A6) and the formula (A7). $J^1$ and $J^2$ are each independently a polymerization reactive group. Examples of $J^1$ and $J^2$ include: a hydroxyl group-containing group such as a hydroxy group or a hydroxyalkyl group; a carboxy group-containing group such as a carboxy group or a carboxyalkyl group; an ester-containing group such as an alkoxycarbonyl group, a phenoxycarbonyl group, or a hydroxy ester group; and an acid halide group. $J^1$ and $J^2$ may be the same or different from each other. $J^1$ and $J^2$ are preferably the same because the monomer represented by the formula (j) tends to be produced in a short process.

The monomer represented by the formula (j) can be used as a raw material of a polymer having a divalent oligofluorene as a repeating unit. The polymerization reactive group is preferably present only at two positions, $J^1$ and $J^2$. That is, it is preferred that $R^{25}$ to $R^{32}$ do not have a substituent that functions as a polymerization reactive group under polymerization conditions for a polycarbonate or a polyester carbonate.

$J^1$ and $J^2$ in the formula (j) are preferably hydroxy groups. The monomer in which $J^1$ and $J^2$ are hydroxy groups can be used for production of a thermoplastic resin having excellent optical performance. The monomer in which $J^1$ and $J^2$ are hydroxy groups is represented by the following formula (j 1).

[Formula 155]

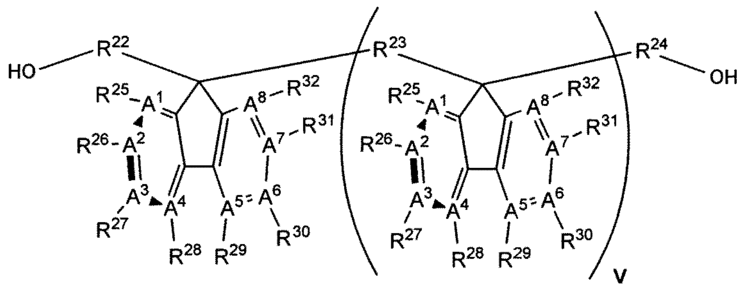

(j1)

Further, $J^1$ and $J^2$ in the formula (j) are preferably ester groups. The monomer in which $J^1$ and $J^2$ are ester groups can be used for production of a polyester carbonate having excellent optical performance. From the viewpoint of easy introduction using industrially-available methyl acrylate, ethyl acrylate, or methyl methacrylate, the ester group is preferably a 2-(methoxycarbonyl)ethyl group, a 2-(ethoxycarbonyl)ethyl group, or a 2-(methoxycarbonyl)propyl group.

From the viewpoint that a transesterification reaction easily proceeds due to an improvement in the activity of ester groups and therefore a polyester carbonate can be synthesized in one step by reacting a diester compound, a dihydroxy compound, and a diester carbonate under the same conditions, the ester group is preferably a phenoxycarbonylalkyl group. Particularly, a 2-(phenoxycarbonyl)methyl group, a 2-(phenoxycarbonyl)ethyl group, and a 2-(phenoxycarbonyl)propyl group are particularly preferred because these groups can be introduced by a method using phenyl 2-bromoacetate, phenyl acrylate, and phenyl methacrylate or a method based on transesterification from a 2-bromoacetic acid ester, a 2-chloroacetic acid ester, a 2-iodoacetic acid ester, an acrylic acid ester, and a methacrylic acid ester. The monomer in which $J^1$ and $J^2$ are ester groups is represented by, for example, the following formula (j2).

[Formula 156]

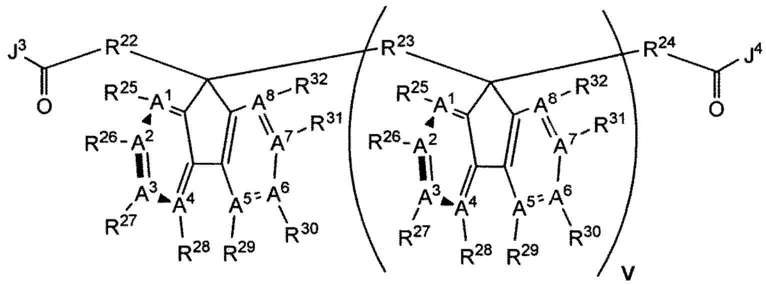

(j2)

In the formula(j2), $J^3$ and $J^4$ are each independently an organic substituent having 1 to 10 carbon atoms or a halogen atom.

Specific examples of the organic substituent having 1 to 10 carbon atoms represented by $J^3$ or $J^4$ include, but are not limited to: linear alkyloxy groups such as a methoxy group, an ethoxy group, a n-propoxy group, a n-butoxy group, a n-pentyloxy group, a n-hexyloxy group, and a n-decyloxy group; alkyloxy groups having a branched chain, such as an isopropyloxy group, a 2-methylpropyloxy group, a 2,2-dimethylpropyloxy group, and a 2-ethylhexyloxy group; cyclic alkyloxy groups such as a cyclopropyloxy group, a cyclopentyloxy group, a cyclohexyloxy group, and a cyclooctyloxy group; aryloxy groups such as a phenoxy group, a 1-naphthyloxy group, and a 2-naphthyloxy group; heteroaryl groups containing a 1-imidazoyl group; heteroaryl groups such as a 2-pyridyloxy group and a 2-furyloxy group; and aralkyloxy groups such as a benzyloxy group, a 2-phenylethoxy group, and a p-methoxybenzyloxy group. Specific examples of the halogen atom include, but are not limited to, a chlorine atom and a bromine atom.

From the viewpoint that a polyester carbonate can efficiently be synthesized by removing a low-boiling alcohol generated by transesterification with a dihydroxy compound, $J^3$ and $J^4$ are preferably methyl groups or ethyl groups. From the viewpoint that a transesterification reaction easily proceeds and therefore a polyester carbonate as a preferred polymer can be synthesized in one step by adding a diester compound, a dihydroxy compound, and a diester carbonate to a reactor at the same time, $J^3$ and $J^4$ are preferably aryl groups. Particularly, $J^3$ and $J^4$ are particularly preferably phenyl groups because a phenyl group has a low molecular weight and can be distilled away as phenol after synthesis of a polyester carbonate. It is noted that when the compound having aryl groups as $J^3$ and $J^4$ is used in a method for producing a thermoplastic resin described later, a diaryl carbonate described later is preferably used as a diester carbonate from the viewpoint of reactivity during polymerization, and it is more preferred that the aryl groups as $J^3$ and $J^4$ and aryl groups in the diaryl carbonate are the same from the viewpoint that a by-product can easily be removed. From the viewpoint that polymerization reactivity is excellent and a thermoplastic resin can be obtained by polymerization using a relatively simple equipment, such as solution polymerization or interfacial polymerization, $J^3$ and $J^4$ are preferably acid chloride-containing groups, and acid chloride or acid bromide is more preferred because it can industrially inexpensively be produced.

Specific examples of the monomer represented by the formula (j) include compounds shown below.

[Formula 157]

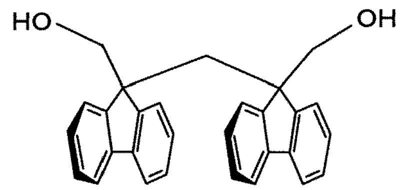
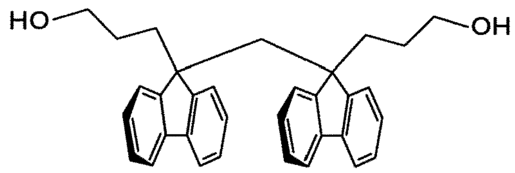
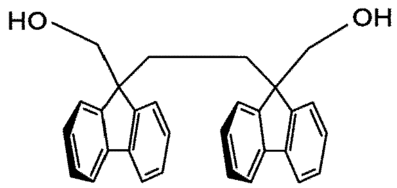
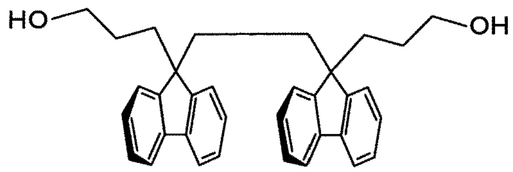
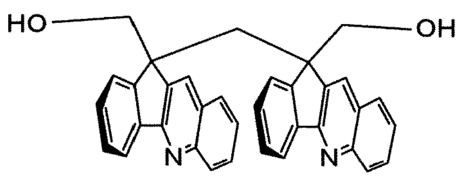
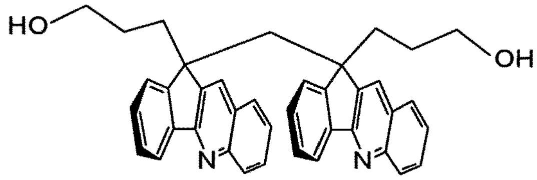
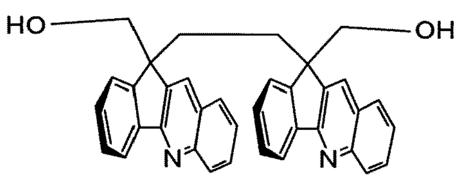
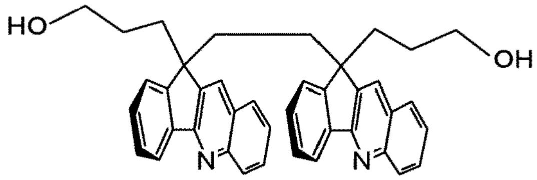
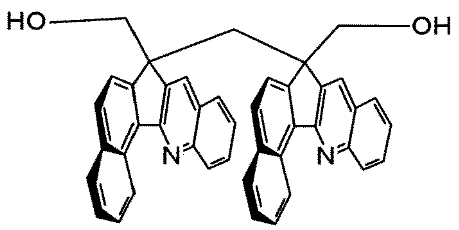
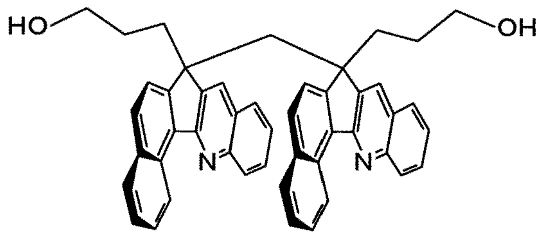
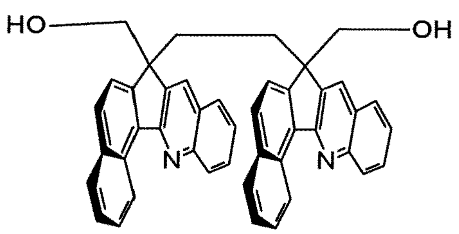
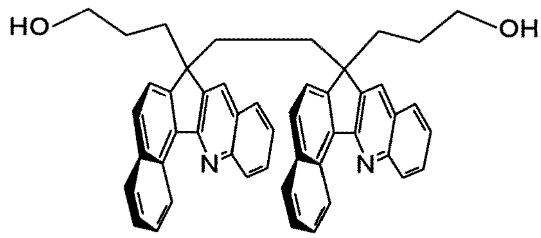

[Formula 158]

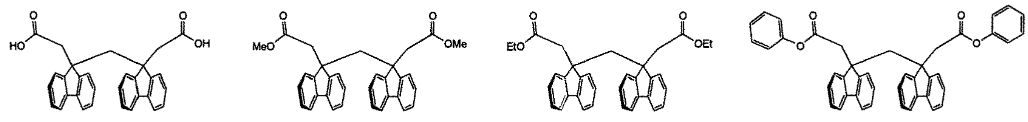

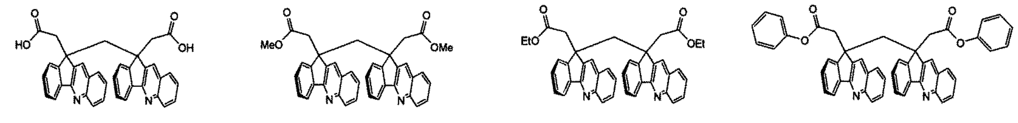

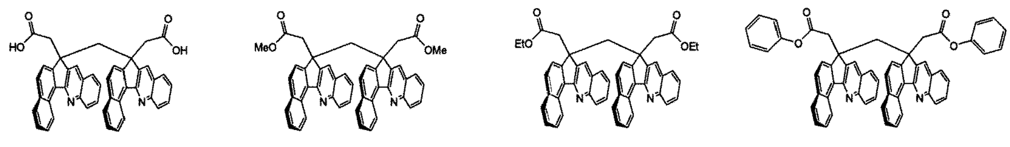

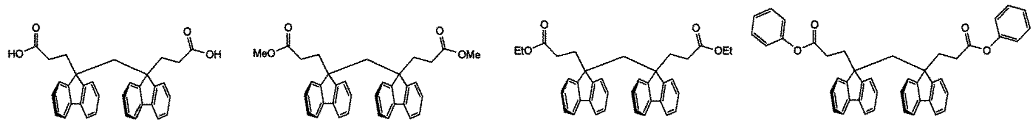

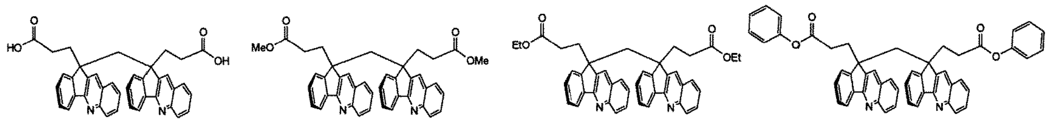

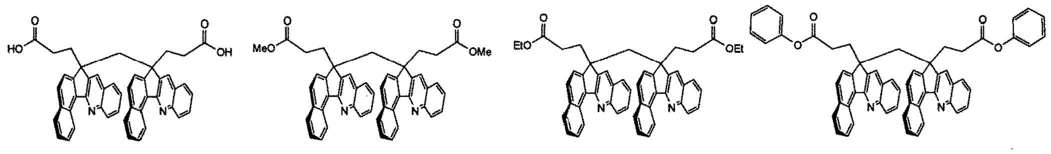

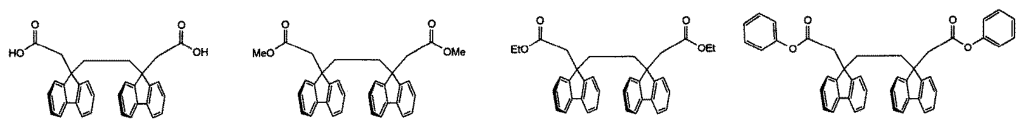

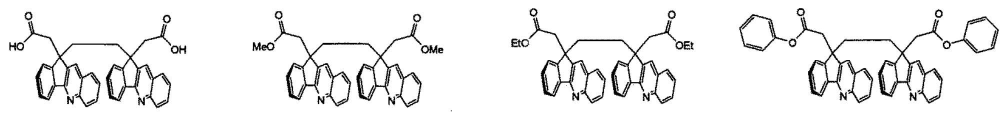

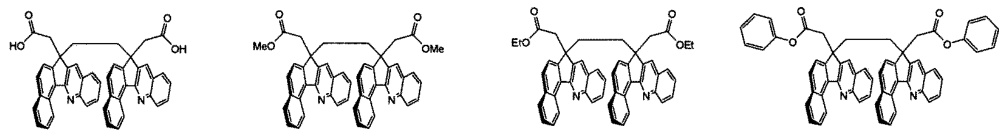

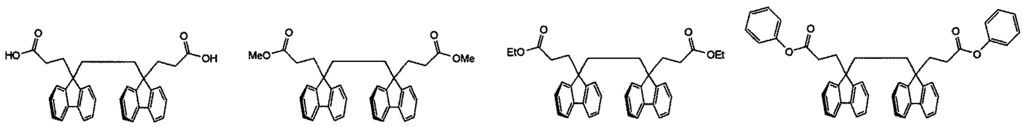

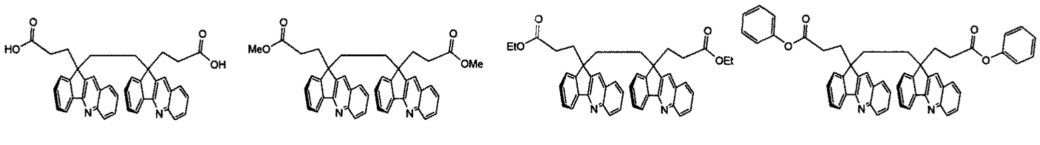

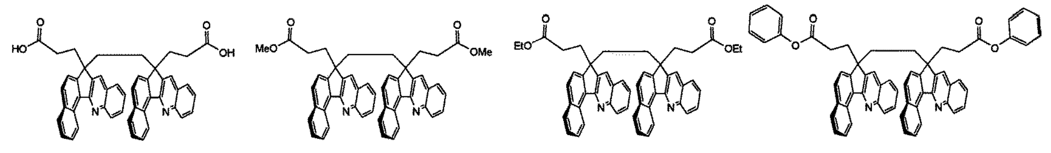

[Formula 159]

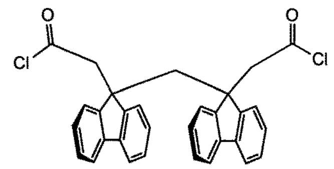

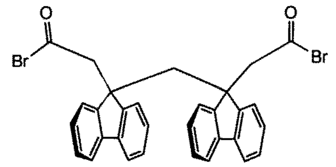

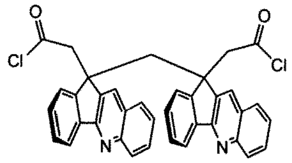

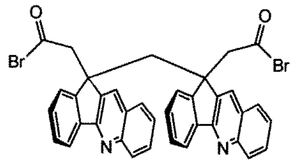

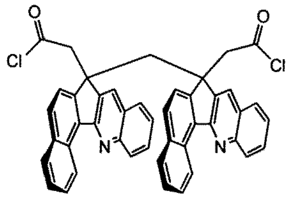

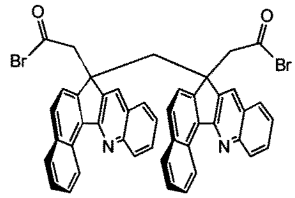

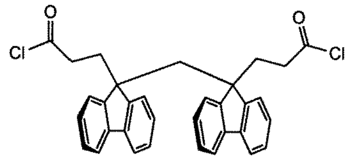

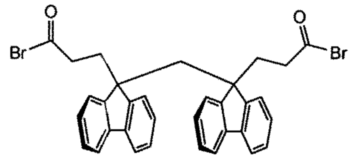

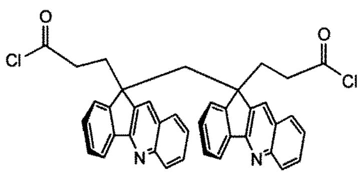

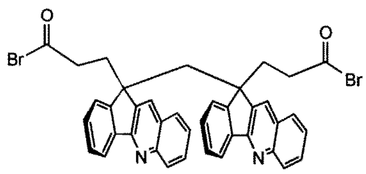

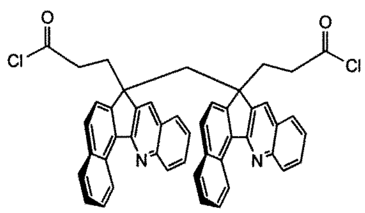

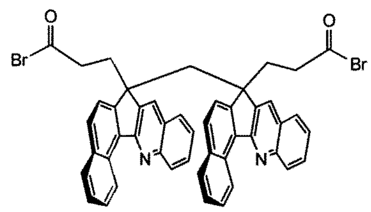

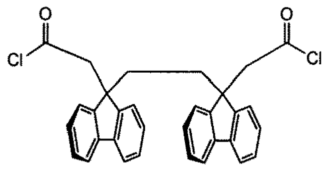
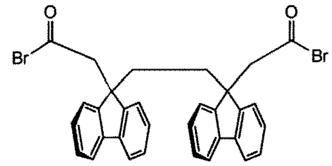

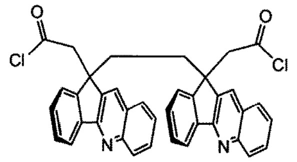
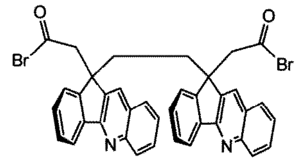

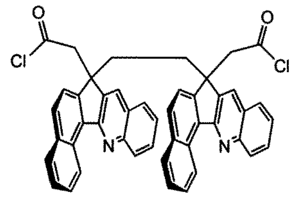
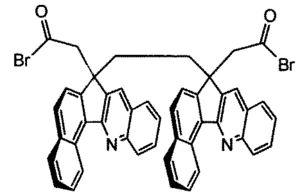

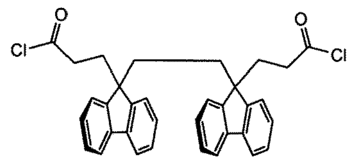
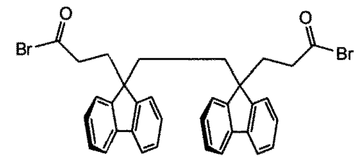

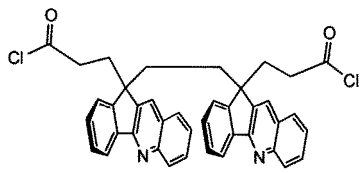
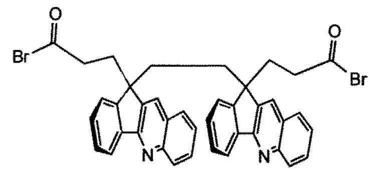

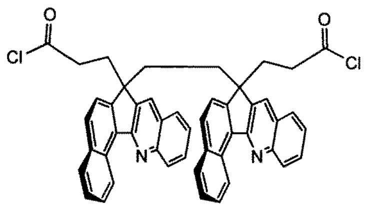
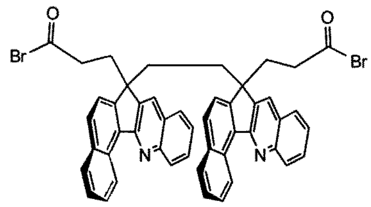

When the polycarbonate resin contains the structural unit (A6) and/or the structural unit (A7), from the viewpoint of improving optical properties, the viewpoint of allowing an optical film formed from the polycarbonate resin to have a preferred wavelength dispersion property, and the viewpoint of adjusting a birefringence to fall within a preferred range, the content of the structural unit (A6) and the content of the structural unit (A7) in the polycarbonate resin are each preferably 0.1 mol% or more, more preferably 1 mol% or more, even more preferably 3 mol% or more, even more preferably 4 mol% or more, particularly preferably 5 mol% or more and are each preferably 45 mol% or less, more preferably 35 mol% or less, even more preferably 25 mol% or less, even more preferably 17.5 mol% or less, particularly preferably 12.5 mol% or less with respect to the total molar quantity of all the structural units based on all the dihydroxy compounds, all the dicarboxylic acid compounds, and all the carbonates in the thermoplastic resin. From the same viewpoint, the content of the structural unit (A6) and the content of the structural unit (A7) are each preferably 2 mass% or more, more preferably 5 mass% or more, even more preferably 7.5 mass% or more, even more preferably 10 mass% or more, particularly preferably 15 mass% or more and are each preferably 90 mass% or less, more preferably 70 mass% or less, even more preferably 50 mass% or less, even more preferably 35 mass% or less, particularly preferably 25 mass% or less with respect to the total mass of the thermoplastic resin based on all the dihydroxy compounds, all the dicarboxylic acid compounds, and all the carbonates in the thermoplastic resin.

The polycarbonate resin may further contain a structural unit represented by the following formula (D6). In this case, the polycarbonate resin exhibits excellent optical properties. The polycarbonate resin may contain one or two or more structural units represented by the formula (D6). The structural unit represented by the formula (D6) is referred to as a "structural unit D6" as appropriate.

[Formula 160]

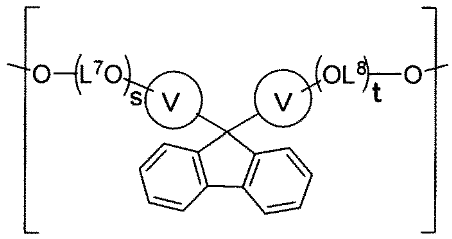

(D6)

In the formula (D6), V is an optionally-substituted arylene group, a substituent of V is a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom, a nitro group, or a cyano group, $L^7$ and $L^8$ are each independently an optionally-substituted alkylene group having 1 to 10 carbon atoms, an optionally-substituted arylene group having 4 to 10 carbon atoms, or an optionally-substituted aralkylene group having 6 to 12 carbon atoms, s is an integer of 0 to 4, and t is an integer of 0 to 4.

In the formula (D6), s and t are each independently an integer of 0 to 4, but from the viewpoint of heat resistance, s and t are preferably each independently an integer of 0 to 3, more preferably an integer of 0 to 2. From the viewpoint of adjusting glass transition temperature to achieve excellent molding processability and the viewpoint that a monomer material is inexpensively synthesized, s and t are particularly preferably each independently 0 or 1.

Further, from the viewpoint of ease of synthesis of a monomer material, the structural unit D6 is preferably represented by the following formula (D6-1).

[Formula 161]

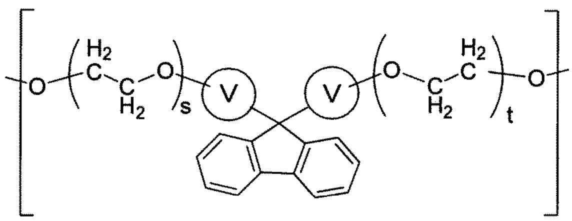

(D6-1)

In the formula (D6-1), V is an optionally-substituted phenylene or naphthylene group, a substituent of V is a hydrogen atom, an alkyl group having 1 to 4 carbon atoms, or an optionally-substituted aryl group having 6 to 20 carbon atoms, s is 0 or 1, and t is 0 or 1.

From the viewpoint of ease of synthesis of a monomer raw material and the viewpoint of adjustment to a preferred optical property (specifically, a preferred wavelength dispersion property), the structural unit D6 is preferably represented by the following formula (D6-2).

[Formula 162]

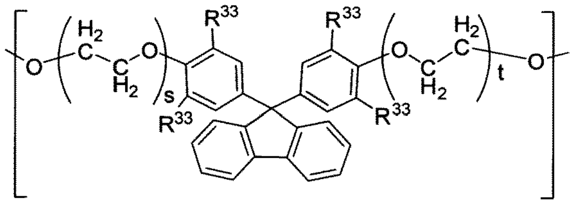

(D6-2)

In the formula (D6-2), $R^{33}$ is a hydrogen atom, a methyl group, or a phenyl group, s is 0 or 1, and t is 0 or 1.

Specific examples of the structural unit D6 include structural units shown below.

[Formula 163]

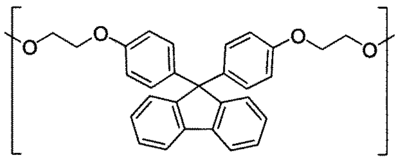

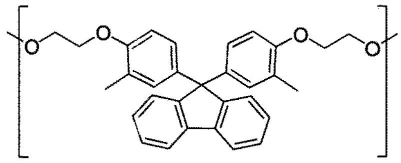

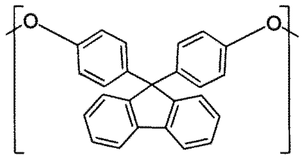

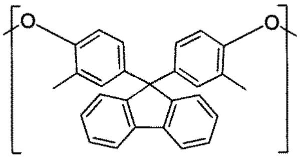

The polycarbonate resin having the structural unit D6 can be produced by, for example, a method such as polymerization of a monomer represented by the following formula (i).

[Formula 164]

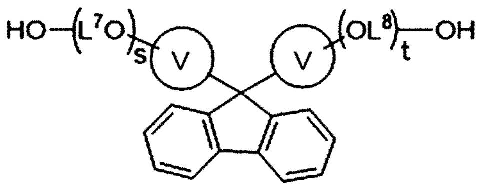

(i)

In the formula (i), V, $L^7$, $L^8$, s, and t are respectively the same as V, $L^7$, $L^8$, s and t in the formula (D6). Specific examples of the monomer represented by the formula (i) include monomers shown below.

[Formula 165]

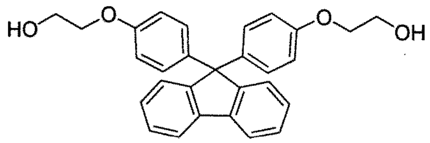

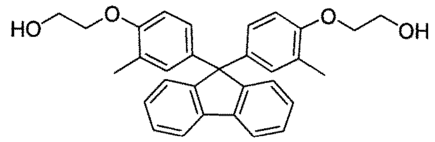

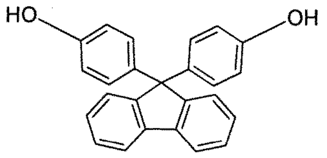

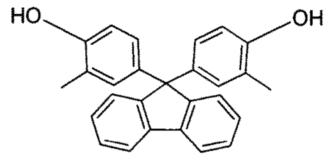

When the polycarbonate resin contains a structural unit derived from the structural unit D6, from the viewpoint of allowing an optical film using the polycarbonate resin to have a preferred wavelength dispersion property, the content of the structural unit is preferably 40 mass% or more, more preferably 45 mass% or more, even more preferably 50 mass% or more, particularly preferably 55 mass% or more, more particularly preferably 60 mass% or more with respect to the total mass of the thermoplastic resin based on all the dihydroxy compounds, all the dicarboxylic acid compounds, and all the carbonates in the thermoplastic resin.

If the content of the structural unit is excessively small, there is a case where an optical film using the polycarbonate resin does not have a preferred wavelength dispersion property. Further, if the content of the structural unit is excessively large, there is a case where an optical film using the polycarbonate resin does not have a preferred optical property because a ratio between a phase difference as measured at a wavelength of 450 nm and a phase difference as measured at a wavelength of 550 nm is excessively large. Therefore, the content of the structural unit is preferably 99 mass% or less, more preferably 95 mass% or less, even more preferably 90 mass% or less, even more preferably 87.5 mass% or less, particularly preferably 85 mass% or less with respect to the total mass of the thermoplastic resin based on all the dihydroxy compounds, all the dicarboxylic acid compounds, and all the carbonates in thermoplastic resin. It is noted that the phase difference ratio of the optical film refers to the ratio between the phase difference of the optical film as measured at a wavelength of 450 nm, λ(450) and the phase difference of the optical film as measured at a wavelength of 550 nm, λ(550)

(λ(450)/λ(550)).

The polycarbonate resin may further contain a structural unit represented by the following formula (D7). In this case, the polycarbonate resin can have improved toughness and heat resistance and absorb less water. Further, a raw material of the structural unit represented by the formula (D7) is inexpensively available. The structural unit represented by the formula (D7) is referred to as a "structural unit D7" as appropriate.

[Formula 166]

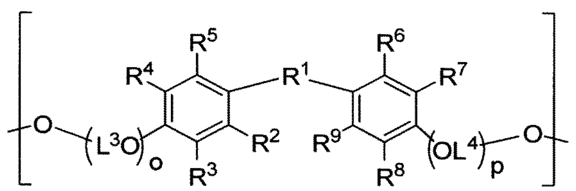

(D7)

In the formula (D7), $R^1$ is a direct bond, an oxygen atom, or an optionally-substituted alkylene group having 1 to 40 carbon atoms. $R^2$ to $R^9$ are each independently a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom having a substituent, a nitro group having a substituent, or a cyano group having a substituent. $L^3$ and $L^4$ are each independently an

optionally-substituted alkylene group having 1 to 10 carbon atoms, an optionally-substituted arylene group having 4 to 10 carbon atoms, or an optionally-substituted aralkylene group having 6 to 12 carbon atoms. o is an integer of 0 to 4, and p is an integer of 0 to 4.

From the viewpoint of heat resistance and ease of synthesis, $L^3$ and $L^4$ in the formula (D7) are preferably each independently an optionally-substituted alkylene group having 1 to 4 carbon atoms, more preferably an ethylene group. From the viewpoint of heat resistance and ease of synthesis, o and p in the formula (D7) are preferably each independently 0 or 1.

From the viewpoint of ease of synthesis, the formula (D7) is preferably represented by the following formula (D7-1).

[Formula 167]

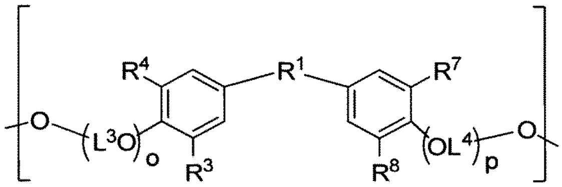

(D7-1)

In the formula (D7-1), $R^1$ is a direct bond, an oxygen atom, or an optionally-substituted alkylene group having 1 to 40 carbon atoms. $R^3$, $R^4$, $R^7$, and $R^8$ are each independently a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom, a nitro group, or a cyano group, $L^3$ and $L^4$ are each independently an optionally-substituted alkylene group having 1 to 10 carbon atoms, o is 0 or 1, and p is 0 or 1.

From the viewpoint of ease of synthesis due to a symmetric structure, the formula (D7) is preferably represented by the following formula (D7-2).

[Formula 168]

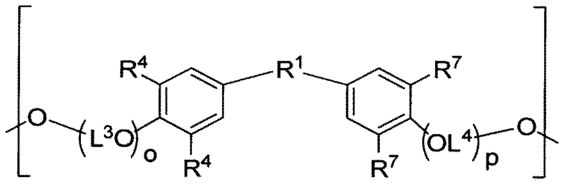

(D7-2)

In the formula (D7-2), $R^1$ is a direct bond, an oxygen atom, or an optionally-substituted alkylene group having 1 to 40 carbon atoms. $R^4$ and $R^7$ are each independently a hydrogen atom, an optionally-substituted alkyl group having 1 to 10 carbon atoms, an optionally-substituted aryl group having 3 to 14 carbon atoms, an optionally-substituted acyl group having 1 to 10 carbon atoms, an optionally-substituted alkoxy group having 1 to 10 carbon atoms, an optionally-substituted aryloxy group having 3 to 14 carbon atoms, an optionally-substituted acyloxy group having 1 to 10 carbon atoms, an optionally-substituted amino group, an optionally-substituted alkenyl group having 2 to 10 carbon atoms, an optionally-substituted alkynyl group having 2 to 10 carbon atoms, a silicon atom having a substituent, a halogen atom having a substituent, a nitro group having a substituent, or a cyano group having a substituent. $L^3$ and $L^4$ are each independently an optionally-substituted alkylene group having 1 to 10 carbon atoms. o is 0 or 1, and p is 0 or 1.

From the viewpoint that raw materials are inexpensively available and the viewpoint of improving heat resistance, the formula (D7) is preferably represented by the following formula (D7-3).

[Formula 169]

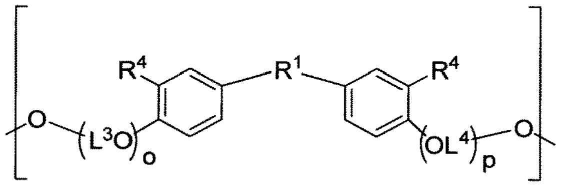

(D7-3)

In the formula (D7-3), $R^1$ is a direct bond, an oxygen atom, an optionally-substituted alkylene group having 1 to 40 carbon atoms. $R^4$ is a hydrogen atom or an optionally-substituted alkyl group having 1 to 4 carbon atoms, $L^3$ and $L^4$ are each independently an optionally-substituted alkylene group having 1 to 10 carbon atoms. o is 0 or 1, and p is 0 or 1.

When the alkylene group as $L^3$ or $L^4$ in the formula (D7-3) is long, heat resistance tends to reduce, and therefore, it is preferred that o and p are 0 or o or p is 1 and $L^3$ or $L^4$ is an alkylene group having 1 to 2 carbon atoms. From the viewpoint of ease of synthesis, the number of carbon atoms of the alkylene group is more preferably 2.

From the viewpoint of high heat resistance, $R^1$ in the formula (D7-3) is preferably a direct bond or an oxygen atom. From the viewpoint of ease of synthesis and adjustment to high toughness, $R^1$ in the formula (D7-3) is preferably a methylene group, an alkylmethylene group having 2 to 40 carbon atoms, or a dialkylmethylene group having 3 to 40 carbon atoms, more preferably an alkylmethylene group having 2 to 40 carbon atoms.

From the viewpoint of heat resistance, $R^1$ in the formula (D7-3) more preferably has a methylene group. From the viewpoint of ease of synthesis and improvement in toughness can be improved, $R^1$ in the formula (D7-3) is more preferably an alkylmethylene group having 2 to 40 carbon atoms. From the viewpoint that raw materials are inexpensively available, the number of carbon atoms of the alkylmethylene group is preferably 2 to 4. From the viewpoint of further improving toughness, the number of carbon atoms of the alkylmethylene group is preferably 3 or more, more preferably 10 or more, even more preferably 12 or more. From the viewpoint of further improving heat resistance, the number of carbon atoms of the alkylmethylene group is preferably 40 or less, more preferably 30 or less, even more preferably 20 or less. From the viewpoint of balance between improvement in toughness and heat resistance, the number of carbon atoms of the alkylmethylene group is preferably 7 to 15.

From the viewpoint of heat stability, $R^1$ in the formula (D7-3) is preferably a dialkylmethylene group having 3 to 40 carbon atoms. From the viewpoint of toughness, the number of carbon atoms of the dialkylmethylene group is preferably 5 or more, more preferably 10 or more, even more preferably 20 or more. From the viewpoint of heat resistance, the number of carbon atoms of the dialkylmethylene group is preferably 40 or less, more preferably 30 or less, even more preferably 20 or less. From the viewpoint of ease of synthesis, the number of carbon atoms of the dialkylmethylene group is preferably 3 to 10.

From the viewpoint of heat resistance, $R^4$ in the formula (D7-3) is preferably a hydrogen atom. Further, from the viewpoint that raw materials are inexpensively available and the viewpoint of toughness, $R^4$ in the formula (D7-3) is preferably a methyl group.

From the viewpoint of high heat resistance, o and p in the formula (D7-3) are preferably 0. Further, from the viewpoint of toughness, o and p in the formula (D7-3) are preferably 1.

A specific example of the formula (D7) is any of structures shown below.

[Formula 170]

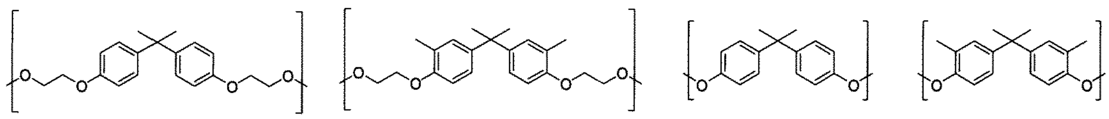

The polycarbonate resin having the structural unit D7 is produced by, for example, polymerization of a monomer represented by the following formula (g).

[Formula 171]

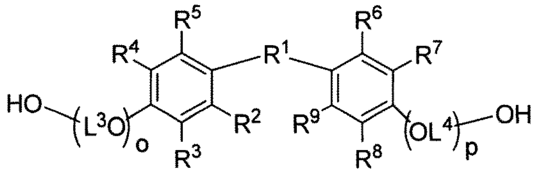

(g)

$R^1$ to $R^9$, $L^3$, $L^4$, o, and p in the formula (g) are respectively the same as $R^1$ to $R^9$, $L^3$, $L^4$, o, and p in the formula (D7). Specific examples of the monomer represented by the formula (g) include monomers shown below.

[Formula 172]

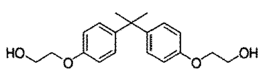 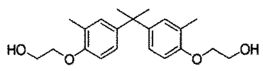 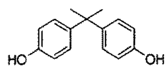 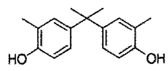

From the viewpoint of improving moist heat resistance and toughness of the polycarbonate resin, the content of the structural unit (D7) is preferably 5 mass% or more, more preferably 10 mass% or more, even more preferably 15 mass% or more, even more preferably 25 mass% or more, particularly preferably 35 mass% or more with respect to the total mass of the polycarbonate resin based on all the dihydroxy compounds and all the carbonates constituting the polycarbonate resin. Further, from the viewpoint of improving weatherability, the content of the structural unit (D7) is preferably 95 mass% or less, more preferably 80 mass% or less, even more preferably 65 mass% or less, even more preferably 55 mass% or less, particularly preferably 45 mass% or less with respect to the total mass of the polycarbonate resin based on all the dihydroxy compounds and all the carbonates constituting the polycarbonate resin.

[Method for producing thermoplastic resin]

The polycarbonate resin or the polyester carbonate resin is produced by a general polymerization method.

<Polycarbonate resin>

The polycarbonate resin is produced by a polymerization method generally used. Specific examples of the polymerization method include solution polymerization using phosgene and melt polymerization involving a reaction between a monomer material and a diester carbonate. Either of these methods may be used, but melt polymerization is preferred which involves a reaction between a raw material monomer (specifically, a dihydroxy compound) and a diester carbonate less toxic to the environment in the presence of a polymerization catalyst.

As the diester carbonate, one represented by the following formula (o) can be used.

[Formula 173]

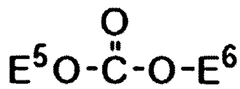

(o)

In the formula (o), $E^5$ and $E^6$ are each independently an optionally-substituted aliphatic group having 1 to 18 carbon atoms or an optionally-substituted aromatic group having 6 to 12 carbon atoms.

Examples of the diester carbonate represented by the formula (o) include diphenyl carbonate and a substituted diphenyl carbonate typified by ditolyl carbonate. Other examples of the diester carbonate represented by the formula (o) include dimethyl carbonate, diethyl carbonate, and di-t-butyl carbonate. Preferred is diphenyl carbonate or a substituted diphenyl carbonate. As the diester carbonate, one of these compounds may be used or two or more of these compounds may be used.

The diester carbonate may be substituted by a dicarboxylic acid or a dicarboxylic acid ester. The amount of the substituted diester carbonate is preferably 50 mol% or less, more preferably 30 mol% or less with respect to the amount of

all the diester carbonates. Examples of the dicarboxylic acid and the dicarboxylic acid ester include terephthalic acid, isophthalic acid, diphenyl terephthalate, and diphenyl isophthalate. A dicarboxylic acid compound as a raw material for introducing the above-described structure (A6) or (A7) may also be used. When the diester carbonate is substituted by such a dicarboxylic acid or dicarboxylic acid ester, a polyester carbonate resin is obtained.

The amount of the diester carbonate used with respect to the amount of all the dihydroxy compounds used in the reaction is preferably 0.90 to 1.10, more preferably 0.96 to 1.04 in terms of mole ratio. When the amount of the diester carbonate used is smaller than 0.90, the amount of terminal OH groups of the polycarbonate resin may increase so that the heat stability of the polymer may deteriorate or a desired high-molecular-weight polymer cannot be obtained. On the other hand, if the amount of the diester carbonate exceeds 1.10, there is a possibility that the speed of a transesterification reaction decreases under the same polymerization conditions or it is difficult to produce a polycarbonate resin having a desired molecular weight, and in addition to that, the amount of the diester carbonate remaining in the polycarbonate resin increases. The remaining diester carbonate becomes a cause of odor during molding or odor of a molded article.

As the raw material dihydroxy compound, a dihydroxy compound for introducing the structural unit A1 and a dihydroxy compound added if necessary to introduce another structural unit are used. As described above, the blending ratio between these dihydroxy compounds can appropriately be adjusted to achieve desired physical properties and characteristics.

As the polymerization catalyst (transesterification catalyst) in melt polymerization, an alkali metal compound and/or an alkaline-earth metal compound are/is used. A basic compound may supplementarily be used together with the alkali metal compound and/or the alkaline-earth metal compound, and examples such a basic compound include a basic boron compound, a basic phosphorus compound, a basic ammonium compound, and an amine-based compound. Preferably, only the alkali metal compound and/or the alkaline-earth metal compound are/is used.

Examples of the alkali metal compound used as the polymerization catalyst include sodium hydroxide, potassium hydroxide, lithium hydroxide, cesium hydroxide, sodium hydrogen carbonate, potassium hydrogen carbonate, lithium hydrogen carbonate, cesium hydrogen carbonate, sodium carbonate, potassium carbonate, lithium carbonate, cesium carbonate, sodium acetate, potassium acetate, lithium acetate, cesium acetate, sodium stearate, potassium stearate, lithium stearate, cesium stearate, sodium boron hydride, potassium boron hydride, lithium boron hydride, cesium boron hydride, sodium phenylboron, potassium phenylboron, lithium phenylboron, cesium phenylboron, sodium benzoate, potassium benzoate, lithium benzoate, cesium benzoate, disodium hydrogen phosphate, dipotassium hydrogen phosphate, dilithium hydrogen phosphate, dicesium hydrogen phosphate, disodium phenyl phosphate, dipotassium phenyl phosphate, dilithium phenyl phosphate, and dicesium phenyl phosphate. Other examples of the alkali metal compound used as the polymerization catalyst include: an alcoholate of sodium, potassium, lithium, or cesium; a disodium salt, a dipotassium salt, a dilithium salt, or a dicesium salt of phenolate; and a disodium salat, a dipotassium salt, a dilithium salt, or a dicesium salt of bisphenol A.

Examples of the alkaline-earth metal compound include potassium hydroxide, barium hydroxide, magnesium hydroxide, strontium hydroxide, calcium hydrogen carbonate, barium hydrogen carbonate, magnesium hydrogen carbonate, strontium hydrogen carbonate, calcium carbonate, barium carbonate, magnesium carbonate, strontium carbonate, calcium acetate, barium acetate, magnesium acetate, strontium acetate, calcium stearate, barium stearate, magnesium stearate, and strontium stearate.

As the alkali metal compound and/or the alkaline earth metal compound, one or two or more of these compounds may be used.

Specific examples of the basic boron compound include metallic salts of tetramethylboron, tetraethylboron, tetrapropylboron, tetrabutylboron, trimethylethylboron, trimethylbenzylboron, trimethylphenylboron, triethylmethylboron, triethylbenzylboron, triethylphenylboron, tributylbenzylboron, tributylphenylboron, tetraphenylboron, benzyltriphenylboron, methyltriphenylboron, and butyltriphenylboron. Examples of the metallic salts include a sodium salt, a potassium salt, a lithium salt, a calcium salt, a barium salt, a magnesium salt, and a strontium salt.

Examples of the basic phosphorus compound include triethylphosphine, tri-n-propylphosphine, triisopropylphosphine, tri-n-butylphosphine, triphenylphosphine, tributylphosphine, and a quaternary phosphonium salt.

Examples of the basic ammonium compound include tetramethylammonium hydroxide, tetraethylammonium hydroxide, tetrapropylammonium hydroxide, tetrabutylammonium hydroxide, trimethylethylammonium hydroxide, trimethylbenzylammonium hydroxide, trimethylphenylammonium hydroxide, triethylmethylammonium hydroxide, triethylbenzylammonium hydroxide, triethylphenylammonium hydroxide, tribuylbenzylammonium hydroxide, tributylphenylammonium hydroxide, tetraphenylammonium hydroxide, benzyltriphenylammonium hydroxide, methyltriphenylammonium hydroxide, and butyltriphenylammonium hydroxide.

Examples of the amine-based compound include 4-aminopyridine, 2-aminopyridine, N,N-dimethyl-4-aminopyridine, 4-diethylaminopyridine, 2-hydroxypyridine, 2-methoxypyridine, 4-methoxypyridine, 2-dimethylaminoimidazole, 2-methoxyimidazole, imidazole, 2-mercaptoimidazole, 2-methylimidazole, and aminoquinoline.

As the basic compound, one or two or more of these compounds can be used.

When the alkali metal compound and/or the alkaline earth metal compound are/is used as the polymerization

catalyst, the amount of the polymerization catalyst used per mole of all the dihydroxy compounds used in the reaction is usually 0.1 to 500 μmol, preferably 0.5 to 300 μmol, more preferably 1 to 250 μmol in terms of the amount of metal. If the amount of the polymerization catalyst used is too small, there is a possibility that polymerization activity to produce a polycarbonate resin having a desired molecular weight cannot be obtained. On the other hand, if the amount of the polymerization catalyst used is too large, there is a possibility that the hue of the polycarbonate resin deteriorates, flowability reduces dur to the generation of a by-product, or a large amount of gel is generated. As a result, it is difficult to produce a polycarbonate resin having desired quality.

Each of the raw material dihydroxy compounds used to produce the polycarbonate resin may be supplied as a solid, or may be supplied in a melt state by heating, or may be supplied as an aqueous solution when soluble in water. When the raw material dihydroxy compound is supplied in a melt state or as an aqueous solution, there is an advantage that it can easily be weighed or transported in industrial production.

The method in which a raw material dihydroxy compound is reacted with a diester carbonate in the presence of a polymerization catalyst is usually performed in a multistage process having two or more stages. Specifically, the reaction of the first stage is performed at a temperature of 140 to 220°C, preferably 150 to 200°C for 0.1 to 10 hours, preferably 0.5 to 3 hours. In and after the second stage, the reaction is performed by increasing the reaction temperature and gradually reducing the pressure as compared to the first stage while generated phenol is discharged to the outside of the reaction system. Finally, a polycondensation reaction is performed at a pressure in the reaction system of 200 Pa or less and a temperature in the range of 210 to 280°C.

Pressure reduction in the polycondensation reaction is preferred performed by controlling a balance between the temperature and the pressure in the reaction system. Particularly, if either of the temperature and the pressure is too quickly changed, there is a possibility that an unreacted monomer distills so that the degree of polymerization decreases due to an inappropriate mole ratio between the dihydroxy compound and the diester carbonate.

Specifically, when, for example, a dihydroxy compound represented by the formula (P1) such as a compound represented by the formula (P24), isosorbide, and 1,4-cyclohexanedimethanol are used as the dihydroxy compounds and the ratio of 1,4-cyclohexanedimethanol used to all the dihydroxy compounds in terms of mole ratio is 50 mol% or more, 1,4-cyclohexanedimethanol is likely to directly distill as a monomer. Therefore, the polycondensation reaction is preferably performed by increasing the temperature at a temperature rise rate of 40°C/h or less until the pressure in the reaction system is reduced to about 13 kPa, then increasing the temperature at a temperature rise rate of 40°C/h or less at a pressure of up to about 6.67 kPa, and finally keeping the temperature at 200 to 250°C at a pressure of 200 Pa or less. In this case, a polycarbonate resin having a sufficiently high polymerization degree is obtained.

When the ratio of 1,4-cyclohexanedimethanol used to all the dihydroxy compounds in terms of mole ratio is less than 50 mol%, a sudden viscosity increase is likely to occur, and when the ratio is 30 mol% or less, a sudden viscosity increase is more likely to occur. Therefore, the polycondensation reaction is preferably performed by increasing the temperature at a temperature rise rate of 40°C/h or less until the pressure in the reaction system is reduced to about 13 kPa, then increasing the temperature at a temperature rise rate of 40°C/h or more at a pressure of up to about 6.67 kPa, and finally keeping the temperature at 220 to 290°C at a reduced pressure of 200 Pa or less. In this case, a polycarbonate resin having a sufficiently high polymerization degree is obtained. It is noted that the temperature rise at a pressure of up to about 6.67 kPa is more preferably performed at a temperature rise rate of 50°C/h.

The reaction may be performed either in batch or continuous manner. Alternatively, the reaction may be performed by a combination of batch and continuous operations.

When the polycarbonate resin is produced by melt polymerization, a phosphate compound, a phosphite compound, a metallic salt of phosphoric acid, or a metallic salt of phosphorous acid may be added during polymerization for the purpose of preventing coloration.

Preferred examples of the phosphate compound include trialkyl phosphates such as trimethyl phosphate and triethyl phosphate. As the phosphate compound, one or two or more of these compounds can be used. The amount of the phosphate compound added is preferably 0.0001 mol% or more and 0.005 mol% or less, more preferably 0.0003 mol% or more and 0.003 mol% or less with respect to the amount of all the dihydroxy compounds used in the reaction. If the amount of the phosphate compound added is smaller than the above lower limit, the effect of preventing coloration is small. On the other hand, if the amount of the phosphate compound added is larger than the above upper limit, an increase in haze may be caused, the addition may promote coloration by the opposite effect, or heat resistance may reduce.

As the phosphite compound, the following compounds used as a heat stabilizer can be used. Specific examples of the phosphite compound include trimethyl phosphite, triethyl phosphite, trisnonylphenyl phosphite, trimethyl phosphate, tris(2,4-di-tert-butylphenyl) phosphite, bis(2,4-di-tert-butylphenyl) pentaerythritol diphosphite. As the phosphite compound, one or two or more of these compounds are used.

The amount of the phosphite compound added is preferably 0.0001 mol% or more and 0.005 mol% or less, more preferably 0.0003 mol% or more and 0.003 mol% or less with respect to the amount of all the dihydroxy compounds used in the reaction. If the amount of the phosphite compound added is smaller than the above lower limit, the effect of preventing coloration is small. On the other hand, if the amount of the phosphite compound added is larger than the above upper limit,

an increase in haze may be caused, the addition may promote coloration by the opposite effect, or heat resistance may reduce.

The phosphate compound and a metallic salt thereof or the phosphite compound and a metallic salt thereof may be used in combination. In this case, the total amount of the compound and the metallic salt added is preferably 0.0001 mol% or more and 0.005 mol% or less, more preferably 0.0003 mol% or more and 0.003 mol% or less with respect to the amount of all the dihydroxy compounds. If the amount added is smaller than the above lower limit, the effect of preventing coloration is small. On the other hand, if the amount added is larger than the above upper limit, an increase in haze may be caused, the addition may promote coloration by the opposite effect, or heat resistance may reduce.

It is noted that the metallic salt of the phosphate compound and the metallic salt of the phosphite compound are preferably alkali metal salts or zinc salts, more preferably zinc salts. Among zinc phosphates, a zinc long-chain alkyl phosphate is preferred.

Further, the polycarbonate resin may contain a heat stabilizer to prevent a reduction in molecular weight or deterioration of hue during molding or the like.

Examples of such a heat stabilizer include phosphorous acid, phosphoric acid, phosphonous acid, phosphonic acid, and esters thereof. Specific examples thereof include triphenyl phosphite, tris(nonylphenyl) phosphite, tris(2,4-di-tert-butylphenyl) phosphite, tridecyl phosphite, trioctyl phosphite, trioctadecyl phosphite, didecylmonophenyl phosphite, dioctylmonophenyl phosphite, diisopropylmonophenyl phosphite, monobutyldiphenyl phosphite, monodecyldiphenyl phosphite, monooctyldiphenyl phosphite, bis(2,6-di-tert-butyl-4-methylphenyl) pentaerythritol diphosphite, 2,2-methylenebis(4,6-di-tert-butylphenyl)octyl phosphite, bis(nonylphenyl) pentaerythritol diphosphite, bis(2,4-di-tert-butylphenyl) pentaerythritol diphosphite, distearylpentaerythritol diphosphite, tributyl phosphate, triethyl phosphate, trimethyl phosphate, triphenyl phosphate, diphenyl monoorthoxenyl phosphate, dibutyl phosphate, dioctyl phosphate, diisopropyl phosphate, tetrakis(2,4-di-tert-butylphenyl) 4,4'-biphenylene diphosphinate, dimethyl benzenephosphonate, diethyl benzenephosphonate, and dipropyl benzenephosphonate.

Among them, trisnonylphenyl phosphite, trimethyl phosphate, tris(2,4-di-tert-butylphenyl) phosphite, bis(2,4-di-tert-butylphenyl) pentaerythritol diphosphite, bis(2,6-di-tert-butyl-4-methylphenyl) pentaerythritol diphosphite, and dimethyl benzenephosphonate are preferably used.

As the heat stabilizer, one of these compounds may be used or two or more of these compounds may be used.

The heat stabilizer may additionally be added after added during melt polymerization.

That is, after a polycarbonate resin is obtained by adding an appropriate amount of a phosphite compound or a phosphate compound, the heat stabilizer such as a phosphite compound may additionally be added. In this case, deterioration of hue of the polycarbonate resin can further be prevented.

The amount of the heat stabilizer added is preferably 0.0001 to 1 part by mass, more preferably 0.0005 to 0.5 parts by mass, even more preferably 0.001 to 0.2 parts by mass per 100 parts by mass of the polycarbonate resin.

[Polycarbonate resin composition]

Various additives may be added to the polycarbonate resin to obtain a resin composition.

An antioxidant may be added to the polycarbonate resin. In this case, oxidation of the polycarbonate resin can be prevented.

Examples of the antioxidant include pentaerythritol tetrakis(3-mercaptopropionate), pentaerythritol tetrakis(3-laurylthiopropionate), glycerol-3-stearylthiopropionate, triethylene glycol-bis[3-(3-tert-butyl-5-methyl-4-hydroxyphenyl) propionate], 1,6-hexanediol-bis[3-(3,5-di-tert-butyl-4-hydroxyphenyl)propionate], pentaerythritol-tetrakis[3-(3,5-di-tert-butyl-4-hydroxyphenyl)propionate], octadecyl-3-(3,5-di-tert-butyl-4-hydroxyphenyl)propionate, 1,3,5-trimethyl-2,4,6-tris(3,5-di-tert-butyl-4-hydroxybenzyl)benzene, N,N-hexamethylenebis(3,5-di-tert-butyl-4-hydroxy-hydrocinnamide), diethyl 3,5-di-tert-butyl-4-hydroxy-benzylphosphonate, tris(3,5-di-tert-butyl-4-hydroxybenzyl) isocyanurate, tetrakis(2,4-di-tert-butylphenyl) 4,4'-biphenylene diphosphinate, and 3,9-bis{1,1-dimethyl-2-[β-(3-tert-butyl-4-hydroxy-5-methylphenyl)propionyloxy]ethyl}-2,4,8,10-tetraoxaspiro(5,5)undecane. As the antioxidant, one or two or more of these compounds can be used.

The amount of the antioxidant added is preferably 0.0001 to 0.5 parts by mass per 100 parts by mass of the polycarbonate resin.

Further, a releasing agent may be added to the polycarbonate resin. In this case, releasability from a mold in melt molding is improved.

Examples of the releasing agent include a higher fatty acid ester of a monohydric or polyhydric alcohol, a higher fatty acid, paraffin wax, bees wax, olefin-based wax, olefin-based wax containing a carboxyl group and/or a carboxylic anhydride group, silicone oil, and organopolysiloxane.

As the higher fatty acid, a partial or total ester of a monohydric or polyhydric alcohol having 1 to 20 carbon atoms and a saturated fatty acid having 10 to 30 carbon atoms is preferred. Examples of the partial or total ester of a monohydric or polyhydric alcohol and a saturated fatty acid include monoglyceride stearate, diglyceride stearate, triglyceride stearate,

monosorbitate stearate, stearyl stearate, monoglyceride behenate, behenyl behenate, pentaerythritol monostearate, pentaerythritol tetrastearate, pentaerythritol tetrapelargonate, propylene glycol monostearate, stearyl stearate, palmityl palmitate, butyl stearate, methyl laurate, isopropyl palmitate, biphenyl biphenate, sorbitan monostearate, and 2-ethylhexyl stearate.

Among them, monoglyceride stearate, triglyceride stearate, pentaerythritol tetrastearate, and behenyl behenate are preferably used.

As the higher fatty acid, a saturated fatty acid having 10 to 30 carbon atoms is preferred. Examples of such a fatty acid include myristic acid, lauric acid, palmitic acid, stearic acid, and behenic acid.

As the releasing agent, one of these compounds may be used or two or more of these compounds may be used.

The amount of the releasing agent added is preferably 0.01 to 5 parts by mass per 100 parts by mass of the polycarbonate resin.

Further, a light stabilizer may be added to the polycarbonate resin.

Examples of the light stabilizer include 2-(2'-hydroxy-5'-tert-octylphenyl)benzotriazole, 2-(3-tert-butyl-5-methyl-2-hydroxyphenyl)-5-chlorobenzotriazole, 2-(5-methyl-2-hydroxyphenyl)benzotriazole, 2-[2-hydroxy-3,5-bis($\alpha$,$\alpha$-dimethylbenzyl)phenyl]-2H-benzotriazole, 2,2'-methylenebis(4-cumyl-6-benzotriazolephenyl), and 2,2'-p-phenylenebis(1,3-benzoxazin-4-one).

As the heat stabilizer, one of these compounds may be used or two or more of these compounds may be used.

The amount of the light stabilizer added is preferably 0.01 to 2 parts by mass per 100 parts by mass of the polycarbonate resin.

Further, a bluing agent may be added to the polycarbonate resin to cancel out yellow tint. As the bluing agent, any bluing agent can be used without any problem as long as it can be used for polycarbonate resins. From the viewpoint of availability, an anthraquinone-based dye is preferred.

Specific typical examples of the bluing agent include, common name, Solvent Violet 13 [CA. No. (color index No.) 60725], common name, Solvent Violet 31 [CA. No. 68210], common name, Solvent Violet 33 [CA. No. 60725], common name, Solvent Blue 94 [CA. No. 61500], common name, Solvent Violet 36 [CA. No. 68210], common name, Solvent blue 97 ["Macrolex Violet RR" manufactured by Bayer AG], and common name, Solvent Blue 45 [CA. No. 61110].

These bluing agents may be used singly or in combination of two or more of them.

The amount of the bluing agent added is usually $0.1 \times 10^{-4}$ to $2 \times 10^{-4}$ parts by mass per 100 parts by mass of the polycarbonate resin.

The polycarbonate resin may be used by blending with a dissimilar material resin such as another thermoplastic resin. From the viewpoint of excellent optical performance and the viewpoint that injection molding tends to become possible, the polycarbonate resin preferably coexists with another thermoplastic resin. Specific examples of another thermoplastic resin allowed to coexist with the polycarbonate resin include a polycondensation-type polymer, an olefin-based polymer, and an addition-polymerization-type polymer, and a polycondensation-type polymer is preferred.

Specific examples of the olefin-based polymer include polyethylene and polypropylene, specific examples of the polycondensation polymer include a polyester, a polyamide, a polyester carbonate, a polyamide, and a polyimide.

The thermoplastic resin and another thermoplastic resin are preferably compatible with each other. In this case, when the thermoplastic resin has excellent transparency, it is possible to prevent a reduction in such transparency.

The resin composition obtained by blending is a product in which a polymer having at least the structural unit B1 and another polymer different from this polymer coexist as polymers. In this method, the divalent structural unit represented by the general formula (B1) may be contained in the resin composition at any molar fraction as long as mechanical properties, heat resistance, and optical properties can be balanced, and from the viewpoint of improving surface hardness of a resulting resin or reducing photoelastic coefficient, the content of the divalent structural unit represented by the general formula (B1) is preferably 1 mol% or more, more preferably 5 mol% or more, even more preferably 15 mol% or more, even more preferably 25 mol% or more, particularly preferably 37.5 mol% or more with respect to the total molar quantity of all the structural units contained in the resin composition. The maximum content of the structural unit (B1) is 50 mol%.

From the same viewpoint, the content of the structural unit (B1) is preferably 1 mass% or more, more preferably 10 mass% or more, even more preferably 40 mass% or more, even more preferably 55 mass% or more, particularly preferably 75 mass% or more with respect to the total mass of all the structural units contained in the resin composition.

On the other hand, from the viewpoint of achieving both moist heat resistance of a resulting resin and film strength, the content of the structural unit (B1) is preferably 0.1 mol% or more, more preferably 1 mol% or more, even more preferably 2 mol% or more, even more preferably 4 mol% or more, particularly preferably 5 mol% or more with respect to the total molar quantity of all the structural units contained in the resin composition. Further, the content of the structural unit (B1) is preferably 50 mol% or less, more preferably 37.5 mol% or less, even more preferably 25 mol% or less, even more preferably 20 mol% or less, particularly preferably 15 mol% or less.

From the same viewpoint, the content of the structural unit (B1) is preferably 1 mass% or more, more preferably 2 mass% or more, even more preferably 5 mass% or more, even more preferably 7 mass% or more, particularly preferably

10 mass% or more with respect to the total mass of all the structural units contained in the resin composition. Further, the content of the structural unit (B1) is preferably 95 mass% or less, more preferably 80 mass% or less, even more preferably 60 mass% or less, even more preferably 50 mass% or less, particularly preferably 40 mass% or less.

<Polyester carbonate resin>

The polyester carbonate resin is produced by a polymerization method usually used. The production method will be described below.

A polyester carbonate is obtained by replacing part of a diester carbonate used for polymerization with a dicarboxylic acid compound. The dicarboxylic acid compound is, for example, a dicarboxylic acid compound represented by the general formula (j2). Examples of another compound having carboxy groups include: an alicyclic dicarboxylic acid such as 1,2-cyclohexane dicarboxylic acid, 1,3-cyclohexane dicarboxylic acid, or 1,4-cyclohexane dicarboxylic acid; and an aliphatic dicarboxylic acid such as malonic acid, succinic acid, glutaric acid, adipic acid, pimelic acid, or suberic acid. Depending on a production method, a dicarboxylic acid ester such as a methyl ester of a dicarboxylic acid or a phenyl ester of a dicarboxylic acid or a dicarboxylic acid derivative such as a dicarboxylic acid halide may also be used as a raw material. From the viewpoint of heat resistance of a resulting polyester carbonate and the viewpoint of reducing photoelastic coefficient, an alicyclic dicarboxylic acid is preferred, and particularly from the viewpoint of handleability and availability, 1,4-cyclohexane dicarboxylic acid is preferred. These dicarboxylic acid components may directly be used as a raw material of the polyester carbonate, but depending on a production method, a dicarboxylic acid ester such as a methyl ester of a dicarboxylic acid or a phenyl ester of a dicarboxylic acid or a dicarboxylic acid derivative such as a dicarboxylic acid halide may also be used as a raw material.

The content of a structural unit derived from a dicarboxylic acid compound in the polyester carbonate is 45 mol% or less, preferably 30 mol% or less, particularly preferably 20 mol% or less when the total amount of structural units derived from all the dihydroxy compounds and all the carboxylic acid compounds is taken as 100 mol%. If the content of the structural unit derived from a dicarboxylic acid compound exceeds the above upper limit, there is a case where polymerizability reduces so that polymerization does not proceed to the extent that a desired molecular weight is achieved.

The polyester carbonate resin may contain additives. As the additives, for example, those described above with reference to the polycarbonate resin may be used.

The thermoplastic resin such as the polycarbonate resin or the polyester carbonate resin and the above-described various additives are mixed by, for example, a method using a tumbler, a V-type blender, a SUPERMIXER, a Nauta mixer, a Banbury mixer, a kneading roll, or an extruder. Another mixing method is a solution blending method in which components are mixed in a state where they are dissolved in a common good solvent such as methylene chloride. The mixing method is not particularly limited, and any mixing method may be used as long as it is a polymer blending method usually used.

The thermoplastic resin or the thermoplastic resin composition obtained by adding an additive or the like thereto may directly be molded or may be molded after once pelletized by a melt extruder. The molding is performed by a commonly-known method such as injection molding, extrusion molding, or compression molding.

In order to obtain stable releasability and various physical properties by increasing miscibility of the thermoplastic resin, a single-screw extruder or a twin-screw extruder is preferably used in melt extrusion. In this case, the use of a solvent or the like can be avoided, and therefore an environmental load can be reduced and productivity can further be improved.

A melt kneading temperature depends on the glass transition temperature of the thermoplastic resin. For example, when the glass transition temperature of the thermoplastic resin is lower than 90°C, the melt kneading temperature of the extruder is usually 130°C to 250°C, preferably 150°C to 240°C. If the melt kneading temperature is lower than 130°C, a heavy load may be imposed on the extruder due to an increase in the melt viscosity of the thermoplastic resin so that productivity may reduce. On the other hand, if the melt kneading temperature is higher than 250°C, the melt viscosity of the thermoplastic resin decreases, which makes it difficult to obtain pellets. As a result, t productivity may reduce.

Further, when the glass transition temperature of the thermoplastic resin is 90°C or higher, the melt kneading temperature is usually 200°C to 300°C, preferably 220°C to 260°C. If the melt kneading temperature is lower than 200°C, a heavy load may be imposed on the extruder due to an increase in the melt viscosity of the thermoplastic resin productivity may reduce. On the other hand, if the melt kneading temperature is higher than 300°C, the thermoplastic resin is likely to deteriorate. Specifically, there is a possibility that yellowing of the thermoplastic resin occurs or the strength of the thermoplastic resin deteriorates due to a reduction in molecular weight.

When the extruder is used, a filter is preferably disposed to prevent burning of the thermoplastic resin or entry of foreign matter during extrusion. The opening of the filter (specifically, the size of foreign matter to be removed) depends on the intended use, physical properties, and characteristics of the thermoplastic resin, but is preferably 100 $\mu$m or less. Particularly, when it is necessary to avoid the entry of foreign matter, the opening of the filter is more preferably 40 $\mu$m or less, even more preferably 10 $\mu$m or less.

The extrusion of the thermoplastic resin is preferably performed in a clean room. In this case, it is possible to prevent the entry of foreign matter into the thermoplastic resin after extrusion.

Further, when the extruded thermoplastic resin is formed into chips by cooling, the cooling is preferably air cooling, water cooling, or the like. In the case of air cooling, air is preferably passed through a hepafilter or the like before use to remove foreign matter. In this case, it is possible to prevent reattachment of foreign matter contained in air. In the case of water cooling, water is preferably passed through an ion-exchange resin or the like to remove metallic components and then through a filter to remove foreign matter before use. The opening of the filter may appropriately be selected, but is preferably 0.45 to 10 μm.

The physical properties and characteristics of the thermoplastic resin can be adjusted depending on the intended use.

<Glass transition temperature of thermoplastic resin>

From the viewpoint of enhancing heat resistance, the glass transition temperature of the thermoplastic resin is preferably 100°C or higher, more preferably 105°C or higher, even more preferably 110°C or higher, even more preferably 115°C or higher, particularly preferably 125°C or higher. From the viewpoint of increasing the glass transition temperature to enhance heat resistance, it is preferred that at least one of the ends of the thermoplastic resin is not a hydroxy group. On the other hand, if the glass transition temperature is too high, the thermoplastic resin may deteriorate due to shear heating during extrusion. Further, in this case, the thermoplastic resin may deteriorate due to an excessive increase in melt viscosity during filtration through a filter. Therefore, the glass transition temperature Tg is preferably 260°C or lower, more preferably 230°C or lower, even more preferably 200°C or lower, particularly preferably 180°C or lower. The glass transition temperature of the thermoplastic resin is adjusted to fall within the above range by adjusting the kind of monomer used for production, the blending ratio thereof, the polymerization temperature, and the amount of an additive added.

The glass transition temperature is measured as a temperature at which glass transition of the thermoplastic resin starts. Specifically, the glass transition temperature of the thermoplastic resin is measured by, for example, a method described in Examples.

<Saturated water absorption rate>

If the saturated water absorption rate of the thermoplastic resin is high, there is a possibility that the physical properties of the resin change under high humidity so that the reliability of a molded article reduces. Therefore, the saturated water absorption rate is preferably 4 wt% or less, more preferably 3.5 wt% or less, even more preferably 3 wt% or less, even more preferably 2.7 wt% or less, particularly preferably 2.5 wt% or less. It is noted that the saturated water absorption rate of the thermoplastic resin is measured by, for example, a method described in Examples. The saturated water absorption rate of the thermoplastic resin is adjusted to fall within the above range by adjusting the kind of monomer used for production, the blending ratio thereof, the polymerization temperature, and the amount of an additive added.

<Boiling water test>

The moist heat resistance of the resin can be evaluated by determining whether or not the resin is deformed by a boiling water test. The boiling water test of the thermoplastic resin is performed by, for example, a method described in Examples.

<Reduced viscosity>

The polymerization degree (specifically, molecular weight) of the resin can be expressed by a reduced viscosity when increased to a certain level or higher. Except for polycarbonate diols or the like used even when having a relatively low polymerization degree (molecular weight), the polymerization degree (specifically, molecular weight) of the thermoplastic resin such as the polycarbonate resin is determined by measuring a reduced viscosity. The reduced viscosity is measured in the following manner. First, a sample is prepared by precisely adjusting the concentration of the resin to 1.00 g/dl with the use of a mixed solvent obtained by mixing phenol and 1,1,2,2-tetrachloroethane in a weight ratio of 1:1. Then, the reduced viscosity of the sample is measured at a temperature of 30.0°C ± 0.1°C.

If the reduced viscosity of the thermoplastic resin is too low, there is a possibility that the characteristics of a molded article obtained after molding, such as heat resistance, chemical resistance, abrasion resistance, and mechanical strength, reduce. Therefore, the reduced viscosity is preferably 0.20 dL/g or more, more preferably 0.30 dL/g or more. On the other hand, if the reduced viscosity is too high, there is a possibility that flowability of the resin during molding reduces so that productivity and moldability reduce or a molded article is highly distorted. Therefore, the reduced viscosity is preferably 1.50 dL/g or less, more preferably 1.20 dL/g or less, even more preferably 1.00 dL/g or less, even more

preferably 0.90 dL/g or less. It is noted that the reduced viscosity of the thermoplastic resin is measured by, for example, a method described in Examples. The reduced viscosity of the thermoplastic resin is adjusted to fall within the above range by adjusting the kind of monomer used for production, the blending ratio thereof, the polymerization temperature, and the amount of an additive added.

<Folding test>

The toughness of the thermoplastic resin is evaluated by, for example, a folding test. When the thermoplastic resin is broken by this test, a molded article may be brittle. It is noted that more specifically, the folding test is performed by a method described in Examples.

<Charpy impact test>

The toughness of the polycarbonate resin is evaluated by, for example, a Charpy impact test. If the Charpy impact strength is low, a molded article may be brittle. Therefore, the value of the Charpy impact test as measured at room temperature (23°C) in accordance with ISO179 (2000) using a specimen having a notch top radius of 0.25R is preferably 1 $kJ/m^2$ or more, more preferably 2 $kJ/m^2$ or more, even more preferably 3 $kJ/m^2$ or more. The value of the Charpy impact test as measured in the same manner except that the test is performed at a low temperature (-23°C) is preferably 1 $kJ/m^2$ or more, more preferably 2 $kJ/m^2$ or more, even more preferably 3 $kJ/m^2$ or more. It is most preferred that the value of the Charpy impact test as measured at room temperature (23°C) and the value of the Charpy impact test as measured at a low temperature (-23°C) are both 3 $kJ/m^2$ or more. It is noted that more specifically, the Charpy impact test is performed by a method described in Examples. The Charpy impact strength of the polycarbonate resin is adjusted to fall within the above range by adjusting the kind of monomer used for production, the blending ratio thereof, the polymerization temperature, and the amount of an additive added.

<Pencil hardness>

If the hardness of the thermoplastic resin is low, a molded article is easily scratched. The pencil hardness of the thermoplastic resin is preferably B or higher, more preferably F or higher. From the viewpoint of further enhancing the scratch resistance of a molded article, the pencil hardness of the thermoplastic resin is even more preferably H or higher. The pencil hardness is measured by a pencil hardness test. It is noted that more specifically, the pencil hardness is measured by a method described in Examples. The pencil hardness of the thermoplastic resin is adjusted to fall within the above range by adjusting the kind of monomer used for production, the blending ratio thereof, the polymerization temperature, and the amount of an additive added.

<Photoelastic coefficient>

If the photoelastic coefficient of the thermoplastic resin having a carbonate bond is high, a phase difference is generated by stress, which reduces optical reliability. The photoelastic coefficient of the thermoplastic resin having a carbonate bond is preferably $18 \times 10^{-12}$ $Pa^{-1}$ or less, more preferably $15 \times 10^{-12}$ $Pa^{-1}$ or less, even more preferably $12 \times 10^{-12}$ $Pa^{-1}$ or less, even more preferably $9 \times 10^{-12}$ $Pa^{-1}$ or less, particularly preferably $4 \times 10^{-12}$ $Pa^{-1}$ or less and is preferably 0 or more. Further, from the viewpoint that a resin having a negative photoelastic coefficient can reduce the photoelastic coefficient of another resin by blending, the photoelastic coefficient is preferably negative.

<5% thermal weight loss temperature (Td5) of monomer>

When the thermoplastic resin such as the polycarbonate resin or the polyester carbonate resin is produced using the polymerizable alicyclic compound, Td5 of the polymerizable alicyclic compound is preferably high from the viewpoint of adjusting a quantitative ratio. Specifically, if the 5% thermal weight loss temperature (Td5) of the polymerizable alicyclic compound is 150°C or lower, the polymerizable alicyclic compound is thermally decomposed under high vacuum during melt polymerization of a polycarbonate so that the mole ratio changes, which is not preferred in that a desired molecular weight is not achieved. On the other hand, Td5 is preferably 170°C or higher, more preferably 200°C or higher, even more preferably 210°C or higher, particularly preferably 220°C or higher.

<5% thermal weight loss temperature (Td5) of thermoplastic resin>

When the 5% thermal weight loss temperature of the thermoplastic resin having a carbonate bond is high, the thermoplastic resin is less likely to be thermally decomposed. The 5% thermal weight loss temperature of the thermoplastic

resin having a carbonate bond is preferably 200°C or higher, more preferably 250°C or higher, particularly preferably 300°C or higher. It is noted that specifically, the 5% thermal weight loss temperature is measured by a method described in Examples.

[Thermoplastic resin composition]

The thermoplastic resin may be kneaded with another synthetic resin, biodegradable resin, rubber, or the like to be used as a thermoplastic resin composition such as a polymer alloy. As the synthetic resin, the biodegradable resin, or the rubber, at least one resin or rubber can be used. Examples of the synthetic resin to be used include an aromatic polycarbonate, an aromatic polyester, an aliphatic polyester, a polyamide, polystyrene, a polyolefin, an acrylic, an amorphous polyolefin, an acrylonitrile-butadienestyrene copolymer (i.e., ABS), and an acrylonitrile-styrene copolymer (i.e., AS, SAN). Examples of the biodegradable resin to be used include polylactic acid and polybutylene succinate.

In addition to the above-described another resin, additives such as a nucleating agent, a flame retarder, a flame retardant aid, an inorganic filler, an impact modifier, a hydrolysis inhibitor, a foaming agent, and a dye or pigment may be added to the thermoplastic resin to produce a thermoplastic resin composition. As such additives, those generally used for thermoplastic resin compositions are used.

[Method for molding thermoplastic resin]

The thermoplastic resin or the resin composition containing the same is molded by a usually-known method such as injection molding, extrusion molding, or compression molding. A molded article is obtained by molding. In this way, it is possible to obtain a molded article having excellent characteristics such as heat resistance, transparency, light resistance, weatherability, and mechanical strength.

[Intended uses of thermoplastic resin having carbonate bond]

The thermoplastic resin having a carbonate bond is suitably used for films, sheets, bottles, various structural materials, optical members, etc. The polycarbonate resin containing at least the structural unit A1 is excellent in mechanical strength and moist heat resistance, and is therefore suitably used for films, sheets, bottles, materials in the field of containers, various structural materials, automobile parts, glass substitutes, and injection molding materials required to have flexibility, surface hardness, and moist heat resistance. Further, the thermoplastic resin containing at least the structural unit A1 has low photoelastic coefficient, and is therefore considered to have excellent optical reliability. For this reason, such a thermoplastic resin having a carbonate bond is suitably used also for optical films used for retardation films, diffusion sheets, polarizing films, and the like used in liquid crystal devices and organic EL devices, optical disks, binders for fixing a pigment or a charge transfer agent, and lenses used in cameras, finders, CCDs, and CMOSs.

[Reasons why effects are produced]

As described above, the thermoplastic resin containing the structural unit A1 and having a carbonate bond is a novel thermoplastic resin having a fused ring. Such a thermoplastic resin having a carbonate bond has a cyclobutane skeleton having a nonplanar structure and two ring structures sandwiching this cyclobutane skeleton.

A fused-ring structure containing a cyclobutane structure has a larger strain as compared to a fused-ring structure containing a cyclopentane structure or a cyclohexane structure and is a very rigid skeleton, and therefore a resulting resin has high heat resistance and surface hardness and causes little local structural change even when subjected to stress, which produces an excellent effect that photoelastic coefficient as one of important optical properties is low. On the other hand, a material having high heat resistance due to its rigid structure generally tends to be brittle due to its high yield stress. However, surprisingly, a polycarbonate resin having excellent toughness was obtained despite that its fused-ring structure containing a cyclobutane structure was a rigid skeleton.

It is generally known that brittle fracture of a resin is less likely to occur when the number of entanglements of the main chain per unit volume is larger, and a polymer having a bulky substituent in its side chain, such as polystyrene, has a short main chain length per molecular weight, and therefore brittle fracture is likely to occur due to a reduction in the number of entanglements of the main chain per unit volume. The study by the present inventors revealed that for the same reason, the polycarbonate resin using DMNDM having a bulky substituent in its side chain had high heat resistance but was very brittle. On the other hand, the polycarbonate resin having a fused ring exhibits high heat resistance and surface hardness while having toughness because the cyclobutane structure sterically occupies a small volume in the resin and therefore the number of entanglements of the main chain is not reduced.

Examples

Hereinbelow, the present invention will be described in more detail with reference to Examples, but the present invention is not limited to the following Examples unless departing from the scope of the invention as defined by the appended claims. It is noted that unless otherwise specified, "%" refers to "mass%".

[Evaluation methods]

The physical properties or characteristics of thermoplastic resin compositions and molded articles were evaluated by the following methods.

(1) Preparation of pressed film

Pellets of a polycarbonate resin were vacuum dried at 90°C for 5 hours or longer. A metallic plate (SUS) having an outer length of 10 cm, an outer width of 10 cm, and a thickness of 0.5 mm was hollowed out in such a manner that a periphery having a width of 1 cm remained to prepare a spacer having an inner length of 8 cm and an inner width of 8 cm. This spacer was sandwiched between two mirror-polished SUS plates having a length of 10 cm, a width of 10 cm, and a thickness of 1.5 mm, and about 4 g of the pellets were placed in the frame of the spacer and hot-pressed. The hot press temperature was 200 to 230°C, the pre-heat time was 5 to 7 minutes, and the pressure during molding was 40 MPa. The pressure time during molding was 1 minute. After hot pressing, a sheet-shaped sample was taken out together with the mirror-polished plates and the spacer and subjected to pressurized cooling by a water cooling-type press at a pressure of 20 MPa for 3 minutes. A film having a thickness of 400 to 500 μm was produced.

(2) Glass transition temperature Tg

A glass transition temperature was measured using a differential scanning calorimeter "EXSTAR 6220" manufactured by SII Nano Technology Inc. The glass transition temperature was measured in accordance with JISK7121: 1987. Specifically, about 10 mg of a measurement sample was heated at a temperature rise rate of 10°C/min, and the sample heated to 250°C was quenched by liquid nitrogen and was again heated to 250°C at a temperature rise rate of 10°C/min. From a DSC date obtained by the second temperature rise, a temperature at a point where a straight line equally distant from an extended line of a low temperature-side base line and an extended line of a high temperature-side base line and a curve in a stepwise change part of glass transition intersect is determined as a middle-point glass transition temperature. This middle-point glass transition temperature is regarded as a glass transition temperature Tg.

(3) Reduced viscosity

A polycarbonate resin was dissolved in a solvent to prepare a polycarbonate solution having a concentration of 1.00 g/dl. As the solvent, a mixed solvent of phenol and 1,1,2,2-tetrachloroethane was used. The mixing mass ratio between phenol and 1,1,2,2-tetrachloroethane is 1:1. The dissolution in the mixed solvent was performed by stirring at 110°C for 30 minutes, and the polycarbonate solution after cooling was used for measurement of reduced viscosity. The measurement of reduced viscosity was performed at a temperature of 30.0°C ± 0.1°C using an Ubbelohde-type viscometer "DT-504 automatic viscometer" manufactured by Chuorika Co., Ltd.). A relative viscosity $\eta_{rel}$ was calculated from the flow time $t_0$ of the solvent and the flow time t of the solution using the following equation (α), and a specific viscosity $n_{sp}$ (unit: g•cm$^{-1}$•sec$^{-1}$) was calculated from the relative viscosity $\eta_{rel}$ using the following equation (β). It is noted that $\eta_0$ in the equation (β) is a viscosity of the solvent. Then, the specific viscosity $\eta_{sp}$ was divided by the concentration c (g/dL) of the polycarbonate solution to determine a reduced viscosity $\eta(\eta=\eta_{sp}/c)$. A higher reduced viscosity indicates a larger molecular weight.

$$\eta_{rel}=t/t_0 \ \cdots(\alpha)$$

$$\eta_{sp}=(\eta-\eta_0)/\eta_0=\eta_{rel}-1 \ \cdots(\beta)$$

(4) Saturated water absorption rate

The film produced by the method described in (1) was cut to have a length of about 40 mm and a width of about 40 mm to prepare a measurement sample. The measurement test piece (film) was dried under vacuum in a vacuum drier at

90°C for 5 hours or longer and then cooled to room temperature under vacuum. Dried air was introduced to return to ordinary pressure, and the dry weight WO of the measurement test piece (film) was quickly measured.
The measurement test piece (film) was immersed in 500 cc of pure water at room temperature (23°C) and left stand for 144 hours. After 144 hours, the measurement test piece (film) was taken out, and water attached to the surface of the measurement test piece (film) was removed by wiping with a cloth, and a weight W1 after water absorption was quickly measured. A saturated water absorption rate was calculated by the following equation.

$$\text{Saturated water absorption rate (\%)} = ((W1 - W0) / W0) \times 100$$

(5) Boiling water test (evaluation of moist heat resistance)

The film produced by the method described in (1) was cut to have a length of about 40 mm and a width of about 40 mm to prepare a measurement sample.
The test piece (film) was placed in a mesh cage, immersed in a water tank heated to 100°C, and left stand for 3 hours. After the lapse of 3 hours, the mesh cage was taken out and cooled, and then the external appearance of the test piece was observed. The test piece (film) not deformed was evaluated as "pass (◦)", and the test piece (film) deformed was evaluated as "fail (×)".

(6) Charpy impact test

Pellets of a polycarbonate resin were vacuum dried at 90°C for 5 hours or longer. Then, the pellets were melted in a nitrogen atmosphere at 240°C for 3 minutes (180 seconds) using a Micro 15cc Twin Screw Compounder manufactured by Rheo Labo Co., Ltd. and injection-molded at a mold temperature of 70°C and a cylinder temperature of 240°C to obtain an ISO test piece for mechanical property test. The obtained test piece was subjected to a Charpy notched impact test in accordance with ISO179 (2000) to determine a Charpy notched impact strength. In this test, the test piece used for measurement had a notch tip radius of 0.25 R. The Charpy impact test was performed at room temperature (23°C) and low temperature (-20°C).

(7) Folding test

The film produced by the method described in (1) was cut to obtain a test piece having a width of 5 mm and a length of 20 mm. The test piece that was foldable when folded by gripping the both ends thereof was evaluated as ◦, and the test piece that was broken when folded by gripping the both ends thereof was evaluated as ×.

(8) Pencil hardness

The film produced by the method described in (1) was used to measure pencil hardness in accordance with JIS K5600-5-4:1999 using a pencil hardness tester (No. 601-B manufactured by MYS-TESTER Co., Ltd.). The measurement was performed under conditions of a load of 750 g and a measurement speed of 60 mm/min.

(9) Photoelastic coefficient

<Sample preparation>

The film produced by the method described in (1) was cut to obtain a sample having a width of 5 mm and a length of 20 mm. <Measurement>
Measurement was performed using an apparatus obtained by combining a birefringence meter including a He-Ne laser, a polarizer, a compensator, an analyzer, and a light detector and an oscillational viscoelastometer (manufactured by UBM) (for more details, see Journal of the Society of Rheology Japan Vol. 19, p. 93-97 (1991)). The sample cut out was fixed to the viscoelastometer, and the storage elastic modulus E' of the sample was measured at a room temperature of 25°C and a frequency of 96 Hz. At the same time, emitted laser light was allowed to pass through the polarizer, the sample, the compensator, and the analyzer in this order, picked up by the light detector (photodiode), and allowed to pass through a rock-in amplifier. From the wave form of an angular frequency of $\omega$ or $2\omega$, an amplitude and a phase difference caused by strain were determined to determine a strain-optic coefficient O'. At this time, the direction of the polarizer and the direction of the analyzer were orthogonal to each other, and an angle between each of them and the stretching direction of the sample was adjusted to $\pi/4$. A photoelastic coefficient C was determined by the following equation using the storage elastic modulus E' and the strain-optic coefficient O'.

C=O'/E'

(10) 5% thermal weight loss temperature (Td5)

TG/DTA7200 manufactured by SII Nano Technology Inc.) was used. About 10 mg of a sample was placed in a container and heated from 40°C to 500°C at a temperature rise rate of 10°C/min in a nitrogen atmosphere (nitrogen flow rate 50 mL/min). A temperature (Td5) at which the weight was reduced by 5% was determined. When this temperature is higher, thermal decomposition is less likely to occur.

(11) NMR (nuclear magnetic resonance)

The NMR of each of resins of Examples 1-1 to 1-5 was measured in the following manner. About 30 mg of a sample was placed in an NMR test tube having an outer diameter of 5 mm and dissolved in 0.7 mL of deuterated chloroform (containing 0.03 v/v% tetramethylsilane). $^1$H-NMR measurement was performed using "AVANCE III 950" manufactured by Bruker at a resonance frequency of 950.3 MHz, a flip angle of 30°, and a measurement temperature of 25°C. It is noted that the NMRs of compounds in Production Examples are measured in the same manner as described above with reference to the resins of Examples except that the instrument was changed to "ECZ400S" manufactured by JEOL Ltd. and the frequency was changed to 400 MHz.

<Production Example 1>

(1) Synthesis of pentacyclopentadeca-3(5),4(6)-diene (N3, N4)

In a nitrogen atmosphere, cyclopentadiene (200 g, 3.03 mol), tris-p-tolylphosphine (41 g, 136 mmol), acetic acid (550 mL), acetonitrile (250 mL), and $Pd(dba)_2$ (26 g, 45.4 mmol) were placed in an autoclave and stirred in the autoclave at 100°C for 3 hours. Water (2000 mL) was added to the reaction liquid in the autoclave, petroleum ether (1500 mL) was added for extraction, and extraction was repeatedly performed three times. Then, the organic layer was concentrated and purified by column chromatography. In this way, 110 g of pentacyclopentadeca-3(5),4(6)-diene was obtained. It is noted that it was confirmed by $^1$H NMR analysis that pentacyclopentadeca-3(5),4(6)-diene was obtained. The analysis result is as follows.

[Formula 174]

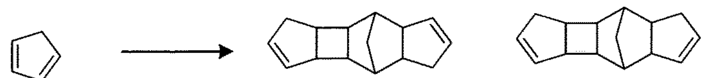

(N2) (N3) (N4)

$^1$H NMR(400MHz,$CDCl_3$):δ= 5.70-5.75(m, 2H), 5.63-5.70(m, 2H), 5.54(dd, J= 5.6, 2.0 Hz, 2H), 5.37-5.45(m, 2H), 2.97(dqt, J=10.4, 5.2, 5.2, 5.2, 2.4, 2.4 Hz, 2H), 2.74(br, J=3.2 Hz, 1H), 2.63-2.69(m, 1H), 2.53-2.63(m, 2H), 2.46-2.53(m, 2H), 2.14-2.28(m, 6H), 2.12(td, J= 4.8, 2.4 Hz, 6H), 1.99-2.06(m, 2H), 1.94(br, J= 6.4 Hz, 1H), 1.78-1.88(m, 2H), 1.67-1.73(m, 1H), 1.46(tt, J= 2.4, 1.2 Hz, 1H) 1.44(dt, J= 3.6, 1.2 Hz, 1H)

(2) Synthesis of compound (N5)

In an autoclave (i.e., a reactor) having an inner capacity of 300 mL and made of stainless steel, 45.7 g (230 mmol) of pentacyclopentadeca-3(5),4(6)-diene, 0.12 g (0.47 mmol) of $Rh(acac)(CO)_2$, 1.37 g (2.1 mmol) of tris(2,4-di-t-butylphenyl) phosphite, and 145 mL of methylcyclohexane were placed, and the reactor was hermetically sealed. The inside of the reactor was purged with nitrogen and then with water gas (specifically, with a mixed gas containing CO and $H_2$ in a mole ratio of 1:1), and was further filled with water gas until the inner pressure of the reactor reached 1 MPa. The contents of the reactor were heated with stirring until the inner temperature reached 70°C, and water gas was injected until the inner pressure reached 5 MPa. The water gas consumed by reaction was continuously supplied from an accumulator via a pressure adjustor so that reaction was performed at 70°C for 6 hours while the inner pressure was maintained at 5 MPa.

The collected reaction liquid was subjected to extraction with 100 mL of a mixed liquid of methanol and water four times. The mixing volume ratio between methanol and water in the mixed liquid was 4/1 (methanol/water). From the

collected aqueous solution containing methanol, methanol was distilled away under reduced pressure to obtain an aqueous phase. The aqueous phase was subjected to extraction with 100 mL of toluene three times. The toluene solution was dehydrated on anhydrous $Na_2SO_4$, the desiccant agent was separated by filtration, and the filtrate was concentrated to obtain pale yellow oily matter. This oily matter was confirmed to be a compound (N5) by $^1H$ NMR. The amount of the compound (N5) obtained was 58.0 g (224 mmol), and the yield was 97.6%. It is noted that it was confirmed by $^1H$ NMR analysis that the obtained compound was a compound (N5). The analysis result is as follows.
$^1H$ NMR(400MHz,$CDCl_3$):$\delta$= 9.9-9.4(m, 2H), 3.2-3.0(m, 0.5H), 2.7-2.35(m, 4H), 2.3-1.9(m, 3.5H), 1.9-1.3(m, 10H)

[Formula 175]

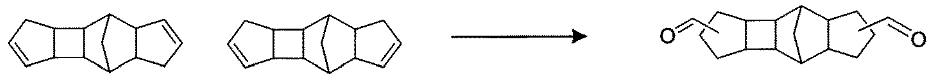

(N3) (N4) (N5)

(3) Synthesis of compound (P2)

A compound (P2) was produced from the compound (N5) by a hydrogenation reaction. Specifically, first, 70.6 g (274 mmol) of the compound (N5), 2.6 g of Ru/C (Ru 0.13 g, 1.2 mmol), and 100 mL of methanol were placed in an autoclave having an inner capacity of 300 mL and made of stainless steel, and the reactor was hermetically sealed. The inside of the reactor was purged with nitrogen and then with hydrogen, and was further filled with hydrogen until the inner pressure reached 1 MPa. The contents of the reactor were heated with stirring until the inner temperature reached 90°C, and hydrogen was injected until the inner pressure reached 8 MPa. The hydrogen consumed by reaction was continuously supplied from an accumulator via a pressure adjuster so that reaction was performed at 100°C for 12 hours while the inner pressure was maintained at 8 MPa. The reaction liquid was filtered through cerite to remove the catalyst, and the filtrate was concentrated in an evaporator. The residue was purified by column chromatography (filler : silica gel, solvent : hexane/ethyl acetate = 1/1 (volume ratio)) to obtain pale yellow oily matter. From the analysis results of $^1H$ NMR and GC-MS, the oily matter was confirmed to be a mixture of isomers of a compound (P2). The amount of the compound (P2) obtained was 19.0 g (72 mmol), and the yield was 26.3%. It is noted that the compound (P2) is referred to as "DA13" as appropriate.

$^1H$ NMR(400MHz,$CDCl_3$): $\delta$=3.8-3.2(m, 4H), 2.7-2.2(m, 2H), 2.1-1.0(m, 20H)
GC-MS(CI+, $NH_3$) $[M+NH_4]^+$ m/z:280, $M^+$=262

$C_{17}H_{26}O_2$ Molecular weight 262.4

The GC-MS analysis (gas chromatography mass spectroscopy analysis) was performed in the following manner. It is noted that ionization was performed by a CI method, and ammonia was used as a reagent gas. The injection temperature was 250°C, and the analysis temperature was increased from 50°C at 10°C/min, then maintained at 220°C for 10 minutes, and then increased up to 300°C at 15°C/min. It is noted that Agilent technology 7890 was used as a GC, and DB-1 manufactured by Agilent Technology (0.25 mm $\varphi\times$ 30 m, film thickness: 0.25 $\mu$m) was used as a column.

[Formula 176]

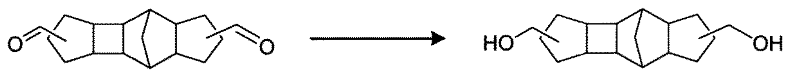

(N5) (P2)

It is noted that in Examples, DA13 produced by the above-described hydrogenation reaction was used, but DA13 can be produced from the compound (N5) also by a $NaBH_3$ reaction described below. First, a dropping funnel was attached to a 500 mL conical flask containing a rotor. In the flask, 31.1 g (120 mmol) of the compound (N5) and 150 mL of methanol were placed and stirred, and then the flask was cooled in an ice bath so that the inner temperature of the flask reached 0°C. From the dropping funnel, an aqueous solution obtained by dissolving 3.6 g (90 mmol) of sodium boron hydride (purity 95%) in 20 mL of a 1% aqueous NaOH solution was dropped into the flask for 40 minutes, and then the mixture in the flask was stirred at room temperature for 1 hour. The reaction liquid in the flask was cooled in an ice bath, and 30 mL of 15% sulfuric acid was added to the reaction liquid to decompose sodium boron hydride remaining in the reaction liquid. The

reaction liquid was concentrated under reduced pressure to obtain a residue. To this residue, 300 mL of ethyl acetate and 100 mL of water were added to separate the mixture into two phases to obtain an aqueous phase. This aqueous phase was subjected to extraction with 100 mL of ethyl acetate twice. Anhydrous $Na_2SO_4$ (i.e., a desiccant agent) was added to the oil phase for dehydration, and then the desiccant agent was removed by filtration. Then, the oil phase was concentrated in an evaporator to obtain a concentrated liquid. Inorganic matter was removed from the concentrated liquid by chromatography using $SiO_2$ as a filler and acetone as a solvent, and the concentrated liquid was further concentrated in an evaporator to obtain pale yellow oily matter. From the analysis result of $^1$H NMR, the oily matter was confirmed to be DA13. The amount of DA13 obtained was 30.2 g (115 mmol), and the yield was 96.0%.

The $^1$H NMR analysis result of the oily matter is as follows.

$^1$H NMR(400MHz,$CDCl_3$): δ=3.8-3.2(m, 4H), 2.7-2.2(m, 2H), 2.1-1.0 (m, 20H)

<Production Example 2>

• Synthesis of NCDDM

Norbornadiene (10 g, 108.53 mmol, 11.04 mL), $Ru_3(CO)_{12}$ (1.39 g, 2.17 mmol), DMAc (i.e., dimethylacetamide) (2.15 g, 21.71 mmol, 2.24 mL), and NMP (6 mL) were added to an autoclave, the reactor was hermetically sealed and purged with nitrogen, and the mixture was stirred at 80°C for 10 hours. The progress of a reaction was monitored by TLC (petroleum ether : ethyl acetate = 10 : 1, Rf = 0.9). The reaction was terminated by adding water, the reaction liquid was subjected to extraction with 30 mL of dichloromethane three times, washed with 20 mL of saturated saline twice, and washed with pure water. The reaction liquid was dehydrated using sodium sulfate, then dried and solidified, and purified with a silica gel column (solvent: hexane) to obtain 1.88 g of NCD as a pale yellow crystal at a yield of 15.6%. From the analysis result of $^1$H NMR, the oily matter was confirmed to be NCD.

$^1$H NMR(400MHz,$CDCl_3$): δ=6.03(d,4H), 2.65(d,4H), 1.71 (d,2H),1.36 (s,4H),1.25 (d,2H)

[Formula 177]

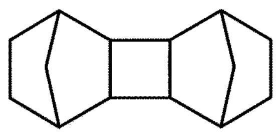

NCD

In an autoclave having an inner capacity of 300 mL and made of stainless steel, 25.0 g (136 mmol) of NCD, 0.09 g (0.35 mmol) of $Rh(acac)(CO)_2$, 0.88 g (1.4 mmol) of tris(2,4-di-t-butylphenyl) phosphite, and 120 mL of methylcyclohexane were placed, and the reactor was hermetically sealed. The inside of the reactor was purged with nitrogen and then with water gas ($CO/H_2$ = 1/1), and was further filled with water gas until the inner pressure reached 1 MPa. The mixture was heated with stirring until the inner temperature reached 80°C, and water gas was injected until the inner pressure of the reactor reached 7 MPa. The water gas consumed by reaction was continuously supplied from an accumulator via a pressure adjuster so that reaction was performed at an inner pressure of 6.5 MPa and 100°C for 1 hour. THF was added to the gel-like reaction mixture to collect it as a homogeneous solution, and the solvent was distilled away under reduced pressure to obtain a brown viscous liquid. It was confirmed by $^1$H NMR analysis that the collected matter was a diformyl derivative of NCD. This was not purified anymore and was used for a next reaction.

$^1$H NMR(400MHz,$CDCl_3$) δ9.7-9.4 (m, 2H), 2.5-2.3 (m, 2H), 2.3-2.0 (m, 4H), 2.0-1.6 (m, 6H), 1.4-1.1 (m, 3H), 1.0-0.8 (m, 3H)

[Formula 178]

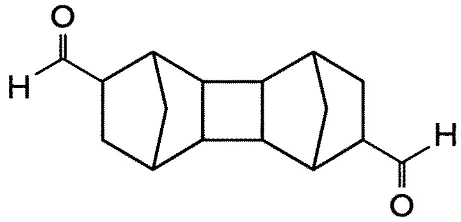

C.F: $C_{16}H_{20}O_2$
F.W.244.33

In a 1000 mL three-necked flask, the diformyl derivative of NCD (all the oxo reaction concentrate, theoretical amount 33.2 g, 136 mmol), 300 mL of THF, and 80 mL of ethanol were placed, and the mixture was stirred in an ice bath to obtain a homogeneous solution. A solution obtained by dissolving 5.60 g (133 mmol) of sodium boron hydride (gravimetric purity 90%) in 150 mL of a 1 mol/L aqueous NaOH solution was added in 10 installments so that the inner temperature of the reaction liquid did not exceed 10°C. After all the solution was added, the reaction liquid was heated to room temperature and subjected to reaction for 1 hour. The reaction liquid was cooled in an ice bath, and 40 mL of a 3 mol/L aqueous HCl solution was added while the inner temperature was maintained at 10°C or lower to decompose unreacted $NaBH_4$.

The solvent was distilled away by an evaporator, and the aqueous phase was subjected to extraction with chloroform. The collected oil phase was washed with demineralized water and then dehydrated with anhydrous $MgSO_4$, the desiccant agent was removed by filtration, and the filtrate was concentrated to obtain a crude product. The crude product was washed with acetonitrile and acetone to isolate 15.5 g (62 mmol) of NCDDM as a white powdery solid at a yield of 45.9%.

$^1$H NMR(400MHz, CDCl3) δ3.5-3.2 (m, 6H), 2.0-1.8 (m, 8H), 1.5-1.3 (m, 2H), 1.3-1.1 (m, 6H), 1.0-0.8 (m, 2H)

[Formula 179]

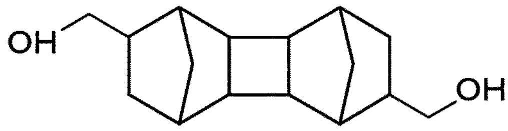

C.F: $C_{16}H_{24}O_2$
F.W.248.37

<Production Example 3>

• Synthesis of TPSA

First, 20 g (50.43 mmol) of pentacyclopentadeca-3(5),4(6)-diene (N3, N4) and 102.96 g (1.01 mmol) of $Ac_2O$ were dissolved in 800 mL of toluene, 216.95 g (690.86 mmol) of $Na_2CO_3$•$3H_2O_2$ was added thereto in four installments every 30 minutes, and the mixture was heated with stirring at 60°C for 5 hours. The reaction liquid was quenched by adding an aqueous saturated $Na_2S_2O_4$ solution, and then water was added to perform extraction with ethyl acetate. A product obtained by dehydration with sodium sulfate and solidification by drying was purified by silica gel chromatography (petroleum ether/ethyl acetate = 8/1) to obtain 12 g of a mixture of isomers of 5,16-dioxaheptacycloheptadecane as a yellow solid at a yield of 67%.

1H NMR (400 MHz, CDCl3) δ3.6 (s, 1H), 3.6 (s, 2H), 3.5 (m, 1H), 3.4 (m, 3H), 3.3 (m, 2H), 3.3 (m, 2H), 2.4-2.3 (m, 19H), 2.2 (m, 6H) , 2.0 (m, 7H), 1.7 (m, 3H), 1.4 (m, 3H)

[Formula 180]

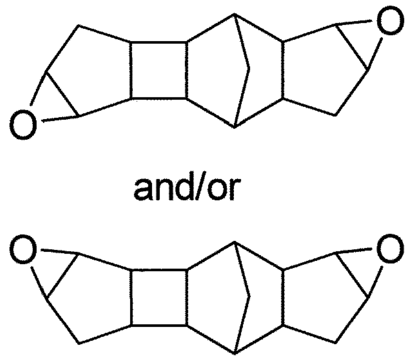

Sulfuric acid was added to an anhydrous methanol solution (600 mL) of the mixture of isomers of 5, 16-dioxaheptacycloheptadecane (30 g, 130.26 mmol), and the mixture was stirred at room temperature for 4 hours. Then, 300 mL of an aqueous saturated sodium hydrogen carbonate solution was added to terminate the reaction, methanol was distilled away under reduced pressure, water was then added, and extraction using ethyl acetate was performed (200 mL x 5). Washing with an aqueous saturated sodium chloride solution and dehydration with sodium sulfate were performed. The solvent was distilled away, and purification was performed by column chromatography (heptane : ethyl acetate = 1 : 3). As a result of recrystallization using an ethyl acetate/heptane solvent, 51 g of TPSA was obtained as a white solid.
$^1$H NMR(400MHz,$CDCl_3$) δ4.1(d, 2H), 3.7 (br, 2H), 3.5 (m, 3H), 3.5 (d, 5H), 3.4 (d, 2H), 3.3 (s, 1H), 2.2(br, 1H), 2.2-1.9 (m, 27H), 1.4 (t, 2H), 1.1 (m, 2H)
Td5:223°C

[Formula 181]

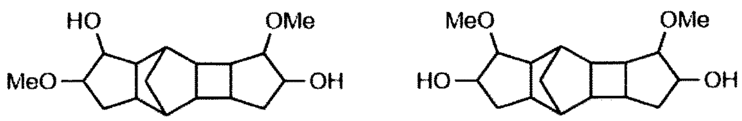

TPSA

<Production Example 4>

• Synthesis of bis[9-(2-phenoxycarbonylethyl)fluoren-9-yl]methane (FN2)

FN2 was synthesized by a method described in JP 2015-25111 A.

[Formula 182]

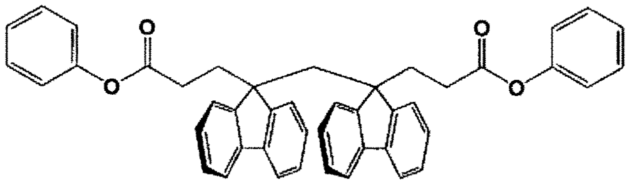

[Raw materials used]

Abbreviations for the compounds used in Production Examples and Examples and manufacturers of the compounds are as follows.

<Dihydroxy compounds>

- ISB: Isosorbide [manufactured by Roquette Freres]
- CHDM: 1,4-cyclohexanedimethanol (cis/trans mixture) [manufactured by SK Chemicals Co., Ltd.)
- TCDDM: Tricyclodecanedimethanol [manufactured by Oxea]
- DA13: Dihydroxy compound synthesized in Production Example 1 (Pentacyclo [7.5.1.02,8.03,7.010, 14]pentadecanedimethanol)
- NBD: Norbornadiene synthesized in Production Example 2
- NCD: Dimer of norbornadiene synthesized in Production Example 2
- NCDDM: Dihydroxy compound of dimer of norbornadiene synthesized in Production Example 2
- TPSA: Dihydroxy compound synthesized in Production Example 3

<Diester carbonate>

• DPC: Diphenyl carbonate [manufactured by Mitsubishi Chemical Corporation]

<Diester component>

• FN2: Diester synthesized in Production Example 4 (bis[9-(2-phenoxycarbonylethyl)fluoren-9-yl]methane

<Polymerization catalyst>

Calcium acetate monohydrate ($Ca(CH_3COO)_2$•$H_2O$) [manufactured by KISHIDA CHEMICAL CO., LTD.]

(Example 1-1)

In this example, a polycarbonate resin was produced using DA13 and ISB as dihydroxy compounds. Specifically, 5.70 g (0.0022 mol) of DA13, 7.41 g (0.051 mol) of isosorbide (hereinafter abbreviated as "ISB"), 15.83 g (0.0739 mol) of diphenyl carbonate (hereinafter abbreviated as "DPC"), and $1.27 \times 10^{-4}$ g ($7.25$ g $\times 10^{-6}$ mol) of calcium acetate monohydrate as a polymerization catalyst in the form of a 0.2% aqueous solution were added to a reaction container, a heating bath was heated to 150°C in a nitrogen atmosphere, and the raw materials were dissolved by increasing the temperature of the heating bath to 220°C in 60 minutes at ordinary pressure while stirred if necessary.

In the first step of a reaction, the temperature of the heating bath was maintained at 220°C for 30 minutes, the pressure was then reduced from ordinary pressure to 13.3 kPa in 40 minutes and then maintained at 13.3 kPa for 60 minutes, and generated phenol was discharged to the outside of the reaction container. In the second step, generated phenol was discharged to the outside of the reaction container while the temperature of the heating bath was increased to 240°C in 20 minutes and the pressure was controlled to be reduced to 0.200 kPa or less in 30 minutes. After a predetermined stirring torque was reached, the reaction was terminated, and a generated reaction product was taken out of the reaction container. In this way, a polycarbonate copolymer (specifically, a polycarbonate copolymer whose mole ratio of structural unit derived from DPC/structural unit derived from DA13/structural unit derived from ISB was 100/30/70). The obtained polycarbonate copolymer had a reduced viscosity of 0.502 dl/g and a glass transition temperature Tg of 143°C. The saturated water absorption rate at room temperature was 1.9 wt%. In the boiling water test, deformation was not observed. The 5% thermal weight loss temperature (Td5) as measured in a nitrogen atmosphere was 345°C. The NMR chart of the polycarbonate copolymer is shown in Fig. 9.

(Example 1-2)

In this example, a polycarbonate resin was produced using DA13 and ISB as dihydroxy compounds. A polycarbonate resin (specifically, a polycarbonate copolymer whose mole ratio of structural unit derived from DPC/structural unit derived from DA13/structural unit derived from ISB was 100/20/80) was produced in the same manner as in Example 1-1 except that 4.03 g (0.0154 mol) of DA13, 8.98 g (0.0614 mol) of ISB, 16.77 g (0.0783 mol) of DPC, and $1.35 \times 10^{-4}$ g ($7.68 \times 10^{-6}$ mol, 0.2% aqueous solution) of calcium acetate monohydrate as a catalyst were used as materials.

The obtained polycarbonate copolymer had a reduced viscosity of 0.638 dl/g and a glass transition temperature Tg of 152°C. The saturated water absorption rate at room temperature was 2.7 wt%. In the boiling water test, deformation was not observed. The 5% thermal weight loss temperature (Td5) as measured in a nitrogen atmosphere was 341°C.

The NMR chart of the polycarbonate copolymer is shown in Fig. 10.

(Example 1-3)

A polycarbonate resin was produced using DA13, ISB, and TCDDM as dihydroxy compounds. A polycarbonate resin (specifically, a polycarbonate copolymer whose mole ratio of structural unit derived from DPC/structural unit derived from DA13/structural unit derived from ISB/structural unit derived from TCDDM was 100/10/70/20) was produced in the same manner as in Example 1-1 except that 13.00 g (0.0495 mol) of DA13, 19.45 g (0.0991 mol) of TCDDM, 50.68 g (0.3468 mol) of ISB, 108.24 g (0.5053 mol) of DPC, and $8.73 \times 10^{-3}$ g ($4.95 \times 10^{-5}$ mol, 2% aqueous solution) of calcium acetate monohydrate as a catalyst were used as materials.
The obtained polycarbonate copolymer had a reduced viscosity of 0.669 dl/g and a glass transition temperature Tg of 134°C. The saturated water absorption rate at room temperature was 1.9 wt%. In the boiling water test, deformation was not observed. The 5% thermal weight loss temperature (Td5) as measured in a nitrogen atmosphere was 346°C.
The NMR chart of the polycarbonate copolymer is shown in Fig. 11.

(Example 1-4)

In this example, a polycarbonate resin was produced using DA13, ISB, and CHDM as dihydroxy compounds. A polycarbonate resin (specifically, a polycarbonate copolymer whose mole ratio of structural unit derived from DPC/structural unit derived from DA13/structural unit derived from ISB/structural unit derived from CHDM was 100/10/70/20) was produced in the same manner as in Example 1-1 except that 21.46 g (0.0818 mol) of DA13, 23.59 g (0.1636 mol) of CHDM, 83.68 g (0.5726 mol) of ISB, 178.73 g (0.8344 mol) of DPC, and $1.44 \times 10^{-2}$ g ($8.18 \times 10^{-5}$ mol, 2% aqueous solution) of calcium acetate monohydrate as a catalyst were used as materials.
The obtained polycarbonate copolymer had a reduced viscosity of 0.689 dl/g and a glass transition temperature Tg of 130°C. The saturated water absorption rate at room temperature was 2.1 wt%. In the boiling water test, deformation was not observed. The 5% thermal weight loss temperature (Td5) as measured in a nitrogen atmosphere was 343°C.
The NMR chart of the polycarbonate copolymer is shown in Fig. 12.

(Example 1-5)

In this example, a polycarbonate resin was produced using DA13, ISB, and CHDM as dihydroxy compounds. A polycarbonate resin (specifically, a polycarbonate copolymer whose mole ratio of structural unit derived from DPC/structural unit derived from DA13/structural unit derived from ISB/structural unit derived from CHDM was 100/20/70/10) was produced in the same manner as in Example 1-1 except that 4.03 g (0.0154 mol) of DA13, 1.11 g (0.0077 mol) of CHDM, 7.86 g (0.0538 mol) of ISB, 16.79 g (0.0784 mol) of DPC, and $3.38 \times 10^{-4}$ g ($1.92 \times 10^{-6}$ mol, 0.2% aqueous solution) of calcium acetate monohydrate as a catalyst were used as materials.
The obtained polycarbonate copolymer had a reduced viscosity of 0.923 dl/g and a glass transition temperature Tg of 140°C. The saturated water absorption rate at room temperature was 1.9 wt%. In the boiling water test, deformation was not observed. The 5% thermal weight loss temperature (Td5) as measured in a nitrogen atmosphere was 348°C.
The NMR chart of the polycarbonate copolymer is shown in Fig. 13.

[Comparative Example 1-1]

In this example, a polycarbonate resin was produced using ISB and CHDM as dihydroxy compounds. A polycarbonate resin (specifically, a polycarbonate copolymer whose mole ratio of structural unit derived from DPC/structural unit derived from ISB/structural unit derived from CHDM was 100/70/30) was produced in the same manner as in Example 1-1 except that the materials were changed to 37.83 g (0.2623 mol) of CHDM, 89.44 g (0.6120 mol) of ISB, 191.05 g (0.8918 mol) of DPC, and $3.85 \times 10^{-3}$ g ($2.19 \times 10^{-5}$ mol, 2% aqueous solution) of calcium acetate monohydrate as a catalyst.
The obtained polycarbonate copolymer had a reduced viscosity of 0.615 dl/g and a glass transition temperature Tg of 122°C. The saturated water absorption rate at room temperature was 2.0 wt%. In the boiling water test, the film was deformed.

(Comparative Example 1-2)

In this example, a polycarbonate resin was produced using ISB and TCDDM as dihydroxy compounds. A polycarbonate resin (specifically, a polycarbonate copolymer whose mole ratio of structural unit derived from DPC/structural unit derived from ISB/structural unit derived from TCDDM was 100/70/30) was produced in the same manner as in Example 1-1 except that the materials were changed to 47.19 g (0.2404 mol) of TCDDM, 81.98 g (0.5610 mol) of ISB, 175.1 g (0.8174 mol) of DPC, and $3.53 \times 10^{-3}$ g ($2.00 \times 10^{-5}$ mol, 2% aqueous solution) of calcium acetate monohydrate as a

catalyst.

The obtained polycarbonate copolymer had a reduced viscosity of 0.602 dl/g and a glass transition temperature Tg of 131°C. The saturated water absorption rate at room temperature was 1.8 wt%. In the boiling water test, the film was deformed.

The glass transition temperature, the result of the folding test, the result of the boiling water test, the saturated water absorption rate, the pencil hardness, and the photoelastic coefficient of each of the polycarbonate resins of Examples 1-1 to 1-4 and Comparative Examples 1-1 and 1-2 are shown in Table 1. Further, the glass transition temperature and photoelastic coefficient of the polycarbonate resin of Example 1-5 are shown in Table 1.

[Table 1]

Table 1

| Example, Comparative Example No. | Diol Component | | | | Carbonate Component | Evaluation Results | | | | | | |
|---|---|---|---|---|---|---|---|---|---|---|---|---|
| | DA13 | ISB | CHDM | TCDDM | DPC | Tg | Folding Test | Saturated Water Absorption Rate | Boiling Water Test | Pencil Hardness | Photoelastic Coefficient |
| | mol% (wt%) | mol% (wt%) | mol% (wt%) | mol% (wt%) | mol% (wt%) | °C | - | wt% | - | - | $\times 10^{-12} Pa^{-1}$ |
| Example 1-1 | 15(37.7) | 35 (48.7) | - | - | 50 (13.5) | 143 | O | 1.9 | O | H | 7 |
| Example 1-2 | (26.7) | 40 (59) | - | - | 50 (14.3) | 152 | O | 2.7 | O | H | 9 |
| Example 1-3 | 5 (13.4) | 35 (52.1) | - | (20.1) | 50 (14.5) | 134 | O | 1.9 | O | H | 9 |
| Example 1-4 | 5 (14.2) | 35 (55) | 10(15.5) | - | 50 (15.3) | 130 | O | 2. 1 | O | F | 15 |
| Example 1-5 | 10 (26.7) | 35 (51.7) | 5 (7.3) | - | 50 (14.4) | 140 | O | 1.9 | O | H | 11 |
| Comparative Example 1-1 | - | 35 (58.8) | 15 (24.9) | - | 50 (16.3) | 122 | O | 2.0 | × | F | 19 |
| Comparative Example 1-2 | - | 35 (53.9) | - | 15 (31.1) | 50 (15.0) | 131 | O | 1.8 | × | F | 11 |

As can be seen from Table 1, the thermoplastic resins (specifically, polycarbonate resins) of Examples 1-1 to 1-5 containing a specific structural unit have a sufficiently high glass transition temperature. Further, in the case of Examples 1-1 to 1-4, the result of the boiling water test is excellent, that is, moist heat resistance is excellent while the glass transition temperature is sufficiently high. On the other hand, in the case of Comparative Examples 1-1 and 1-2 containing no specific structural unit, the glass transition temperature was low, and the result of the boiling water test was failed, that is, moist heat resistance was poor.

Further, as can be seen from Table 1, the thermoplastic resins of Examples 1-1 to 1-4 (specifically, polycarbonate resins) containing a specific structural unit have excellent results of the folding test and the boiling water test, that is, have excellent toughness and moist heat resistance while having a sufficiently high glass transition temperature. On the other hand, in the case of Comparative Examples 1-1 and 1-2 containing no specific structural unit, the glass transition temperature was low, toughness was poor, and moist heat resistance was poor.

Further, as can be seen from Table 1, the thermoplastic resins of Examples 1-1 to 1-5 containing a specific structural unit have an excellent result of measurement of photoelastic coefficient while having a sufficiently high glass transition temperature, that is, have excellent optical reliability and heat resistance. On the other hand, in the case of Comparative Examples 1-1 and 1-2 containing no specific structural unit, the glass transition temperature was low and the photoelastic coefficient was insufficient. Particularly, the result of Example 1-1 indicates that the thermoplastic resin having a specific structural unit has a very low photoelastic coefficient and excellent optical reliability. Further, Examples 1-1 to 1-4, have an excellent result of measurement of pencil hardness, that is, have excellent mechanical strength.

Further, although not shown in Table 1, the results of the Charpy impact test of Example 1-3 and Comparative Example 1-2 (specifically, the Charpy impact strength at room temperature and the Charpy impact strength at low temperature) were compared, and as a result, the Charpy impact strength of Example 3 as measured at room temperature was 3 $kJ/m^2$ and the Charpy impact strength of Example 3 as measured at low temperature was 3 $kJ/m^2$. On the other hand, the Charpy impact strength of Comparative Example 1-2 as measured at room temperature was 3 $kJ/m^2$, and the Charpy impact strength of Comparative Example 1-2 as measured at low temperature was 2 $kJ/m^2$. That is, the impact resistance of Example 1-3 was equal to or higher than that of Comparative Example 1-2. That is, it can be said that Example 1-3 has excellent toughness.

(Example 2-1)

In this example, a polycarbonate resin was produced using DA13 and ISB as dihydroxy compounds. A polycarbonate resin (specifically, a polycarbonate copolymer whose mole ratio of structural unit derived from DPC/structural unit derived from DA13/structural unit derived from ISB was 100/70/30) was produced in the same manner as in Example 1-1 except that the materials of Example 1-1 were changed to 10.87 g (0.0414 mol) of DA13, 2.59 g (0.0178 mol) of ISB, 12.93 g (0.0604 mol) of DPC, and $3.13 \times 10^{-3}$ g ($1.78 \times 10^{-5}$ mol, 2% aqueous solution) of calcium acetate monohydrate as a catalyst.

The obtained polycarbonate copolymer had a reduced viscosity of 0.621 dl/g and a glass transition temperature Tg of 125°C. The saturated water absorption rate at room temperature was 0.7 wt%. In the boiling water test, deformation was not observed.

The physical properties and evaluation results of the obtained polycarbonate copolymer are shown in Table 2.

(Example 2-2)

In this example, a polycarbonate resin was produced using DA13 and TCDDM as dihydroxy compounds. A polycarbonate resin (specifically, a polycarbonate copolymer whose mole ratio of structural unit derived from DPC/structural unit derived from DA13/structural unit derived from TCDDM was 100/75/25) was produced in the same manner as in Example 1-1 except that the materials of Example 1-1 were changed to 10.86 g (0.0414 mol) of DA13, 2.71 g (0.0138 mol) of TCDDM, 12.06 g (0.0563 mol) of DPC, and $2.92 \times 10^{-3}$ g ($1.66 \times 10^{-5}$ mol, 2% aqueous solution) of calcium acetate monohydrate as a catalyst.

The obtained polycarbonate copolymer had a reduced viscosity of 1.256 dl/g and a glass transition temperature Tg of 107°C. The saturated water absorption rate at room temperature was 0.3 wt%. In the boiling water test, deformation was observed.

The physical properties and evaluation results of the obtained polycarbonate copolymer are shown in Table 2.

(Example 2-3)

In this example, a polyester carbonate resin was produced using DA13 and ISB as dihydroxy compounds and FN2 as a diester component. As materials, 40.31 parts by weight (0.154 mol) of DA13, 31.35 parts by weight (0.215 mol) of ISB, 28.19 parts by weight (0.044 mol) of FN2, 69.44 parts by weight (0.324 mol) of DPC, and $1.30 \times 10^{-3}$ parts by weight

($7.36 \times 10^{-6}$ mol) of calcium acetate monohydrate were added to a reaction container, and the inside of the reaction container was purged with nitrogen under reduced pressure. In a nitrogen atmosphere, the raw materials were dissolved with stirring at 150°C for about 10 minutes. In the first step of a reaction, the pressure was adjusted to 53.3 kPa, and then the temperature was increased to 220°C in 30 minutes. After 30 minutes from when the temperature reached 220°C, the pressure was reduced to 13.3 kPa in 60 minutes. Generated phenol was discharged to the outside of the reaction system. Then, in the second step of the reaction, the temperature of a heat medium was increased to 245°C in 15 minutes while the pressure was maintained at 13.3 kPa, and then the pressure was reduced to 0.10 kPa or less in 30 minutes. After a predetermined stirring torque was reached, the pressure was returned to ordinary pressure by nitrogen to terminate the reaction, a generated polyester carbonate was extruded into water, and a strand was cut to obtain pellets. In this way, a polyester carbonate resin (specifically, a polyester carbonate copolymer whose mole ratio of structural unit derived from DPC/structural unit derived from DA13/structural unit derived from ISB/structural unit derived from FN2 was 100/40/40/20) was produced. It is noted that the structural unit derived from FN2 is shown below.

The physical properties and evaluation results of the obtained polyester carbonate resin are shown in Table 2.

[Formula 183]

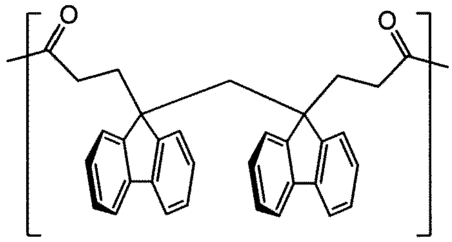

FN2

[Table 2]

Table 2

| Example, Comparative Example No. | Diol Component | | | | Diester Component | Carbonate Component | Evaluation Results | | | | | |
|---|---|---|---|---|---|---|---|---|---|---|---|---|
| | DA13 | ISB | CHDM | TCDDM | FN2 | DPC | Tg | Folding Test | Saturated Water Absorption Rate | Boiling Water Test | Pencil Hardness | Photoelastic Coefficient |
| | mol% (wt%) | mol% (wt%) | mol% (wt%) | mol% (wt%) | mol% (wt%) | mol% (wt%) | °C | - | wt% | - | - | $\times 10^{-12} Pa^{-1}$ |
| Example 2-1 | 35 (72) | 15 (17) | - | - | - | 50 (11) | 125 | O | 0.7 | O | HB | - |
| Example 2-2 | 37.5 (72) | - | - | 12.5 (18) | - | 50 (10) | 107 | O | 0.3 | × | B | - |
| Example 2-3 | 20 (33) | 20 (19) | - | - | 10 (39) | 50 (9) | 137 | - | - | O | - | 0.9 |

As can be seen from Table 2, for example, Example 2-1 has a sufficiently high glass transition temperature and has excellent results of the folding test and the boiling water test, that is, has excellent toughness and moist heat resistance. Further, the saturated water absorption rate is low. Example 2-2 has a glass transition temperature within a practical range and has an excellent result of the folding test, that is, has excellent toughness. Further, the saturated water absorption rate is very low. On the other hand, in the case of Comparative Example 4-2 containing no specific structural unit, the glass transition temperature was low and the result of the boiling water test was failed, that is, moist heat resistance was poor. The polyester carbonate of Example 2-3 has a sufficiently high glass transition temperature and has an excellent result of the boiling water test, that is, has excellent moist heat resistance. Further, the photoelastic coefficient is sufficiently low, and therefore the polyester carbonate of Example 2-3 is suitable for optics applications.

(Example 3-1)

In this example, a polycarbonate resin was produced using NCDDM and ISB as dihydroxy compounds. A polycarbonate resin (specifically, a polycarbonate copolymer whose mole ratio of structural unit derived from DPC/structural unit derived from NCDDM/structural unit derived from ISB was 100/30/70) was produced in the same manner as in Example 1-1 except that the materials of Example 1-1 were changed to 5.51 g (0.0222 mol) of NCDDM, 7.58 g (0.0518 mol) of ISB, 16.16 g (0.0754 mol) of DPC, and 1.30 $\times$ $10^{-3}$ g (7.40 $\times$ $10^{-4}$ mol, 2% aqueous solution) of calcium acetate monohydrate as a catalyst.

The obtained polycarbonate copolymer had a reduced viscosity of 0.282 dl/g and a glass transition temperature Tg of 145°C.

The physical properties and evaluation results of the obtained polycarbonate copolymer are shown in Table 3.

(Example 3-2)

In this example, a polycarbonate resin was produced using NCDDM, ISB, and CHDM as dihydroxy compounds. A polycarbonate resin (specifically, a polycarbonate copolymer whose mole ratio of structural unit derived from DPC/structural unit derived from NCDDM/structural unit derived from ISB/structural unit derived from CHDM was 100/10/70/20) was produced in the same manner as in Example 1-1 except that the materials of Example 1-1 were changed to 2.05 g (0.0082 mol) of NCDDM, 2.38 g (0.0165 mol) of CHDM, 8.43 g (0.0577 mol) of ISB, 18.01 g (0.0841 mol) of DPC, and 1.45 $\times$ $10^{-3}$ g (8.24 $\times$ $10^{-6}$ mol, 0.2% aqueous solution) of calcium acetate monohydrate as a catalyst.

The obtained polycarbonate copolymer had a reduced viscosity of 0.804 dl/g and a glass transition temperature Tg of 139°C. The saturated water absorption rate at room temperature was 2.2 wt%. In the boiling water test, deformation was not observed.

The physical properties and evaluation results of the obtained polycarbonate copolymer are shown in Table 3.

(Example 3-3)

In this example, a polycarbonate resin was produced using TPSA, ISB, and TCDDM as dihydroxy compounds. A polycarbonate resin (specifically, a polycarbonate copolymer whose mole ratio of structural unit derived from DPC/structural unit derived from TPSA/structural unit derived from ISB/structural unit derived from TCDDM was 100/10/70/20) was produced in the same manner as in Example 1-1 except that the materials of Example 1-1 were changed to 2.24 g (0.0076 mol) of TPSA, 2.99 g (0.0152 mol) of TCDDM, 7.79 g (0.0533 mol) of ISB, 16.64 g (0.0777 mol) of DPC, and 1.34 $\times$ $10^{-3}$ g (7.62 $\times$ $10^{-6}$ mol, 0.2% aqueous solution) of calcium acetate monohydrate as a catalyst.

The obtained polycarbonate copolymer had a reduced viscosity of 0.795 dl/g and a glass transition temperature Tg of 146°C. The saturated water absorption rate at room temperature was 2.7 wt%. In the boiling water test, deformation was not observed.

The physical properties and evaluation results of the obtained polycarbonate copolymer are shown in Table 3.

(Example 3-4)

In this example, a polycarbonate resin was produced using TPSA, ISB, TCDDM as dihydroxy compounds. A polycarbonate resin (specifically, a polycarbonate copolymer whose mole ratio of structural unit derived from DPC/structural unit derived from TPSA/structural unit derived from ISB/structural unit derived from TCDDM was 100/20/60/20) was produced in the same manner as in Example 1-1 except that the materials of Example 1-1 were changed to 4.17 g (0.0142 mol) of TPSA, 2.78 g (0.0142 mol) of TCDDM, 6.21 g (0.0425 mol) of ISB, 15.47 g (0.0722 mol) of DPC, and 1.25 $\times$ $10^{-3}$ g (7.08 $\times$ $10^{-6}$ mol, 0.2% aqueous solution) of calcium acetate monohydrate as a catalyst.

The obtained polycarbonate copolymer had a reduced viscosity of 0.640 dl/g and a glass transition temperature Tg of 148°C. The saturated water absorption rate at room temperature was 2.4 wt%. In the boiling water test, deformation

was not observed.

The physical properties and evaluation results of the obtained polycarbonate copolymer are shown in Table 3.

(Example 3-5)

In this example, a polycarbonate resin was produced using TPSA, ISB, and CHDM as dihydroxy compounds. A polycarbonate resin (specifically, a polycarbonate copolymer whose mole ratio of structural unit derived from DPC/structural unit derived from TPSA/structural unit derived from ISB/structural unit derived from CHDM was 100/10/70/20) was produced in the same manner as in Example 1-1 except that the materials of Example 1-1 were changed to 2.37 g (0.0080 mol) of TPSA, 2.32 g (0.0161 mol) of CHDM, 8.22 g (0.0563 mol) of ISB, 17.57 g (0.0820 mol) of DPC, and $1.42 \times 10^{-3}$ g ($8.04 \times 10^{-6}$ mol, 0.2% aqueous solution) of calcium acetate monohydrate as a catalyst.

The obtained polycarbonate copolymer had a reduced viscosity of 0.876 dl/g and a glass transition temperature Tg of 141°C. The saturated water absorption rate at room temperature was 2.9 wt%. In the boiling water test, deformation was not observed.

The physical properties and evaluation results of the obtained polycarbonate copolymer are shown in Table 3.

(Example 3-6)

In this example, a polycarbonate resin was produced using TPSA, ISB, and CHDM as dihydroxy compounds. A polycarbonate resin (specifically, a polycarbonate copolymer whose mole ratio of structural unit derived from DPC/structural unit derived from TPSA/structural unit derived from ISB/structural unit derived from CHDM was 100/20/70/10) was produced in the same manner as in Example 1-1 except that the materials of Example 1-1 were changed to 4.38 g (0.0149 mol) of TPSA, 1.07 g (0.0074 mol) of CHDM, 7.61 g (0.0521 mol) of ISB, 16.26 g (0.0759 mol) of DPC, and $1.31 \times 10^{-3}$ g ($7.44 \times 10^{-6}$ mol, 0.2% aqueous solution) of calcium acetate monohydrate as a catalyst.

The obtained polycarbonate copolymer had a reduced viscosity of 0.600 dl/g and a glass transition temperature Tg of 158°C. The saturated water absorption rate at room temperature was 3.5 wt%. In the boiling water test, deformation was not observed. The physical properties and evaluation results of the obtained polycarbonate copolymer are shown in Table 3.

[Table 3]

Table 3

| Example, Comparative Example No. | Diol Component | | | | | | Carbonate Component | Evaluation Results | | | | | |
|---|---|---|---|---|---|---|---|---|---|---|---|---|---|
| | DA13 | NCDDM | TPSA | ISB | CHDM | TCDDM | DPC | Tg | Folding Test | Saturated Water Absorption Rate | Boiling Water Test | Pencil Hardness | Photoelastic Coefficient |
| | mol% (wt%) | mol% (wt%) | mol% (wt%) | mol% (wt%) | mol% (wt%) | mol% (wt%) ) | mol% (wt%) | °C | - | wt% | - | - | $\times 10^{-12}Pa^{-1}$ |
| Example 3-1 | - | 15 (36) | - | 35 (50) | - | - | 50 (14) | 145 | × | - | - | - | - |
| Example 3-2 | - | 5 (14) | - | 35 (57) | 10 (13) | - | 50 (16) | 139 | O | 2.2 | O | H | 17 |
| Example 3-3 | - | - | 5 (16) | 35 (50) | - | 10 (20) | 50 (14) | 146 | O | 2.7 | O | H | - |
| Example 3-4 | - | - | 10 (29) | 30 (40) | - | 10 (18) | 50 (13) | 148 | × | 2.4 | O | H | - |
| Example 3-5 | - | - | 5 (17) | 35 (55) | 10 (13) | - | 50 (15) | 141 | O | 2.9 | O | H | 14.2 |
| Example 3-6 | - | - | 10 (28) | 30 (39) | 20 (21) | - | 50 (12) | 158 | × | 3.5 | O | H | 12.1 |

As can be seen from Table 3, for example, Example 3-1 has a high glass transition temperature and excellent heat resistance. Example 3-2 has a sufficiently high glass transition temperature and has excellent results of the folding test and the boiling water test, that is, has excellent toughness and moist heat resistance. The carbonate-based resins of Examples 3-3 to 3-6 have a large molecular weight and contain a structure having an alkoxy group, and therefore have excellent heat resistance and moist heat resistance.

(Example 4-1)

In this example, a polycarbonate resin was produced using DA13 as a dihydroxy compound. A polycarbonate resin (specifically, a polycarbonate copolymer whose mole ratio of structural unit derived from DPC/structural unit derived from DA13 was 100/100) was produced in the same manner as in Example 1-1 except that the materials of Example 1-1 were changed to 13.65 g (0.0520 mol) of DA13, 11.37 g (0.0531 mol) of DPC, and $2.75 \times 10^{-3}$ g ($1.56 \times 10^{-6}$ mol, 2% aqueous solution) of calcium acetate monohydrate as a catalyst.

The obtained polycarbonate copolymer had a reduced viscosity of 0.639 dl/g and a glass transition temperature Tg of 116°C. The saturated water absorption rate at room temperature was 0.31 wt%. In the boiling water test, deformation was not observed.

The physical properties and evaluation results of the obtained polycarbonate homopolymer are shown in Table 4.

(Comparative Example 4-1)

In this example, a polycarbonate resin was produced using CHDM as a dihydroxy compound. A polycarbonate resin (specifically, a polycarbonate copolymer whose mole ratio of structural unit derived from DPC/structural unit derived from CHDM was 100/100) was produced in the same manner as in Example 1-1 except that the materials of Example 1-1 were changed to 12.71 g (0.0881 mol) of CHDM, 19.26 g (0.0899 mol) of DPC, and $4.66 \times 10^{-3}$ g ($2.64 \times 10^{-5}$ mol, 2% aqueous solution) of calcium acetate monohydrate as a catalyst and that in the second step, the reaction was continued at 220°C without increasing the temperature of the heating bath to 240°C in 20 minutes.

The obtained polycarbonate copolymer had a reduced viscosity of 1.051 dl/g and a glass transition temperature Tg of 40°C. The saturated water absorption rate at room temperature was 0.41 wt%. In the boiling water test, deformation was observed.

The physical properties and evaluation results of the obtained polycarbonate homopolymer are shown in Table 4.

(Comparative Example 4-2)

In this example, a polycarbonate resin was produced using TCDDM as a dihydroxy compound. A polycarbonate resin (specifically, a polycarbonate copolymer whose mole ratio of structural unit derived from DPC/structural unit derived from TCDDM was 100/100) was produced in the same manner as in Example 1-1 except that the materials of Example 1-1 were changed to 13.25 g (0.0675 mol) of TCDDM, 14.46 g (0.0675 mol) of DPC, and $3.57 \times 10^{-3}$ g ($2.02 \times 10^{-5}$ mol, 2% aqueous solution) of calcium acetate monohydrate as a catalyst.

The obtained polycarbonate copolymer had a reduced viscosity of 0.836 dl/g and a glass transition temperature Tg of 77°C. The saturated water absorption rate at room temperature was 0.36 wt%. In the boiling water test, deformation was observed.

The physical properties and evaluation results of the obtained polycarbonate homopolymer are shown in Table 4.

[Table 4]

Table 4

| Example, Comparative Example No. | Diol Component | | | | Carbonate Component | Evaluation Results | | | | |
|---|---|---|---|---|---|---|---|---|---|---|
| | DA13 | NCDDM | CHDM | TCDDM | DPC | Tg | Saturated Water Absorption Rate | Boiling Water Test | Pencil Hardness | Photoelastic Coefficient |
| | mol% (wt%) | mol% (wt%) | mol% (wt%) | mol% (wt%) | mol% (wt%) | °C | wt% | - | - | $\times 10^{-12}Pa^{-1}$ |
| Example 4-1 | 50 (90) | - | - | - | 50 (10) | 116 | 0.31 | O | B | 0.8 |
| Comparative Example 4-1 | - | - | 50 (81) | - | 50 (19) | 40 | 0.41 | × | <6B | 49 |
| Comparative Example 4-2 | - | - | - | 50 (88) | 50 (12) | 77 | 0.36 | × | 2B | 6 |

As can be seen from Table 4, the thermoplastic resin (homopolymer) formed from the alicyclic monomer and DPC shown in Example 4-1 has a high glass transition temperature and high moist heat resistance. Further, the thermoplastic resin has a very low photoelastic coefficient. On the other hand, in the case of Comparative Examples 4-1 and 4-2, the glass transition temperature is 100°C or lower, and therefore moist heat resistance is significantly impaired.

(Experimental example)

In this example, resistance to thermal decomposition of DA13, ISB, TCDDM, and SPG was evaluated. Specifically, the 5% thermal weight loss temperature (i.e., Td5) of each of the monomers was measured. SPG is spiro-glycol. The above-described measurement method is used. The results are shown in Table 5.

[Table 5]

Table 5

| Monomer Species | | DA13 | ISB | TCDDM | SPG |
|---|---|---|---|---|---|
| Td5 | °C | 220 | 145 | 174 | 202 |

As can be seen from Table 5, Td5 of DA13 is higher than those of other monomers. Therefore, it can be said that DA13 has excellent resistance to thermal decomposition, and is suitable for synthesis of a thermoplastic resin such as a carbonate-based resin. That is, when DA13 is used as a monomer of a thermoplastic resin, thermal decomposition of the monomer is prevented during polymerization, and therefore a gap between the initial composition of monomers in a polymerization step and the composition of a thermoplastic resin obtained in the polymerization step can be reduced. This makes it possible to stably produce a thermoplastic resin having certain physical properties.

## Claims

1. A thermoplastic resin containing a structure represented by the following formula (A1):

[Formula 1]

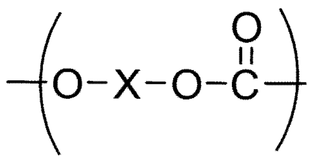

(A1)

[Formula 2]

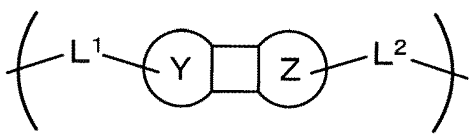

(B1)

(in the formula (A1), X represents the above formula (B1), a ring Y and a ring Z in the formula (B1) forming a fused ring with cyclobutane are each independently an optionally-substituted alicyclic carbon ring having 15 or less carbon atoms, the alicyclic carbon ring optionally contains a hetero atom, $L^1$ and $L^2$ in the formula (B1) are each independently a direct bond or a divalent hydrocarbon group having 1 to 5 carbon atoms).

2. The thermoplastic resin according to claim 1, wherein $L^1$ and $L^2$ in the formula (B1) are direct bonds or methylene groups.

3. The thermoplastic resin according to claim 1 or 2, wherein the alicyclic carbon ring has at least one carbon ring constituted of 4 or more carbon atoms.

4. The thermoplastic resin according to any one of claims 1 to 3, wherein the formula (B1) is represented by the following formula (B1') or (B1"):

[Formula 3]

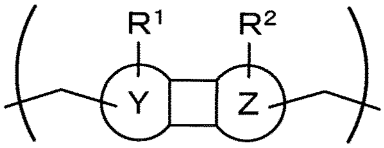

(B1')

[Formula 4]

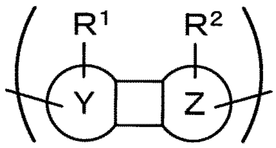

(B1'')

(in the formula (B1') and (B1"), a ring Y and a ring Z forming a fused ring with cyclobutane are each independently an alicyclic carbon ring constituted of 4 or more and 15 or less carbon atoms, the alicyclic carbon ring optionally contains a hetero atom, $R^1$ and $R^2$ are each independently a hydrogen atom, a hydrocarbon group having 1 to 14 carbon atoms, or an alkoxy group having 1 to 12 carbon atoms).

5. The thermoplastic resin according to any one of claims 1 to 4, wherein the ring Y and the ring Z in the formula (B1) are each independently an alicyclic carbon ring selected from among the following formulas (C1) to (C11):

[Formula 5]

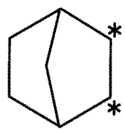

(C1)

[Formula 6]

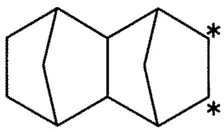

(C2)

[Formula 7]

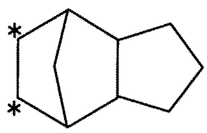

(C3)

[Formula 8]

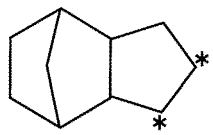

(C4)

[Formula 9]

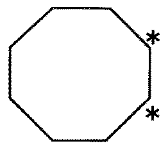

(C5)

[Formula 10]

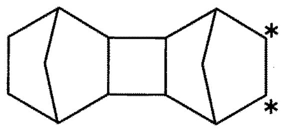

(C6)

[Formula 11]

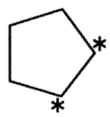

(C7)

[Formula 12]

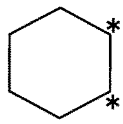

(C8)

[Formula 13]

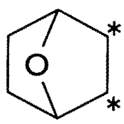

(C9)

[Formula 14]

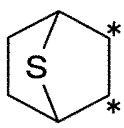

(C10)

[Formula 15]

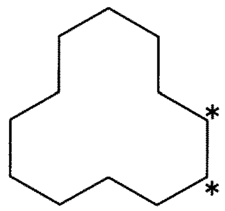

(C11)

(in the formulas (C1) to (C11), * represents a site where the fused ring is formed).

**6.** The thermoplastic resin according to claim 5, wherein the ring Y and the ring Z in the formula (B1) are each independently an alicyclic carbon ring selected from among the formulas (C1) to (C7).

**7.** The thermoplastic resin according to any one of claims 1 to 6, wherein the formula (B1) is selected from among the following formulas (B2) to (B12) and the following formulas (B16) to (B26):

[Formula 16]

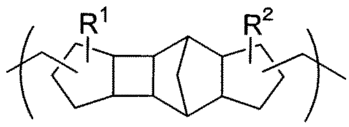

(B2)

[Formula 17]

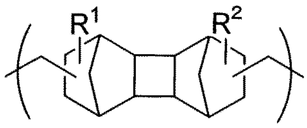

(B3)

[Formula 18]

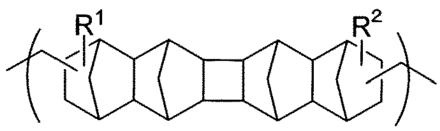

(B4)

[Formula 19]

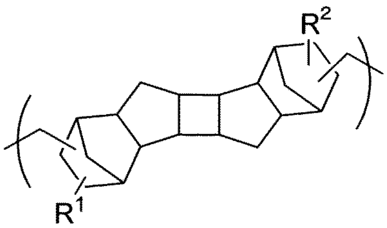

(B5)

[Formula 20]

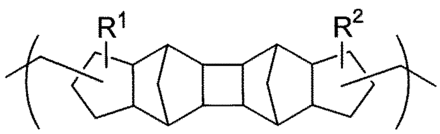

(B6)

[Formula 21]

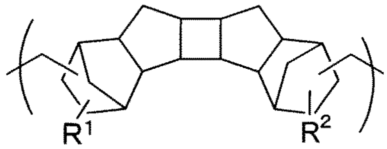

(B7)

[Formula 22]

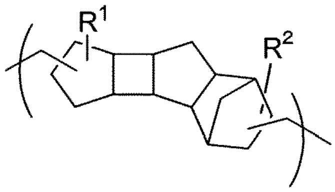

(B8)

[Formula 23]

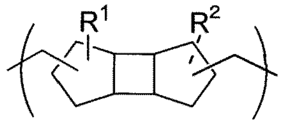

(B9)

[Formula 24]

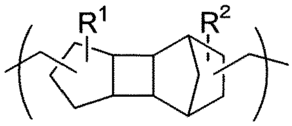

(B10)

[Formula 25]

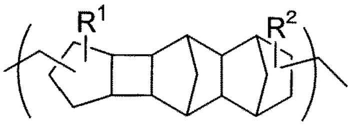

(B11)

[Formula 26]

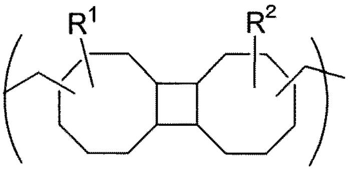

(B12)

[Formula 27]

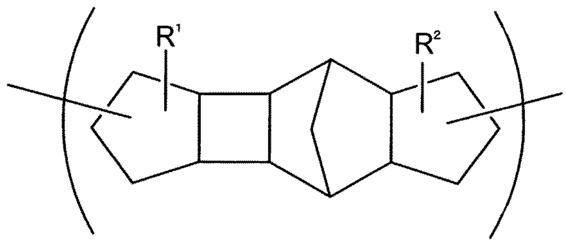

(B16)

[Formula 28]

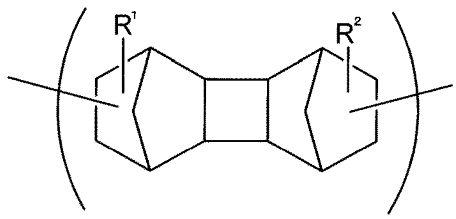

(B17)

[Formula 29]

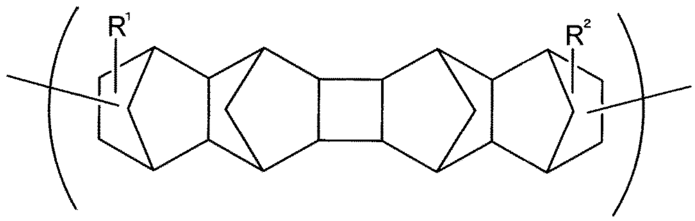

(B18)

[Formula 30]

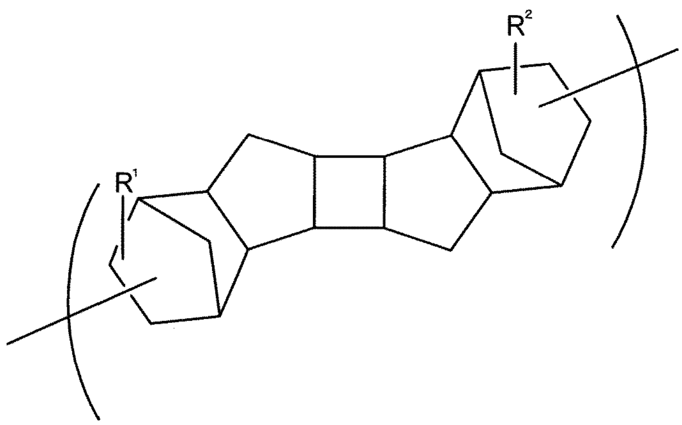

(B19)

[Formula 31]

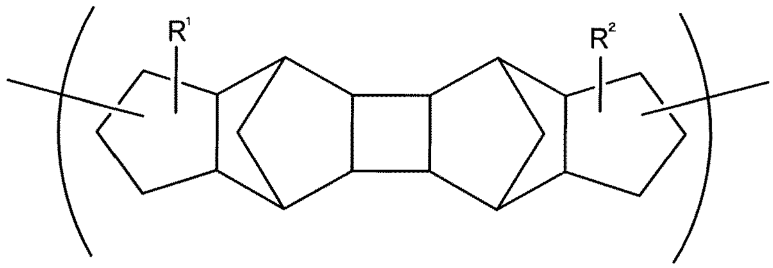

(B20)

[Formula 32]

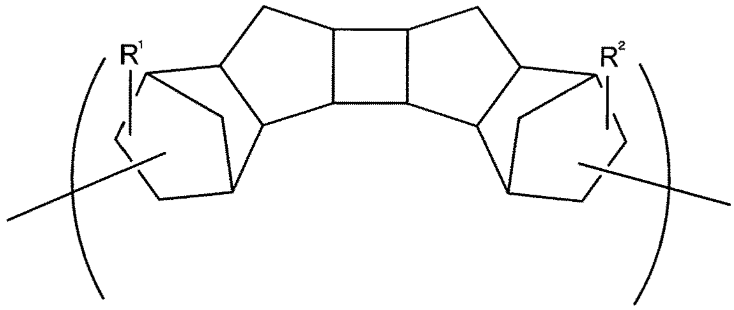

(B21)

[Formula 33]

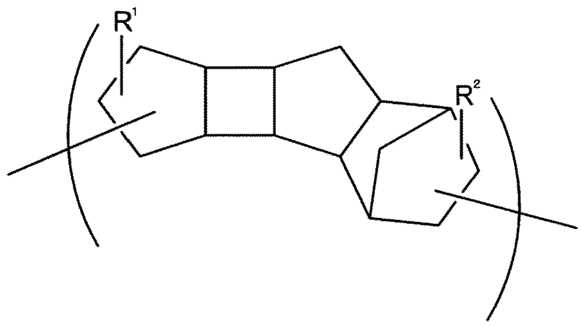

(B22)

[Formula 34]

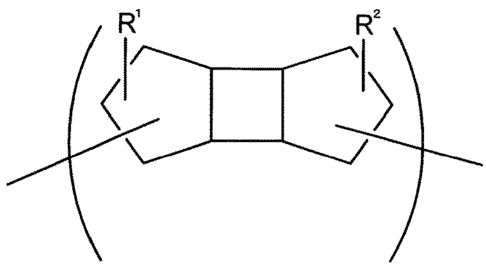

(B23)

[Formula 35]

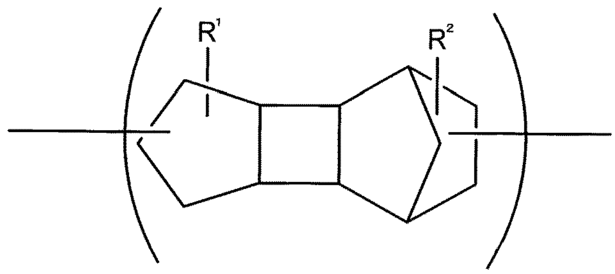

(B24)

[Formula 36]

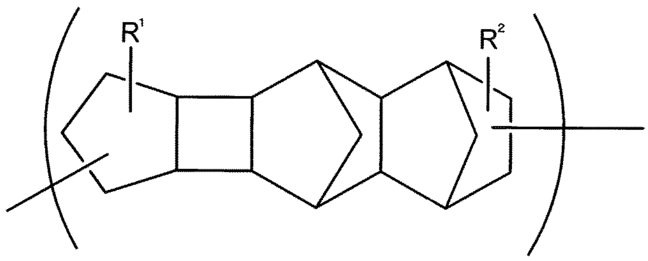

(B25)

[Formula 37]

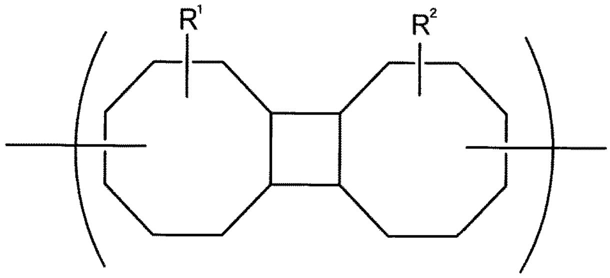

(B26)

(in the formulas (B2) to (B12) and (B16) to (B26), $R^1$ and $R^2$ are each independently a hydrogen atom, a hydrocarbon group having 1 to 14 carbon atoms, or an alkoxy group having 1 to 12 carbon atoms).

**8.** The thermoplastic resin according to claim 7, wherein $R^1$ and $R^2$ in the formulas (B2) to (B12) and (B16) to (B26) are each independently a hydrogen atom, a methyl group, a methoxy group, or an ethoxy group.

**9.** The thermoplastic resin according to any one of claims 1 to 8, wherein the ring Y and the ring Z in the formula (B1) are different in alicyclic skeleton from each other.

**10.** The thermoplastic resin according to any one of claims 1 to 9, whose glass transition temperature is 100°C or higher.

**11.** The thermoplastic resin according to any one of claims 1 to 10, whose reduced viscosity is 0.2 dl/g or more and 1.50 dl/g or less.

**12.** The thermoplastic resin according to any one of claims 1 to 11, further containing a structural unit derived from at least one compound selected from the group consisting of isosorbide, isomannide, and isoidide.

**13.** The thermoplastic resin according to any one of claims 1 to 12, being a polycarbonate resin or a polyester carbonate resin.

**14.** A molded article formed from the thermoplastic resin according to any one of claims 1 to 13.

**Patentansprüche**

**1.** Ein thermoplastisches Harz, das eine Struktur enthält, die durch die folgende Formel (A1) dargestellt ist:

[Formel 1]

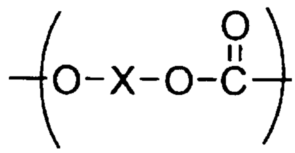

(A1)

[Formel 2]

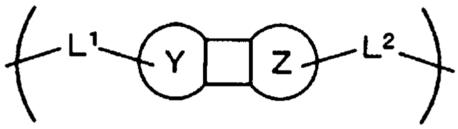

(B1)

(in der Formel (A1) steht X für die vorstehende Formel (B1), ein Ring Y und ein Ring Z in der Formel (B1), die einen kondensierten Ring mit Cyclobutan bilden, sind jeweils unabhängig ein gegebenenfalls substituierter alicyclischer Kohlenstoffring mit 15 oder weniger Kohlenstoffatomen, der alicyclische Kohlenstoffring enthält gegebenenfalls ein Heteroatom, $L^1$ und $L^2$ in der Formel (B1) sind jeweils unabhängig eine direkte Bindung oder eine zweiwertige Kohlenwasserstoffgruppe mit 1 bis 5 Kohlenstoffatomen).

**2.** Das thermoplastische Harz nach Anspruch 1, wobei $L^1$ und $L^2$ in der Formel (B1) direkte Bindungen oder Methylengruppen sind.

**3.** Das thermoplastische Harz nach Anspruch 1 oder 2, wobei der alicyclische Kohlenstoffring mindestens einen Kohlenstoffring aus 4 oder mehr Kohlenstoffatomen aufweist.

**4.** Das thermoplastische Harz nach einem der Ansprüche 1 bis 3, wobei die Formel (B1) durch die folgende Formel (B1') oder (B1") dargestellt ist:

[Formel 3]

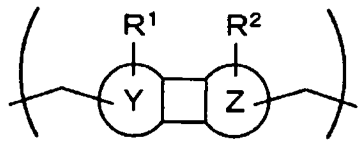

(B1')

[Formel 4]

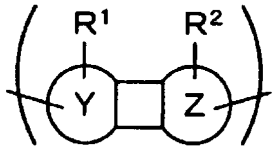

(B1'')

(in der Formel (B1') und (B1") sind ein Ring Y und ein Ring Z, die mit Cyclobutan einen kondensierten Ring bilden, jeweils unabhängig ein alicyclischer Kohlenstoffring aus 4 oder mehr und 15 oder weniger Kohlenstoffatomen, der alicyclische Kohlenstoffring enthält gegebenenfalls ein Heteroatom, $R^1$ und $R^2$ sind jeweils unabhängig ein Wasserstoffatom, eine Kohlenwasserstoffgruppe mit 1 bis 14 Kohlenstoffatomen oder eine Alkoxygruppe mit 1 bis 12 Kohlenstoffatomen).

**5.** Das thermoplastische Harz nach einem der Ansprüche 1 bis 4, wobei der Ring Y und der Ring Z in der Formel (B1) jeweils unabhängig ein alicyclischer Kohlenstoffring sind, der aus den folgenden Formeln (C1) bis (C11) ausgewählt ist:

[Formel 5]

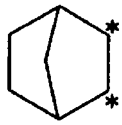

(C1)

[Formel 6]

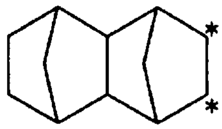

(C2)

[Formel 7]

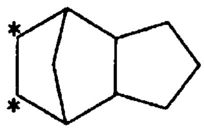

(C3)

[Formel 8]

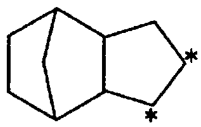

(C4)

[Formel 9]

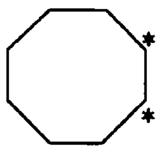

(C5)

[Formel 10]

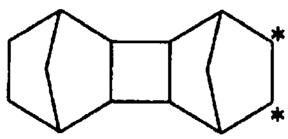

(C6)

[Formel 11]

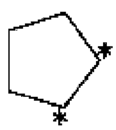

(C7)

[Formel 12]

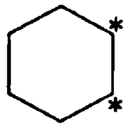

(C8)

[Formel 13]

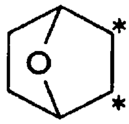

(C9)

[Formel 14]

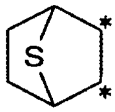

(C10)

[Formel 15]

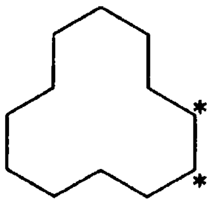

(C11)

(in den Formeln (C1) bis (C11) steht * für eine Stelle, an der der kondensierte Ring gebildet ist).

**6.** Das thermoplastische Harz nach Anspruch 5, wobei der Ring Y und der Ring Z in der Formel (B1) jeweils unabhängig ein alicyclischer Kohlenstoffring sind, der aus den Formeln (C1) bis (C7) ausgewählt ist.

**7.** Das thermoplastische Harz nach einem der Ansprüche 1 bis 6, wobei die Formel (B1) aus den folgenden Formeln (B2) bis (B12) und den folgenden Formeln (B16) bis (B26) ausgewählt ist:

[Formel 16]

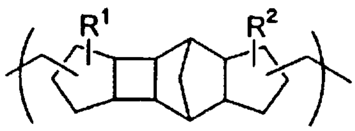

(B2)

[Formel 17]

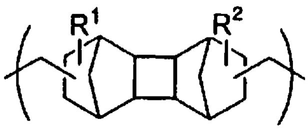

(B3)

[Formel 18]

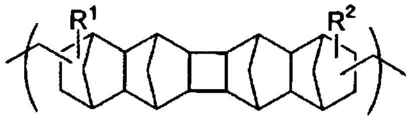

(B4)

[Formel 19]

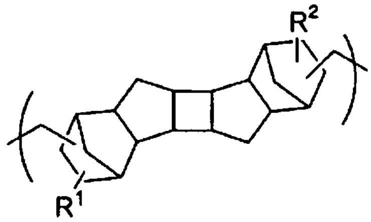

(B5)

[Formel 20]

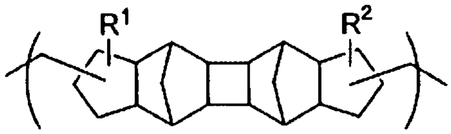

(B6)

[Formel 21]

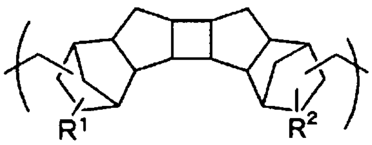

(B7)

[Formel 22]

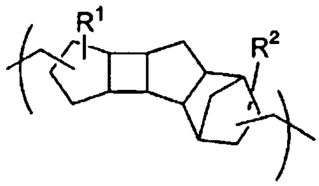

(B8)

[Formel 23]

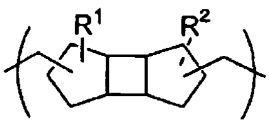

(B9)

[Formel 24]

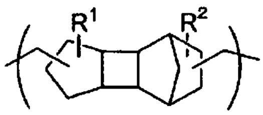

(B10)

[Formel 25]

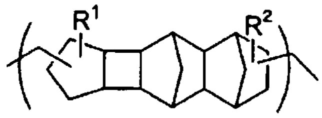

(B11)

[Formel 26]

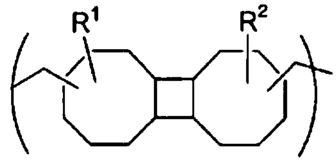

(B12)

[Formel 27]

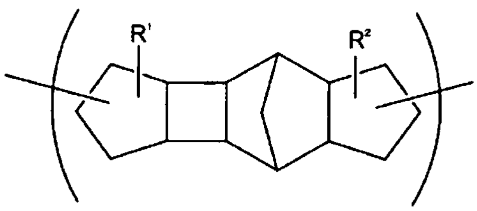

(B16)

[Formel 28]

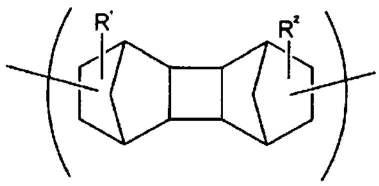

(B17)

[Formel 29]

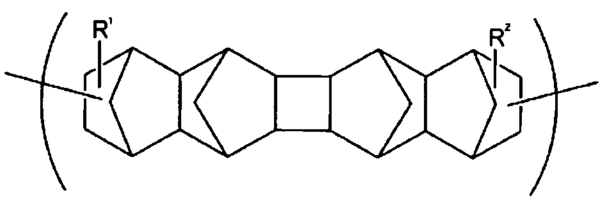

(B18)

[Formel 30]

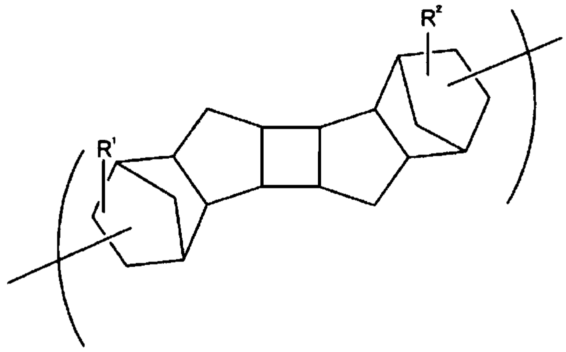

(B19)

[Formel 31]

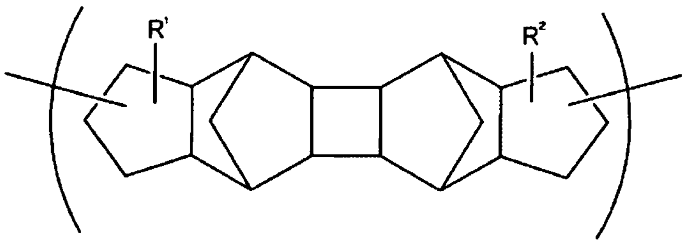

(B20)

[Formel 32]

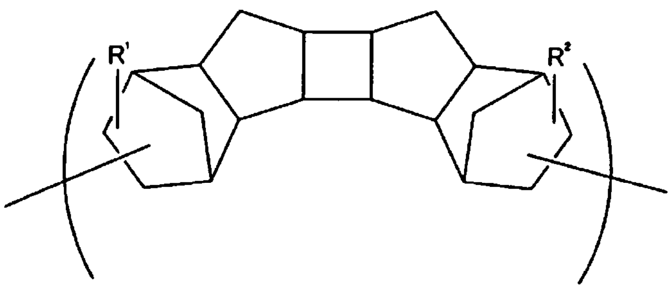

(B21)

[Formel 33]

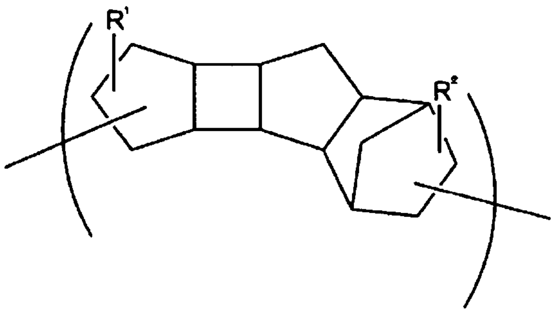

(B22)

[Formel 34]

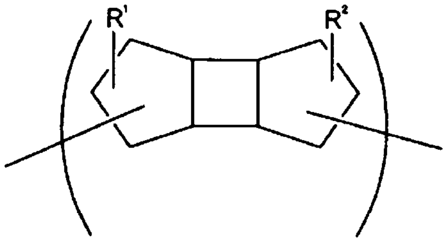

(B23)

[Formel 35]

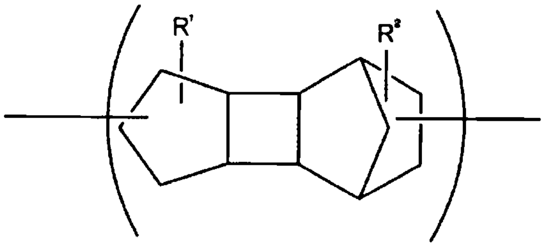

(B24)

[Formel 36]

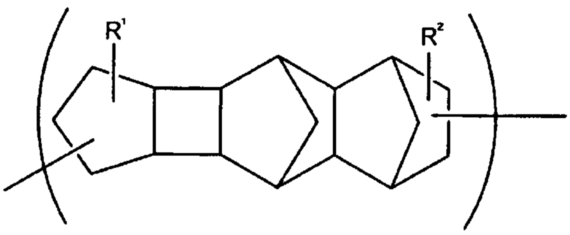

(B25)

[Formel 37]

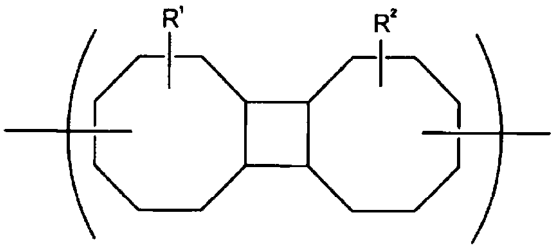

(B26)

(in den Formeln (B2) bis (B12) und (B16) bis (B26) sind $R^1$ und $R^2$ jeweils unabhängig ein Wasserstoffatom, eine Kohlenwasserstoffgruppe mit 1 bis 14 Kohlenstoffatomen oder eine Alkoxygruppe mit 1 bis 12 Kohlenstoffatomen).

**8.** Das thermoplastische Harz nach Anspruch 7, wobei $R^1$ und $R^2$ in den Formeln (B2) bis (B12) und (B16) bis (B26) jeweils unabhängig ein Wasserstoffatom, eine Methylgruppe, eine Methoxygruppe oder eine Ethoxygruppe sind.

**9.** Das thermoplastische Harz nach einem der Ansprüche 1 bis 8, wobei sich der Ring Y und der Ring Z in der Formel (B1) in ihrem alicyclischen Gerüst voneinander unterscheiden.

**10.** Das thermoplastische Harz nach einem der Ansprüche 1 bis 9, dessen Glasübergangstemperatur 100°C oder höher ist.

**11.** Das thermoplastische Harz nach einem der Ansprüche 1 bis 10, dessen reduzierte Viskosität 0,2 dl/g oder mehr und

1,50 dl/g oder weniger beträgt.

12. Das thermoplastische Harz nach einem der Ansprüche 1 bis 11, das ferner eine Struktureinheit enthält, die von mindestens einer Verbindung, ausgewählt aus der Gruppe bestehend aus Isosorbid, Isomannid und Isoidid, abgeleitet ist.

13. Das thermoplastische Harz nach einem der Ansprüche 1 bis 12, bei dem es sich um ein Polycarbonatharz oder ein Polyestercarbonatharz handelt.

14. Ein Formgegenstand, gebildet aus dem thermoplastischen Harz nach einem der Ansprüche 1 bis 13.

**Revendications**

1. Résine thermoplastique contenant une structure représentée par la formule (A1) suivante :

Formule 1

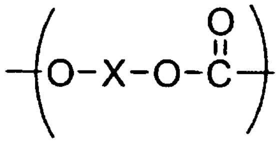

(A1)

Formule 2

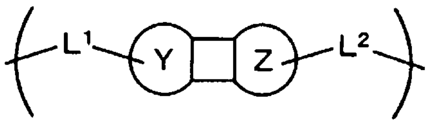

(B1)

(dans la formule (A1), X représente la formule (B1) ci-dessus, un cycle Y et un cycle Z dans la formule (B1) formant un cycle condensé avec un cyclobutane sont chacun indépendamment un cycle carboné alicyclique éventuellement substitué ayant 15 atomes de carbone ou moins, le cycle carboné alicyclique contient éventuellement un hétéroatome, $L^1$ et $L^2$ dans la formule (B1) sont chacun indépendamment une liaison directe ou un groupe hydrocarboné divalent ayant 1 à 5 atomes de carbone).

2. Résine thermoplastique selon la revendication 1, dans laquelle $L^1$ et $L^2$ dans la formule (B1) sont des liaisons directes ou des groupes méthylène.

3. Résine thermoplastique selon la revendication 1 ou 2, dans laquelle le cycle carboné alicyclique a au moins un cycle carboné constitué de 4 atomes de carbone ou plus.

4. Résine thermoplastique selon l'une quelconque des revendications 1 à 3, dans laquelle la formule (B1) est représentée par la formule (B1') ou (B1") suivante :

Formule 3

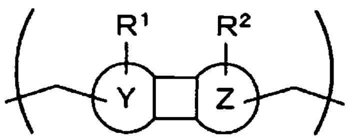

(B1')

Formule 4

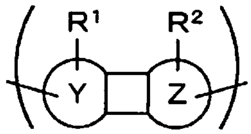

(B1'')

(dans la formule (B1') et (B1"), un cycle Y et un cycle Z formant un cycle condensé avec un cyclobutane sont chacun indépendamment un cycle carboné alicyclique constitué de 4 atomes de carbone ou plus et de 15 atomes de carbone ou moins, le cycle carboné alicyclique contient éventuellement un hétéroatome, $R^1$ et $R^2$ sont chacun indépendamment un atome d'hydrogène, un groupe hydrocarboné ayant 1 à 14 atomes de carbone, ou un groupe alcoxy ayant 1 à 12 atomes de carbone).

**5.** Résine thermoplastique selon l'une quelconque des revendications 1 à 4, dans laquelle le cycle Y et le cycle Z dans la formule (B1) sont chacun indépendamment un cycle carboné alicyclique choisi parmi les formules (C1) à (C11) suivantes :

Formule 5

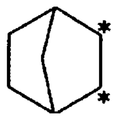

(C1)

Formule 6

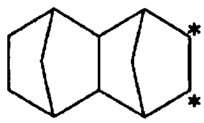

(C2)

Formule 7

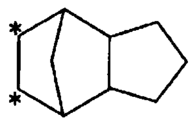

(C3)

Formule 8

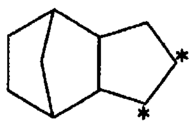

(C4)

Formule 9

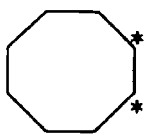

(C5)

Formule 10

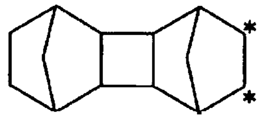

(C6)

Formule 11

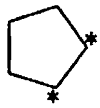

(C7)

Formule 12

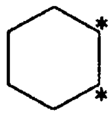

(C8)

Formule 13

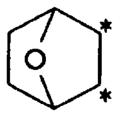

(C9)

Formule 14

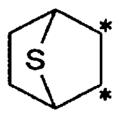

(C10)

Formule 15

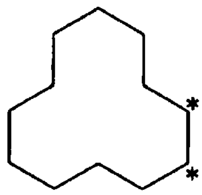

(C11)

(dans les formules (C1) à (C11), * représente un site où le cycle condensé est formé).

6. Résine thermoplastique selon la revendication 5, dans laquelle le cycle Y et le cycle Z dans la formule (B1) sont chacun indépendamment un cycle carboné alicyclique choisi parmi les formules (C1) à (C7).

7. Résine thermoplastique selon l'une quelconque des revendications 1 à 6, dans laquelle la formule (B1) est choisie parmi les formules (B2) à (B12) suivantes et les formules (B16) à (B26) suivantes :

Formule 16

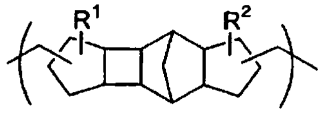

(B2)

Formule 17

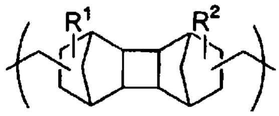

(B3)

Formule 18

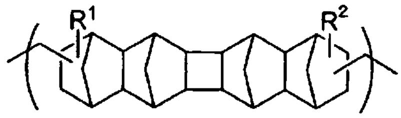

(B4)

Formule 19

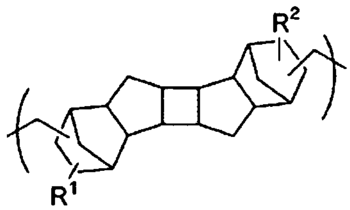

(B5)

Formule 20

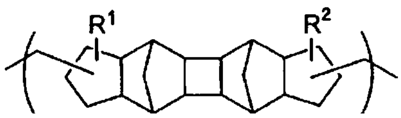

(B6)

Formule 21

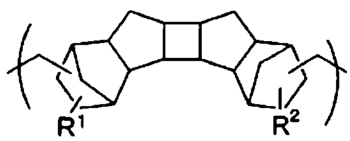

(B7)

Formule 22

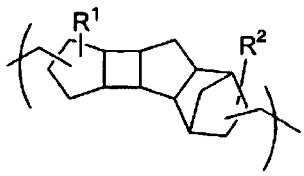

(B8)

Formule 23

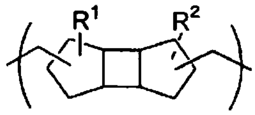

(B9)

Formule 24

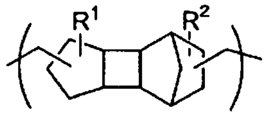

(B10)

Formule 25

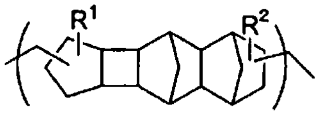

(B11)

Formule 26

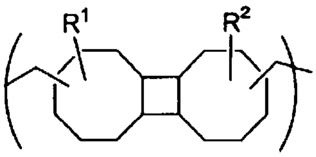

(B12)

Formule 27

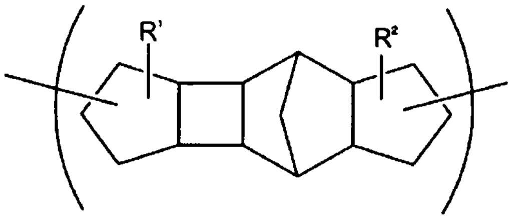

(B16)

Formule 28

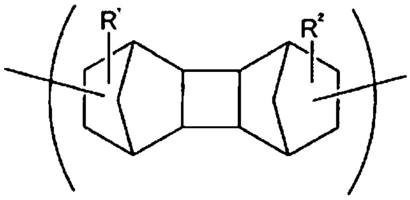

(B17)

Formule 29

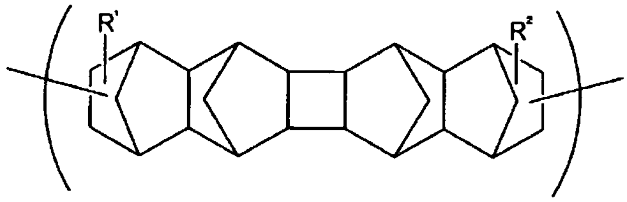

(B18)

Formule 30

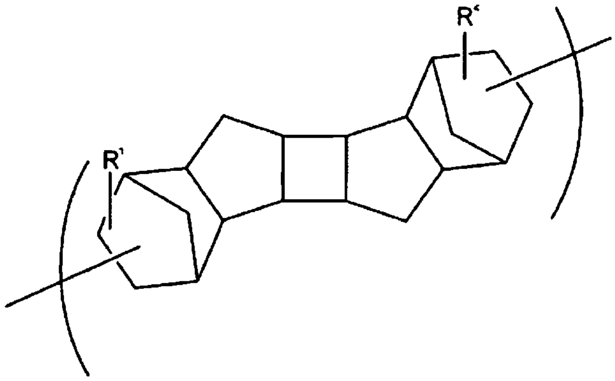

(B19)

Formule 31

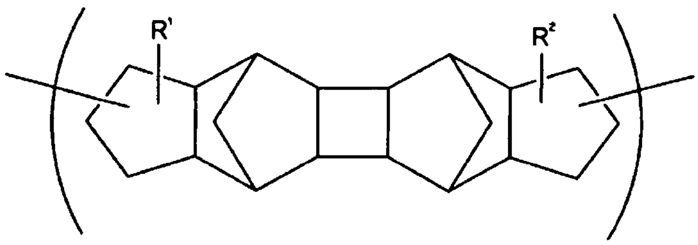

(B20)

Formule 32

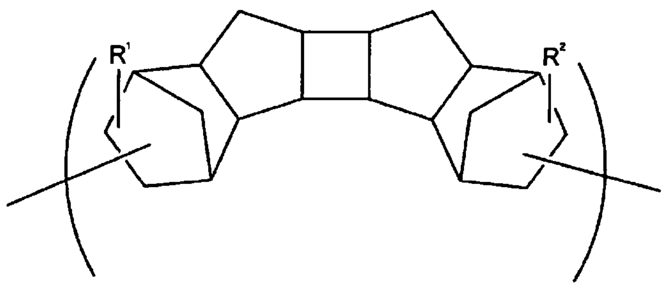

(B21)

Formule 33

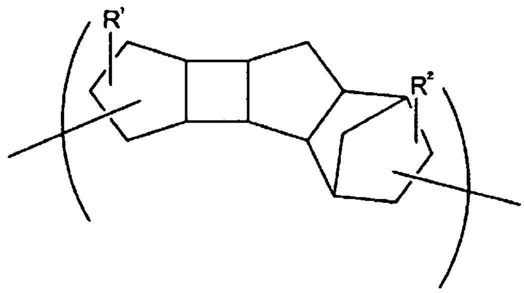

(B22)

Formule 34

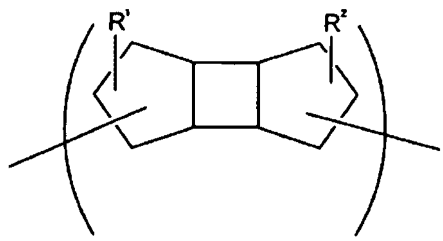

(B23)

Formule 35

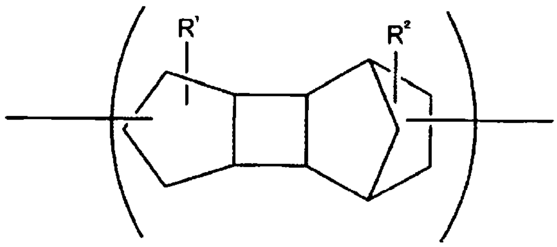

(B24)

Formule 36

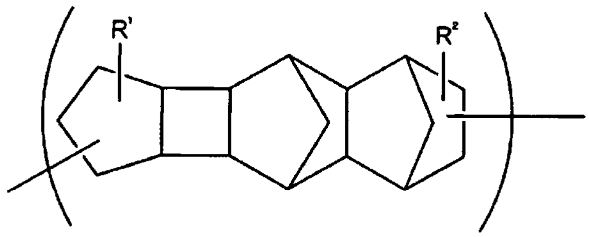

(B25)

Formule 37

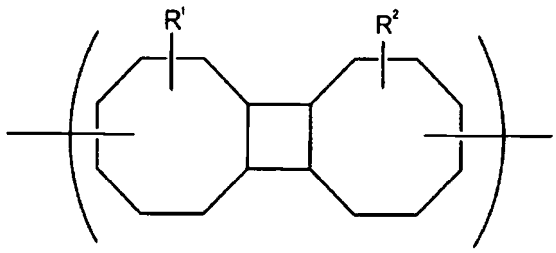

(B26)

(dans les formules (B2) à (B12) et (B16) à (B26), $R^1$ et $R^2$ sont chacun indépendamment un atome d'hydrogène, un groupe hydrocarboné ayant de 1 à 14 atomes de carbone, ou un groupe alcoxy ayant de 1 à 12 atomes de carbone).

8. Résine thermoplastique selon la revendication 7, dans laquelle $R^1$ et $R^2$ dans les formules (B2) à (B12) et (B16) à (B26) sont chacun indépendamment un atome d'hydrogène, un groupe méthyle, un groupe méthoxy, ou un groupe éthoxy.

9. Résine thermoplastique selon l'une quelconque des revendications 1 à 8, dans laquelle le cycle Y et le cycle Z dans la formule (B1) sont différents l'un de l'autre au niveau du squelette alicyclique.

10. Résine thermoplastique selon l'une quelconque des revendications 1 à 9, dont la température de transition vitreuse est de 100°C ou plus.

11. Résine thermoplastique selon l'une quelconque des revendications 1 à 10, dont la viscosité réduite est de 0,2 dl/g ou plus et de 1,50 dl/g ou moins.

12. Résine thermoplastique selon l'une quelconque des revendications 1 à 11, contenant en outre un motif structural dérivé d'au moins un composé choisi dans le groupe consistant en l'isosorbide, l'isomannide et l'isoidide.

13. Résine thermoplastique selon l'une quelconque des revendications 1 à 12, qui est une résine de polycarbonate ou une résine de carbonate de polyester.

14. Article moulé formé à partir de la résine thermoplastique selon l'une quelconque des revendications 1 à 13.

FIG. 1

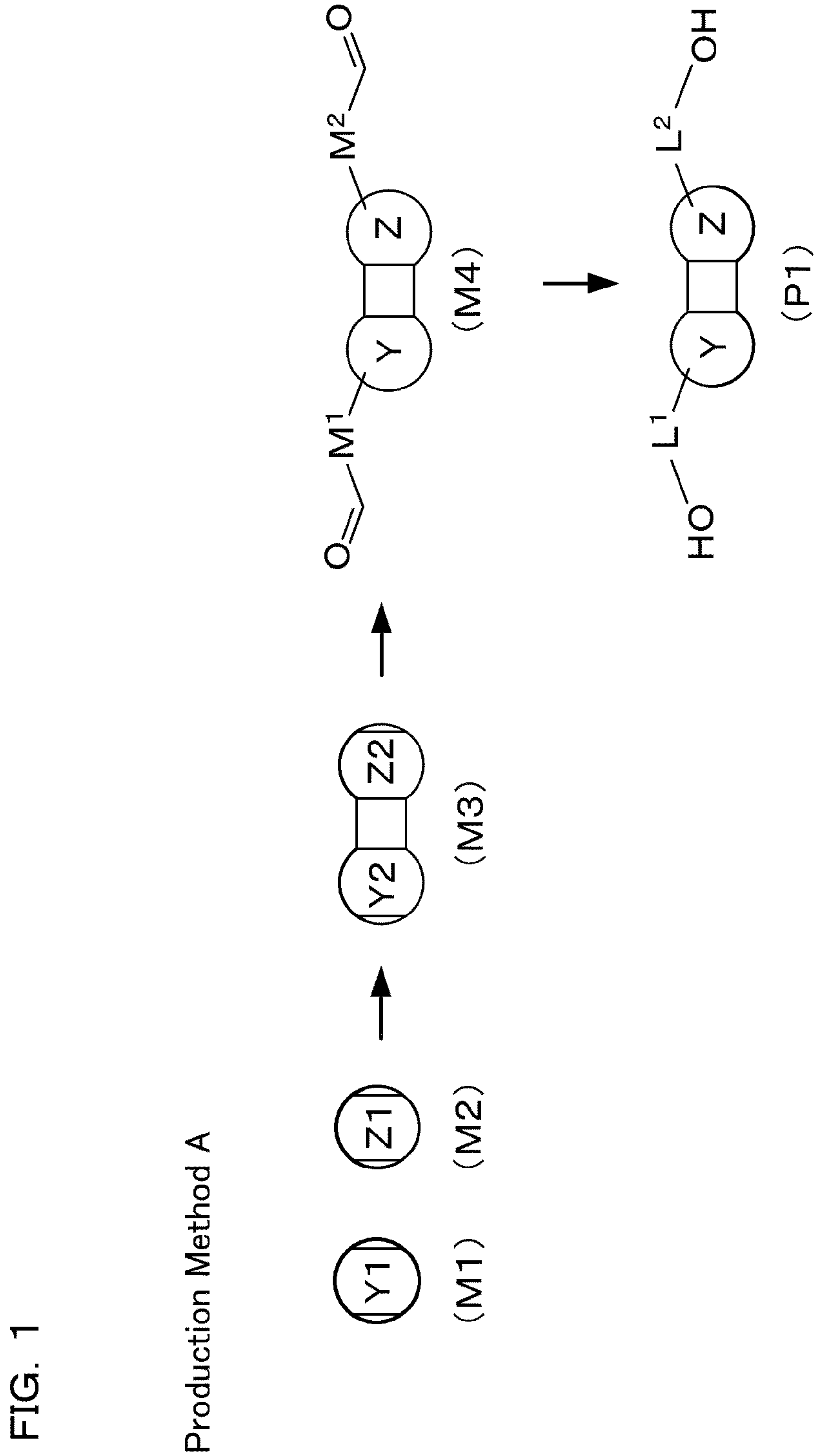

FIG. 2

FIG. 2

Production Method B

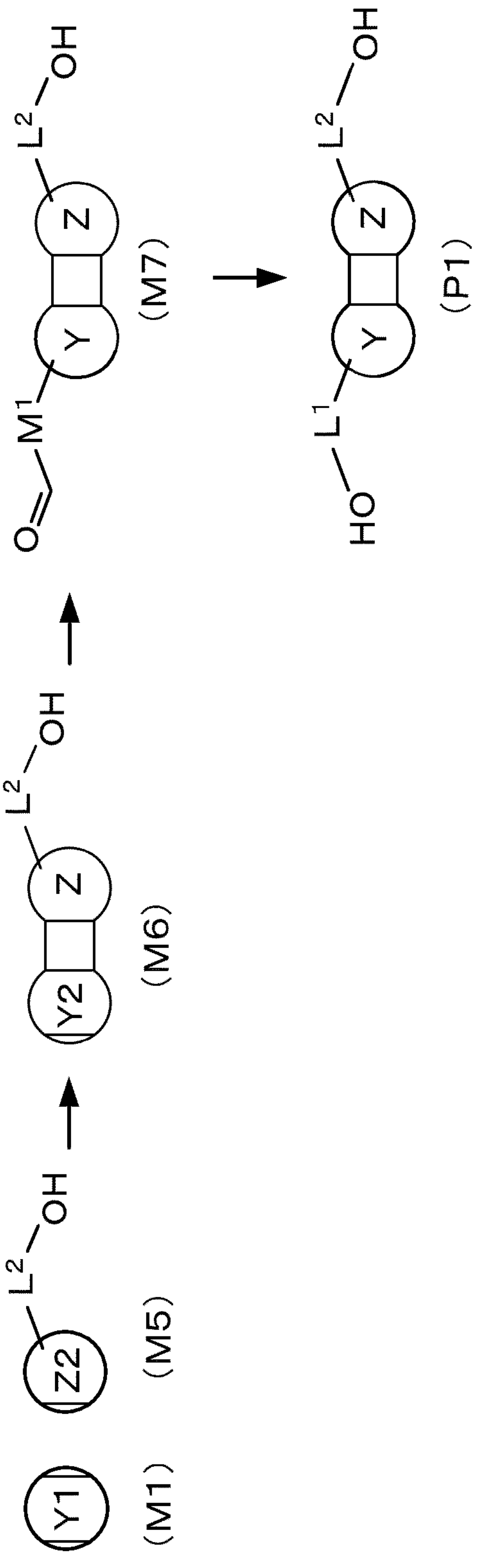

(M1) (M5) (M6) (M7) (P1)

FIG. 3

FIG. 3

Production Method C

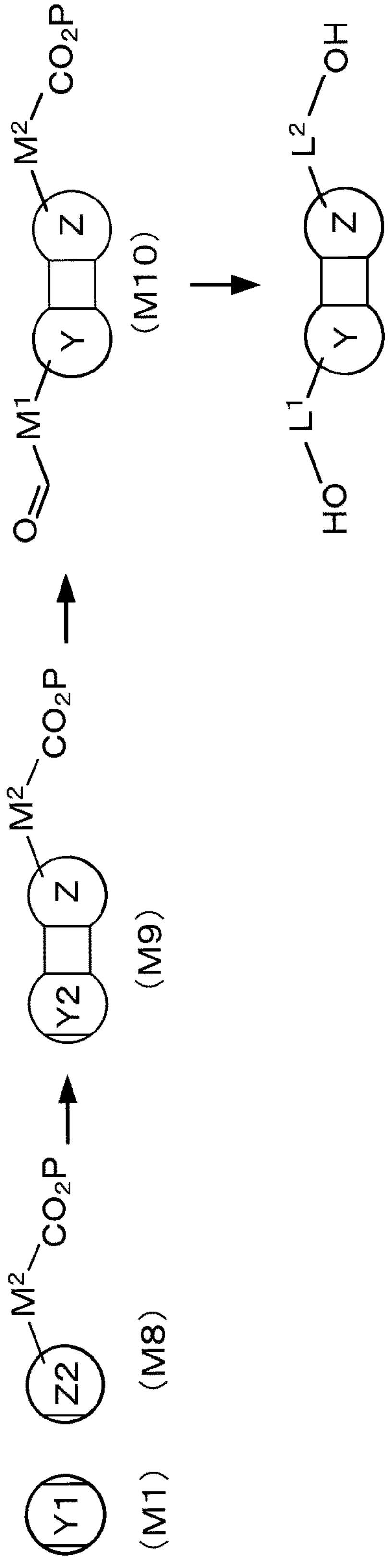

(M1) (M8) (M9) (M10) (P1)

FIG. 4

FIG. 4

Production Method D

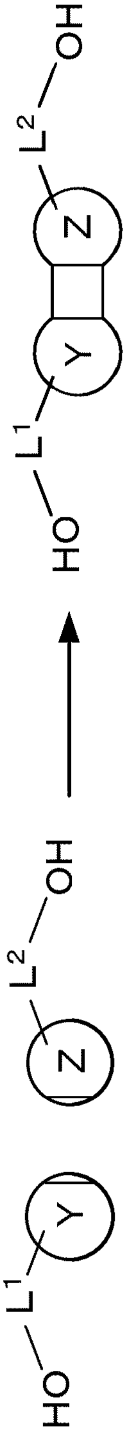

(M5')

(M5)

(P1)

FIG. 5

FIG. 5

Production Method E

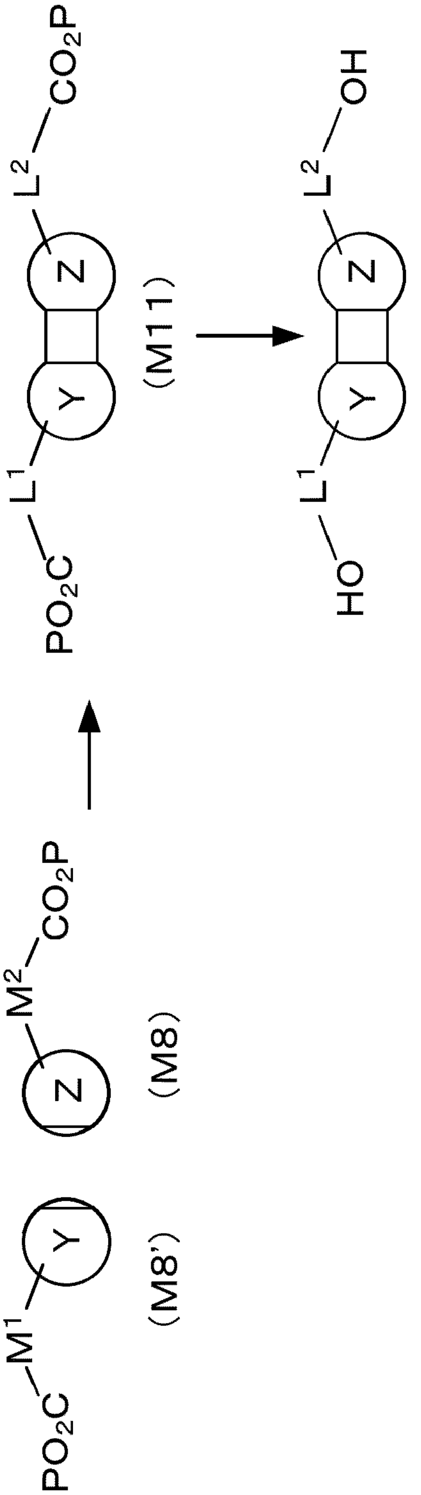

(M8') (M8) (M11) (P1)

FIG. 6

FIG. 6

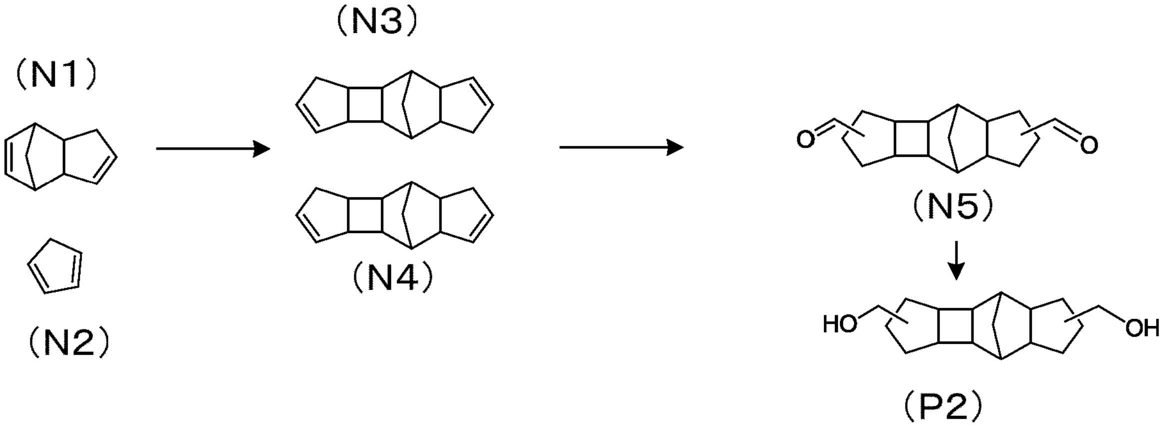

(N1)

(N2)

(N3)

(N4)

(N5)

(P2)

FIG. 7

FIG. 7

(a1)

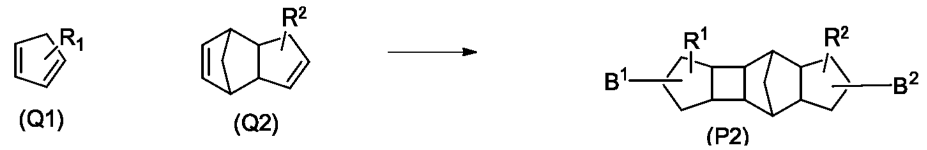

(Q1) (Q2) (P2)

(a2)

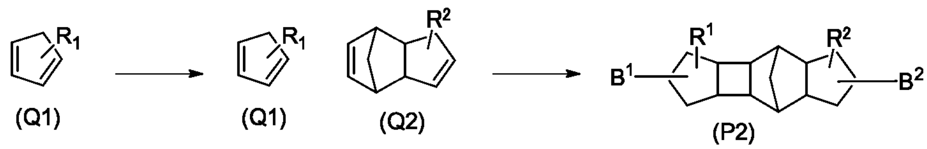

(Q1) (Q1) (Q2) (P2)

(a3)

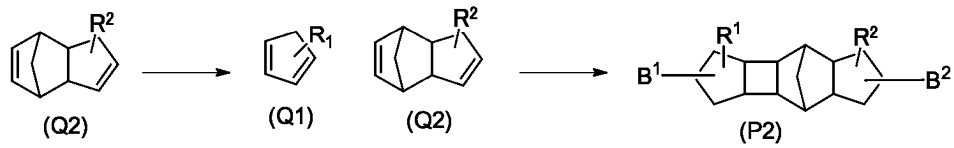

(Q2) (Q1) (Q2) (P2)

(b)

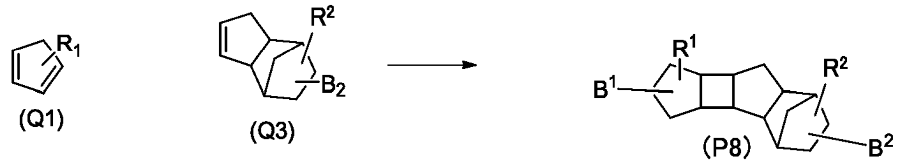

(Q1) (Q3) (P8)

(c)

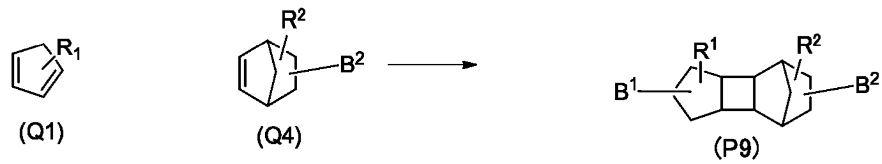

(Q1) (Q4) (P9)

(d)

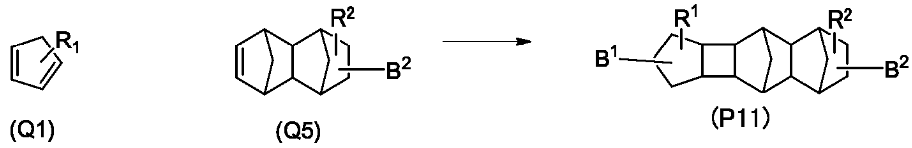

(Q1) (Q5) (P11)

(e)

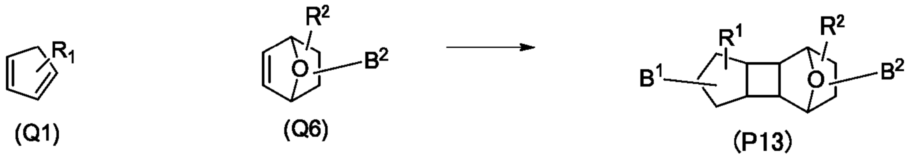

(Q1) (Q6) (P13)

(f)

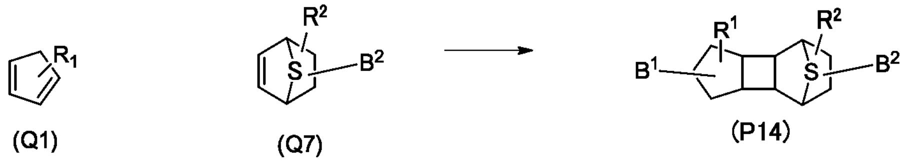

(Q1) (Q7) (P14)

FIG. 8

FIG. 8

(a)

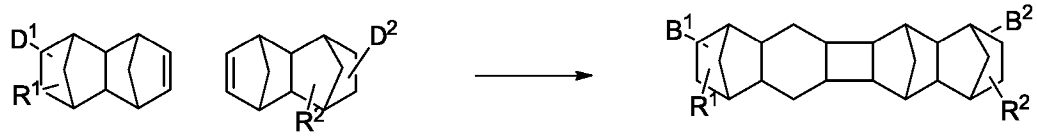

(Q8) (Q8') (P4)

(b)

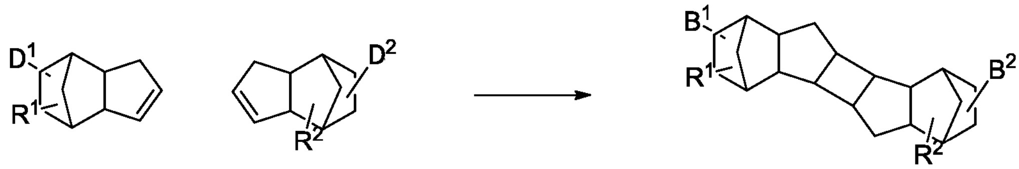

(Q9) (Q8') (P5)

(c)

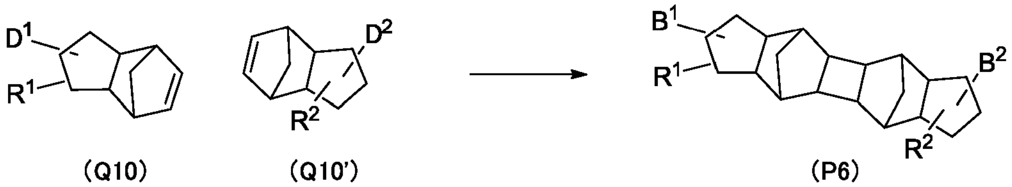

(Q10) (Q10') (P6)

(d)

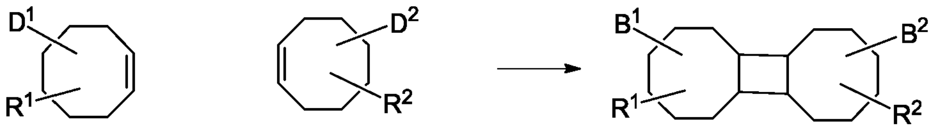

(Q11) (Q11') (P12)

(e)

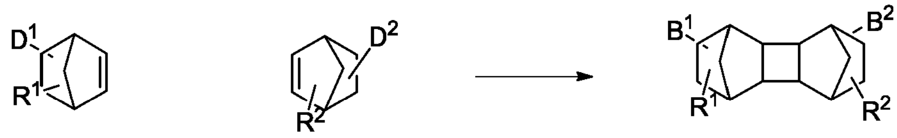

(Q12) (Q12') (P15)

FIG. 9

FIG. 9

Production Method A

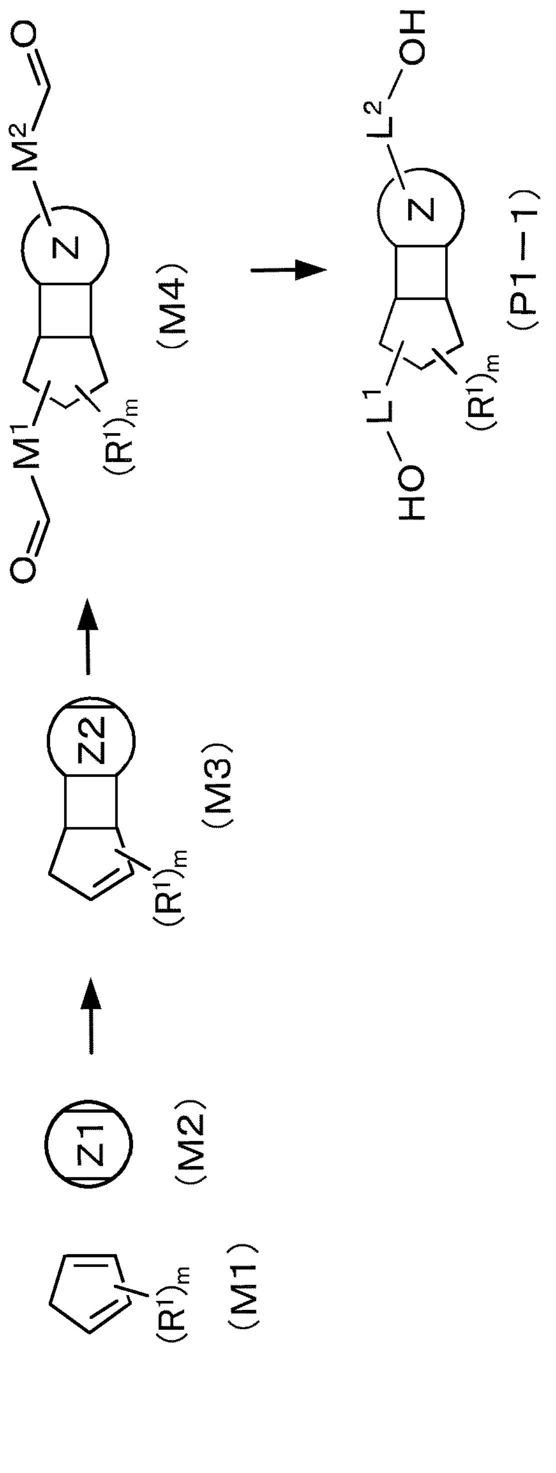

(M1) (M2) (M3) (M4) (P1−1)

FIG. 10

FIG. 10

Production Method B

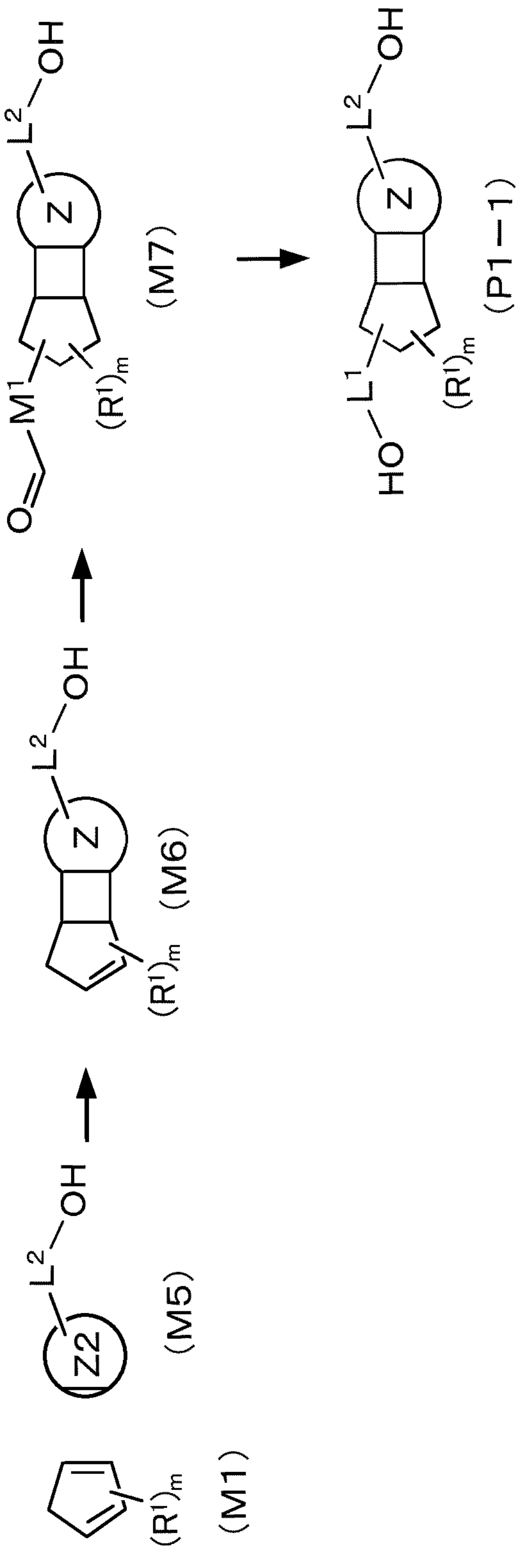

(M1) (M5) (M6) (M7) (P1−1)

FIG. 11

FIG. 11

Production Method C

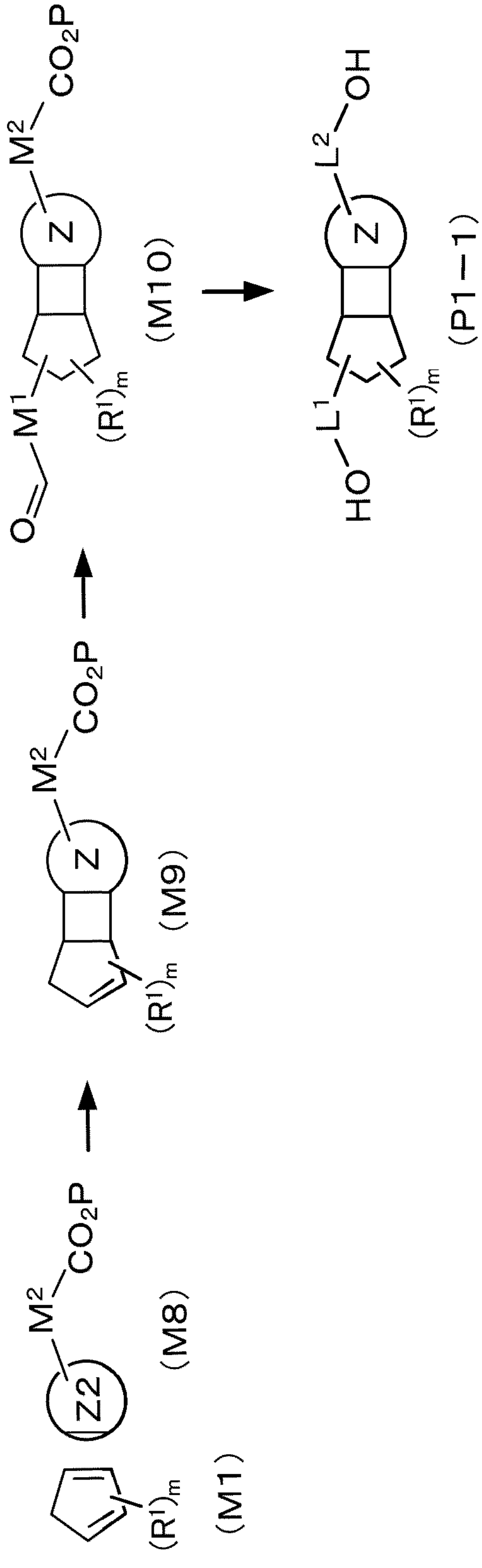

(M1) (M8) (M9) (M10) (P1−1)

FIG. 12

FIG. 12

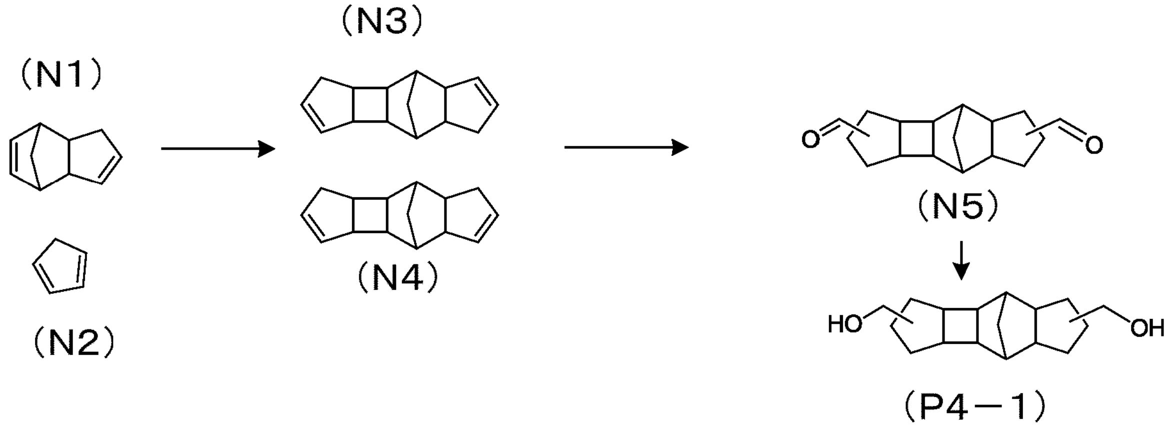

(N1) (N2) (N3) (N4) (N5) (P4－1)

FIG. 13

FIG. 13

(a)

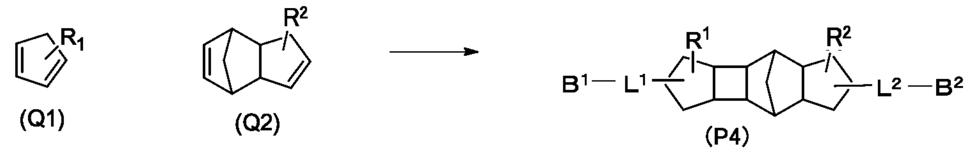

(Q1) (Q2) (P4)

(b)

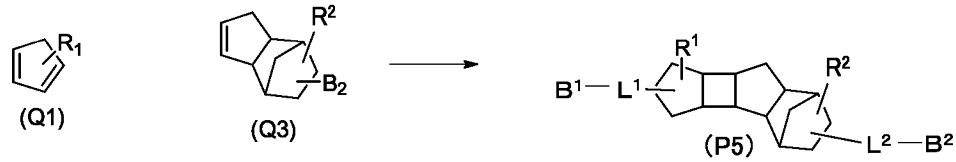

(Q1) (Q3) (P5)

(c)

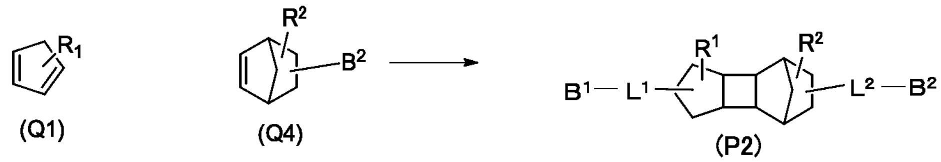

(Q1) (Q4) (P2)

(d)

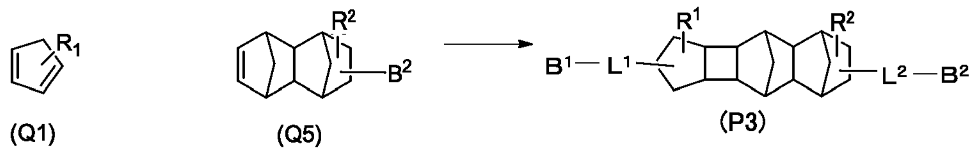

(Q1) (Q5) (P3)

(e)

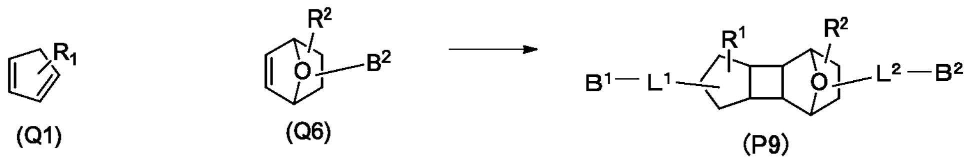

(Q1) (Q6) (P9)

(f)

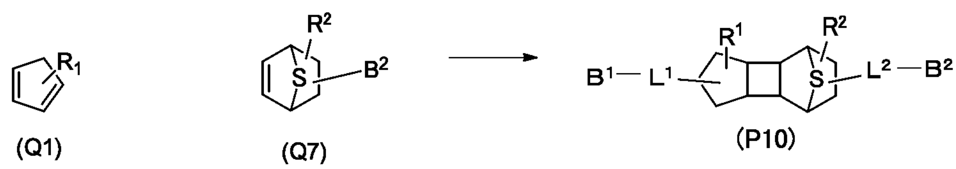

(Q1) (Q7) (P10)

FIG. 14

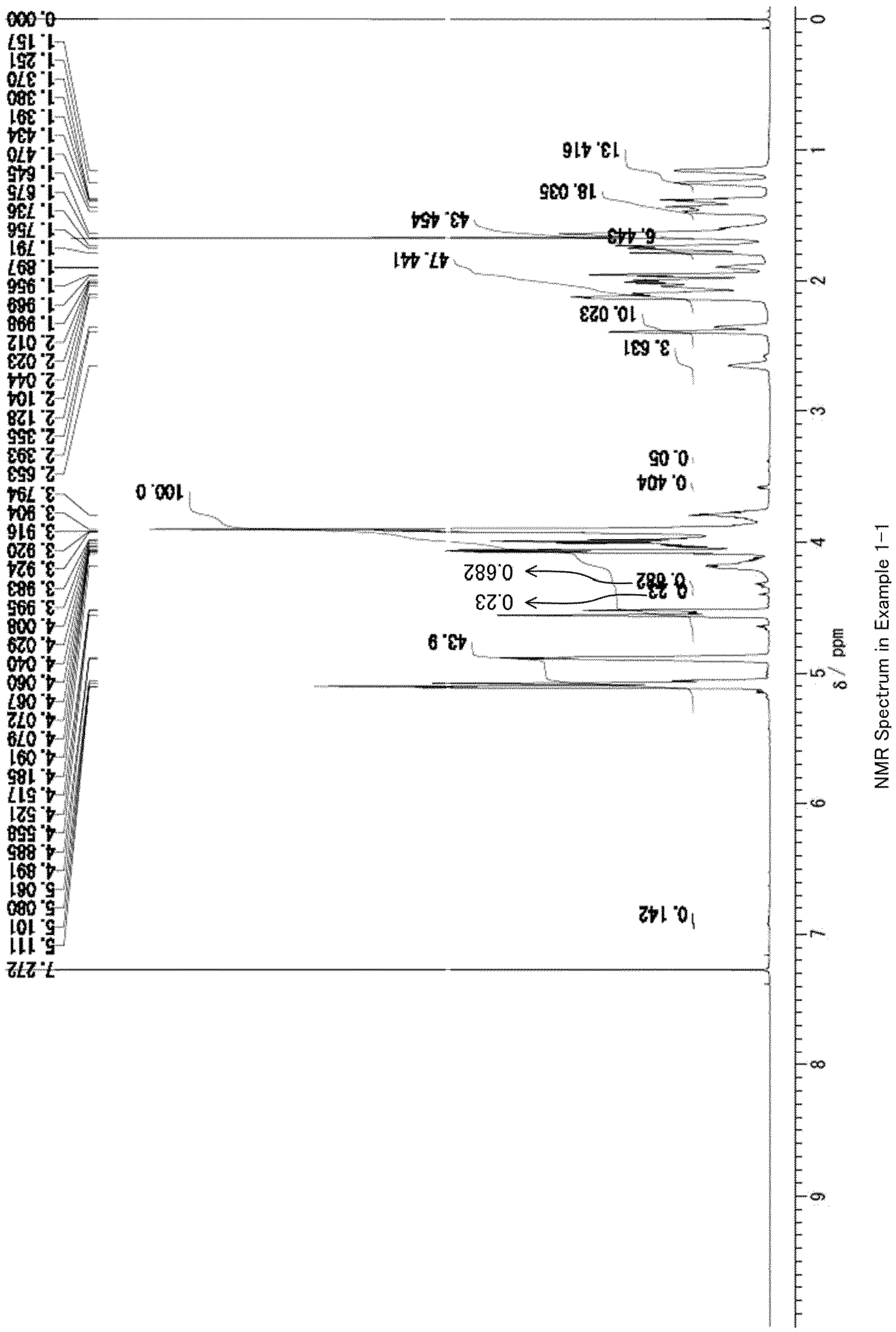


NMR Spectrum in Example 1-1

FIG. 15

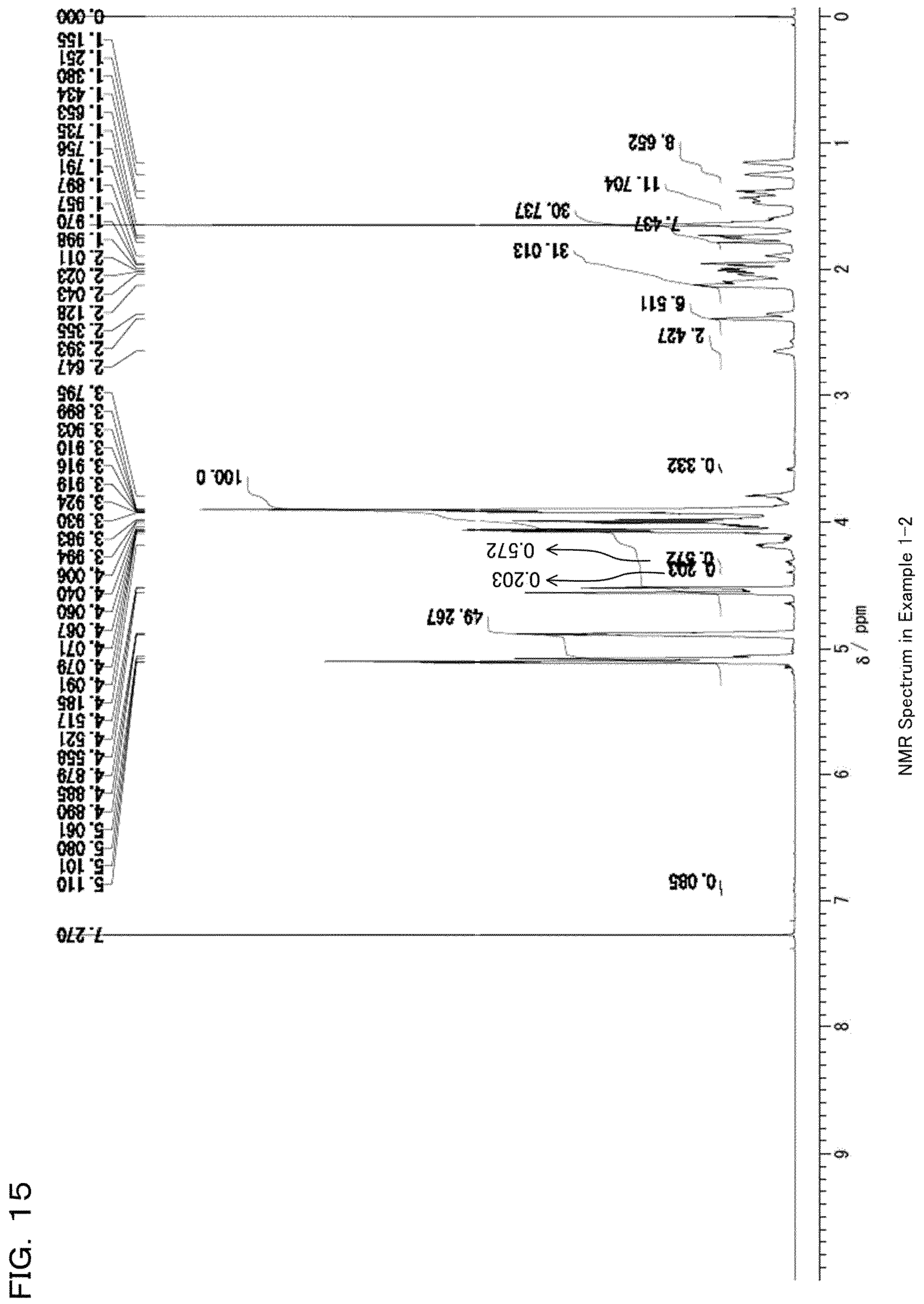

FIG. 16

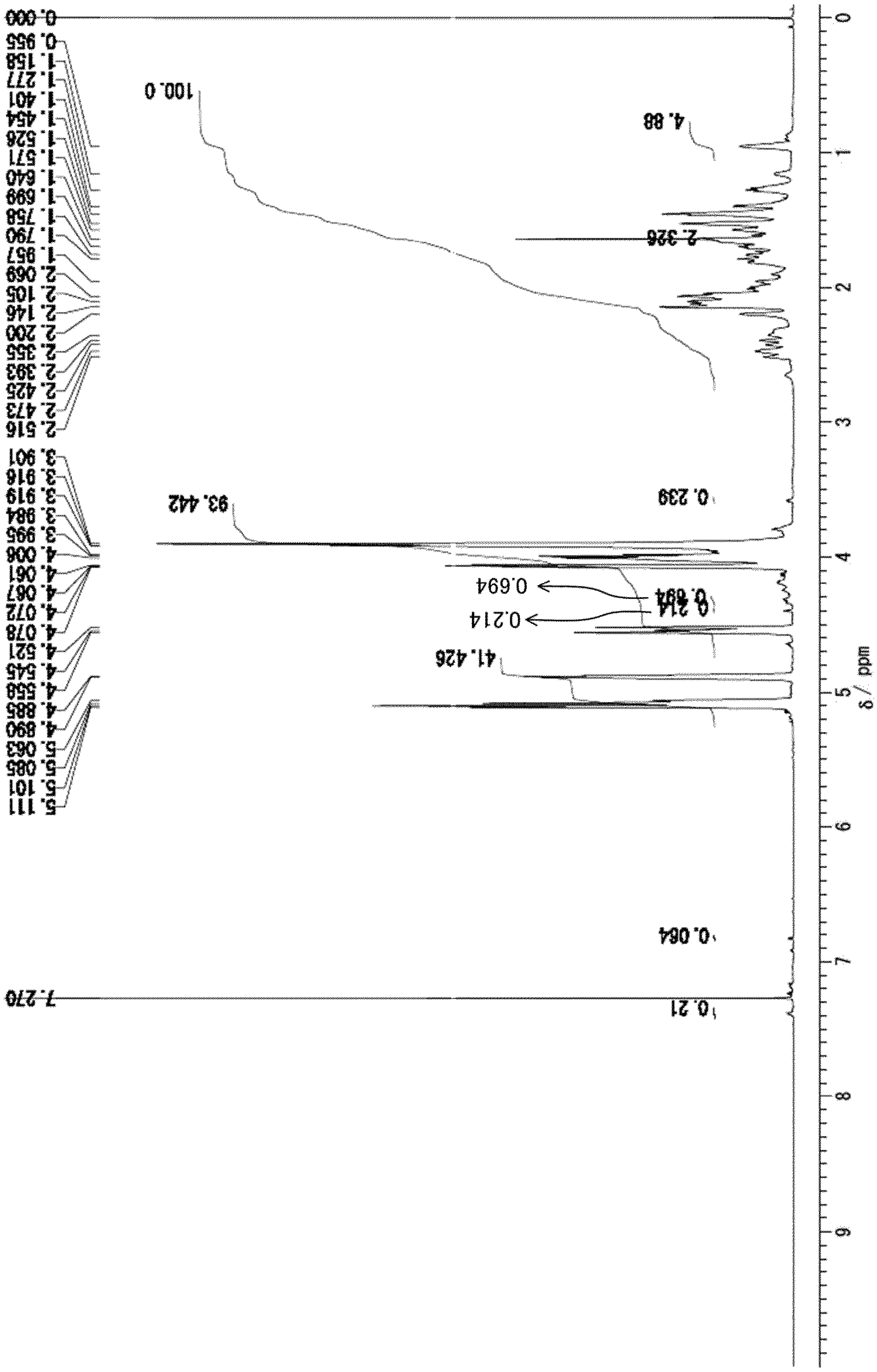


NMR Spectrum in Example 1-3

FIG. 16

FIG. 17

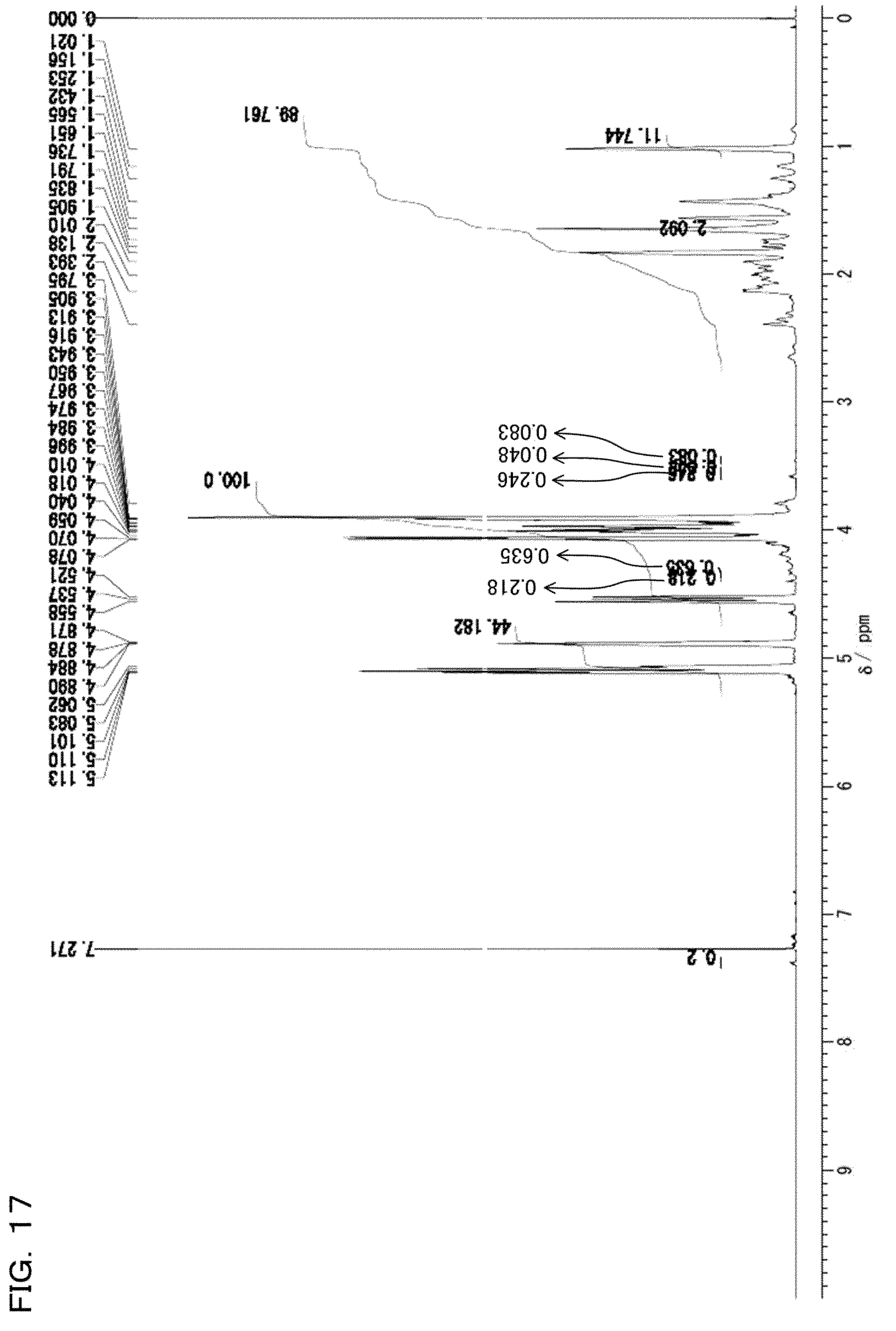

NMR Spectrum in Example 1-4

FIG. 18

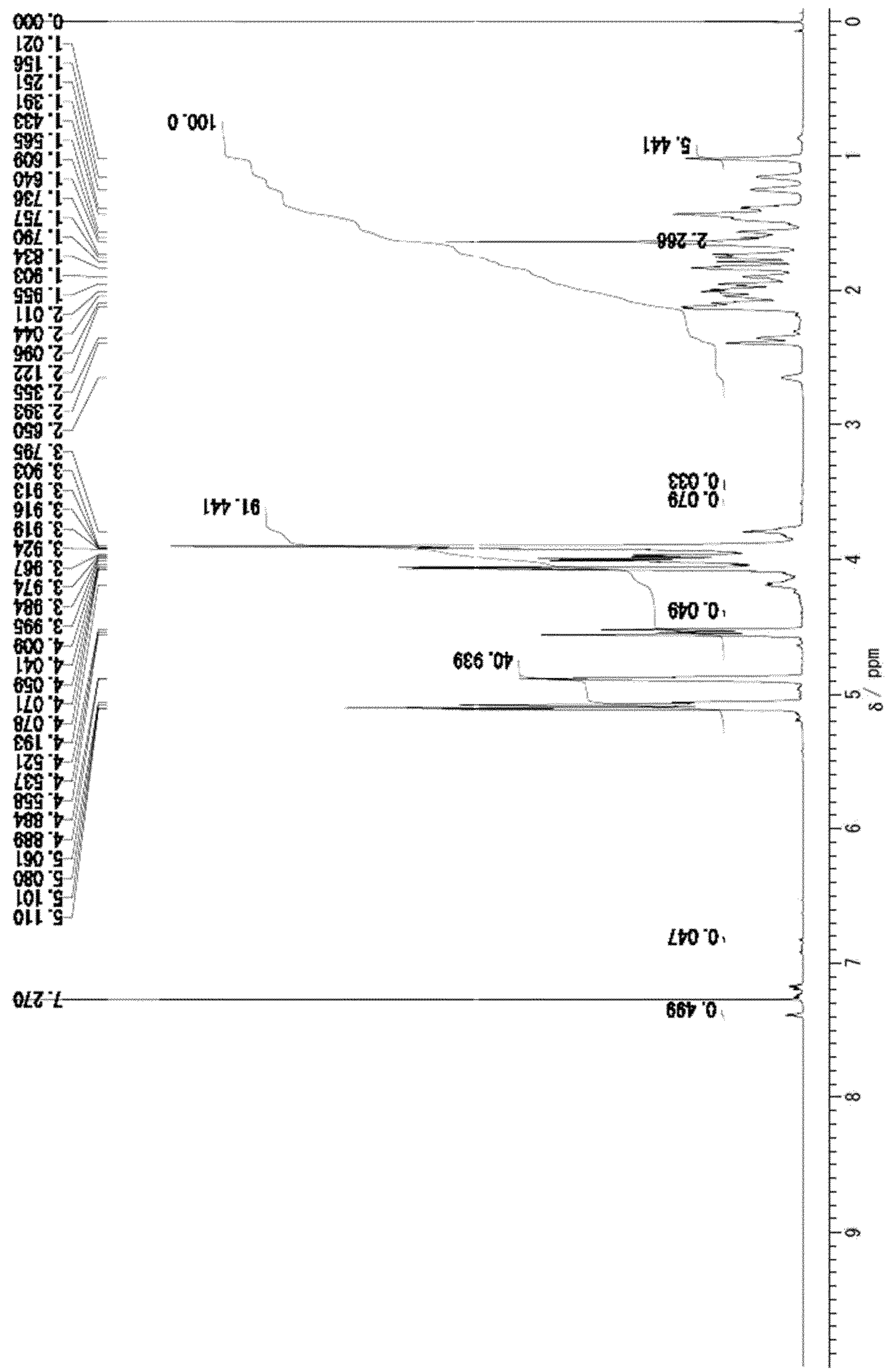


FIG. 18

NMR Spectrum in Example 1-5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004111106 A
- WO 2007063823 A
- JP 5119250 B
- JP 5204200 B
- JP 2015025111 A
- JP 6043302 A
- JP 2002322267 A
- JP 2002265584 A
- WO 2017159525 A


### Non-patent literature cited in the description


- **SIEMS K. et al.** Rigidol, an unusual diterpene from Sapium rigidifolium. *PHYTOCHEMISTRY*, 1993, vol. 33 (6), 1465-1468
- **ITOH KENJI**. *Organotransition Metal Catalyzed Cycloaddition*, 2002, vol. 60 (1), 28, 41
- **JORDAN M. HOYT et al.** Iron-catalyzed intermolecular [2+2]cycloadditions of unactivated alkenes. *Science*, 28 August 2015, vol. 349 (6251), 960-963
- **DAW-JEN HUANG et al.** 2+2]Dimerization of norbornadiene and its derivatives in the presence of nickel complexes and zinc metal. *Journal of Organometallic Chemistry*, 1995, vol. 490, C1-C7
- **ROBERT G. SALOMON et al.** Copper(I) catalysis in photocycloadditions. I. Norbornene. *Journal of the American Chemical Society*, 1974, vol. 96 (4), 1137
- **KYUNG-SUN SON**. Selective synthesis of tricyclopentadiene from dicyclopentadiene with homogeneous Pd catalysts. *Applied Organometallic Chemistry*, 2014, vol. 28, 151-155
- **ITOH KENJI**. *Organotransition Metal Catalyzed Cycloaddition*, 2002, vol. 60 (1), 28-41
- **DAW-JEN HUANG et al.** 2+2]Dimerization of norbornadiene and its derivatives in the presence of nickel complexes and zinc metal. *Journal of Organometallic Chemistry*, 1995, vol. 490, C1-C7
- *Journal of the Society of Rheology Japan*, 1991, vol. 19, 93-97